# EUROPÄISCHE PATENTSCHRIFT

(11) **EP 1 765 816 B1**
(45) Veröffentlichungstag und Bekanntmachung des Hinweises auf die Patenterteilung: **10.03.2010**
(21) Anmeldenummer: 05756539.2
(22) Anmeldetag: 05.07.2005
(51) Int. Cl.: C07D 417/04, A61K 31/495, A61P 25/22

(54) **SUBSTITUIERTE 1-PROPINOYL-PIPERAZINE MIT AFFINITÄT FÜR DEN MGLUR5 REZEPTOR ZUR BEHANDLUNG VON SCHMERZZUSTÄNDEN**
SUBSTITUTED 1-PROPIOLYLPIPERAZINES HAVING AN AFFINITY FOR THE MGLUR5 RECEPTOR IN ORDER TO TREAT PAINFUL CONDITIONS
1-PROPIOLYLPIPERAZINES SUBSTITUEES PRESENTANT UNE AFFINITE POUR LE RECEPTEUR MGLUR5, UTILISEES POUR LE TRAITEMENT DE DOULEURS

(30) Priorität: 05.07.2004 DE 102004032567
(43) Veröffentlichungstag der Anmeldung: 28.03.2007
(73) Patentinhaber: Grünenthal GmbH, 52078 Aachen (DE)
(72) Erfinder: KÜHNERT, Sven, 52355 Düren (DE); OBERBÖRSCH, Stefan, 52074 Aachen (DE); HAURAND, Michael, 52078 Aachen (DE); JOSTOCK, Ruth, 52223 Stolberg (DE); SCHIENE, Klaus, 40227 Düsseldorf (DE)
(74) Vertreter: Brosch, Oliver
(86) Internationale Anmeldenummer: PCT/EP2005/007248
(87) Internationale Veröffentlichungsnummer: WO 2006/002981

(56) Entgegenhaltungen:
- WO-A-03/093236
- WO-A-2004/029044
- MORALES-ALCELAY S ET AL: "AMPA GLUTAMATE RECEPTORS AND NEUROPATHIC PAIN" MINI REVIEWS IN MEDICINAL CHEMISTRY, BENTHAM SCIENCE PUBLISHERS, HILVERSUM, NL, Bd. 3, Nr. 7, November 2003 (2003-11), Seiten 757-763, XP001205152 ISSN: 1389-5575
- WILLIAMS, M. ET AL.: "Emerging Molecular Approaches to Pain Therapy" JOURNAL OF MEDICINAL CHEMISTRY, Bd. 42, Nr. 9, 1999, Seiten 1481-1500, XP002344884
- WERMUTH ET AL: "The Practise of Medicinal Chemistry" PRACTICE OF MEDICINAL CHEMISTRY, 1996, Seiten 203-237, XP002190259

## Beschreibung

Die vorliegende Erfindung betrifft substituierte 1-Propiolyl-piperazine, Verfahren zu ihrer Herstellung, Arzneimittel enthaltend diese Verbindungen sowie deren Verwendung zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nozizeption in letzter Zeit erschienen sind.

Klassische Opioide, wie beispielsweise Morphin, sind bei der Therapie starker bis sehr starker Schmerzen wirksam, führen jedoch oftmals zu unerwünschten Begleiterscheinungen wie beispielsweise Atemdepression, Erbrechen, Sedierung, Obstipation oder Toleranzentwicklung, Des Weiteren sind sie bei neuropathischen Schmerzen, unter denen insbesondere Tumorpatienten leiden, häufig nicht ausreichend wirksam.

Aus der WO 03/093236 sind 1-(Pyrid-2-yl)-piperazinverbindungen bekannt, die sich als Inhibitoren von metabotropischen Glutamatrezeptoren unter anderem zur Bekämpfung von Schmerz eignen. Die WO 2004/029044 offenbart 2-Pyrimidinylpiperazinverbindungen mit entsprechenden Eigenschaften.

Eine Aufgabe der vorliegenden Erfindung bestand daher darin, neue Verbindungen zur Verfügung zu stellen, die sich insbesondere als pharmazeutische Wirkstoffe in Arzneimitteln eignen, bevorzugt in Arzneimitteln zur Behandlung von Schmerzen.

Es wurde nun überraschenderweise gefunden, dass sich die substituierten 1-Propiolyl-piperazine der nachstehend angegebenen allgemeinen Formel I zur mGluR5-Rezeptor-Regulation eignen und daher insbesondere als pharmazeutische Wirkstoffe in Arzneimitteln zur Prophylaxe und/oder Behandlung von mit diesen Rezeptoren bzw. Prozessen in Verbindung stehenden Störungen oder Erkrankungen eingesetzt werden können.

Ein Gegenstand der vorliegenden Erfindung sind daher substituierte 1-Propiolyl-piperazine der allgemeinen Formel I, 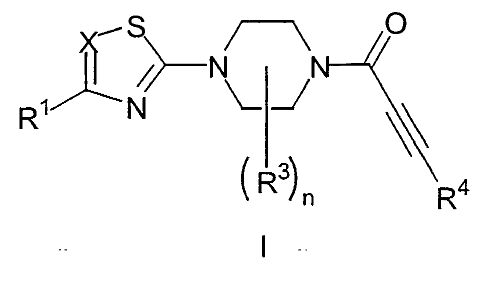 worin
X für N oder C-R² steht,
R¹ und R², unabhängig voneinander, jeweils für einen Wasserstoff-Rest, für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine -NH-R⁵-Gruppe, für eine-NR⁶R⁷-Gruppe, für eine -C(=O)-R⁸-Gruppe, für eine -C(=O)-O-R⁹-Gruppe, für eine-O-C(=O)-R¹⁰-Gruppe, für eine -NH-C(=O)-R¹¹-Gruppe, für eine -NR¹²-C(=O)-R¹³-Gruppe, für eine -C(=O)-NH₂-Gruppe, für eine -C(=O)-NH-R¹⁴-Gruppe, für eine-C(=O)-NR¹⁵R¹⁶-Gruppe, für eine -O-R¹⁷-Gruppe, für eine -S-R¹⁸-Gruppe, für eine-S(=O)-R¹⁹-Gruppe, für eine -S(=O)₂-R²⁰-Gruppe, für eine -NH-C(=O)-NH-R²¹-Gruppe, für eine -NH-C(=S)-NH-R²²-Gruppe, für eine -NH-S(=O)₂-R²³-Gruppe, für eine -NR²⁴-S(=O)₂-R²⁵-Gruppe, für einen linearen oder verzweigten, gesättigten oder ungesättigten, unsubstituierten oder wenigstens einfach substituierten, ggf. wenigstens ein Heteroatom als Kettenglied aufweisenden aliphatischen Rest, für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, stehen,
R³ für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo-Gruppe (=O), für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=0)-R¹⁰-Gruppe, für eine -NH-C(=O)-R¹¹-Gruppe, für eine -NR¹²-C(=O)-R¹³-Gruppe, für eine -C(=O)-NH₂-Gruppe, für eine -C(=O)-NH-R¹⁴-Gruppe, für eine -C(=O)-NR¹⁵R¹⁶-Gruppe, für eine O-R¹⁷-Gruppe, für eine -S-R¹⁸-Gruppe, für eine -S(=O)-R¹⁹-Gruppe, für eine -S(=O)₂-R²⁰-Gruppe, für eine -NH-C(=O)-NH-R²¹-Gruppe, für eine -NH-C(=S)-NH-R²²-Gruppe, für eine -NH-S(=O)₂-R²³-Gruppe, für eine -NR²⁴-S(=O)₂-R²⁵-Gruppe, für einen linearen oder verzweigten, gesättigten oder ungesättigten, unsubstituierten oder wenigstens einfach substituierten, ggf. wenigstens ein Heteroatom als Kettenglied aufweisenden aliphatischen Rest, für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, steht,
R⁴ für einen Wasserstoff-Rest, für ein Fluoratom, für ein Chloratom, für ein Bromatom, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine -NH-R⁵-Gruppe, für eine-NR⁶R⁷-Gruppe, für eine -C(=O)-R⁸-Gruppe, für eine -C(=O)-O-R⁹-Gruppe, für eine-O-C(=O)-R¹⁰-Gruppe, für eine -NH-C(=O)-R¹¹Gruppe, für eine -NR¹²-C(=O)-R¹³-Gruppe, für eine -C(=O)-NH₂-Gruppe, für eine -C(=O)-NH-R¹⁴-Gruppe, für eine-C(=O)-NR¹⁵R¹⁶-Gruppe, für eine -O-R¹⁷-Gruppe, für eine -S-R¹⁸-Gruppe, für eine-S(=O)-R¹⁹-Gruppe, für eine -S(=O)₂-R²⁰-Gruppe, für eine -NH-C(=O)-NH-R²¹-Gruppe, für eine -NH-C(=S)-NH-R²²-Gruppe, für eine -NH-S(=O)₂-R²³-Gruppe, für eine -NR²⁴-S(=O)₂-R²⁵-Grüppe, für einen linearen oder verzweigten, gesättigten oder ungesättigten, unsubstituierten oder wenigstens einfach substituierten, ggf. wenigstens ein Heteroatom als Kettenglied aufweisenden aliphatischen Rest, für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, steht,
R⁵ bis R²⁵, unabhängig voneinander, jeweils für einen linearen oder verzweigten, gesättigten oder ungesättigten, unsubstituierten oder wenigstens einfach substituierten, ggf. wenigstens ein Heteroatom als Kettenglied aufweisenden aliphatischen Rest, für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden sein kann, stehen,
n = 0, 1, 2, 3, 4, 5, 6, 7 oder 8 ist,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

Sofern einer oder mehrere der Substituenten R¹ bis R²⁵ für einen gesättigten oder ungesättigten aliphatischen Rest, d.h. für einen Alkyl-, Alkenyl- oder Alkinyl-Rest, stehen, der einfach oder mehrfach substituiert ist, kann dieser bevorzugt mit 1, 2, 3, 4 oder 5 Substituenten unabhängig voneinander ausgewählt aus der Gruppe bestehend aus F, Cl, Br, -NO₂, -CN, -OH, -SH und -NH₂ substituiert sein. Alkenyl-Reste weisen wenigstens eine, vorzugsweise 1, 2, 3 oder 4 C-C-Doppelbindungen und Alkinyl-Reste wenigstens eine, vorzugsweise 1, 2, 3 oder 4 C-C-Dreifachbindungen auf.

Als geeignete Alkyl-, Alkenyl- und Alkinyl-Reste, die einfach oder mehrfach substituiert sein können, seien beispielsweise Methyl, Ethyl, n-Propyl, Isopropyl, n-Butyl, Isobutyl, sec-Butyl, tert-Butyl, n-Pentyl, 2-Pentyl, 3-Pentyl, iso-Pentyl, neo-Pentyl, n-Hexyl, 2-Hexyl, 3-Hexyl, n-Heptyl, n-Octyl, n-Nonyl, n-Decyl, -C(H)(C₂H₅)₂,-C(H)(n-C₃H₇)₂, -CH₂-CH₂-C(H)(CH₃)-(CH₂)₃-CH₃, Ethenyl, Ethinyl, 1-Propenyl, 2-Propenyl, 1-Propinyl, 2-Propinyl, 1-Butenyl, 2-Butenyl, 3-Butenyl, 1-Butinyl, 2-Butinyl, 3-Butinyl, 1-Pentenyl, 2-Pentenyl, 3-Pentenyl, 4-Pentenyl, 1-Pentinyl, 2-Pentinyl, 3-Pentinyl, 4-Pentinyl, Hexenyl, Hexinyl, -CH=CH-CH=CH-CH₃ und -CH₂-CH₂-CH=CH₂ genannt.

Als geeignete substituierte Alkyl- und Alkenyl-Reste seien beispielsweise Trifluormethyl, Difluormethyl, Monofluormethyl, -(CH₂)-OH, -(CH₂)-NH₂, -(CH₂)-CN,-(CH₂)-(CF₃), -(CH₂)-(CHF₂), -(CH₂)-(CH₂F), -(CH₂)-(CH₂)-OH, -(CH₂)-(CH₂)-NH₂,-(CH₂)-(CH₂)-CN, -(CF₂)-(CF₃), -(CH₂)-(CH₂)-(CF₃), -CH=CH-(CH₂)-OH, -CH=CH-(CH₂)-NH₂, -CH=CH-CN und -(CH₂)-(CH₂)-(CH₂)-OH genannt.

Sofern einer oder mehrere der Substituenten R¹ bis R²⁵ für einen gesättigten oder ungesättigten aliphatischen Rest, d. h. für einen Alkyl-, Alkenyl- oder Alkinyl-Rest, stehen, der eines oder mehrere, vorzugsweise 1, 2 oder 3, Heteroatom(e) als Kettenglied(er) aufweist, können diese Heteroatome, jeweils unabhängig voneinander, bevorzugt ausgewählt werden aus der Gruppe bestehend aus Sauerstoff, Schwefel und Stickstoff (NH).

Vorzugsweise befinden sich diese Heteroatome in einer nicht-endständigen Position des jeweiligen Restes. Beispielhaft seien Reste wie -(CH₂)-(CH₂)-O-(CH₃), -(CH₂)-(CH₂)-S-(CH₃), -(CH₂)-S-(CH₂)-(CH₂)-S-(CH₃), -(CH₂)-O-(CH₂)-(CH2)-O-(CH₃), -(CH₂)-O-(CH₃) oder -(CH₂)-S-(CH₃) genannt.

Sofern einer oder mehrere der Substituenten R¹ bis R²⁵ für einen cycloaliphatischen Rest stehen oder einen cycloaliphatischen Rest aufweisen, der einfach oder mehrfach substituiert ist, kann dieser bevorzugt mit 1, 2, 3, 4 oder 5 Substituenten unabhängig voneinander ausgewählt aus der Gruppe bestehend aus F, Cl, Br, I, -CN, -CF₃, -OH, -NH₂, -O-CF₃, -SH, -O-C₁₋₅-Alkyl, -(CH₂)-O-C₁₋₅-Alkyl, -S-C₁₋₅-Alkyl,-C₁₋₅-Alkyl, -C₂₋₅-Alkenyl, -C₂₋₅-Alkinyl, -C≡C-Si(CH₃)₃, -C=C-Si(C₂H₅)₃, -C(=O)-O-C₁₋₅-Alkyl, -C(=O)-CF₃, -S(=O)₂-C₁₋₅-Alkyl, -S(=O)-C₁₋₅-Alkyl, -S(=O)₂-Phenyl, Oxo (=O), Thioxo (=S), -N(C₁₋₅-Alkyl)₂, -N(H)(C₁₋₅-Alkyl), -NO₂, -S-CF₃, -C(=O)-OH, -NH-S(=O)₂-C₁₋₅-Alkyl, -NH-C(=O)-C₁₋₅-Alkyl, -C(=O)-H, -C(=O)-C₁₋₅-Alkyl, -C(=O)-NH₂, -C(=O)-N(C₁₋₅-Alkyl)₂, -C(=O)-N(H)(C₁₋₅-Alkyl) und Phenyl substituiert sein, wobei die vorstehend genannten C₁₋₅-Alkyl-Reste jeweils linear oder verzweigt sein können und die Phenyl-Reste jeweils unsubstituiert oder mit 1, 2, 3, 4 oder 5, bevorzugt mit 1, 2, 3 oder 4, Substituenten unabhängig voneinander ausgewählt aus der Gruppe bestehend aus F, Cl, Br, I, -CN, -CF₃, -OH, -NH₂, -O-CF₃, -SH, -O-C₁₋₅-Alkyl, -(CH₂)-O-C₁₋₅-Alkyl, -S-C₁₋₅-Alkyl, -C₁₋₅-Alkyl, -C₂₋₅-Alkenyl, -C₂₋₅-Alkinyl, -C≡C-Si(CH₃)₃,-C≡C-Si(C₂H₅)₃, -C(=O)-O-C₁₋₅-Alkyl und -C(=O)-CF₃ substituiert sein können.

Besonders bevorzugt können die Substituenten, jeweils unabhängig voneinander, ausgewählt werden aus der Gruppe bestehend aus F, Cl, Br, I, -CN, Methyl, Ethyl, n-Propyl, Isopropyl, n-Butyl, sec-Butyl, Isobutyl, tert-Butyl, Ethenyl, Allyl, Ethinyl, Propinyl, -C=C-Si(CH₃)₃, -C=C-Si(C₂H₅)₃, -OH, Oxo, Thioxo, -O-CH₃, -O-C₂H₅, -O-C₃H₇, -(CH₂)-O-CH₃, -(CH₂)-O-C₂H₅, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -S(=O)-CH₃, -S(=O)₂-CH₃,-S(=O)-C₂H₅, -S(=O)₂-C₂H₅, -NH-S(=O)₂-CH₃, -C(=O)-OH, -C(=O)-H; -C(=O)-CH₃,-C(=O)-C₂H₅, -C(=O)-N(CH₃)₂, -C(=O)-NH-CH₃, -C(=O)-NH₂, -NH-C(=O)-CH₃, -NH-C(=O)-C₂H₅, -C(=O)-O-CH₃, -C(=O)-O-C₂H₅, -C(=O)-O-C(CH₃)₃ und Phenyl, wobei der Phenyl-Rest mit 1, 2, 3, 4 oder 5, bevorzugt 1, 2 oder 3, Substituenten unabhängig voneinander ausgewählt aus der Gruppe bestehend aus F, Cl, Br, -CN,-CF₃, -OH, -NH₂, -O-CF₃, -SH, -O-CH₃, -O-C₂H₅, -O-C₃H₇, Methyl, Ethyl, n-Propyl, Isopropyl, n-Butyl, sec-Butyl, Isobutyl, tert-Butyl, Ethenyl, Allyl, Ethinyl, Propinyl,-C=C-Si(CH₃)₃, -C=C-Si(C₂H₅)₃, -C(=O)-O-C₁₋₅-Alkyl und -C(=O)-CF₃ substituiert sein kann.

Sofern die cycloaliphatischen Reste eines oder mehrere Heteroatome als Ringglieder aufweisen, können diese vorzugsweise 1, 2, 3, 4 oder 5, besonders bevorzugt 1, 2 oder 3, Heteroatom(e) als Ringglied(er) aufweisen, die jeweils unabhängig voneinander, ausgewählt werden können aus der Gruppe bestehend aus Stickstoff, Sauerstoff und Schwefel.

Als geeignete cycloaliphatische Reste, die einfach oder mehrfach substituiert sein können, seien beispielsweise Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Imidazolidinyl, Tetrahydrofuranyl (Tetrahydrofuryl), Piperidinyl, Piperazinyl, Morpholinyl, Pyrrolidinyl, Tetrahydrothiophenyl, Tetrahydropyranyl, Thiomorpholinyl, Dioxolanyl, Azepanyl, Diazepanyl und Dithiolanyl genannt.

Sofern einer oder mehrere der Substituenten R¹ bis R²⁵ für einen Aryl- oder Heteroaryl-Rest stehen oder einen Aryl- oder Heteroaryl-Rest aufweisen, der einfach oder mehrfach substituiert ist, kann dieser bevorzugt mit ggf. 1, 2, 3, 4 oder 5 Substituenten unabhängig voneinander ausgewählt aus der Gruppe bestehend aus Halogen, -CN, -NO₂, -OH, -SH, -NH₂, -C(=O)-OH, -C₁₋₅-Alkyl, -(CH₂)-O-C₁₋₅-Alkyl,-C₂₋₅-Alkenyl, -C₂₋₅-Alkinyl, -C≡C-Si(CH₃)₃, -C=C-Si(C₂H₅)₃, -S-C₁₋₅-Alkyl, -O-C₁₋₅-Alkyl, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, -S(=O)₂-C₁₋₅-Alkyl, -S(=O)-C₁₋₅-Alkyl, -NH-C₁₋₅-Alkyl, N(C₁₋₅ Alkyl)₂, -C(=O)-O-C₁₋₅-Alkyl, -C(=O)-H; -C(=O)-C₁₋₅-Alkyl, -CH₂-O-C(=O)-Phenyl, -O-C(=O)-Phenyl, -NH-S(=O)₂-C₁₋₅-Alkyl, -NH-C(=O)-C₁₋₅-Alkyl, -C(=O)-NH₂, -C(=O)-NH-C₁₋₅-Alkyl, -C(=O)-N(C₁₋₅-Alkyl)₂, Pyrazolyl, Phenyl, Furyl (Furanyl), Thiazolyl, Thiadiazolyl, Thiophenyl (Thienyl), Phenoxy, Benzyl und Phenethyl substituiert sein,
wobei die zyklischen Substituenten bzw. die zyklischen Reste dieser Substituenten selbst mit 1, 2, 3, 4 oder 5, bevorzugt mit 1, 2, 3 oder 4, Substituenten unabhängig voneinander ausgewählt aus der Gruppe bestehend aus F, Cl, Br, I, -CN, -NO₂, -OH, -SH, -NH₂, -C(=O)-OH, -C₁₋₅-Alkyl, -(CH₂)-O-C₁₋₅-Alkyl, -C₂₋₅-Alkenyl, -C₂₋₅-Alkinyl,-C≡C-Si(CH₃)₃, -C≡C-Si(C₂H₅)₃, -S-C₁₋₅-Alkyl, -O-C₁₋₅-Alkyl, -CF₃, -CHF₂, -CH₂F, -O-CF₃, -O-CHF₂, -O-CH₂F, -C(=O)-CF₃, -S-CF₃, -S-CHF₂ und -S-CH₂F substituiert sein können.

Besonders bevorzugt können die Substituenten, jeweils unabhängig voneinander ausgewählt werden aus der Gruppe bestehend aus F, Cl, Br, I, -CN, -NO₂, -OH, -SH, Methyl, Ethyl, n-Propyl, Isopropyl, n-Butyl, Isobutyl, sec-Butyl, tert.-Butyl, n-Pentyl, neo-Pentyl, Ethenyl, Allyl, Ethinyl, Propinyl, -C≡C-Si(CH₃)₃, -C≡C-Si(C₂H₅)₃, -CH₂-O-CH₃, -CH₂-O-C₂H₅, -OH, -SH, -NH₂, -C(=O)-OH, -S-CH₃, -S-C₂H₅, -S(=O)-CH₃,-S(=O)₂-CH₃, -S(=O)-C₂H₅, -S(=O)₂-C₂H₅, -O-CH₃, -O-C₂H₅, -O-C₃H₇, -O-C(CH₃)₃,-CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, Pyrazolyl, Phenyl, -N(CH₃)₂, -N(C₂H₅)₂, -NH-CH₃, -NH-C₂H₅,-CH₂-O-C(=O)-Phenyl, -NH-S(=O)₂-CH₃, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -O-C(=O)-Phenyl, -C(=O)-NH₂, -C(=O)-NH-CH₃, -C(=O)-N(CH₃)₂, Phenyl, Furyl (Furanyl), Thiadiazolyl, Thiophenyl (Thienyl), Phenoxy und Benzyl, wobei die zyklischen Substituenten bzw. die zyklischen Reste dieser Substituenten selbst jeweils mit 1, 2, 3, 4, oder 5, bevorzugt mit 1, 2, 3 oder 4, Substituenten unabhängig voneinander ausgewählt aus der Gruppe bestehend aus F, Cl, Br, I, -CN, -NO₂, -OH, -SH, -NH₂, -C(=O)-OH, Methyl, Ethyl, n-Propyl, Isopropyl, n-Butyl, Isobutyl, sec-Butyl, tert.-Butyl, n-Pentyl, neo-Pentyl, Ethenyl, Allyl, Ethinyl, Propinyl, -C≡C-Si(CH₃)₃, C=C-Si(C₂H₅)₃, -CH₂-O-CH₃, -CH₂-O-C₂H₅, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃,-CHF₂, -CH₂F, -O-CF₃, -O-CHF₂, -O-CH₂F, -C(=O)-CF₃, -S-CF₃, -S-CHF₂ und -S-CH₂F substituiert sein können.

Als geeignete Aryl-Reste seien beispielsweise Phenyl-, 1-Naphthyl, 2-Naphthyl und Anthracenyl genannt.

Sofern einer oder mehrere der Substituenten R¹ bis R²⁵ für einen Heteroaryl-Rest stehen oder einen Heteroaryl-Rest aufweisen, können dessen Heteroatom(e), jeweils unabhängig voneinander, bevorzugt ausgewählt werden aus der Gruppe bestehend aus Sauerstoff, Schwefel und Stickstoff. Bevorzugt kann ein Heteroaryl-Rest ggf. 1, 2, 3, 4 oder 5, besonders bevorzugt 1, 2 oder 3, Heteroatome aufweisen.

Als geeignete Heteroaryl-Reste seien beispielsweise Pyrrolyl, Indolyl, Furyl (Furanyl), Benzo[b]furanyl, Thienyl (Thiophenyl), Benzo[b]thienyl, Pyrazolyl, Imidazolyl, Thiazolyl, Dithiazolyl, Thiadiazolyl, Triazolyl, Oxazolyl, Oxadiazolyl, Isoxazolyl, Pyridinyl, Pyridazinyl, Pyrimidinyl, Pyrazinyl, Pyranyl, Indazolyl, Purinyl, Indolizinyl, Chinolinyl, Isochinolinyl und Chinazolinyl genannt.

Unter einem mono- bzw. polyzyklischen Ringsystem werden im Sinne der vorliegende Erfindung mono- bzw. polyzyklische Kohlenwasserstoffreste verstanden, die gesättigt, ungesättigt oder aromatisch sein und ggf. eines oder mehrere Heteroatome als Ringglieder aufweisen können. Ein solches mono- bzw. polyzyklisches Ringsystem kann beispielsweise mit einem cycloaliphatischen Rest, einem Aryl-Rest oder einem Heteroaryl-Rest kondensiert (anneliert) sein. Sofern ein polyzyklisches Ringsystem vorliegt, können die verschiedenen Ringe, jeweils unabhängig voneinander, einen unterschiedlichen Sättigungsgrad aufweisen, d.h. gesättigt, ungesättigt oder aromatisch sein. Die Heteroatome jedes Ringes können, jeweils unabhängig voneinander, bevorzugt ausgewählt werden aus der Gruppe bestehend aus Sauerstoff, Stickstoff und Schwefel. Vorzugsweise enthält ein Ring 0, 1, 2, 3, 4 oder 5, besonders bevorzugt 0, 1, 2 oder 3, Heteroatome. Vorzugsweise sind die jeweiligen Ringe des mono- oder polyzyklischen Ringsystems 4-, 5- oder 6-gliedrig.

Sofern einer oder mehrere der Substituenten R¹ bis R²⁵ ein monozyklisches oder polyzyklisches Ringsystem aufweisen, das einfach oder mehrfach substituiert ist, kann dieses bevorzugt mit ggf. 1, 2, 3, 4 oder 5 Substituenten substituiert sein, die unabhängig voneinander ausgewählt werden können aus der Gruppe bestehend aus Halogen, -CN, -NO₂, -OH, -SH, -NH₂, Oxo, Thioxo (=S), -C(=O)-OH, C₁₋₅-Alkyl, -C₂₋₅-Alkenyl, -C₂₋₅-Alkinyl, -C≡C-Si(CH₃)₃, -C≡C-Si(C₂H₅)₃, -(CH₂)-O-C₁₋₅-Alkyl, -S-C₁₋₅-Alkyl, -O-C₁₋₅-Alkyl, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, -S(=O)₂-C₁₋₅-Alkyl, -S(=O)-C₁₋₅-Alkyl, -NH-C₁₋₅-Alkyl, N(C₁₋₅Alkyl)(C₁₋₅-Alkyl -C(=O)-O-C₁₋₅-Alkyl, -C(=O)-H, -C(=O)-C₁₋₅-Alkyl,-CH₂-O-C(=O)-Phenyl, -O-C(=O)-Phenyl, -NH-S(=O)₂-C₁₋₅-Alkyl, -NH-C(=O)-C₁₋₅-Alkyl, -C(=O)-NH₂, -C(=O)-NH-C₁₋₅-Alkyl, -C(=O)-N(C₁₋₅-Alkyl)₂, Pyrazolyl, Phenyl, Furyl (Furanyl), Thiadiazolyl, Thiophenyl (Thienyl), Phenoxy und Benzyl, wobei die zyklischen Substituenten bzw. die zyklischen Reste dieser Substituenten selbst jeweils mit 1, 2, 3, 4 oder 5, bevorzugt mit 1, 2, 3 oder 4, Substituenten unabhängig voneinander ausgewählt aus der Gruppe bestehend aus F, Cl, Br, I, -CN, -CF₃, -OH, -NH₂, -O-CF₃, -SH, -O-C₁₋₅-Alkyl, -(CH₂)-O-C₁₋₅-Alkyl, -S-C₁₋₅-Alkyl, -C₁₋₅-Alkyl, -C₂₋₅-Alkenyl, -C₂₋₅-Alkinyl, -C≡C-Si(CH₃)₃, -C≡C-Si(C₂H₅)₃, -C(=O)-O-C₁₋₅-Alkyl und-C(=O)-CF₃ substituiert sein können.

Besonders bevorzugt können die Substituenten, jeweils unabhängig voneinander, ausgewählt werden aus der Gruppe bestehend aus F, Cl, Br, I, -CN, -NO₂, -OH, -SH, Methyl, Ethyl, n-Propyl, Isopropyl, n-Butyl, Isobutyl, sec-Butyl, tert.-Butyl, n-Pentyl, neo-Pentyl, Ethenyl, Allyl, Ethinyl, Propinyl, -C≡C-Si(CH₃)₃, -C≡C-Si(C₂H₅)₃, -C=C-Si(CH₃)₃, -C≡C-Si(C₂H₅)₃, -CH₂-O-CH₃, -CH₂-O-C₂H₅, -OH, -SH, -NH₂, oxo, -C(=O)-OH, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F,-C(=O)-CF₃, -S-CF₃, -S-CHF₂, -S-CH₂F, -S(=O)₂-Phenyl, Pyrazolyl, Phenyl, -N(CH₃)₂, -N(C₂H₅)₂, -NH-CH₃, -NH-C₂H₅, -CH₂-O-C(=O)-Phenyl, -NH-S(=O)₂-CH₃, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -O-C(=O)-Phenyl, -C(=O)-NH₂, -C(=O)-NH-CH₃,-C(=O)-N(CH₃)₂, Phenyl, Furyl (Furanyl), Thiadiazolyl, Thiophenyl (Thienyl), Phenoxy und Benzyl, wobei die zyklischen Substituenten bzw. die zyklischen Reste dieser Substituenten selbst mit 1, 2, 3, 4 oder 5, bevorzugt mit 1, 2, 3 oder 4, Substituenten unabhängig voneinander ausgewählt aus der Gruppe bestehend aus F, Cl, Br, I,-CN, -CF₃, -OH, -NH₂, -O-CF₃, -SH, -O-CH₃, -O-C₂H₅, -O-C₃H₇, Methyl, Ethyl, n-Propyl, Isopropyl, n-Butyl, sec-Butyl, Isobutyl, tert-Butyl, Ethenyl, Allyl, Ethinyl, Propinyl, -C≡C-Si(CH₃)₃, -C≡C-Si(C₂H₅)₃, -C(=O)-O-C₁-₅-Alkyl und -C(=O)-CF₃ substituiert sein können.

Sofern einer der vorstehend genannten Substituenten R¹ bis R²⁵ eine lineare oder verzweigte Alkylen-, Alkenylen- oder Alkinylen-Gruppe aufweist, die einfach oder mehrfach substituiert ist, kann diese bevorzugt ggf. 1, 2, 3, 4 oder 5, besonders bevorzugt 1, 2 oder 3 Substituenten aufweisen, die jeweils unabhängig voneinander, bevorzugt ausgewählt werden aus der Gruppe bestehend aus F, Cl, Br, -OH und unsubstituiertem Phenyl.

Sofern eine Alkylen-, Alkenylen- oder Alkinylen-Gruppe eines oder mehrere, vorzugsweise 1, 2 oder 3 Heteroatome, als Kettenglied(er) aufweist, können diese bevorzugt ausgewählt werden aus der Gruppe bestehend aus Sauerstoff, Schwefel und Stickstoff (NH).

Beispielhaft seien Alkylen-, Alkenylen- oder Alkinylen-Gruppen wie -(CH₂)-, -(CH₂)₂-, -C(H)(CH₃)-, -(CH₂)₃-, -(CH₂)₄-, -(CH₂)₅-, -C(H)(CH₂-Phenyl)-, -C(H)(Phenyl),-C(H)(C(H)(CH₃)₂)- -C(C₂H₅)(H)-, -(CH₂)-O-, -(CH₂)₂-O-, -(CH₂)₃-O-, -((CH₂)₄-O-, -O-(CH₂)-, -O-(ch₂)₂-, -O-(CH₂)₃-, -O-(CH₂)₄- -C(C₂H₅)(H)-O-, -O-C(C₂H₅)(H)-, -CH₂-O-CH₂-, -CH₂-S-CH₂-, -C(CH₃)₂-, -C(H)(CH₃)-, -CH=CH- und -C≡C- genannt.

Bevorzugt sind substituierte 1-Propiolyl-piperazine der vorstehend genannten allgemeinen Formel I,
worin
X für N oder C-R² steht,
R¹ für einen Wasserstoff-Rest, für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine -NH-R⁵-Gruppe, für eine -NR ⁶R⁷-Gruppe, für eine -C(=O)-R⁸-Gruppe, für eine -C(=O)-O-R⁹-Gruppe, für eine -O-C(=O)-R¹⁰-Gruppe, für eine -NH-C(=O)-R¹¹-Gruppe, für eine -NR¹²C(=0)-R¹³-Gruppe, für eine -C(=O)-NH₂-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für eine -NH-C(=O)-NH-R²¹-Gruppe, für eine -NH-C(=S)-NH-R²²-Gruppe, für eine -NH-S(=O)₂-R²³-Gruppe, für eine -NR²⁴-S(=O)₂-R²⁵-Gruppe, für einen linearen oder verzweigten, gesättigten oder ungesättigten, unsubstituierten oder wenigstens einfach substituierten, ggf. 1, 2 oder 3 Heteroatome unabhängig voneinander ausgewählt aus der Gruppe bestehend aus Sauerstoff, Schwefel- und Stickstoff als Kettenglied(er) aufweisenden aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte ggf. 1, 2 oder 3 Heteroatome unabhängig voneinander ausgewählt aus der Gruppe bestehend aus Sauerstoff, Schwefel- und Stickstoff als Kettenglied(er) aufweisende C₁₋₅-Alkylen-, C₂₋₅ -Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 Heteroatome unabhängig voneinander ausgewählt aus der Gruppe bestehend aus Sauerstoff, Schwefel- und Stickstoff als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, steht
R² für einen Wasserstoff-Rest, für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -NH-C(=O)-R¹¹-Gruppe, für eine -NR¹²C(=O)-R¹³-Gruppe, für eine -C(=O)-NH₂-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für eine -NH-C(=O)-NH-R²¹-Gruppe, für eine -NH-C(=S)-NH-R²²-Gruppe, für eine -NH-S(=O)₂-R²³-Gruppe, für eine -NR²⁴-S(=O)₂-R²⁵-Gruppe, für einen linearen oder verzweigten, gesättigten oder ungesättigten, unsubstituierten oder wenigstens einfach substituierten, ggf. 1, 2 oder 3 Heteroatome unabhängig voneinander ausgewählt aus der Gruppe bestehend aus Sauerstoff, Schwefel- und Stickstoff als Kettenglied(er) aufweisenden aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte ggf. 1, 2 oder 3 Heteroatome unabhängig voneinander ausgewählt aus der Gruppe bestehend aus Sauerstoff, Schwefel- und Stickstoff als Kettenglied(er) aufweisende C₁₋₅-Alkylen-, C₂₋₅ -Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 Heteroatome unabhängig voneinander ausgewählt aus der Gruppe bestehend aus Sauerstoff, Schwefel- und Stickstoff als Kettenglied(er) aufweisende C₁₋₅-Atkyien-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, steht
R³ für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo-Gruppe (=O), für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -NH-C(=O)-R¹¹-Gruppe, für -NR¹²-C(=O)-R¹³-Gruppe, für eine -C(=O)-NH₂-Gruppe, für eine -C(=O)-NH-R¹⁴-Gruppe, für eine -C(=O)-NR¹⁵SR¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für eine -NH-C(=O)-NH-R²¹-Gruppe, für eine -NH-C(=S)-NH-R²²-Gruppe, für eine -NH-S(=O)₂-R²³-Gruppe, für eine -NR²⁴-S(=O)₂-R²⁵-Gruppe, für einen linearen oder verzweigten, gesättigten oder ungesättigten, unsubstituierten oder wenigstens einfach substituierten, ggf. 1, 2 oder 3 Heteroatome unabhängig voneinander ausgewählt aus der Gruppe bestehend aus Sauerstoff, Schwefel- und Stickstoff als Kettenglied(er) aufweisenden aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 Heteroatome unabhängig voneinander ausgewählt aus der Gruppe bestehend aus Sauerstoff, Schwefel- und Stickstoff als Kettenglied(er) aufweisende C₁₋₅-Alkylen-, C₂_ ₅-Alkenylen- oder C₂₋₅-Alkinylen-Gruppe gebunden und/oder mit einem unsubstituierten oder wenigstens einfach substituierten mono- oder polyzyklischen Ringsystem kondensiert und/oder sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 Heteroatome unabhängig voneinander ausgewählt aus der Gruppe bestehend aus Sauerstoff, Schwefel- und Stickstoff als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, steht,
R⁴ für einen Wasserstoff-Rest, für einen linearen oder verzweigten, gesättigten oder ungesättigten, unsubstituierten oder wenigstens einfach substituierten aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 Heteroatome unabhängig voneinander ausgewählt aus der Gruppe bestehend aus Sauerstoff, Schwefel- und Stickstoff als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 Heteroatome unabhängig voneinander ausgewählt aus der Gruppe bestehend aus Sauerstoff, Schwefel- und Stickstoff als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, steht,
R⁵ bis R²⁵, unabhängig voneinander, jeweils für einen linearen oder verzweigten, gesättigten oder ungesättigten, unsubstituierten oder wenigstens einfach substituierten aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 Heteroatome unabhängig voneinander ausgewählt aus der Gruppe bestehend aus Sauerstoff, Schwefel- und Stickstoff als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 Heteroatome unabhängig voneinander ausgewählt aus der Gruppe bestehend aus Sauerstoff, Schwefel- und Stickstoff als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, stehen,
und
- n: gleich 0, 1, 2, 3, 4, 5, 6, 7 oder 8 ist,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 1-Propiolyl-piperazine der vorstehend genannten allgemeinen Formel I,
worin
X für N oder C-R² steht,
n gleich 0, 1, 2, 3, 4, 5, 6, 7 oder 8 ist,
R¹ und R², unabhängig voneinander, jeweils für einen Wasserstoff-Rest, für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine -NH-R⁵-Gruppe, für eine-NR⁶R⁷-Gruppe, für eine -C(=O)-R⁸-Gruppe, für eine -C(=O)-O-R⁹-Gruppe, für eine-O-C(=O)-R¹⁰-Gruppe, für eine -NH-C(=O)-R¹¹-Gruppe, für eine -NR¹²-C(=O)-R¹³-Gruppe, für eine -C(=O)-NH₂-Gruppe, für eine -C(=O)-NH-R¹⁴-Gruppe, für eine-C(=O)-NR¹⁵R¹⁶-Gruppe, für eine -O-R¹⁷-Gruppe, für eine -S-R¹⁸-Gruppe, für eine-S(=O)-R¹⁹-Gruppe, für eine -S(=O)₂-R²⁰-Gruppe, für eine -NH-C(=O)-NH-R²¹-Gruppe, für eine -NH-C(=S)-NH-R²²-Gruppe, für eine -NH-S(=O)₂-R²³-Gruppe, für eine -NR²⁴-S(=O)₂-R²⁵-Gruppe, für einen linearen oder verzweigten, gesättigten oder ungesättigten, unsubstituierten oder wenigstens einfach substituierten, ggf. 1, 2 oder 3 unabhängig voneinander aus der Gruppe bestehend aus Sauerstoff, Schwefel und Stickstoff ausgewählte Heteroatome als Kettenglied(er) aufweisenden aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, stehen,
R³ für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo-Gruppe (=O), für eine -NH-R⁵-Gruppe, für eine -NR⁶R⁷-Gruppe, für eine -C(=O)-R⁸-Gruppe, für eine -C(=O)-O-R⁹-Gruppe, für eine O-C(=O)-R¹⁰-Gruppe, für eine -NH-C(=O)-R¹¹-Gruppe, für -NR¹²-C(=O)-R¹³-Gruppe, für eine -C(=O)-NH₂-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für eine -NH-C(=O)-NH-R²¹-Gruppe, für eine -NH-C(=S)-NH-R²²-Gruppe, für eine -NH-S(=O)₂-R²³-Gruppe, für eine -NR²⁴-S(=O)₂-R²⁵-Gruppe, für einen linearen oder verzweigten, gesättigten oder ungesättigten, unsubstituierten oder wenigstens einfach substituierten, ggf. 1, 2 oder 3 unabhängig voneinander aus der Gruppe bestehend aus Sauerstoff, Schwefel und Stickstoff ausgewählte Heteroatome als Kettenglied(er) aufweisenden aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, steht,
R⁴ für einen Wasserstoff-Rest, für eine -C(=O)-NH₂-Gruppe, für eine -C(=O)-NH-R¹⁴-Gruppe, für eine -C(=O)-NR¹⁵R¹⁶-Gruppe, für einen linearen oder verzweigten, gesättigten oder ungesättigten, unsubstituierten oder wenigstens einfach substituierten aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, steht,
und
R⁵ bis R²⁵, unabhängig voneinander, jeweils für einen linearen oder verzweigten, gesättigten oder ungesättigten, unsubstituierten oder wenigstens einfach substituierten aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, stehen;
wobei
die vorstehend genannten cycloaliphatischen Reste ggf. 1, 2, 3, 4 oder 5 Heteroatom(e) als Ringglied(er) aufweisen können, die jeweils unabhängig voneinander, ausgewählt werden können aus der Gruppe bestehend aus Stickstoff, Sauerstoff und Schwefel,
die Ringe der vorstehend genannten mono- oder bizyklischen Ringsysteme jeweils 4-, 5- oder 6-gliedrig sind und jeweils ggf. 0, 1, 2, 3, 4 oder 5 Heteroatom(e) als Ringglied(er) aufweisen, die unabhängig voneinander aus der Gruppe bestehend aus Sauerstoff, Stickstoff und Schwefel ausgewählt sind;
und
die vorstehend genannten Heteroaryl-Reste ggf. 1, 2, 3, 4 oder 5 Heteroatom(e) als Ringglied(er) aufweisen können, die jeweils unabhängig voneinander, ausgewählt werden können aus der Gruppe bestehend aus Sauerstoff, Schwefel und Stickstoff;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze,
bevorzugt in Form entsprechender Hydrochloride, oder jeweils in Form entsprechender Solvate.

Bevorzugt sind ferner substituierte 1-Propiolyl-piperazine der vorstehend angegebenen allgemeinen Formel I, in denen der Rest R¹
für einen Wasserstoff-Rest, für einen Halogen-Rest ausgewählt aus der Gruppe bestehend aus F, Cl, Br und I, für eine Nitro-Gruppe, für eine CF₃-Gruppe, für eine Cyano-Gruppe, für eine Amino-Gruppe, für eine Hydroxy-Gruppe, für eine Thiol-Gruppe, für eine Carboxy-Gruppe, für eine -NH-R⁵-Gruppe, für eine -NR⁶R⁷-Gruppe, für eine -C(=O)-R⁸-Gruppe, für eine -C(=O)-O-R⁹-Gruppe, für eine -O-G(=O)-R¹⁰-Gruppe, für eine -C(=O)-NH₂-Gruppe, für eine -C(=O)-NH-R¹⁴-Gruppe, für eine-C(=O)-NR¹⁵R¹⁶-Gruppe, für eine -O-R¹⁷-Gruppe, für eine -S-R¹⁸-Gruppe, für eineS(=O)-R¹⁹-Gruppe, für eine -S(=O)₂-R²⁰-Gruppe, für einen linearen oder verzweigten C₁₋₈-Alkyl-Rest, für einen linearen oder verzweigten C₂₋₈-Alkenyl-Rest, für einen linearen oder verzweigten C₂₋₈-Alkinyl-Rest, für einen unsubstituierten oder wenigstens einfach substituierten, ungesättigten oder gesättigten, ggf. 1, 2 oder 3 unabhängig voneinander aus der Gruppe bestehend aus Sauerstoff, Schwefel und Stickstoff ausgewählte Heteroatome als Kettenglied(er) aufweisenden 3-, 4-, 5-, 6- oder 7-gliedrigen cycloaliphatischen Rest, der über eine lineare oder verzweigte, unsubstituierte oder wenigstens einfach substituierte C₁₋₅-Alkylen-, C₂₋₅-Alkenylen- oder C₂₋₅-Alkinylen-Gruppe gebunden sein kann oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte C₁₋₅ -Alkylen-, C₂₋₅-Alkenylen- oder C₂₋₅-Alkinylen-Gruppe gebunden sein kann, steht,
besonders bevorzugt für einen Wasserstoff-Rest, für einen Halogen-Rest ausgewählt aus der Gruppe bestehend aus F, Cl und Br, für eine CF₃-Gruppe, eine Nitro-Gruppe, für eine Cyano-Gruppe, für eine Amino-Gruppe, für eine Hydroxy-Gruppe, für eine Thiol-Gruppe, für eine Carboxy-Gruppe, für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -C(=O)-NH-R¹⁴ -Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für einen linearen oder verzweigten C₁₋₄-Alkyl-Rest, für einen linearen oder verzweigten C₂₋₄-Alkenyl-Rest, für einen linearen oder verzweigten C₂₋₄-Alkinyl-Rest, oder für einen Rest ausgewählt aus der Gruppe bestehend aus Cyclopropyl-, Cyclobutyl-, Cyclopentyl-, Cyclohexyl-, Pyrrolidinyl-, Tetrahydrofuranyl-, Tetrahydrothiophenyl- Piperidinyl-, Tetrahydropyranyl-, Piperazinyl-, Morpholinyl-, Thiomorpholinyl-, Dioxolanyl-, Azepanyl-, Diazepanyl-, Imidazolidinyl-, Phenyl-, Naphthyl-, Anthracenyl-, Pyrrolyl-, Indolyl-, Furanyl-, Benzo[b]furanyl-, Thiophenyl-, Benzo[b]thiophenyl-, Pyrazolyl-, Imidazolyl-, Thiazolyl-, Dithiazolyl-, Thiadiazolyl-, Triazolyl-, Oxazolyl-, Oxadiazolyl-, Isoxazolyl-, Pyridinyl-, Pyridazinyl-, Pyrimidinyl-, Pyrazinyl-, Pyranyl-, Indazolyl-, Purinyl-, Indolizinyl-, Chinolinyl-, Isochinolinyl- und Chinazolinyl-Rest, der jeweils über eine C₁₋₃-Alkylen-, C₂₋₃-Alkenylen- oder C₂₋₃-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅, -O-C₃H₇, NH₂, -N(CH₃)₂, -N(C₂H₅)₂, -NH-CH₃, -NH-C₂H₅, -NO₂, -CF₃, -O-CF₃, -S-CF₃, -SH,-NH-S(=O)₂-CH₃, -C(=O)-OH, -C(=O)-CH₃, -C(=O)-C₂H₅, -C(=O)-N(CH₃)₂, -C(=O)-NH-CH₃, -NH-C(=O)-CH₃, -NH-C(=O)-C₂H₅, -C(=O)-O-CH₃ und -C(=O)-O-C₂H₅ substituiert ist, steht,
ganz besonders bevorzugt für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Monomethylamino-Gruppe, für eine Monoethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R ²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht;
und jeweils die übrigen Reste X, n, R², R³, R⁴ und R⁵ bis R ²⁵ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

Weiterhin bevorzugt sind substituierte 1-Propiolyl-piperazine der vorstehend angegebenen allgemeinen Formel I, worin der Rest R²
für einen Wasserstoff-Rest, für einen Halogen-Rest ausgewählt aus der Gruppe bestehend aus F, Cl, Br und I, für eine Nitro-Gruppe, für eine CF₃-Gruppe, für eine Cyano-Gruppe, für eine Amino-Gruppe, für eine Hydroxy-Gruppe, für eine Thiol-Gruppe, für eine Carboxy-Gruppe, für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -C(=O)-NH₂-Gruppe, für eine -C(=O)-NH-R¹⁴-Gruppe, für eine-C(=O)-NR¹⁵R¹⁶-Gruppe, für eine -O-R¹⁷-Gruppe, für eine -S-R¹⁸-Gruppe, für eineS(=O)-R¹⁹-Gruppe, für eine -S(=O)₂-R²⁰-Gruppe, für einen linearen oder verzweigten C₁₋₈-Alkyl-Rest, für einen linearen oder verzweigten C₂₋₈-Alkenyl-Rest oder für einen linearen oder verzweigten C₂₋₈-Alkinyl-Rest, für einen unsubstituierten oder wenigstens einfach substituierten, ungesättigten oder gesättigten, ggf. 1, 2 oder 3 unabhängig voneinander aus der Gruppe bestehend aus Sauerstoff, Schwefel und Stickstoff ausgewählte Heteroatome als Ringglied(er) aufweisenden, 3-, 4-, 5-, 6- oder 7-gliedrigen cycloaliphatischen Rest, der über eine lineare oder verzweigte, unsubstituierte oder wenigstens einfach substituierte C₁₋₅-Alkylen-, C₂₋₅-Alkenylen- oder C₂₋₅-Alkinylen-Gruppe gebunden sein kann oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte C₁₋₅-Alkylen-, C₂₋₅-Alkenylen- oder C₂₋₅-Alkinylen-Gruppe gebunden sein kann, steht,
besonders bevorzugt für einen Wasserstoff-Rest, für einen Halogen-Rest ausgewählt aus der Gruppe bestehend aus F, Cl und Br, für eine CF₃-Gruppe, eine Nitro-Gruppe, für eine Cyano-Gruppe, für eine Amino-Gruppe, für eine Hydroxy-Gruppe, für eine Thiol-Gruppe, für eine Carboxy-Gruppe, für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -C(=O)-NH-R¹⁴-Gruppe, für eine -C(=O)-NR¹⁵R¹⁶-Gruppe, für eine -S(=O)-R¹⁹-Gruppe, für eine -S(=O)₂-R ²⁰-Gruppe, für eine O-R¹⁷-Gruppe, für eine -S-R¹⁸-Gruppe, für einen linearen oder verzweigten C₁₋₄-Alkyl-Rest, für einen linearen oder verzweigten C₂₋₄-Alkenyl-Rest, für einen linearen oder verzweigten C₂₋₄-Alkinyl-Rest, oder für einen Rest ausgewählt aus der Gruppe bestehend aus Cyclopropyl-, Cyclobutyl-, Cyclopentyl-, Cyclohexyl-, Pyrrolidinyl-, Tetrahydrofuranyl-, Tetrahydrothiophenyl- Piperidinyl-, Tetrahydropyranyl-, Piperazinyl-, Morpholinyl-, Thiomorpholinyl-, Dioxolanyl-, Azepanyl-, Diazepanyl-, Imidazolidinyl-, Phenyl-, Naphthyl-, Anthracenyl-, Pyrrolyl-, Indolyl-, Furanyl-, Benzo[b]furanyl-, Thiophenyl-, Benzo[b]thiophenyl-, Pyrazolyl-, Imidazolyl-, Thiazolyl-, Dithiazolyl-, Thiadiazolyl-, Triazolyl-, Oxazolyl-, Oxadiazolyl-, Isoxazolyl-, Pyridinyl-, Pyridazinyl-, Pyrimidinyl-, Pyrazinyl-, Pyranyl-, Indazolyl-, Purinyl-, Indolizinyl-, Chinolinyl-, Isochinolinyl- und Chinazolinyl-Rest, der jeweils über eine C₁₋₃-Alkylen-, C₂₋₃-Alkenylen- oder C₂₋₃-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅, O-C₃H₇,-NH₂, -N(CH₃)₂, -N(C₂H₅)₂, -NH-CH₃, -NH-C₂H₅, -NO₂, -CF₃, -O-CF₃, -S-CF₃, -SH,-NH-S(=O)₂-C_{H3}, -C(=O)-OH, -C(=O)-CH₃, -C(=O)-C₂H₅, -C(=O)-N(CH₃)₂, -C(=O)-NH-CH₃, -NH-C(=O)-CH₃, -NH-C(=O)-C₂H₅, -C(=O)-O-CH₃ und -C(=O)-O-C₂H₅ substituiert ist, steht,
ganz besonders bevorzugt für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Monomethylamino-Gruppe, für eine Monoethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht;
und jeweils die übrigen Reste X, n, R¹, R³, R⁴ und R⁵ bis R²⁵ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

Ebenfalls bevorzugt sind substituierte 1-Propiolyl-piperazine der vorstehend angegebenen allgemeinen Formel I, in denen R³
für einen Halogen-Rest ausgewählt aus der Gruppe bestehend aus F, Cl und Br, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Oxo-Gruppe (=O), oder für einen linearen oder verzweigten C₁₋₄-Alkyl-Rest, oder für einen Rest ausgewählt aus der Gruppe bestehend aus Cyclopropyl-, Cyclobutyl-, Cyclopentyl-, Cyclohexyl-, Pyrrolidinyl-, Tetrahydrofuranyl-, Tetrahydrothiophenyl- Piperidinyl-, Tetrahydropyranyl-, Piperazinyl-, Morpholinyl-, Thiomorpholinyl-, Dioxolanyl-, Azepanyl-, Diazepanyl-, Imidazolidinyl-, Phenyl-, Naphthyl-, Anthracenyl-, Pyrrolyl-, Indolyl-, Furanyl-, Benzo[b]furanyl-, Thiophenyl-, Benzo[b]thiophenyl-, Pyrazolyl-, Imidazolyl-, Thiazolyl-, Dithiazolyl-, Thiadiazolyl-, Triazolyl-, Oxazolyl-, Oxadiazolyl-, Isoxazolyl-, Pyridinyl-, Pyridazinyl-, Pyrimidinyl-, Pyrazinyl-, Pyranyl-, Indazolyl-, Purinyl-, Indolizinyl-, Chinolinyl-, Isochinolinyl- und Chinazolinyl-Rest, der unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht,
besonders bevorzugt für eine Oxo-Gruppe (=O), für einen Alkyl-Rest ausgewählt aus der Gruppe bestehend aus Methyl, Ethyl, n-Propyl, Isopropyl, n-Butyl, sec-Butyl, Isobutyl und tert-Butyl oder für einen Phenyl-Rest, der unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht;
und jeweils die übrigen Reste X, n, R¹, R², R⁴ und R⁵ bis R²⁵ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

Der Fachmann versteht, dass jeweils ggf. 1, 2, 3, 4, 5, 6, 7 oder 8 gleiche oder zumindest teilweise verschiedene Reste R³ ausgewählt werden können.

Besonders bevorzugt sind die vorstehend genannten Reste R³ wie folgend an den Piperazinyl-Rest der vorstehend angegebenen allgemeinen Formel I gebunden, so dass sich die folgenden allgemeinen Formeln Ib bis li ergeben: 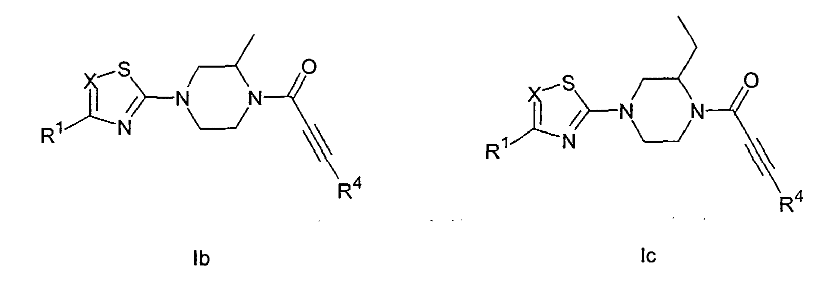 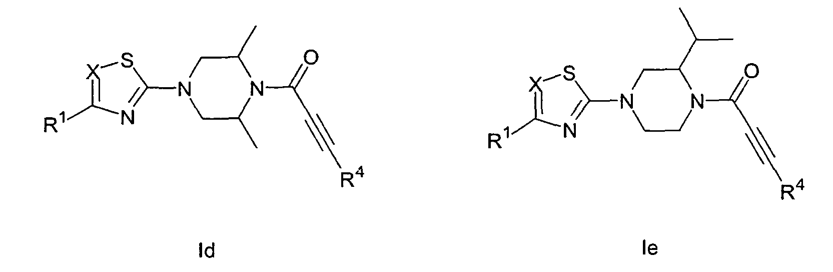 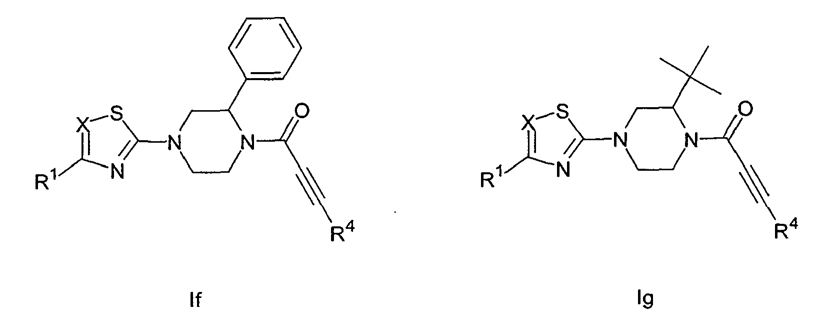 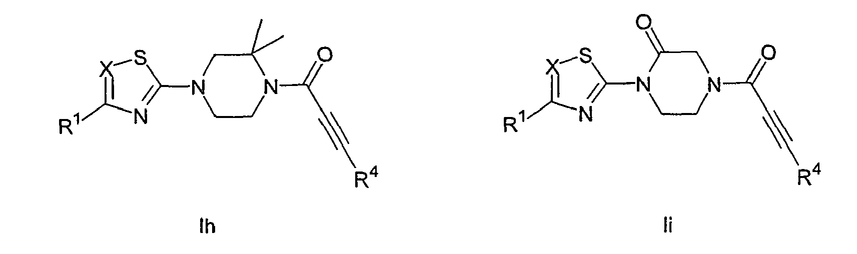 jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

Weiterhin bevorzugt sind substituierte 1-Propiolyl-piperazine der vorstehend angegebenen allgemeinen Formel I, in denen R⁴
für einen Wasserstoff-Rest, für eine -C(=O)-NH₂-Gruppe, für eine -C(=O)-NH-R¹⁴-Gruppe, für eine -C(=O)-NR¹⁵R¹⁶-Gruppe, für einen linearen oder verzweigten, unsubstituierten oder wenigstens einfach substituierten C₁₋₈-Alkyl-Rest, für einen linearen oder verzweigten, unsubstituierten oder wenigstens einfach substituierten C₂₋₈-Alkenyl-Rest, für einen linearen oder verzweigten, unsubstituierten oder wenigstens einfach substituierten C₂₋₈-Alkinyl-Rest, für einen unsubstituierten oder wenigstens einfach substituierten, ungesättigten oder gesättigten 4-, 5-, 6- oder 7-gliedrigen cycloaliphatischen Rest, der mit einem ungesättigten, gesättigten oder aromatischen, unsubstituierten oder wenigstens einfach substituierten, mono- oder bizyklischen Ringsystem kondensiert sein kann, wobei jeder der Ringe 1, 2 oder 3 Heteroatome aufweisen kann, die unabhängig voneinander ausgewählt werden können aus der Gruppe bestehend aus Sauerstoff, Stickstoff und Schwefel und wobei die Ringe des mono- oder bizyklischen Ringsystems jeweils 4-, 5- oder 6-gliedrig sind, oder für einen unsubstituierten oder wenigstens einfach substituierten 5- oder 6-gliedrigen Aryl- oder Heteroaryl-Rest, der mit einem gesättigten, ungesättigten oder aromatischen, unsubstituierten oder wenigstens einfach substituierten, mono- oder bizyklischen Ringsystem kondensiert sein kann, wobei der Heteroaryl-Rest und ggf. einer oder beide Ringe des mono- oder bizyklischen Ringsystems jeweils 1, 2 oder 3 Heteroatome aufweisen, die unabhängig voneinander ausgewählt werden können aus der Gruppe bestehend aus Sauerstoff, Stickstoff und Schwefel und wobei die Ringe des mono- oder bizyklischen Ringsystems jeweils 4-, 5- oder 6-gliedrig sind, steht,
besonders bevorzugt für einen Wasserstoff-Rest, für eine -C(=O)-NH₂-Gruppe, für eine -C(=O)-NH-R¹⁴-Gruppe, für eine -C(=O)-NR¹⁵R¹⁶-Gruppe, für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, n-Heptyl und n-Octyl, oder für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Pyrrolidinyl, Tetrahydrofuranyl, Tetrahydrothiophenyl, Piperidinyl, Tetrahydropyranyl, Piperazinyl, Morpholinyl, Thiomorpholinyl, Dioxolanyl, Azepanyl, Diazepanyl, Imidazolidinyl, Phenyl, Naphthyl, Anthracenyl, Furanyl, Thiophenyl, Pyrazolyl, Pyrazinyl, Pyrimidinyl, Pyridinyl, Pyrrolyl, Oxazolyl, Isoxazolyl, Thiazolyl, Dithiazolyl, Oxadiazolyl, Triazolyl, Imidazolyl, Indolyl, Benzo[b]thiophenyl, Benzo[b]furanyl, Chinolinyl, Isochinolinyl und Chinazolyl steht, der jeweils ggf. mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, Ethenyl, Allyl, Ethinyl, Propinyl, -C≡C-Si(CH₃)₃, -C=C-Si(C₂H₅)₃,-CH₂-O-CH₃, -CH₂-O-C₂H₅, -OH, -O-CH₃, -O-C₂H₅, -O-C₃H₇, -S-CH₃, -S-C₂H₅, -S(=O)-CH₃, -S(=O)₂-CH₃, -S(=O)-C₂H₅, -S(=O)₂-C₂H₅, -NH₂, -N(CH₃)₂, -N(C₂H₅)₂, -NH-CH₃, -NH-C₂H₅, -NO₂, -CF₃, -CHF₂, -CH₂F, -O-CF₃, -S-CF₃, -SH, -NH-S(=O)₂-CH₃, -C(=O)-OH, -C(=O)-H; -C(=O)-CH₃, -C(=O)-C₂H₅, -C(=O)-NH₂, -C(=O)-N(CH₃)₂, -C(=O)-NH-CH₃, -NH-C(=O)-CH₃, -NH-C(=O)-C₂H₅, -C(=O)-O-CH₃, -C(=O)-O-C₂H₅, -C(=O)-O-C(CH₃)₃ und Phenyl substituiert sein kann, steht,
ganz besonders bevorzugt für einen Wasserstoff-Rest, für eine -C(=O)-NH₂-Gruppe, für eine -C(=O)-NH-R¹⁴-Gruppe, für eine -C(=O)-NR¹⁵R¹⁶-Gruppe, für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, neo-Pentyl, n-Hexyl, n-Heptyl und n-Octyl, oder für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Phenyl, Pyrid-2-yl, Pyrid-3-yl, Pyrid-4-yl, Pyrrol-2-yl, Pyrrol-3-yl, Thiophen-2-yl, Thiophen-3-yl, 4-Methyl-thiophen-2-yl, 3-Methyl-thiophen-2-yl, 5-Methyl-thiophen-2-yl, Furan-2-yl, Furan-3-yl, lmidazol-2-yl, Imidazol-4-yl, Thiazol-2-yl, Thiazol-4-yl, Thiazol-5-yl, Oxazol-2-yl, Oxazol-4-yl, Oxazol-5-yl, (1,2,4)-Thiadiazol-5-yl, (1,2,4)-Oxadiazol-5-yl, Napht-1-yl, Napht-2-yl, Anthracen-1-yl, Anthracen-2-yl, Anthracen-9-yl, Indol-2-yl, Indol-3-yl, Indol-4-yl, Indol-5-yl, Indol-6-yl, Indol-7-yl, 1-Methyl-indol-2-yl, 1-Methyl-indol-3-yl, 1-Methyl-indol-4-yl, 1-Methyl-indol-5-yl, 1-Methyl-indol-6-yl, 1-Methyl-indol-7-yl, Chinolin-3-yl, Chinolin-4-yl, Chinolin-2-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, Isochinolin-8-yl, 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Hydroxyphenyl, 3-Hydroxy-phenyl, 4-Hydroxy-phenyl, 2-Amino-phenyl, 3-Amino-phenyl, 4-Amino-phenyl, 2-Dimethylamino-phenyl, 3-Dimethylamino-phenyl, 4-Dimethylaminophenyl, 2-Methylamino-phenyl, 3-Methylamino-phenyl, 4-Methylamino-phenyl, 2-Acetyl-phenyl, 3-Acetyl-phenyl, 4-Acetyl-phenyl, 2-Methylsulfinyl-phenyl, 3-Methylsulfinyl-phenyl, 4-Methylsulfinyl-phenyl, 2-Methylsulfonyl-phenyl, 3-Methylsulfonyl-phenyl, 4-Methylsulfonyl-phenyl, 2-Methoxy-phenyl, 3-Methoxyphenyl, 4-Methoxy-phenyl, 2-Chlor-phenyl, 3-Chlor-phenyl, 4-Chlor-phenyl, 2-Ethoxyphenyl, 3-Ethoxy-phenyl, 4-Ethoxyphenyl, 2-Trifluormethyl-phenyl, 3-Trifluormethyl-phenyl, 4-Trifluormethyl-phenyl, 2-Difluormethyl-phenyl, 3-Difluormethyl-phenyl, 4-Difluormethyl-phenyl, 2-Fluormethyl-phenyl, 3-Fluormethyl-phenyl, 4-Fluormethyl-phenyl, 2-Nitro-phenyl, 3-Nitro-phenyl, 4-Nitro-phenyl, 2-Ethyl-phenyl, 3-Ethyl-phenyl, 4-Ethyl-phenyl, 2-Propyl-phenyl, 3-Propyl-phenyl, 4-Propyl-phenyl, 2-Isopropylphenyl, 3-Isopropyl-phenyl, 4-Isopropyl-phenyl, 2-tert-Butyl-phenyl, 3-tert-Butylphenyl, 4-tert-Butyl-phenyl, 2-Carboxyphenyl, 3-Carboxy-phenyl, 4-Carboxyphenyl, 2-Ethenyl-phenyl, 3-Ethenyl-phenyl, 4-Ethenyl-phenyl, 2-Ethinyl-phenyl, 3-Ethinylphenyl, 4-Ethinyl-phenyl, 2-Allyl-phenyl, 3-Allyl-phenyl, 4-Allyl-phenyl, 2-Trimethylsilanylethinyl-phenyl, 3-Trimethylsilanylethinyl-phenyl, 4-Trimethylsilanylethinyl-phenyl, 2-Formyl-phenyl, 3-Formyl-phenyl, 4-Formyl-phenyl, 2-Acetamino-phenyl, 3-Acetamino-phenyl, 4-Acetamino-phenyl, 2-Dimethylaminocarbonyl-phenyl, 3-Dimethylaminocarbonyl-phenyl, 4-Dimethylaminocarbonyl-phenyl, 2-Methoxymethyl-phenyl, 3-Methoxymethyl-phenyl, 4-Methoxymethyl-phenyl, 2-Ethoxymethyl-phenyl, 3-Ethoxymethyl-phenyl, 4-Ethoxymethyl-phenyl, 2-Aminocarbonyl-phenyl, 3-Aminocarbonyl-phenyl, 4-Aminocarbonyl-phenyl, 2-Methylaminocarbonyl-phenyl, 3-Methylaminocarbonylphenyl, 4-Methylaminocarbonyl-phenyl, 2-Carboxymethylester-phenyl, 3-Carboxymethylester-phenyl, 4-Carboxymethylester-phenyl, 2-Carboxyethylesterphenyl, 3-Carboxyethylester-phenyl, 4-Carboxyethylester-phenyl, 2-Carboxy-tert-butylester-phenyl, 3-Carboxy-tert-butylester-phenyl, 4-Carboxy-tert-butylester-phenyl, 2-Methylmercapto-phenyl, 3-Methylmercapto-phenyl, 4-Methylmercapto-phenyl, 2-Ethylmercapto-phenyl, 3-Ethylmercapto-phenyl, 4-Ethylmercaptophenyl, 2-Biphenyl, 3-Biphenyl, 4-Biphenyl, 2-Bromphenyl, 3-Bromphenyl, 4-Bromphenyl, 2-lod-phenyl, 3-lodphenyl, 4-lodphenyl, 2-Trifluormethoxy-phenyl, 3-Trilfuormethoxy-phenyl, 4-Trifluormethoxy-phenyl, 2-Fluor-3-trifluormethylphenyl, 2-Fluor-4-methyl-phenyl, (2,3)-Difluorphenyl, (2,3)-Dimethyl-phenyl, (2,3)-Dichlorphenyl, 3-Fluor-2-trifluormethylphenyl, (2,4)-Dichlor-phenyl, (2,4)-Difluorphenyl, 4-Fluor-2-trifluormethyl-phenyl, (2,4)-Dimethoxyphenyl, 2-Chlor-4-fluor-phenyl, 2-Chlor-4-nitro-phenyl, 2-Chlor-4-methyl-phenyl, 2-Chlor-5-trifluormethyl-phenyl, 2-Chlor-5-methoxy-phenyl, 2-Brom-5-trifluormethyl-phenyl, 2-Brom-5-methoxy-phenyl, (2,4)-Dibrom-phenyl, (2,4)-Dimethyl-phenyl, 2-Fluor-4-trifluormethyl-phenyl, (2,5)-Difluor-phenyl, 2-Fluor-5-trifluormethyl-phenyl, 5-Fluor-2-trifluormethyl-phenyl 5-Chlor-2-trifluormethyl-phenyl, 5-Brom-2-trifluormethyl-phenyl, (2,5)-Dimethoxy-phenyl, (2,5)-Bis-trifluormethyl-phenyl, (2,5)-Dichlor-phenyl, (2,5)-Dibrom-phenyl, 2-Methoxy-5-nitro-phenyl, 2-Fluor-6-trifluormethyl-phenyl, (2,6)-Dimethoxy-phenyl, (2,6)-Dimethyl-phenyl, (2,6)-Dichlorphenyl, 2-Chlor-6-fluor-phenyl, 2-Brom-6-chlor-phenyl, 2-Brom-6-fluor-phenyl, (2,6)-Difluor-phenyl, (2,6)-Difluor-3-methyl-phenyl, (2,6)-Dibrom-phenyl, (2,6)-Dichlorphenyl, 3-Chlor-2-fluor-phenyl, 3-Chlor-5-methyl-phenyl, (3,4)-Dichlorphenyl, (3,4)-Dimethyl-phenyl, 3-Methyl-4-methoxy-phenyl, 4-Chlor-3-nitro-phenyl, (3,4)-Dimethoxy-phenyl, 4-Fluor-3-trifluormethylphenyl, 3-Fluor-4-trifluormethyl-phenyl, (3,4)-Difluor-phenyl, 3-Cyano-4-fluor-phenyl, 3-Cyano-4-methyl-phenyl, 3-Cyano-4-methoxy-phenyl, 3-Brom-4-fluor-phenyl, 3-Brom-4-methyl-phenyl, 3-Brom-4-methoxyphenyl, 4-Chlor-2-fluor-phenyl, 4-Chlor-3-trifluormethyl, 4-Brom-3-methyl-phenyl, 4-Brom-5-methyl-phenyl, 3-Chlor-4-fluor-phenyl, 4-Fluor-3-nitro-phenyl, 4-Brom-3-nitrophenyl, (3,4)-Dibrom-phenyl, 4-Chlor-3-methyl-phenyl, 4-Brom-3-methyl-phenyl, 4-Fluor-3-methyl-phenyl, 3-Fluor-4-methyl-phenyl, 3-Fluor-5-methyl-phenyl, 2-Fluor-3-methyl-phenyl, 4-Methyl-3-nitro-phenyl, (3,5)-Dimethoxy-phenyl, (3,5)-Dimethylphenyl, (3,5)-Bis-trifluormethyl-phenyl, (3,5)-Difluor-phenyl, (3,5)-Dinitro-phenyl, (3,5)-Dichlor-phenyl, 3-Fluor-5-trifluormethyl-phenyl, 5-Fluor-3-trifluormethyl-phenyl, (3,5)-Dibrom-phenyl, 5-Chlor-4-fluor-phenyl, 5-Chlor-4-fluor-phenyl, 5-Brom-4-methylphenyl, (2,3,4)-Trifluorphenyl, (2,3,4)-Trichlorphenyl, (2,3,6)-Trifluor-phenyl, 5-Chlor-2-methoxy-phenyl, (2,3)-Difluor-4-methyl, (2,4,5)-Trifluor-phenyl, (2,4,5)-Trichlorphenyl, (2,4)-Dichlor-5-fluor-phenyl, (2,4,6)-Trichlor-phenyl, (2,4,6)-Trimethylphenyl, (2,4,6)-Trifluor-phenyl, (2,4,6)-Trimethoxy-phenyl, (3,4,5)-Trimethoxy-phenyl, (2,3,4,5)-Tetrafluor-phenyl, 4-Methoxy-(2,3,6)-trimethyl-phenyl, 4-Methoxy-(2,3,6)-trimethyl-phenyl, 4-Chlor-2,5-dimethyl-phenyl, 2-Chlor-6-fluor-3-methyl-phenyl, 6-Chlor-2-fluor-3-methyl, (2,4,6)-Trimethylphenyl, (2,3,4,5,6)-Pentafluor-phenyl, 3-Methyl-pyrid-2-yl, 4-Methyl-pyrid-2-yl, 5-Methyl-pyrid-2-yl, 6-Methyl-pyrid-2-yl, 2-Methyl-pyrid-3-yl, 4-Methyl-pyrid-3-yl, 5-Methyl-pyrid-3-yl, 6-Methyl-pyrid-3-yl, 2-Methyl-pyrid-4-yl, 3-Methyl-pyrid-4-yl, 3-Fluor-pyrid-2-yl, 4-Fluor-pyrid-2-yl, 5-Fluorpyrid-2-yl, 6-Fluor-pyrid-2-yl, 3-Chlor-pyrid-2-yl, 4-Chlor-pyrid-2-yl, 5-Chlor-pyrid-2-yl, 6-Chlor-pyrid-2-yl, 3-Trifluormethyl-pyrid-2-yl, 4-Trifluormethyl-pyrid-2-yl, 5-Trifluormethyl-pyrid-2-yl, 6-Trifluormethyl-pyrid-2-yl, 3-Methoxy-pyrid-2-yl, 4-Methoxypyrid-2-yl, 5-Methoxy-pyrid-2-yl, 6-Methoxy-pyrid-2-yl, 4-Methyl-thiazol-2-yl, 5-Methylthiazol-2-yl, 4-Trifluormethyl-thiazol-2-yl, 5-Trifluormethyl-thiazol-2-yl, 4-Chlor-thiazol-2-yl, 5-Chlor-thiazol-2-yl, 4-Brom-thiazol-2-yl, 5-Brom-thiazol-2-yl, 4-Fluor-thiazol-2-yl, 5-Fluor-thiazol-2-yl, 4-Cyano-thiazol-2-yl, 5-Cyano-thiazol-2-yl, 4-Methoxy-thiazol-2-yl, 5-Methoxy-thiazol-2-yl, 4-Methyl-oxazol-2-yl, 5-Methyl-oxazol-2-yl, 4-Trifluormethyl-oxazol-2-yl, 5-Trifluormethyl-oxazol-2-yl, 4-Chlor-oxazol-2-yl, 5-Chloroxazol-2-yl, 4-Brom-oxazol-2-yl, 5-Brom-oxazol-2-yl, 4-Fluor-oxazol-2-yl, 5-Fluoroxazol-2-yl, 4-Cyano-oxazol-2-yl, 5-Cyano-oxazol-2-yl, 4-Methoxy-oxazol-2-yl, 5-Methoxy-oxazol-2-yl, 2-Methyl-(1,2,4)-thiadiazol-5-yl, 2-Trifluormethyl-(1,2,4)-thiadiazol-5-yl, 2-Chlor-(1,2,4)-thiadiazol-5-yl, 2-Fluor-(1,2,4)-thiadiazol-5-yl, 2-Methoxy-(1,2,4)-thiadiazol-5-yl, 2-Cyano-(1,2,4)-thiadiazol-5-yl, 2-Methyl-(1,2,4)-oxadiazol-5-yl, 2-Trifluormethyl-(1,2,4)-oxadiazol-5-yl, 2-Chlor-(1,2,4)-oxadiazol-5-yl, 2-Fluor-(1,2,4)-oxadiazol-5-yl, 2-Methoxy-(1,2,4)-oxadiazol-5-yl und 2-Cyano-(1,2,4)-oxadiazol-5-yl steht;
und jeweils die übrigen Reste X, n, R¹, R², R³ und R⁵ bis R²⁵ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

Weiterhin bevorzugt sind substituierte 1-Propiolyl-piperazine der vorstehend angegebenen allgemeinen Formel I in denen n gleich 0, 1, 2, 3 oder 4 ist, besonders bevorzugt gleich 0, 1 oder 2 ist, ganz besonders bevorzugt gleich 0 ist,
und jeweils die übrigen Reste X, R¹, R², R³ R⁴ und R⁵ bis R²⁵ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

Der Fachmann versteht, dass sich für n gleich 0 die allgemeine Formel Ia ergibt:

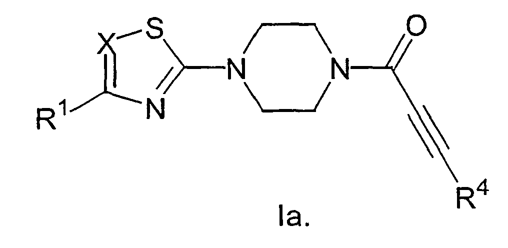

Ferner sind substituierte 1-Propiolyl-piperazine der vorstehend angegebenen allgemeinen Formel I bevorzugt, in denen R⁵ bis R²⁵, unabhängig voneinander,
für einen linearen oder verzweigten, unsubstituierten oder wenigstens einfach substituierten C₁₋₈-Alkyl-Rest, für einen linearen oder verzweigten, unsubstituierten oder wenigstens einfach substituierten C₂₋₈-Alkenyl-Rest, für einen linearen oder verzweigten, unsubstituierten oder wenigstens einfach substituierten C₂₋₈-Alkinyl-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden sein kann, stehen,
besonders bevorzugt für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl, oder für einen Rest ausgewählt aus der Gruppe bestehend aus Cyclopropyl-, Cyclobutyl-, Cyclopentyl-, Cyclohexyl-, Pyrrolidinyl-, Tetrahydrofuranyl-, Tetrahydrothiophenyl- Piperidinyl-, Tetrahydropyranyl-, Piperazinyl-, Morpholinyl-, Thiomorpholinyl-, Dioxolanyl-, Azepanyl-, Diazepanyl-, lmidazolidinyl-, Phenyl-, Naphthyl-, Anthracenyl-, Pyrrolyl-, Indolyl-, Furanyl-, Benzo[b]furanyl-, Thiophenyl-, Benzo[b]thiophenyl-, Pyrazolyl-, Imidazolyl-, Thiazolyl-, Dithiazolyl-, Thiadiazolyl-, Triazolyl-, Oxazolyl-, Oxadiazolyl-, Isoxazolyl-, Pyridinyl-, Pyridazinyl-, Pyrimidinyl-, Pyrazinyl-, Pyranyl-, Indazolyl-, Purinyl-, Indolizinyl-, Chinolinyl-, Isochinolinyl- und Chinazolinyl-, wobei der jeweilige zyklische Rest über eine, ggf. 1 oder 2 Heteroatome ausgewählt aus der Gruppe bestehend aus Sauerstoff, Schwefel und Stickstoff als Kettenglied(er) aufweisende C₁₋₃-Alkylen-, C₂₋₃-Alkenylen- oder C₂₋₃-Alkinylen-Gruppe gebunden sein kann und/oder unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅, -O-C₃H₇, -NH₂, -N(CH₃)₂, -N(C₂H₅)₂, -NH-CH₃, -NH-C₂H₅, -NO₂, -CF₃, -O-CF₃, -S-CF₃, -SH, -NH-S(=O)₂-CH₃, -C(=O)-OH, -C(=O)-CH₃,-C(=O)-C₂H₅, -C(=O)-N(CH₃)₂, -C(=O)-NH-CH₃, -NH-C(=O)-CH₃, -NH-C(=O)-C₂H₅,-C(=O)-O-CH₃ und -C(=O)-O-C₂H₅ substituiert ist, steht;
und jeweils die übrigen Reste X, n, R¹, R², R³ und R⁴ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

Ferner sind substituierte 1-Propiolyl-piperazine der vorstehend angegebenen allgemeinen Formel I bevorzugt, in denen X für C-R² steht;
und jeweils die übrigen Reste n und R¹ bis R²⁵ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

Bevorzugt sind ferner substituierte 1-Propiolyl-piperazine der vorstehend angegebenen allgemeinen Formel I, in denen der Rest R¹ für einen Wasserstoff-Rest, für einen Halogen-Rest ausgewählt aus der Gruppe bestehend aus F, Cl, Br und I, für eine Nitro-Gruppe, für eine CF₃-Gruppe, für eine Cyano-Gruppe, für eine Amino-Gruppe, für eine Hydroxy-Gruppe, für eine Thiol-Gruppe, für eine Carboxy-Gruppe, für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine-C(=O)-NH₂-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für einen linearen oder verzweigten C₁₋₈-Alkyl-Rest, für einen linearen oder verzweigten C₂₋₈-Alkenyl-Rest oder für einen linearen oder verzweigten C₂₋₈-Alkinyl-Rest, für einen unsubstituierten oder wenigstens einfach substituierten, ungesättigten oder gesättigten, ggf. 1, 2 oder 3 Heteroatome unabhängig voneinander ausgewählt aus der Gruppe bestehend aus Sauerstoff, Schwefel- und Stickstoff als Ringglied(er) 3-, 4-, 5-, 6- oder 7-gliedrigen cycloaliphatischen Rest, der über eine lineare oder verzweigte, unsubstituierte oder wenigstens einfach substituierte C₁₋₅-Alkylen-, C₂₋₅-Alkenylen- oder C₂₋₅-Alkinylen-Gruppe gebunden sein kann oder für einen unsubstituierten oder wenigstens einfach substituierten 5- bis 14- gliedrigen Aryl- oder Heteroaryl-Rest, der über eine lineare oder verzweigte, unsubstituierte oder wenigstens einfach substituierte C₁₋₅-Alkylen-, C₂₋₅-Alkenylen- oder C₂₋₅-Alkinylen-Gruppe gebunden sein kann, steht,
bevorzugt für einen Wasserstoff-Rest, für einen Halogen-Rest ausgewählt aus der Gruppe bestehend aus F, Cl und Br, für eine CF₃-Gruppe, eine Nitro-Gruppe, für eine Cyano-Gruppe, für eine Amino-Gruppe, für eine Hydroxy-Gruppe, für eine Thiol-Gruppe, für eine Carboxy-Gruppe, für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -O-R¹⁷-Gruppe, für eine -S-R¹⁸-Gruppe, für einen linearen oder verzweigten C₁₋₄-Alkyl-Rest, für einen linearen oder verzweigten C₂₋₄-Alkenyl-Rest, für einen linearen oder verzweigten C₂₋₄-Alkinyl-Rest, oder für einen Rest ausgewählt aus der Gruppe bestehend aus Cyclopropyl-, Cyclobutyl-, Cyclopentyl-, Cyclohexyl-, Pyrrolidinyl-, Tetrahydrofuranyl-, Tetrahydrothiophenyl- Piperidinyl-, Tetrahydropyranyl-, Piperazinyl-, Morpholinyl-, Thiomorpholinyl-, Dioxolanyl-, Azepanyl-, Diazepanyl-, Imidazolidinyl-, Phenyl-, Naphthyl-, Anthracenyl-, Pyrrolyl-, Indolyl-, Furanyl-, Benzo[b]furanyl-, Thiophenyl-, Benzo[b]thiophenyl-, Pyrazolyl-, Imidazolyl-, Thiazolyl-, Dithiazolyl-, Thiadiazolyl-, Triazolyl-, Oxazolyl-, Oxadiazolyl-, Isoxazolyl-, Pyridinyl-, Pyridazinyl-, Pyrimidinyl-, Pyrazinyl-, Pyranyl-, Indazolyl-, Purinyl-, Indolizinyl-, Chinolinyl-, Isochinolinyl- und Chinazolinyl-Rest, der jeweils über eine C₁₋₃-Alkylen-, C₂₋₃-Alkenylen- oder C₂₋₃-Alkinylen-Gruppe gebunden sein kann und/oder unsubstituiert oder mit 1, 2 oder 3 Substituenten, unabhängig voneinander ausgewählt aus der Gruppe bestehend aus F, Cl, Br, I, -CN, Methyl, Ethyl, n-Propyl, iso-Propyl, n-Butyl, sec-Butyl, iso-Butyl, tert-Butyl, -OH, -O-CH₃, -O-C₂H₅, -O-C₃H₇,-NH₂, -N(CH₃)₂, -N(C₂H₅)₂, -NH-CH₃, -NH-C₂H₅, -NO₂, -CF₃, -O-CF₃, -S-CF₃, -SH,-NH-S(=O)₂-CH₃, -C(=O)-OH, -C(=O)-CH₃, -C(=O)-C₂H₅, -C(=O)-N(CH₃)₂, -C(=O)-NH-CH₃, -NH-C(=O)-CH₃, -NH-C(=O)-C₂H₅, -C(=O)-O-CH₃ und -C(=O)-O-C₂H₅ substituiert ist, steht,
besonders bevorzugt für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Monomethylamino-Gruppe, für eine Monoethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für einen Alkyl-Rest ausgewählt aus der Gruppe bestehend aus Methyl, Ethyl, n-Propyl, iso-Propyl, n-Butyl, sec-Butyl, iso-Butyl und tert-Butyl, für einen Alkenyl-Rest ausgewählt aus der Gruppe bestehend aus Vinyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen unsubstituierten Rest ausgewählt aus der Gruppe bestehend aus (1,3)-Dioxolan-2-yl, Phenyl, Benzyl, Phenethyl, 2-Pyridyl, 3-Pyridyl, 4-Pyridyl, 2-Thienyl, 3-Thienyl, 2-Furyl und 3-Furyl steht,
und jeweils die übrigen Reste X, n, R², R³, R⁴ und R⁵ bis R²⁵ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte 1-Propiolyl-piperazine der vorstehend angegebenen allgemeinen Formel I, worin der Rest R² für einen Wasserstoff-Rest, für einen Halogen-Rest ausgewählt aus der Gruppe bestehend aus F, Cl, Br und I, für eine Nitro-Gruppe, für eine CF₃-Gruppe, für eine Cyano-Gruppe, für eine Amino-Gruppe, für eine Hydroxy-Gruppe, für eine Thiol-Gruppe, für eine Carboxy-Gruppe, für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -C(=O)-NH₂-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für einen linearen oder verzweigten C₁₋₈-Alkyl-Rest, für einen linearen oder verzweigten C₂₋₈-Alkenyl-Rest oder für einen linearen oder verzweigten C₂₋₈-Alkinyl-Rest, für einen unsubstituierten oder wenigstens einfach substituierten, ungesättigten oder gesättigten, ggf. 1, 2 oder 3 Heteroatome unabhängig voneinander ausgewählt aus der Gruppe bestehend aus Sauerstoff, Schwefel- und Stickstoff als Ringglied(er) 3-, 4-, 5-, 6- oder 7-gliedrigen cycloaliphatischen Rest, der über eine lineare oder verzweigte, unsubstituierte oder wenigstens einfach substituierte C₁₋₅-Alkylen-, C₂₋₅-Alkenylen- oder C₂₋₅-Alkinylen-Gruppe gebunden sein kann oder für einen unsubstituierten oder wenigstens einfach substituierten 5- bis 14- gliedrigen Aryl- oder Heteroaryl-Rest, der über eine lineare oder verzweigte, unsubstituierte oder wenigstens einfach substituierte C₁₋₅-Alkylen-, C₂₋₅-Alkenylen- oder C₂₋₅-Alkinylen-Gruppe gebunden sein kann, steht,
bevorzugt für einen Wasserstoff-Rest, für einen Halogen-Rest ausgewählt aus der Gruppe bestehend aus F, Cl und Br, für eine CF₃-Gruppe, eine Nitro-Gruppe, für eine Cyano-Gruppe, für eine Amino-Gruppe, für eine Hydroxy-Gruppe, für eine Thiol-Gruppe, für eine Carboxy-Gruppe, für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -O-R¹⁷-Gruppe, für eine -S-R¹⁸-Gruppe, für einen linearen oder verzweigten C₁₋₄-Alkyl-Rest, für einen linearen oder verzweigten C₂₋₄-Alkenyl-Rest, für einen linearen oder verzweigten C₂₋₄-Alkinyl-Rest, oder für einen Rest ausgewählt aus der Gruppe bestehend aus Cyclopropyl-, Cyclobutyl-, Cyclopentyl-, Cyclohexyl-, Pyrrolidinyl-, Tetrahydrofuranyl-, Tetrahydrothiophenyl- Piperidinyl-, Tetrahydropyranyl-, Piperazinyl-, Morpholinyl-, Thiomorpholinyl-, Dioxolanyl-, Azepanyl-, Diazepanyl-, Imidazolidinyl-, Phenyl-, Naphthyl-, Anthracenyl-, Pyrrolyl-, Indolyl-, Furanyl-, Benzo[b]furanyl-, Thiophenyl-, Benzo[b]thiophenyl-, Pyrazolyl-, Imidazolyl-, Thiazolyl-, Dithiazolyl-, Thiadiazolyl-, Triazolyl-, Oxazolyl-, Oxadiazolyl-, Isoxazolyl-, Pyridinyl-, Pyridazinyl-, Pyrimidinyl-, Pyrazinyl-, Pyranyl-, Indazolyl-, Purinyl-, Indolizinyl-, Chinolinyl-, Isochinolinyl- und Chinazolinyl-Rest, der jeweils über eine C₁₋₃-Alkylen-, C₂₋₃-Alkenylen- oder C₂₋₃-Alkinylen-Gruppe gebunden sein kann und/oder unsubstituiert oder mit 1, 2 oder 3 Substituenten, unabhängig voneinander ausgewählt aus der Gruppe bestehend aus F, Cl, Br, I, -CN, Methyl, Ethyl, n-Propyl, iso-Propyl, n-Butyl, sec-Butyl, iso-Butyl, tert-Butyl, -OH, -O-CH₃, -O-C₂H₅, -O-C₃H₇,-NH₂, -N(CH₃)₂, -N(C₂H₅)₂, -NH-CH₃, -NH-C₂H₅, -NO₂, -CF₃, -O-CF₃, -S-CF₃, -SH,-NH-S(=O)₂-CH₃, -C(=O)-OH, -C(=O)-CH₃, -C(=O)-C₂H₅, -C(=O)-N(CH₃)₂, -C(=O)-NH-CH₃, -NH-C(=O)-CH₃, -NH-C(=O)-C₂H₅ -C(=O)-O-CH₃ und -C(=O)-O-C₂H₅ substituiert ist, steht,
besonders bevorzugt für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Monomethylamino-Gruppe, für eine Monoethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für einen Alkyl-Rest ausgewählt aus der Gruppe bestehend aus Methyl, Ethyl, n-Propyl, iso-Propyl, n-Butyl, sec-Butyl, iso-Butyl und tert-Butyl, für einen Alkenyl-Rest ausgewählt aus der Gruppe bestehend aus Vinyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen unsubstituierten Rest ausgewählt aus der Gruppe bestehend aus (1,3)-Dioxolan-2-yl, Phenyl, Benzyl, Phenethyl, 2-Pyridyl, 3-Pyridyl, 4-Pyridyl, 2-Thienyl, 3-Thienyl, 2-Furyl und 3-Furyl steht,
und jeweils die übrigen Reste X, n, R¹, R³, R⁴ und R⁵ bis R²⁵ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 1-Propiolyl-piperazine der vorstehend angegebenen allgemeinen Formel I, in denen R³ für einen Halogen-Rest ausgewählt aus der Gruppe bestehend aus F, Cl und Br, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Oxo-Gruppe (=O) oder für einen linearen oder verzweigten C₁₋₄-Alkyl-Rest steht,
vorzugsweise für eine Oxo-Gruppe (=O) oder für einen Alkyl-Rest ausgewählt aus der Gruppe bestehend aus Methyl, Ethyl, n-Propyl, iso-Propyl, n-Butyl, sec-Butyl, iso-Butyl und tert-Butyl steht,
und jeweils die übrigen Reste X, n, R¹, R², R⁴ und R⁵ bis R²⁵ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte 1-Propiolyl-piperazine der vorstehend angegebenen allgemeinen Formel I, in denen R⁴ für einen unsubstituierten oder wenigstens einfach substituierten, ungesättigten oder gesättigten 4-, 5-, 6- oder 7-gliedrigen cycloaliphatischen Rest, der mit einem ungesättigten, gesättigten oder aromatischen, unsubstituierten oder wenigstens einfach substituierten, mono- oder bizyklischen Ringsystem kondensiert sein kann, wobei jeder der Ringe 1, 2 oder 3 Heteroatome aufweisen kann, die unabhängig voneinander ausgewählt werden können aus der Gruppe bestehend aus Sauerstoff, Stickstoff und Schwefel und wobei die Ringe des mono- oder bizyklischen Ringsystems jeweils 4-, 5- oder 6-gliedrig sind, oder für einen unsubstituierten oder wenigstens einfach substituierten 5- oder 6-gliedrigen Aryl- oder Heteroaryl-Rest, der mit einem ungesättigten oder aromatischen, unsubstituierten oder wenigstens einfach substituierten, mono- oder bizyklischen Ringsystem kondensiert sein kann, wobei der Heteroaryl-Rest und ggf. der Ring des monozyklischen Ringsystems jeweils 1, 2 oder 3 Heteroatome aufweisen, die unabhängig voneinander ausgewählt werden können aus der Gruppe bestehend aus Sauerstoff, Stickstoff und Schwefel und wobei die Ringe des mono- oder bizyklischen Ringsystems jeweils 4-, 5- oder 6- gliedrig sind, steht,
bevorzugt für einen Rest ausgewählt aus der Gruppe bestehend aus Cyclopropyl, Cyclobutyl, Cyclopentyl, Cyclohexyl, Pyrrolidinyl, Tetrahydrofuranyl, Tetrahydrothiophenyl, Piperidinyl, Tetrahydropyranyl, Piperazinyl, Morpholinyl, Thiomorpholinyl, Dioxolanyl, Azepanyl, Diazepanyl, Imidazolidinyl, Phenyl, Naphthyl, Anthracenyl, Furanyl, Thiophenyl, Pyrazolyl, Pyrazinyl, Pyrimidinyl, Pyridinyl, Pyrrolyl, Oxazolyl, Isoxazolyl, Thiazolyl, Dithiazolyl, Oxadiazolyl, Triazolyl, Imidazolyl, Indolyl, Benzo[b]thiophenyl, Benzo[b]furanyl, Chinolinyl, Isochinolinyl und Chinazolyl steht, der mit 1, 2 oder 3 Substituenten, unabhängig voneinander ausgewählt aus der Gruppe bestehend aus F, Cl, Br, I, -CN, Methyl, Ethyl, n-Propyl, iso-Propyl, n-Butyl, sec-Butyl, iso-Butyl, tert-Butyl, -OH, -O-CH₃, -O-C₂H₅, -O-C₃H₇, -NH₂, -N(CH₃)₂,-N(C₂H₅)₂, -NH-CH₃, -NH-C₂H₅, -NO₂, -CF₃, -O-CF₃, -S-CF₃, -SH, -NH-S(=O)₂-CH₃,-C(=O)-OH, -C(=O)-CH₃, -C(=O)-C₂H₅, -C(=O)-N(CH₃)₂, -C(=O)-NH-CH₃, -NH-C(=O)-CH₃, -NH-C(=O)-C₂H₅, -C(=O)-O-CH₃ und -C(=O)-O-C₂H₅ substituiert sein kann, steht,
besonders bevorzugt für einen Rest ausgewählt aus der Gruppe bestehend aus Phenyl, Pyrid-2-yl, Pyrid-3-yl, Pyrid-4-yl, Pyrrol-2-yl, Pyrrol-3-yl, Thiophen-2-yl, Thiophen-3-yl, Furan-2-yl, Furan-3-yl, Imidazol-2-yl, Imidazol-4-yl, Thiazol-2-yl, Thiazol-4-yl, Thiazol-5-yl, Oxazol-2-yl, Oxazol-4-yl, Oxazol-5-yl, 1,2,4-Thiadiazol-5-yl, 1,2,4-Oxadiazol-5-yl, Napht-1-yl, Napht-2-yl, Anthracen-1-yl, Anthracen-2-yl, Anthracen-9-yl, Indol-2-yl, Indol-3-yl, Indol-4-yl, Indol-5-yl, Indol-6-yl, Indol-7-yl, Chinolin-3-yl, Chinolin-4-yl, Chinolin-2-yl, Isochinolin-1-yl, Isochinolin-3-yl, Isochinolin-4-yl, 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Hydroxy-phenyl, 3-Hydroxy-phenyl, 4-Hydroxy-phenyl, 2-Amino-phenyl, 3-Aminophenyl, 4-Amino-phenyl, 2-Acetyl-phenyl, 3-Acetyl-phenyl, 4-Acetyl-phenyl, 2,4-Dimethyl-phenyl, 2,4,6-Trimethylphenyl, 3,5-Dimethyl-phenyl, 2-Methoxy-phenyl, 3-Methoxy-phenyl, , 4-Methoxy-phenyl, 2-Chlor-phenyl, 3-Chlor-phenyl, 4-Chlor-phenyl, 2-Ethoxy-phenyl, 3-Ethoxy-phenyl, 4-Ethoxyphenyl, 2,4-Difluor-phenyl, 2-Trifluormethyl-phenyl, 3-Trifluormethyl-phenyl, 4-Trifluormethyl-phenyl, 2-Nitrophenyl, 3-Nitro-phenyl, 4-Nitro-phenyl, 3-Methyl-pyrid-2-yl, 4-Methyl-pyrid-2-yl, 5-Methyl-pyrid-2-yl, 6-Methyl-pyrid-2-yl, 2-Methyl-pyrid-3-yl, 4-Methyl-pyrid-3-yl, 5-Methyl-pyrid-3-yl, 6-Methyl-pyrid-3-yl, 2-Methyl-pyrid-4-yl, 3-Methyl-pyrid-4-yl, 3-Fluor-pyrid-2-yl, 4-Fluor-pyrid-2-yl, 5-Fluor-pyrid-2-yl, 6-Fluor-pyrid-2-yl, 3-Chlorpyrid-2-yl, 4-Chlor-pyrid-2-yl, 5-Chlor-pyrid-2-yl, 6-Chlor-pyrid-2-yl, 3-Trifluormethylpyrid-2-yl, 4-Trifluormethyl-pyrid-2-yl, 5-Trifluormethyl-pyrid-2-yl, 6-Trifluormethylpyrid-2-yl, 3-Methoxy-pyrid-2-yl, 4-Methoxy-pyrid-2-yl, 5-Methoxy-pyrid-2-yl, 6-Methoxy-pyrid-2-yl, 4-Methyl-thiazol-2-yl, 5-Methyl-thiazol-2-yl, 4-Trifluormethylthiazol-2-yl, 5-Trifluormethyl-thiazol-2-yl, 4-Chlor-thiazol-2-yl, 5-Chlor-thiazol-2-yl, 4-Brom-thiazol-2-yl, 5-Brom-thiazol-2-yl, 4-Fluor-thiazol-2-yl, 5-Fluor-thiazol-2-yl, 4-Cyano-thiazol-2-yl, 5-Cyano-thiazol-2-yl, 4-Methoxy-thiazol-2-yl, 5-Methoxy-thiazol-2-yl, 4-Methyl-oxazol-2-yl, 5-Methyl-oxazol-2-yl, 4-Trifluormethyl-oxazol-2-yl, 5-Trifluormethyl-oxazol-2-yl, 4-Chlor-oxazol-2-yl, 5-Chlor-oxazol-2-yl, 4-Brom-oxazol-2-yl, 5-Brom-oxazol-2-yl, 4-Fluor-oxazol-2-yl, 5-Fluor-oxazol-2-yl, 4-Cyano-oxazol-2-yl, 5-Cyano-oxazol-2-yl, 4-Methoxy-oxazol-2-yl, 5-Methoxy-oxazol-2-yl, 2-Methyl-(1,2,4)-thiadiazol-5-yl, 2-Trifluormethyl-(1,2,4)-thiadiazol-5-yl, 2-Chlor-(1,2,4)-thiadiazol-5-yl, 2-Fluor-(1,2,4)-thiadiazol-5-yl, 2-Methoxy-(1,2,4)-thiadiazol-5-yl, 2-Cyano-(1,2,4)-thiadiazol-5-yl, 2-Methyl-(1,2,4)-oxadiazol-5-yl, 2-Trifluormethyl-(1,2,4)-oxadiazol-5-yl, 2-Chlor-(1,2,4)-oxadiazol-5-yl, 2-Fluor-(1,2,4)-oxadiazol-5-yl, 2-Methoxy-(1,2,4)-oxadiazol-5-yl und 2-Cyano-(1,2,4)-oxadiazol-5-yl steht,
und jeweils die übrigen Reste X, n, R¹, R², R³ und R⁵ bis R²⁵ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte 1-Propiolyl-piperazine der vorstehend angegebenen allgemeinen Formel I in denen n gleich 0, 1 oder 2 ist, besonders bevorzugt gleich 0 ist,
und jeweils die übrigen Reste X, R¹, R², R³ R⁴ und R⁵ bis R²⁵ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ferner sind substituierte 1-Propiolyl-piperazine der vorstehend angegebenen allgemeinen Formel I bevorzugt, in denen R⁵ bis R²⁵, unabhängig voneinander, jeweils für einen linearen oder verzweigten, unsubstituierten oder wenigstens einfach substituierten C₁₋₈-Alkyl-Rest, für einen linearen oder verzweigten, unsubstituierten oder wenigstens einfach substituierten C₂₋₈-Alkenyl-Rest, für einen linearen oder verzweigten, unsubstituierten oder wenigstens einfach substituierten C₂₋₈-Alkinyl-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 Heteroatome unabhängig voneinander ausgewählt aus der Gruppe bestehend aus Sauerstoff, Schwefel- und Stickstoff als Kettenglied(er) aufweisende C₁₋₅-Alkylen-, C₂₋₅ -Alkenylen- oder C₂₋₅-Alkinylen-Gruppe gebunden sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 Heteroatome unabhängig voneinander ausgewählt aus der Gruppe bestehend aus Sauerstoff, Schwefel- und Stickstoff als Kettenglied(er) aufweisende C₁₋₅-Alkylen-, C₂₋₅-Alkenylen- oder C₂₋₅-Alkinylen-Gruppe gebunden sein kann, stehen,
bevorzugt für einen Alkyl-Rest ausgewählt aus der Gruppe bestehend aus Methyl, Ethyl, n-Propyl, iso-Propyl, n-Butyl, sec-Butyl, iso-Butyl und tert-Butyl, für einen Alkenyl-Rest ausgewählt aus der Gruppe bestehend aus Vinyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl, für einen Rest ausgewählt aus der Gruppe bestehend aus Cyclopropyl-, Cyclobutyl-, Cyclopentyl-, Cyclohexyl-, Pyrrolidinyl-, Tetrahydrofuranyl-, Tetrahydrothiophenyl- Piperidinyl-, Tetrahydropyranyl-, Piperazinyl-, Morpholinyl-, Thiomorpholinyl-, Dioxolanyl-, Azepanyl-, Diazepanyl-, Imidazolidinyl-, Phenyl-, Naphthyl-, Anthracenyl-, Pyrrolyl-, Indolyl-, Furanyl-, Benzo[b]furanyl-, Thiophenyl-, Benzo[b]thiophenyl-, Pyrazolyl-, Imidazolyl-, Thiazolyl-, Dithiazolyl-, Thiadiazolyl-, Triazolyl-, Oxazolyl-, Oxadiazolyl-, Isoxazolyl-, Pyridinyl-, Pyridazinyl-, Pyrimidinyl-, Pyrazinyl-, Pyranyl-, Indazolyl-, Purinyl-, Indolizinyl-, Chinolinyl-, Isochinolinyl- und Chinazolinyl-Rest, wobei der jeweilige zyklische Reste über eine, ggf. 1 oder 2 Heteroatome ausgewählt aus der Gruppe bestehend aus Sauerstoff, Schwefel und Stickstoff als Kettengleid(er) aufweisende C₁₋₃-Alkylen-, C₂₋₃-Alkenylen- oder C₂₋₃-Alkinylen-Gruppe gebunden sein kann und/oder unsubstituiert oder mit 1, 2 oder 3 Substituenten, unabhängig voneinander ausgewählt aus der Gruppe bestehend aus F, Cl, Br, I, -CN, Methyl, Ethyl, n-Propyl, iso-Propyl, n-Butyl, sec-Butyl, iso-Butyl, tert-Butyl, -OH, -O-CH₃, -O-C₂H₅, -O-C₃H₇, -NH₂, -N(CH₃)₂, -N(C₂H₅)₂, -NH-CH₃, -NH-C₂H₅, -NO₂, -CF₃, -O-CF₃, -S-CF₃, -SH, -NH-S(=O)₂-CH₃, -C(=O)-OH, -C(=O)-CH₃,-C(=O)-C₂H₅, -C(=O)-N(CH₃)₂, -C(=O)-NH-CH₃, -NH-C(=O)-CH₃, -NH-C(=O)-C₂H₅,-C(=O)-O-CH₃ und -C(=O)-O-C₂H₅ substituiert ist, stehen,
und jeweils die übrigen Reste X, n, R¹, R², R³ und R⁴ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind substituierte 1-Propiolyl-piperazine der allgemeinen Formel I,
worin
n = 0, 1, 2, 3 oder 4 ist,
X für N oder C-R² steht,
R¹ für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht,
R² für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht,
R³ für eine Oxo-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl oder für einen Phenyl-Rest, der unsubstituiert oder mit 1, 2, 3, 4 oder 5 Substituenten unabhängig voneinander ausgewählt aus der Gruppe bestehend aus F, Cl, Br, I,-CN, Methyl, Ethyl, n-Propyl, Isopropyl, n-Butyl, sec-Butyl, Isobutyl, tert-Butyl, -OH,-O-CH₃, -O-C₂H₅ und -O-C₃H₇ substituiert ist, steht,
R⁴ für einen Wasserstoff-Rest, für eine -C(=O)-NH₂-Gruppe, für eine -C(=O)-NH-R¹⁴-Gruppe, für eine -C(=O)-NR¹⁵R¹⁶-Gruppe, für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, n-Heptyl und n-Octyl, oder für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Pyrrolidinyl, Tetrahydrofuranyl, Tetrahydrothiophenyl, Piperidinyl, Tetrahydropyranyl, Piperazinyl, Morpholinyl, Thiomorpholinyl, Dioxolanyl, Azepanyl, Diazepanyl, Imidazolidinyl, Phenyl, Naphthyl, Anthracenyl, Furanyl, Thiophenyl, Pyrazolyl, Pyrazinyl, Pyrimidinyl, Pyridinyl, Pyrrolyl, Oxazolyl, Isoxazolyl, Thiazolyl, Dithiazolyl, Oxadiazolyl, Triazolyl, Imidazolyl, Indolyl, Benzo[b]thiophenyl, Benzo[b]furanyl, Chinolinyl, Isochinolinyl und Chinazolyl steht, der jeweils ggf. mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, Ethenyl, Allyl, Ethinyl, Propinyl, -C≡C-Si(CH₃)₃, -C≡C-Si(C₂H₅)₃, -CH₂-O-CH₃,-CH₂-O-C₂H₅, -OH, -O-CH₃, -O-C₂H₅, -O-C₃H₇, -S-CH₃, -S-C₂H₅, -S(=O)-CH₃,-S(=O)₂-CH₃, -S(=O)-C₂H₅, -S(=O)₂-C₂H₅, -NH₂, -N(CH₃)₂, -N(C₂H₅)₂, -NH-CH₃, -NH-C₂H₅, -NO₂, -CF₃, -CH₂F, -CHF₂, -O-CF₃, -S-CF₃, -SH, -NH-S(=O)₂-CH₃, -C(=O)-OH, -C(=O)-H; -C(=O)-CH₃, -C(=O)-C₂H₅, -C(=O)-NH₂, -C(=O)-N(CH₃)₂, -C(=O)-NH-CH₃, -NH-C(=O)-CH₃, -NH-C(=O)-C₂H₅, -C(=O)-O-CH₃, -C(=O)-O-C₂H₅, -C(=O)-O-C(CH₃)₃ und Phenyl substituiert sein kann, steht;
und
R¹⁴, R¹⁵, R¹⁶, R¹⁷, R¹⁸, R¹⁹ und R²⁰, unabhängig voneinander, jeweils für einen Alkyl-Rest ausgewählt aus der Gruppe bestehend aus Methyl, Ethyl, n-Propyl, Isopropyl, n-Butyl, sec-Butyl, Isobutyl und tert-Butyl stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

Besonders bevorzugt sind substituierte 1-Propiolyl-piperazine der allgemeinen Formel I, 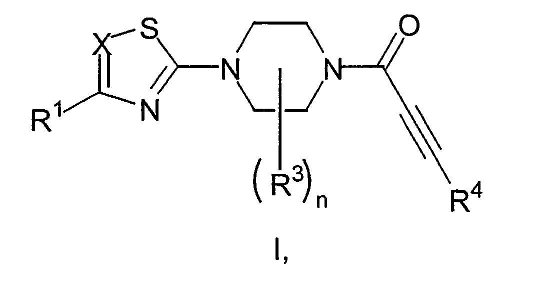 worin
n = 0 ist,
X für N oder C-R² steht,
R¹ für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für einen Alkyl-Rest ausgewählt aus der Gruppe bestehend aus Methyl, Ethyl, n-Propyl, iso-Propyl, n-Butyl, sec-Butyl, iso-Butyl und tert-Butyl, für einen Alkenyl-Rest ausgewählt aus der Gruppe bestehend aus Vinyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder ür einen unsubstituierten Rest ausgewählt aus der Gruppe bestehend aus (1,3)-Dioxolan-2-yl, Phenyl, Benzyl, Phenethyl, 2-Pyridyl, 3-Pyridyl, 4-Pyridyl, 2-Thienyl, 3-Thienyl, 2-Furyl und 3-Furyl steht,
R² für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für einen Alkyl-Rest ausgewählt aus der Gruppe bestehend aus Methyl, Ethyl, n-Propyl, iso-Propyl, n-Butyl, sec-Butyl, iso-Butyl und tert-Butyl, für einen Alkenyl-Rest ausgewählt aus der Gruppe bestehend aus Vinyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen unsubstituierten Rest ausgewählt aus der Gruppe bestehend aus (1,3)-Dioxolan-2-yl, Phenyl, Benzyl, Phenethyl, 2-Pyridyl, 3-Pyridyl, 4-Pyridyl, 2-Thienyl, 3-Thienyl, 2-Furyl und 3-Furyl steht,
R⁴ für einen Rest ausgewählt aus der Gruppe bestehend aus Phenyl, Pyrid-2-yl, Pyrid-3-yl, Pyrid-4-yl, Pyrrol-2-yl, Pyrrol-3-yl, Thiophen-2-yl, Thiophen-3-yl, Furan-2-yl, Furan-3-yl, Imidazol-2-yl, Imidazol-4-yl, Thiazol-2-yl, Thiazol-4-yl, Thiazol-5-yl, Oxazol-2-yl, Oxazol-4-yl, Oxazol-5-yl, 1,2,4-Thiadiazol-5-yl, 1,2,4-Oxadiazol-5-yl, Napht-1-yl, Napht-2-yl, Anthracen-1-yl, Anthracen-2-yl, Anthracen-9-yl, Indol-2-yl, Indol-3-yl, Indol-4-yl, Indol-5-yl, Indol-6-yl, Indol-7-yl, Chinolin-3-yl, Chinolin-4-yl, Chinolin-2-yl, Isochinolin-1-yl, Isochinolin-3-yl, Isochinolin-4-yl, 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Hydroxy-phenyl, 3-Hydroxyphenyl, 4-Hydroxy-phenyl, 2-Amino-phenyl, 3-Amino-phenyl, 4-Amino-phenyl, 2-Acetyl-phenyl, 3-Acetyl-phenyl, 4-Acetyl-phenyl, 2,4-Dimethyl-phenyl, 2,4,6-Trimethylphenyl, 3,5-Dimethyl-phenyl, 2-Methoxy-phenyl, 3-Methoxy-phenyl, 4-Methoxy-phenyl, 2-Chlor-phenyl, 3-Chlor-phenyl, 4-Chlor-phenyl, 2-Ethoxy-phenyl, 3-Ethoxy-phenyl, 4-Ethoxyphenyl, 2,4-Difluor-phenyl, 2-Trifluormethyl-phenyl, 3-Trifluormethyl-phenyl, 4-Trifluormethyl-phenyl, 2-Nitro-phenyl, 3-Nitro-phenyl, 4-Nitrophenyl, 3-Methyl-pyrid-2-yl, 4-Methyl-pyrid-2-yl, 5-Methyl-pyrid-2-yl, 6-Methyl-pyrid-2-yl, 2-Methyl-pyrid-3-yl, 4-Methyl-pyrid-3-yl, 5-Methyl-pyrid-3-yl, 6-Methyl-pyrid-3-yl, 2-Methyl-pyrid-4-yl, 3-Methyl-pyrid-4-yl, 3-Fluor-pyrid-2-yl, 4-Fluor-pyrid-2-yl, 5-Fluorpyrid-2-yl, 6-Fluor-pyrid-2-yl, 3-Chlor-pyrid-2-yl, 4-Chlor-pyrid-2-yl, 5-Chlor-pyrid-2-yl, 6-Chlor-pyrid-2-yl, 3-Trifluormethyl-pyrid-2-yl, 4-Trifluormethyl-pyrid-2-yl, 5-Trifluormethyl-pyrid-2-yl, 6-Trifluormethyl-pyrid-2-yl, 3-Methoxy-pyrid-2-yl, 4-Methoxypyrid-2-yl, 5-Methoxy-pyrid-2-yl, 6-Methoxy-pyrid-2-yl, 4-Methyl-thiazol-2-yl, 5-Methylthiazol-2-yl, 4-Trifluormethyl-thiazol-2-yl, 5-Trifluormethyl-thiazol-2-yl, 4-Chlor-thiazol-2-yl, 5-Chlor-thiazol-2-yl, 4-Brom-thiazol-2-yl, 5-Brom-thiazol-2-yl, 4-Fluor-thiazol-2-yl, 5-Fluor-thiazol-2-yl, 4-Cyano-thiazol-2-yl, 5-Cyano-thiazol-2-yl, 4-Methoxy-thiazol-2-yl, 5-Methoxy-thiazol-2-yl, 4-Methyl-oxazol-2-yl, 5-Methyl-oxazol-2-yl, 4-Trifluormethyl-oxazol-2-yl, 5-Trifluormethyl-oxazol-2-yl, 4-Chlor-oxazol-2-yl, 5-Chloroxazol-2-yl, 4-Brom-oxazol-2-yl, 5-Brom-oxazol-2-yl, 4-Fluor-oxazol-2-yl, 5-Fluoroxazol-2-yl, 4-Cyano-oxazol-2-yl, 5-Cyano-oxazol-2-yl, , 4-Methoxy-oxazol-2-yl, 5-Methoxy-oxazol-2-yl, 2-Methyl-(1,2,4)-thiadiazol-5-yl, 2-Trifluormethyl-(1,2,4)-thiadiazol-5-yl, 2-Chlor-(1,2,4)-thiadiazol-5-yl, 2-Fluor-(1,2,4)-thiadiazol-5-yl, 2-Methoxy-(1,2,4)-thiadiazol-5-yl, 2-Cyano-(1,2,4)-thiadiazol-5-yl, 2-Methyl-(1,2,4)-oxadiazol-5-yl, 2-Trifluormethyl-(1,2,4)-oxadiazol-5-yl, 2-Chlor-(1,2,4)-oxadiazol-5-yl, 2-Fluor-(1,2,4)-oxadiazol-5-yl, 2-Methoxy-(1,2,4)-oxadiazol-5-yl und 2-Cyano-(1,2,4)-oxadiazol-5-yl steht,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls besonders bevorzugt sind substituierte 1-Propiolyl-piperazine der allgemeinen Formel I, 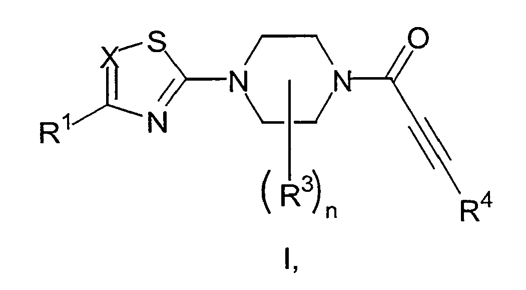 worin
n = 0, 1, 2, 3 oder 4 ist,
X für N oder C-R² steht,
R¹ für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht,
R² für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht,
R³ für eine Oxo-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl oder für einen Phenyl-Rest, der unsubstituiert oder mit 1, 2, 3, 4 oder 5 Substituenten unabhängig voneinander ausgewählt aus der Gruppe bestehend aus F, Cl, Br, I,-CN, Methyl, Ethyl, n-Propyl, Isopropyl, n-Butyl, sec-Butyl, Isobutyl, tert-Butyl, -OH,-O-CH₃, -O-C₂H₅ und -O-C₃H₇ substituiert ist, steht,
R⁴ für einen Wasserstoff-Rest, für eine -C(=O)-NH₂-Gruppe, für eine -C(=O)-NH-R¹⁴-Gruppe, für eine -C(=O)-NR¹⁵R¹⁶-Gruppe, für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, neo-Pentyl, n-Hexyl, n-Heptyl und n-Octyl, oder für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Phenyl, Pyrid-2-yl, Pyrid-3-yl, Pyrid-4-yl, Pyrrol-2-yl, Pyrrol-3-yl, Thiophen-2-yl, Thiophen-3-yl, 4-Methyl-thiophen-2-yl, 3-Methyl-thiophen-2-yl, 5-Methyl-thiophen-2-yl, Furan-2-yl, Furan-3-yl, Imidazol-2-yl, Imidazol-4-yl, Thiazol-2-yl, Thiazol-4-yl, Thiazol-5-yl, Oxazol-2-yl, Oxazol-4-yl, Oxazol-5-yl, (1,2,4)-Thiadiazol-5-yl, (1,2,4)-Oxadiazol-5-yl, Napht-1-yl, Napht-2-yl, Anthracen-1-yl, Anthracen-2-yl, Anthracen-9-yl, Indol-2-yl, Indol-3-yl, Indol-4-yl, Indol-5-yl, Indol-6-yl, Indol-7-yl, 1-Methyl-indol-2-yl, 1-Methyl-indol-3-yl, 1-Methyl-indol-4-yl, 1-Methyl-indol-5-yl, 1-Methyl-indol-6-yl, 1-Methyl-indol-7-yl, Chinolin-3-yl, Chinolin-4-yl, Chinolin-2-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, Isochinolin-8-yl, 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Hydroxy-phenyl, 3-Hydroxyphenyl, 4-Hydroxy-phenyl, 2-Amino-phenyl, 3-Amino-phenyl, 4-Amino-phenyl, 2-Dimethylamino-phenyl, 3-Dimethylamino-phenyl, 4-Dimethylamino-phenyl, 2-Methylamino-phenyl, 3-Methylamino-phenyl, 4-Methylamino-phenyl, 2-Acetyl-phenyl, 3-Acetyl-phenyl, 4-Acetyl-phenyl, 2-Methylsulfinyl-phenyl, 3-Methylsulfinyl-phenyl, 4-Methylsulfinyl-phenyl, 2-Methylsulfonyl-phenyl, 3-Methylsulfonyl-phenyl, 4-Methylsulfonyl-phenyl, 2-Methoxy-phenyl, 3-Methoxy-phenyl, 4-Methoxy-phenyl, 2-Chlor-phenyl, 3-Chlor-phenyl, 4-Chlor-phenyl, 2-Ethoxy-phenyl, 3-Ethoxy-phenyl, 4-Ethoxyphenyl, 2-Trifluormethyl-phenyl, 3-Trifluormethyl-phenyl, 4-Trifluormethyl-phenyl, 2-Difluormethyl-phenyl, 3-Difluormethyl-phenyl, 4-Difluormethyl-phenyl, 2-Fluormethyl-phenyl, 3-Fluormethyl-phenyl, 4-Fluormethyl-phenyl, 2-Nitro-phenyl, 3-Nitro-phenyl, 4-Nitro-phenyl, 2-Ethyl-phenyl, 3-Ethyl-phenyl, 4-Ethyl-phenyl, 2-Propylphenyl, 3-Propyl-phenyl, 4-Propyl-phenyl, 2-Isopropyl-phenyl, 3-Isopropyl-phenyl, 4-Isopropyl-phenyl, 2-tert-Butyl-phenyl, 3-tert-Butyl-phenyl, 4-tert-Butyl-phenyl, 2-Carboxyphenyl, 3-Carboxy-phenyl, 4-Carboxyphenyl, 2-Ethenyl-phenyl, 3-Ethenylphenyl, 4-Ethenyl-phenyl, 2-Ethinyl-phenyl, 3-Ethinyl-phenyl, 4-Ethinyl-phenyl, 2-Allylphenyl, 3-Allyl-phenyl, 4-Allyl-phenyl, 2-Trimethylsilanylethinyl-phenyl, 3-Trimethylsilanylethinyl-phenyl, 4-Trimethylsilanylethinyl-phenyl, 2-Formyl-phenyl, 3-Formyl-phenyl, 4-Formyl-phenyl, 2-Acetamino-phenyl, 3-Acetamino-phenyl, 4-Acetamino-phenyl, 2-Dimethylaminocarbonyl-phenyl, 3-Dimethylaminocarbonylphenyl, 4-Dimethylaminocarbonyl-phenyl, 2-Methoxymethyl-phenyl, 3-Methoxymethyl-phenyl, 4-Methoxymethyl-phenyl, 2-Ethoxymethyl-phenyl, 3-Ethoxymethyl-phenyl, 4-Ethoxymethyl-phenyl, 2-Aminocarbonyl-phenyl, 3-Aminocarbonyl-phenyl, 4-Aminocarbonyl-phenyl, 2-Methylaminocarbonyl-phenyl, 3-Methylaminocarbonyl-phenyl, 4-Methylaminocarbonyl-phenyl, 2-Carboxymethylesterphenyl, 3-Carboxymethylester-phenyl, 4-Carboxymethylester-phenyl, 2-Carboxyethylester-phenyl, 3-Carboxyethylester-phenyl, 4-Carboxyethylester-phenyl, 2-Carboxy-tert-butylester-phenyl, 3-Carboxy-tert-butylester-phenyl, 4-Carboxy-tert-butylester-phenyl, 2-Methylmercapto-phenyl, 3-Methylmercapto-phenyl, 4-Methylmercapto-phenyl, 2-Ethylmercapto-phenyl, 3-Ethylmercapto-phenyl, 4-Ethylmercaptophenyl, 2-Biphenyl, 3-Biphenyl, 4-Biphenyl, 2-Bromphenyl, 3-Bromphenyl, 4-Bromphenyl, 2-lod-phenyl, 3-lodphenyl, 4-lodphenyl, 2-Trifluormethoxy-phenyl, 3-Trilfuormethoxy-phenyl, 4-Trifluormethoxy-phenyl, 2-Fluor-3-trifluormethylphenyl, 2-Fluor-4-methyl-phenyl, (2,3)-Difluorphenyl, (2,3)-Dimethyl-phenyl, (2,3)-Dichlorphenyl, 3-Fluor-2-trifluormethylphenyl, (2,4)-Dichlorphenyl, (2,4)-Difluorphenyl, 4-Fluor-2-trifluormethyl-phenyl, (2,4)-Dimethoxyphenyl, 2-Chlor-4-fluor-phenyl, 2-Chlor-4-nitro-phenyl, 2-Chlor-4-methyl-phenyl, 2-Chlor-5-trifluormethyl-phenyl, 2-Chlor-5-methoxy-phenyl, 2-Brom-5-trifluormethyl-phenyl, 2-Brom-5-methoxy-phenyl, (2,4)-Dibrom-phenyl, (2,4)-Dimethyl-phenyl, 2-Fluor-4-trifluormethyl-phenyl, (2,5)-Difluor-phenyl, 2-Fluor-5-trifluormethyl-phenyl, 5-Fluor-2-trifluormethyl-phenyl, 5-Chlor-2-trifluormethyl-phenyl, 5-Brom-2-trifluormethyl-phenyl, (2,5)-Dimethoxy-phenyl, (2,5)-Bis-trifluormethyl-phenyl, (2,5)-Dichlor-phenyl, (2,5)-Dibrom-phenyl, 2-Methoxy-5-nitro-phenyl, 2-Fluor-6-trifluormethyl-phenyl, (2,6)-Dimethoxy-phenyl, (2,6)-Dimethyl-phenyl, (2,6)-Dichlor-phenyl, 2-Chlor-6-fluor-phenyl, 2-Brom-6-chlor-phenyl, 2-Brom-6-fluor-phenyl, (2,6)-Difluor-phenyl, (2,6)-Difluor-3-methyl-phenyl, (2,6)-Dibrom-phenyl, (2,6)-Dichlorphenyl, 3-Chlor-2-fluor-phenyl, 3-Chlor-5-methyl-phenyl, (3,4)-Dichlorphenyl, (3,4)-Dimethyl-phenyl, 3-Methyl-4-methoxy-phenyl, 4-Chlor-3-nitro-phenyl, (3,4)-Dimethoxy-phenyl, 4-Fluor-3-trifluormethylphenyl, 3-Fluor-4-trifluormethyl-phenyl, (3,4)-Difluor-phenyl, 3-Cyano-4-fluor-phenyl, 3-Cyano-4-methyl-phenyl, 3-Cyano-4-methoxy-phenyl, 3-Brom-4-fluor-phenyl, 3-Brom-4-methyl-phenyl, 3-Brom-4-methoxy-phenyl, 4-Chlor-2-fluor-phenyl, 4-Chlor-3-trifluormethyl, 4-Brom-3-methyl-phenyl, 4-Brom-5-methyl-phenyl, 3-Chlor-4-fluor-phenyl, 4-Fluor-3-nitro-phenyl, 4-Brom-3-nitro-phenyl, (3,4)-Dibrom-phenyl, 4-Chlor-3-methyl-phenyl, 4-Brom-3-methyl-phenyl, 4-Fluor-3-methyl-phenyl, 3-Fluor-4-methyl-phenyl, 3-Fluor-5-methyl-phenyl, 2-Fluor-3-methyl-phenyl, 4-Methyl-3-nitrophenyl, (3,5)-Dimethoxy-phenyl, (3,5)-Dimethyl-phenyl, (3,5)-Bis-trifluormethyl-phenyl, (3,5)-Difluor-phenyl, (3,5)-Dinitro-phenyl, (3,5)-Dichlor-phenyl, 3-Fluor-5-trifluormethyl-phenyl, 5-Fluor-3-trifluormethyl-phenyl, (3,5)-Dibrom-phenyl, 5-Chlor-4-fluor-phenyl, 5-Chlor-4-fluor-phenyl, 5-Brom-4-methyl-phenyl, (2,3,4)-Trifluorphenyl, (2,3,4)-Trichlorphenyl, (2,3,6)-Trifluor-phenyl, 5-Chlor-2-methoxy-phenyl, (2,3)-Difluor-4-methyl, (2,4,5)-Trifluor-phenyl, (2,4,5)-Trichlor-phenyl, (2,4)-Dichlor-5-fluor-phenyl, (2,4,6)-Trichlor-phenyl, (2,4,6)-Trimethylphenyl, (2,4,6)-Trifluor-phenyl, (2,4,6)-Trimethoxy-phenyl, (3,4,5)-Trimethoxy-phenyl, (2,3,4,5)-Tetrafluor-phenyl, 4-Methoxy-(2,3,6)-trimethyl-phenyl, 4-Methoxy-(2,3,6)-trimethyl-phenyl, 4-Chlor-2,5-dimethyl-phenyl, 2-Chlor-6-fluor-3-methyl-phenyl, 6-Chlor-2-fluor-3-methyl, (2,4,6)-Trimethylphenyl, (2,3,4,5,6)-Pentafluor-phenyl, 3-Methyl-pyrid-2-yl, 4-Methyl-pyrid-2-yl, 5-Methyl-pyrid-2-yl, 6-Methyl-pyrid-2-yl, 2-Methyl-pyrid-3-yl, 4-Methyl-pyrid-3-yl, 5-Methyl-pyrid-3-yl, 6-Methyl-pyrid-3-yl, 2-Methyl-pyrid-4-yl, 3-Methyl-pyrid-4-yl, 3-Fluor-pyrid-2-yl, 4-Fluor-pyrid-2-yl, 5-Fluor-pyrid-2-yl, 6-Fluor-pyrid-2-yl, 3-Chlorpyrid-2-yl, 4-Chlor-pyrid-2-yl, 5-Chlor-pyrid-2-yl, 6-Chlor-pyrid-2-yl, 3-Trifluormethylpyrid-2-yl, 4-Trifluormethyl-pyrid-2-yl, 5-Trifluormethyl-pyrid-2-yl, 6-Trifluormethylpyrid-2-yl, 3-Methoxy-pyrid-2-yl, 4-Methoxy-pyrid-2-yl, 5-Methoxy-pyrid-2-yl, 6-Methoxy-pyrid-2-yl, 4-Methyl-thiazol-2-yl, 5-Methyl-thiazol-2-yl, 4-Trifluormethylthiazol-2-yl, 5-Trifluormethyl-thiazol-2-yl, 4-Chlor-thiazol-2-yl, 5-Chlor-thiazol-2-yl, 4-Brom-thiazol-2-yl, 5-Brom-thiazol-2-yl, 4-Fluor-thiazol-2-yl, 5-Fluor-thiazol-2-yl, 4-Cyano-thiazol-2-yl, 5-Cyano-thiazol-2-yl, 4-Methoxy-thiazol-2-yl, 5-Methoxy-thiazol-2-yl, 4-Methyl-oxazol-2-yl, 5-Methyl-oxazol-2-yl, 4-Trifluormethyl-oxazol-2-yl, 5-Trifluormethyl-oxazol-2-yl, 4-Chlor-oxazol-2-yl, 5-Chlor-oxazol-2-yl, 4-Brom-oxazol-2-yl, 5-Brom-oxazol-2-yl, 4-Fluor-oxazol-2-yl, 5-Fluor-oxazol-2-yl, 4-Cyano-oxazol-2-yl, 5-Cyano-oxazol-2-yl, , 4-Methoxy-oxazol-2-yl, 5-Methoxy-oxazol-2-yl, 2-Methyl-(1,2,4)-thiadiazol-5-yl, 2-Trifluormethyl-(1,2,4)-thiadiazol-5-yl, 2-Chlor-(1,2,4)-thiadiazol-5-yl, 2-Fluor-(1,2,4)-thiadiazol-5-yl, 2-Methoxy-(1,2,4)-thiadiazol-5-yl, 2-Cyano-(1,2,4)-thiadiazol-5-y1, 2-Methyl-(1,2,4)-oxadiazol-5-yl, 2-Trifluormethyl-(1,2,4)-oxadiazol-5-yl, 2-Chlor-(1,2,4)-oxadiazol-5-yl, 2-Fluor-(1,2,4)-oxadiazol-5-yl, 2-Methoxy-(1,2,4)-oxadiazol-5-yl und 2-Cyano-(1,2,4)-oxadiazol-5-yl steht,
und
R¹⁴, R¹⁵, R¹⁶, R¹⁷, R¹⁸, R¹⁹ und R²⁰, unabhängig voneinander, jeweils für einen Alkyl-Rest ausgewählt aus der Gruppe bestehend aus Methyl, Ethyl, n-Propyl, Isopropyl, n-Butyl, sec-Butyl, Isobutyl und tert-Butyl stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

Ganz besonders bevorzugt sind substituierte 1-Propiolyl-piperazine der allgemeinen Formel I,
worin
n = 0, 1 oder 2 ist,
X für N oder C-R² steht,

R¹ für einen Wasserstoff-Rest, für einen Halogen-Rest ausgewählt aus der Gruppe bestehend aus F, Cl und Br, für eine CF₃-Gruppe, für eine Nitro-Gruppe, für eine Carboxy-Gruppe, für eine -C(=O)-O-CH₃-Gruppe, für eine -C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, oder für einen Rest ausgewählt aus der Gruppe bestehend aus Phenyl und Oxadiazolyl, der jeweils unsubstituiert oder mit ggf. 1, 2, 3, 4 oder 5 Substituenten unabhängig voneinander ausgewählt aus der Gruppe bestehend aus F, Cl, Br, I, -O-CH₃, -O-C₂H₅ und -O-C₃H₇ substituiert ist, steht,
R² für einen Wasserstoff-Rest, für einen Halogen-Rest ausgewählt aus der Gruppe bestehend aus F, Cl und Br, für eine CF₃-Gruppe, für eine Nitro-Gruppe, für eine Carboxy-Gruppe, für eine -C(=O)-O-CH₃-Gruppe, für eine -C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)₂₋R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, oder für einen Rest ausgewählt aus der Gruppe bestehend aus Phenyl und Oxadiazolyl, der jeweils unsubstituiert oder mit ggf. 1, 2, 3, 4 oder 5 Substituenten unabhängig voneinander ausgewählt aus der Gruppe bestehend aus F, Cl, Br, I, -O-CH₃, -O-C₂H₅ und -O-C₃H₇ substituiert ist, steht,
R³ für eine Oxo-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl oder für einen unsubstituierten Phenyl-Rest steht,
R⁴ für einen Wasserstoff-Rest, für eine -C(=O)-NH₂-Gruppe, für eine -C(=O)-NH-R¹⁴-Gruppe, für eine -C(=O)-NR¹⁵R¹⁶-Gruppe, für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, neo-Pentyl, n-Hexyl, n-Heptyl und n-Octyl oder für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Phenyl, Pyrid-2-yl, Pyrid-3-yl, Pyrid-4-yl, Pyrrol-2-yl, Pyrrol-3-yl, Thiophen-2-yl, Thiophen-3-yl, 4-Methyl-thiophen-2-yl, 3-Methyl-thiophen-2-yl, 5-Methyl-thiophen-2-yl, Furan-2-yl, Furan-3-yl, Imidazol-2-yl, Imidazol-4-yl, Thiazol-2-yl, Thiazol-4-yl, Thiazol-5-yl, Oxazol-2-yl, Oxazol-4-yl, Oxazol-5-yl, (1,2,4)-Thiadiazol-5-yl, (1,2,4)-Oxadiazol-5-yl, Napht-1-yl, Napht-2-yl, Indol-3-yl, Indol-4-yl, Indol-5-yl, Indol-6-yl, Indol-7-yl, 1-Methyl-indol-2-yl, 1-Methyl-indol-3-yl, 1-Methyl-indol-4-yl, 1-Methyl-indol-5-yl, 1-Methyl-indol-6-yl, 1-Methyl-indol-7-yl, Chinolin-3-yl, Chinolin-4-yl, Chinolin-2-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5'yl, Isochinolin-6-yl, Isochinolin-7-yl, Isochinolin-8-yl, 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Hydroxyphenyl, 3-Hydroxy-phenyl, 4-Hydroxy-phenyl, 2-Amino-phenyl, 3-Amino-phenyl, 4-Amino-phenyl, 2-Dimethylamino-phenyl, 3-Dimethylamino-phenyl, 4-Dimethylaminophenyl, 2-Methylamino-phenyl, 3-Methylamino-phenyl, 4-Methylamino-phenyl, 2-Acetyl-phenyl, 3-Acetyl-phenyl, 4-Acetyl-phenyl, 2-Methylsulfinyl-phenyl, 3-Methylsulfinyl-phenyl, 4-Methylsulfinyl-phenyl, 2-Methylsulfonyl-phenyl, 3-Methylsulfonyl-phenyl, 4-Methylsulfonyl-phenyl, 2-Methoxy-phenyl, 3-Methoxyphenyl, 4-Methoxy-phenyl, 2-Chlor-phenyl, 3-Chlor-phenyl, 4-Chlor-phenyl, 2-Ethoxyphenyl, 3-Ethoxy-phenyl, 4-Ethoxyphenyl, 2-Trifluormethyl-phenyl, 3-Trifluormethyl-phenyl, 4-Trifluormethyl-phenyl, 2-Difluormethyl-phenyl, 3-Difluormethyl-phenyl, 4-Difluormethyl-phenyl, 2-Fluormethyl-phenyl, 3-Fluormethyl-phenyl, 4-Fluormethyl-phenyl, 2-Nitro-phenyl, 3-Nitro-phenyl, 4-Nitro-phenyl, 2-Ethyl-phenyl, 3-Ethyl-phenyl, 4-Ethyl-phenyl, 2-Propyl-phenyl, 3-Propyl-phenyl, 4-Propyl-phenyl, 2-Isopropylphenyl, 3-Isopropyl-phenyl, 4-Isopropyl-phenyl, 2-tert-Butyl-phenyl, 3-tert-Butylphenyl, 4-tert-Butyl-phenyl, 2-Carboxyphenyl, 3-Carboxy-phenyl, 4-Carboxyphenyl, 2-Ethenyl-phenyl, 3-Ethenyl-phenyl, 4-Ethenyl-phenyl, 2-Ethinyl-phenyl, 3-Ethinylphenyl, 4-Ethinyl-phenyl, 2-Allyl-phenyl, 3-Allyl-phenyl, 4-Allyl-phenyl, 2-Trimethylsilanylethinyl-phenyl, 3-Trimethylsilanylethinyl-phenyl, 4-Trimethylsilanylethinyl-phenyl, 2-Formyl-phenyl, 3-Formyl-phenyl, 4-Formyl-phenyl, 2-Acetamino-phenyl, 3-Acetamino-phenyl, 4-Acetamino-phenyl, 2-Dimethylaminocarbonyl-phenyl, 3-Dimethylaminocarbonyl-phenyl, 4-Dimethylaminocarbonyl-phenyl, 2-Methoxymethyl-phenyl, 3-Methoxymethyl-phenyl, 4-Methoxymethyl-phenyl, 2-Ethoxymethyl-phenyl, 3-Ethoxymethyl-phenyl, 4-Ethoxymethyl-phenyl, 2-Aminocarbonyl-phenyl, 3-Aminocarbonyl-phenyl, 4-Aminocarbonyl-phenyl, 2-Methylaminocarbonyl-phenyl, 3-Methylaminocarbonylphenyl, 4-Methylaminocarbonyl-phenyl, 2-Carboxymethylester-phenyl, 3-Carboxymethylester-phenyl, 4-Carboxymethylester-phenyl, 2-Carboxyethylesterphenyl, 3-Carboxyethylester-phenyl, 4-Carboxyethylester-phenyl, 2-Carboxy-tert-butylester-phenyl, 3-Carboxy-tert-butylester-phenyl, 4-Carboxy-tert-butylester-phenyl, 2-Methylmercapto-phenyl, 3-Methylmercapto-phenyl, 4-Methylmercapto-phenyl, 2-Ethylmercapto-phenyl, 3-Ethylmercapto-phenyl, 4-Ethylmercaptophenyl, 2-Biphenyl, 3-Biphenyl, 4-Biphenyl, 2-Bromphenyl, 3-Bromphenyl, 4-Bromphenyl, 2-lod-phenyl, 3-lodphenyl, 4-lodphenyl, 2-Trifluormethoxy-phenyl, 3-Trilfuormethoxy-phenyl, 4-Trifluormethoxy-phenyl, 2-Fluor-4-methyl-phenyl, (2,3)-Dimethyl-phenyl, (2,4)-Dichlor-phenyl, (2,4)-Difluorphenyl, 2-Chlor-4-methyl-phenyl, 2-Chlor-5-trifluormethyl-phenyl, 2-Chlor-5-methoxy-phenyl, 2-Brom-5-trifluormethyl-phenyl, 2-Brom-5-methoxy-phenyl, (2,4)-Dimethyl-phenyl, (2,6)-Dimethyl-phenyl, 3-Chlor-5-methylphenyl, (3,4)-Dimethyl-phenyl, 3-Methyl-4-methoxy-phenyl, (3,4)-Difluor-phenyl, 3-Cyano-4-fluor-phenyl, 3-Cyano-4-methyl-phenyl, 3-Cyano-4-methoxy-phenyl, 3-Brom-4-fluor-phenyl, 3-Brom-4-methyl-phenyl, 3-Brom-4-methoxy-phenyl, 4-Chlor-2-fluor-phenyl, 4-Fluor-3-methyl-phenyl, 3-Fluor-4-methyl-phenyl, 3-Fluor-5-methylphenyl, 2-Fluor-3-methyl-phenyl, (3,5)-Dimethyl-phenyl und (3,5)-Dichlor-phenyl,
und
R¹⁴, R¹⁵, R¹⁶, R¹⁷, R¹⁸ und R²⁰, unabhängig voneinander, jeweils für einen Alkyl-Rest ausgewählt aus der Gruppe bestehend aus Methyl, Ethyl, n-Propyl, Isopropyl, n-Butyl, sec-Butyl, Isobutyl und tert-Butyl stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

Ganz besonders bevorzugt ist die erfindungsgemäße Verbindung 1-(3-Phenylpropiolyl)-4-(thiazol-2-yl)-piperazin der folgenden Formel: 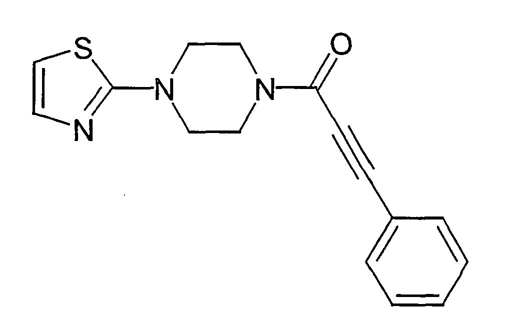 und deren entsprechende Salze sowie ggf. jeweils entsprechende Solvate.

Noch weiter bevorzugt sind Verbindungen der allgemeinen Formel I ausgewählt aus der Gruppe bestehend aus

| | |
|---|---|
| [AAA00100] | 4-(3-Methylmercapto-1,2,4-thiadiazol-5-yl)-1-(3-phenyl-propiolyl)-piperazin |
| [AAA00101] | 4-(3-Methansulfonyl-1,2,4-thiadiazol-5-yl)-1-(3-phenyl-propiolyl)-piperazin |
| [AAA100102] | 4-(3-Methoxy-1,2,4-thiadiazol-5-yl)-1-(3-phenyl-propiolyl)-piperazin |
| [AAA00103] | 4-(1,2,4-Thiadiazol-5-yl)-1-(3-phenyl-propiolyl)-piperazin |
| [AAA00104] | 4-(3-Methyl-1,2,4-thiadiazol-5-yl)-1-(3-phenyl-propiolyl)-piperazin |
| [AAA00105] | 4-(3-Trifluormethyl-1,2,4-thiadiazol-5-yl)-1-(3-phenyl-propiolyl)-piperazin |
| [AAA100106] | 4-(5-(3-Methyl-1,2,4-oxadiazol-5-yl)-thiazol-2-yl)-1-(3-phenylpropiolyl)-piperazin |
| [AAA00107] | 4-(4-tert-Butyl-thiazol-2-yl)-1-(3-(4-amino-phenyl)-propiolyl)-piperazin |
| [AAA1] | 4-(Thiazol-2-yl)-1-(3-(2-carboxy-phenyl)-propiolyl)-piperazin methylester |
| [AAA10] | 4-(Thiazol-2-yl)-1-(3-(2-thienyl)-propiolyl)-piperazin |
| [AAA11] | 4-(Thiazol-2-yl)-1-(3-(3-thienyl)-propiolyl)-piperazin |
| [AAA12] | 4-(Thiazol-2-yl)-1-(3-(2-methoxy-phenyl)-propiolyl)-piperazin |
| [AAA13] | 4-(Thiazol-2-yl)-1-(3-(3-methoxy-phenyl)-propiolyl)-piperazin |
| [AAA14] | 4-(Thiazol-2-yl)-1-(3-(4-methoxy-phenyl)-propiolyl)-piperazin |
| [AAA15] | 4-(Thiazol-2-yl)-1-(3-(2-cyano-phenyl)-propiolyl)-piperazin |
| [AAA116] | 4-(Thiazol-2-yl)-1-(3-(3-cyano-phenyl)-propiolyl)-piperazin |
| [AAA17] | 4-(Thiazol-2-yl)-1-(3-(4-cyano-phenyl)-propiolyl)-piperazin |
| [AAA18] | 4-(Thiazol-2-yl)-1-(3-(2,4-dimethyl-phenyl)-propiolyl)-piperazin |
| [AAA19] | 4-(Thiazol-2-yl)-1-(3-(3,5-dimethyl-phenyl)-propiolyl)-piperazin |
| [AAA2] | 4-(Thiazol-2-yl)-1-(3-(4-carboxy-phenyl)-propiolyl)-piperazin methylester |
| [AAA20] | 4-(Thiazol-2-yl)-1-(3-(2,6-dimethyl-phenyl)-propiolyl)-piperazin |
| [AAA21] | 4-(Thiazol-2-yl)-1-(3-(2-fluor-phenyl)-propiolyl)-piperazin |
| [AAA22] | 4-(Thiazol-2-yl)-1-(3-(3-fluor-phenyl)-propiolyl)-piperazin |
| [AAA23] | 4-(Thiazol-2-yl)-1-(3-(2-chlor-phenyl)-propiolyl)-piperazin |
| [AAA24] | 4-(Thiazol-2-yl)-1-(3-(3-chlor-phenyl)-propiolyl)-piperazin |
| [AAA25] | 4-(Thiazol-2-yl)-1-(3-naphthyl-propiolyl)-piperazin |
| [AAA26] | 4-(Thiazol-2-yl)-1-(3-(2,3-dimethyl-phenyl)-propiolyl)-piperazin |
| [AAA27] | 4-(Thiazol-2-yl)-1-(3-(3,4-dimethyl-phenyl)-propiolyl)-piperazin |
| [AAA28] | 4-(Thiazol-2-yl)-1-(3-(3-nitro-phenyl)-propiolyl)-piperazin |
| [AAA29] | 4-(Thiazol-2-yl)-1-(3-(2-nitro-phenyl)-propiolyl)-piperazin |
| [AAA3] | 4-(Thiazol-2-yl)-1-(3-(3-carboxy-phenyl)-propiolyl)-piperazin ethylester |
| [AAA30] | 4-(Thiazol-2-yl)-1-(3-(3-formyl-phenyl)-propiolyl)-piperazin |
| [AAA31] | 4-(Thiazol-2-yl)-1-(3-(3-ethenyl-phenyl)-propiolyl)-piperazin |
| [AAA32] | 4-(Thiazol-2-yl)-1-(3-pyrid-2-yl-propiolyl)-piperazin |
| [AAA33] | 4-(Thiazol-2-yl)-1-(3-pyrid-3-yl-propiolyi)-piperazin |
| [AAA34] | 4-(Thiazol-2-yl)-1-(3-pyrid-4-yl-propiolyl)-piperazin |
| [AAA35] | 4-(Thiazol-2-yl)-1-(3-(chinolin-6-yl)-propiolyi)-piperazin |
| [AAA36] | 4-(Thiazol-2-yl)-1-(3-(3-isopropyl-phenyl)-propiolyl)-piperazin |
| [AAA37] | 4-(Thiazol-2-yl)-1-(3-biphenyl-3-yl-propiolyl)-piperazin |
| [AAA38] | 4-(Thiazol-2-yl)-1-(3-naphth-2-yl-propiolyl)-piperazin |
| [AAA39] | 4-(Thiazol-2-yl)-1-(3-(1-methyl-indol-5-yl)-propiolyl)-piperazin |
| [AAA4] | 4-(Thiazol-2-yl)-1-(3-(2-hydroxy-phenyl)-propiolyl)-piperazin |
| [AAA40] | 4-(Thiazol-2-yl)-1-(3-(3-methylmercapto-phenyl)-propiolyl)-piperazin |
| [AAA41] | 4-(Thiazol-2-yl)-1-(3-(3-cyano-4-fluor-phenyl)-propiolyl)-piperazin |
| [AAA42] | 4-(Thiazol-2-yl)-1-(3-(3-methoxymethyl-phenyl)-propiolyl)-piperazin |
| [AAA43] | 4-(Thiazol-2-yl)-1-(3-(3-hydroxy-phenyl)-propiolyl)-piperazin |
| [AAA44] | 4-(Thiazol-2-yl)-1-(3-(3-acetamino-phenyl)-propiolyl)-piperazin |
| [AAA45] | 4-(Thiazol-2-yl)-1-(3-(4-acetamino-phenyl)-propiolyl)-piperazin |
| [AAA46] | 4-(Thiazol-2-yl)-1-(3-(2-carboxy-phenyl)-propiolyl)-piperazin |
| [AAA47] | 4-(Thiazol-2-yl)-1-(3-(3-carboxy-phenyl)-propiolyl)-piperazin |
| [AAA48] | 4-(Thiazol-2-yl)-1-(3-(4-carboxy-phenyl)-propiolyl)-piperazin |
| [AAA49] | 4-(Thiazol-2-yl)-1-(3-(3-carboxy-phenyl)-propiolyl)-piperazinmethylester |
| [AAA5] | 4-(Thiazol-2-yl)-1-(3-(4-hydroxy-phenyl)-propiolyl)-piperazin |
| [AAA50] | 4-(Thiazol-2-yl)-1-(3-(3-aminocarbonyl-phenyl)-propiolyl)-piperazin |
| [AAA51] | 4-(Thiazol-2-yl)-1-(3-(3-methylaminocarbonyl-phenyl)-propiolyl)-piperazin |
| [AAA52] | 4-(Thiazol-2-yl)-1-(3-(3-dimethylaminocarbonyl-phenyl)-propiolyl)-piperazin |
| [AAA53] | 4-(Thiazol-2-yl)-1-(3-(2-tolyl)-propiolyl)-piperazin Hydrochlorid |
| [AAA54] | 4-(Thiazol-2-yl)-1-(3-(3-tolyl)-propiolyl)-piperazin Hydrochlorid |
| [AAA55] | 4-(Thiazol-2-yl)-1-(3-(4-tolyl)-propiolyl)-piperazin Hydrochlorid |
| [AAA56] | 4-(Thiazol-2-yl)-1-(3-phenyl-propiolyl)-piperazin Hydrochlorid |
| [AAA57] | 4-(Thiazol-2-yl)-1-(3-(2-trifluormethyl-phenyl)-propiolyl)-piperazin Hydrochlorid |
| [AAA58] | 4-(Thiazol-2-yl)-1-(3-(3-trifluormethyl-phenyl)-propiolyl)-piperazin Hydrochlorid |
| [AAA59] | 4-(Thiazol-2-yl)-1-(3-(4-trifluormethyl-phenyl)-propiolyl)-piperazin Hydrochlorid |
| [AAA6] | 4-(Thiazol-2-yl)-1-(3-(2-amino-phenyl)-propiolyl)-piperazin |
| [AAA60] | 4-(Thiazol-2-yl)-1-(3-pentyl-propiolyl)-piperazin |
| [AAA61] | 4-(Thiazol-2-yl)-1-(3-(4-fluor-phenyl)-propiolyi)-piperazin |
| [AAA62] | 4-(Thiazol-2-yl)-1-(3-(4-chlor-phenyl)-propiolyl)-piperazin |
| [AAA63] | 2-Methyl-4-(thiazol-2-yl)-1-(3-phenyl-propiolyl)-piperazin |
| [AAA64] | (S)-2-Methyl-4-(thiazol-2-yl)-1-(3-phenyl-propiolyl)-piperazin |
| [AAA65] | (R)-2-Methyl-4-(thiazol-2-yl)-1-(3-phenyl-propiolyl)-piperazin |
| [AAA66] | 2-Methyl-4-(thiazol-2-yl)-1-(3-phenyl-propiolyl)-piperazin Hydrochlorid |
| [AAA67] | 2-Methyl-4-(thiazol-2-yl)-1-(3-(3-tolyl)-propiolyl)-piperazin |
| [AAA68] | 2-Methyl-4-(thiazol-2-yl)-1-(3-(3-tolyl)-propiolyl)-piperazin Hydrochlorid |
| [AAA69] | 4-(Thiazol-2-yl)-1-(3-cyclohexyl-propiolyl)-piperazin |
| [AAA7] | 4-(Thiazol-2-yl)-1-(3-(3-amino-phenyl)-propiolyl)-piperazin |
| [AAA70] | 4-(Thiazol-2-yl)-1-(3-methyl-propiolyl)-piperazin |
| [AAA71] | 2-Ethyl-4-(thiazol-2-yl)-1-(3-phenyl-propiolyl)-piperazin |
| [AAA72] | 2-Phenyl-4-(thiazol-2-yl)-1-(3-phenyl-propiolyl)-piperazin |
| [AAA73] | 4-(Thiazol-2-yl)-1-(3-(2-furyl)-propiolyl)-piperazin |
| [AAA74] | 4-(Thiazol-2-yl)-1-(3-(3-furyl)-propiolyl)-piperazin |
| [AAA75] | cis-2,6-Dimethyl-4-(thiazol-2-yl)-1-(3-phenyl-propiolyl)-piperazin ethylester |
| [AAA76] | 4-(5-Carboxy-thiazol-2-yl)-1-(3-phenyl-propiolyl)-piperazin ethylester |
| [AAA77] | 4-(4-Carboxy-thiazol-2-yl)-1-(3-phenyl-propiolyl)-piperazin |
| [AAA78] | 4-(5-Carboxy-thiazol-2-yl)-1-(3-phenyl-propiolyl)-piperazin |
| [AAA79] | 4-(4-Carboxy-thiazol-2-yl)-1-(3-phenyl-propiolyl)-piperazin |
| [AAA8] | 4-(Thiazol-2-yl)-1-(3-(4-amino-phenyl)-propiolyl)-piperazin |
| [AAA80] | 4-(5-Methylaminocarbonyl-thiazol-2-yl)-1-(3-phenyl-propiolyl)-piperazin |
| [AAA81] | 4-(5-Dimethylaminocarbonyl-thiazol-2-yl)-1-(3-phenyl-propiolyl)-piperazin |
| [AAA82] | 4-(3-Phenyl-1,2,4-thiadiazol-5-yl)-1-(3-phenyl-propiolyl)-piperazin |
| [AAA83] | 4-(Thiazol-2-yl)-1-(3-(chinol-7-yl)-propiolyl)-piperazin |
| [AAA84] | 4-(Thiazol-2-yl)-1-(3-phenyl-propiolyl)-piperazin-3-on |
| [AAA85] | 4-(3-(4-Fluorbenzyl)-1,2,4-thiadiazol-5-yl)-1-(3-phenyl-propiolyl)-piperazin |
| [AAA86] | 4-(5-Nitro-thiazol-2-yl)-1-(3-phenyl-propiolyl)-piperazin |
| [AAA87] | 4-(4-tert-Butyl-thiazol-2-yl)-1-(3-phenyl-propiofyl)-piperazin |
| [AAA88] | 4-(5-Methyl-thiazol-2-yl)-1-(3-phenyl-propiolyl)-piperazin |
| [AAA89] | 4-(4,5-Dimethyl-thiazol-2-yl)-1-(3-phenyl-propiolyl)-piperazin |
| [AAA9] | 4-(Thiazol-2-yl)-1-(3-(indol-5-yl)-propiolyl)-piperazin |
| [AAA90] | 4-(5-Brom-4-phenyl-thiazol-2-yl)-1-(3-phenyl-propiolyl)-piperazin |
| [AAA91] | 4-(5-Brom-4-methyl-thiazol-2-yl)-1-(3-phenyl-propiolyl)-piperazin |
| [AAA92] | 4-(4-Methyl-thiazol-2-yl)-1-(3-phenyl-propiolyl)-piperazin |
| [AAA93] | 4-(4-Trifluormethyl-thiazol-2-yl)-1-(3-phenyl-propiolyl)-piperazin |
| [AAA94] | 4-(4-Chlor-thiazol-2-yl)-1-(3-phenyl-propiolyl)-piperazin |
| [AAA95] | 4-(5-Chlor-thiazol-2-yl)-1-(3-phenyl-propiolyl)-piperazin |
| [AAA96] | 4-(4-Brom-thiazol-2-yl)-1-(3-phenyl-propiolyl)-piperazin |
| [AAA97] | 4-(5-Brom-thiazol-2-yl)-1-(3-phenyl-propiolyl)-piperazin |
| [AAA98] | 4-(5-Phenyl-thiazol-2-yl)-1-(3-phenyl-propiolyl)-piperazin |
| [AAA99] | 4-(4-Phenyl-thiazol-2-yl)-1-(3-phenyl-propiolyl)-piperazin |
| [BBB2] | 4-(Thiazol-2-yl)-1-propiolyl-piperazin; |
| [CCC1] | 4-(3-Phenyl-1,2,4-thiadiazol-5-yl)-1-(3-(2-methoxy-phenyl)-propiolyl)-piperazin |
| [CCC2] | 4-(3-Phenyl-1,2,4-thiadiazol-5-yl)-1-(3-(4-methoxy-phenyl)-propiolyl)-piperazin |
| [CCC3] | 4-(3-Phenyl-1,2,4-thiadiazol-5-yl)-1-(3-(2-fluor-phenyl)-propiolyl)-piperazin |
| [CCC4] | 4-(3-Phenyl-1,2,4-thiadiazol-5-yl)-1-(3-(3-fluor-phenyl)-propiolyl)-piperazin |
| [CCC5] | 4-(3-Phenyl-1,2,4-thiadiazol-5-yl)-1-(3-(4-fluor-phenyl)-propiolyl)-piperazin |
| [CCC6] | 4-(3-Phenyl-1,2,4-thiadiazol-5-yl)-1-(3-(2,4-dichlor-phenyl)-propiolyl)-piperazin |
| [CCC7] | 4-(3-Phenyl-1,2,4-thiadiazol-5-yl)-1-(3-(3,5-dichlor-phenyl)-propiolyl)-piperazin |
| [CCC8] | 4-(3-(4-Fluor-phenyl)-1,2,4-thiadiazol-5-yl)-1-(3-(tol-2-yl)-propiolyl)-piperazin |
| [CCC9] | 4-(3-(4-Fluor-phenyl)-1,2,4-thiadiazol-5-yl)-1-(3-(3-methoxy-phenyl)-propiolyl)-piperazin |
| [CCC10] | 4-(3-(4-Fluor-phenyl)-1,2,4-thiadiazol-5-yl)-1-(3-(3-fluor-4-methyl-phenyl)-propiolyl)-piperazin |
| [CCC11] | 4-(3-(4-Fluor-phenyl)-1,2,4-thiadiazol-5-yl)-1-(3-(2,4-difluor-phenyl)-propiolyl)-piperazin |
| [CCC12] | 4-(3-(4-Fluor-phenyl)-1,2,4-thiadiazol-5-yl)-1-(3-(tol-3-yl)-propiolyl)-piperazin |
| [CCC13] | 4-(3-(4-Fluor-phenyl)-1,2,4-thiadiazol-5-yl)-1 -(3-(4-trifluormethyl-phenyl)-propiolyl)-piperazin |
| [CCC14] | 4-(3-(4-Fluor-phenyl)-1,2,4-thiadiazol-5-yl)-1-(3-(3-trifluormethyl-phenyl)-propiolyl)-piperazin |
| [CCC15] | 4-(3-(4-Fluor-phenyl)-1,2,4-thiadiazol-5-yl)-1-(3-(2-chlor-5-trifluormethyl-phenyl)-propiolyl)-piperazin |
| [CCC16] | 4-(3-tert-Butyl-1,2,4-thiadiazol-5-yl)-1-(3-(thiophen-2-yl)-propiolyl)-piperazin |
| [CCC17] | 4-(3-tert-Butyl-1,2,4-thiadiazol-5-yl)-1-(3-(3-trifluormethyl-phenyl)-propiolyl)-piperazin |
| [CCC18] | 4-(3-tert-Butyl-1,2,4-thiadiazol-5-yl)-1-(3-(2-methoxy-phenyl)-propiolyl)-piperazin |
| [CCC19] | 4-(3-tert-Butyl-1,2,4-thiadiazol-5-yl)-1-(3-(tol-2-yl)-propiolyl)-piperazin |
| [CCC20] | 4-(3-tert-Butyl-1,2,4-thiadiazol-5-yl)-1-(3-(3-fluor-4-methyl-phenyl)-propiolyl)-piperazin |
| [CCC21] | 4-(3-tert-Butyl-1,2,4-thiadiazol-5-yl)-1-(3-(2-chlor-5-trifluormethyl-phenyl)-propiolyl)-piperazin |
| [CCC22] | 4-(Thiazol-2-yl)-1-(3-(2,4-difluor-phenyl)-propiolyl)-piperazin |
| [CCC23] | 4-(Thiazol-2-yl)-1-(3-(2-brom-5-methoxy-phenyl)-propiolyl)-piperazin |
| [CCC24] | 4-(Thiazol-2-yl)-1-(3-(3-brom-4-methoxy-phenyl)-propiolyl)-piperazin |
| [CCC25] | 4-(Thiazol-2-yl)-1-(3-(3,5-dichlor-phenyl)-propiolyl)-piperazin |
| [CCC26] | 4-(Thiazo!-2-yl)-1-(3-(4-fluor-3-methyl-phenyl)-propiolyl)-piperazin |
| [CCC27] | 4-(4-tert-Butyl-thiazol-2-yl)-1-(3-(3-cyano-phenyl)-propiolyl)-piperazin |
| [CCC28] | 4-(4-tert-Butyl-thiazol-2-yl)-1-(3-(2-triffuormethyl-phenyl)-propiolyl)-piperazin |
| [CCC29] | 4-(4-Phenyl-thiazol-2-yl)-1-(3-(2,3-dimethyl-phenyl)-propiolyl)-piperazin |
| [CCC30] | 4-(4-Phenyl-thiazol-2-yl)-1-(3-(4-fluor-phenyl)-propiolyl)-piperazin |
| [CCC31] | 4-(4-Methyl-thiazol-2-yl)-1-(3-(tol-3-yl)-propiolyl)-piperazin |
| [CCC32] | 4-(4-Methyl-thiazol-2-yl)-1-(3-(thiophen-2-yl)-propiolyl)-piperazin |
| [CCC33] | 4-(5-Methyl-thiazol-2-yl)-1-(3-(tol-4-yl)-propiolyl)-piperazin |
| [CCC34] | 4-(5-Methyl-thiazol-2-yl)-1-(3-(2-cyano-phenyl)-propiolyl)-piperazin |
| [CCC35] | 4-(4,5-Dimethyl-thiazol-2-yl)-1-(3-(3-cyano-phenyl)-propiolyl)-piperazin |
| [CCC36] | 4-(4,5-Dimethyl-thiazol-2-yl)-1-(3-(3,4-dimethyl-phenyl)-propiolyl)-piperazin |
| [CCC37] | 4-(4-(4-Methoxy-phenyl)-thiazol-2-yl)-1-(3-(2,4-dimethyl-phenyl)-propiolyl)-piperazin |
| [CCC38] | 4-(4-(4-Methoxy-phenyl)-thiazol-2-yl)-1-(3-(4-trifluormethyl-phenyl)-propiolyl)-piperazin |
| [CCC39] | 4-(4-(4-Fluor-phenyl)-thiazol-2-yl)-1-(3-(2-cyano-phenyl)-propiolyl)-piperazin |
| [CCC40] | 4-(4-(4-Chlor-phenyl)-thiazol-2-yl)-1-(3-(4-cyano-phenyl)-propiolyl)-piperazin |
| [AAA00108] | 4-(Thiazol-2-yl)-1-(3-(1-Methyl-indol-6-yl)-propiolyl)-piperazin |
| [AAA00109] | 4-(Thiazol-2-yl)-1-(3-(3-acetyl-phenyl)-propiolyl)-piperazin |
| [AAA00110] | 4-(Thiazol-2-yl)-1-(3-(3-fluor-5-methyl-phenyl)-propiolyl)-piperazin |
| [AAA00111] | 4-(Thiazol-2-yl)-1-(3-(2-fluor-3-methyl-phenyl)-propiolyl)-piperazin |
| [AAA00112] | 4-(Thiazol-2-yl)-1-(3-(3-methylamino-phenyl)-propiolyl)-piperazin |
| [AAA00113] | 4-(Thiazol-2-yl)-1-(3-(3-dimethylamino-phenyl)-propiolyl)-piperazin |
| [AAA00114] | 4-(Thiazol-2-yl)-1-(dimethylcarbamoyl-propiolyl)-piperazin |
| [AAA00115] | 4-(Thiazol-2-yl)-1-(3-(3-methylsulfinyl-phenyl)-propiolyl)-piperazin |
| [AAA00116] | 4-(Thiazol-2-yl)-1-(3-(3-methylsulfonyl-phenyl)-propiolyl)-piperazin |
| [AAA00117] | 4-(Thiazol-2-yl)-1-(3-(3-ethinyl-phenyl)-propiolyl)-piperazin |
| [AAA00118] | 4-(Thiazol-2-yl)-1-(3-(4-methyl-thiophen-2-yl)-propiolyl)-piperazin |
| [AAA00119] | 2-Methyl-4-(thiazol-2-yl)-1-(3-(3-chlor-phenyl)-propiolyl)-piperazin |
| [AAA00120] | 4-(Thiazol-2-yl)-1-(3-(3-ethyl-phenyl)-propiolyl)-piperazin |
| [AAA00121] | 2-tert-Butyl-4-(thiazol-2-yl)-1-(3-phenyl-propiolyl)-piperazin |
| [AAA00122] | 4-(Thiazol-2-yl)-1-(3-(3-difluormethyl-phenyl)-propiolyl)-piperazin |
| [AAA00123] | 2-Methyl-4-(thiazol-2-y1)-1-(3-(3-cyano-phenyl)-propiolyl)-piperazin |
| [AAA00124] | 2-Isopropyl-4-(thiazol-2-yl)-1-(3-phenyl-propiolyl)-piperazin |
| [AAA00125] | 4-(Thiazol-2-yl)-1-(3-(3-trimethylsilanylethinyl-phenyl)-propiolyl)-piperazin; |

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

Ein weiterer Gegenstand der vorliegenden Erfindung ist ein Verfahren zur Herstellung von Verbindungen der vorstehend angegebenen allgemeinen Formel I gemäß dem wenigstens eine Verbindung der allgemeinen Formel II, 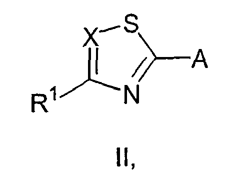 worin die Reste X und R¹ die vorstehend genannte Bedeutung haben und A für eine Abgangsgruppe, vorzugsweise für einen Halogen-Rest oder einen Sulfonsäureester, besonders bevorzugt für Chlor oder Brom steht, mit wenigstens einer Verbindung der allgemeinen Formel III, 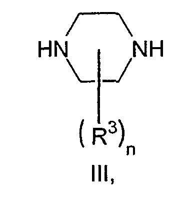 worin R³ und n die vorstehend genannte Bedeutung haben, ggf. in einem Reaktionsmedium, ggf. in Gegenwart wenigstens einer Base und/oder wenigstens einer metallorganischen Verbindung und/oder wenigstens eines Metallhydrid-Reagenzes, vorzugsweise bei einer Temperatur von - 70 °C bis 300 °C, bevorzugt-70 °C bis 150 °C, in wenigstens eine entsprechende Verbindung der allgemeinen Formel IV, ggf. in Form eines entsprechenden Salzes überführt wird, 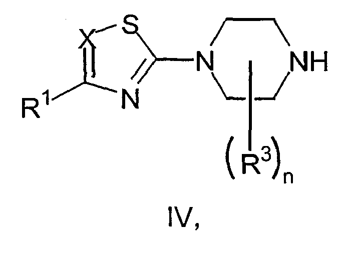 worin X, R¹, R³ und n die vorstehend genannte Bedeutung haben, und diese ggf. gereinigt und/oder isoliert wird
oder wenigstens eine Verbindung der allgemeinen Formel II mit wenigstens einer Verbindung der allgemeinen Formel V, 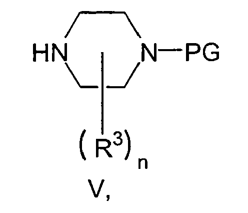 worin R³ und n die vorstehend genannte Bedeutung haben und PG für eine Schutzgruppe, bevorzugt eine Schutzgruppe ausgewählt aus der Gruppe bestehend aus tert-Butyloxy-carbonyl, Benzyl, Benzyloxycarbonyl und 9-Fluorenylmethyloxycarbonyl, steht ggf. in einem Reaktionsmedium, ggf. in Gegenwart wenigstens einer Base und/oder wenigstens einer metallorganischen Verbindung und/oder wenigstens eines Metallhydrid-Reagenzes, vorzugsweise bei einer Temperatur von - 70 °C bis 300 °C in wenigstens eine entsprechende Verbindung der allgemeinen Formel VI überführt wird, 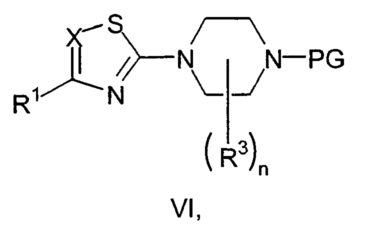 worin R¹, R³, X, n und PG die vorstehend genannte Bedeutung haben, und diese ggf. gereinigt und/oder isoliert wird;
oder wenigstens eine Verbindung der allgemeinen Formel VII, 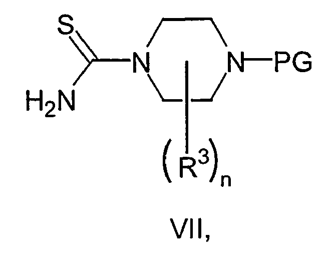 worin R³ und n die vorstehend genannte Bedeutung haben und PG für eine Schutzgruppe, bevorzugt eine Schutzgruppe ausgewählt aus der Gruppe bestehend aus tert-Butyloxy-carbonyl, Benzyl, Benzyloxycarbonyl und 9-Fluorenylmethyloxycarbonyl, steht durch Umsetzung mit wenigstens einer Verbindung der allgemeinen Formel R¹C(=O)-CH₂-A oder (C₁₋₅Alkyl-O)₂₋CH₂-A, worin R¹ die vorstehend genannte Bedeutung hat und A für eine Abgangsgruppe, vorzugsweise für einen Halogen-Rest, besonders bevorzugt für ein Brom-Atom steht, in einem Reaktionsmedium, ggf. in Gegenwart wenigstens einer organischen Base oder in Gegenwart wenigstens einer Säure, vorzugsweise in Gegenwart wenigstens einer Base ausgewählt aus der Gruppe bestehend aus Triethylamin, Diisopropylethylamin, N-Methylmorpholin, Dimethylaminopyridin und Pyridin oder in Gegenwart wenigstens einer Säure ausgewählt aus der Gruppe bestehend aus Essigsäure, Trifluoressigsäure und Salzsäure, vorzugsweise bei einer Temperatur zwischen - 70 °C bis 300 °C in wenigstens eine entsprechende Verbindung der allgemeinen Formel VI, ggf. in Form eines entsprechenden Salzes überführt wird, worin R¹, R³ und n die vorstehend genannte Bedeutung haben, PG die vorstehend genannte Bedeutung hat und X für CH steht, und diese ggf. gereinigt und/oder isoliert wird;
und wenigstens eine Verbindung der allgemeinen Formel VI für den Fall, dass PG für eine tert-Butoxycarbonyl- oder 9-Fluorenylmethyloxycarbonyl-Gruppe steht, in einem Reaktionsmedium, in Gegenwart wenigstens einer Säure, vorzugsweise in Gegenwart wenigstens einer Säure ausgewählt aus der Gruppe bestehend aus Salzsäure und Trifluoressigsäure, vorzugsweise bei einer Temperatur zwischen -70 °C bis 100 °C oder für den Fall, dass PG für eine Benzyl-Gruppe oder Benzyloxycarbonyl-Gruppe steht, in einem Reaktionsmedium, in Gegenwart von Wasserstoff und in Gegenwart wenigstens eines Katalysators, vorzugsweise in Gegenwart von Palladium auf Kohle, vorzugsweise bei einer Temperatur zwischen - 70 °C bis 100 °C in wenigstens eine entsprechende Verbindung der allgemeinen Formel IV, ggf. in Form eines entsprechenden Salzes überführt wird, worin X, R¹, R³ und n die vorstehend genannte Bedeutung haben, und diese ggf. gereinigt und/oder isoliert wird;
und wenigstens eine Verbindung der allgemeinen Formel IV durch Umsetzung mit wenigstens einer Verbindung der allgemeinen Formel R⁴-C≡C-C(=O)-OH, worin R⁴ die vorstehend genannte Bedeutung hat, in einem Reaktionsmedium, ggf. in Gegenwart wenigstens eines geeigneten Kopplungsmittels, ggf. in Gegenwart wenigstens einer Base, vorzugsweise bei einer Temperatur von - 70 °C bis 100 °C, oder durch Umsetzung mit wenigstens einer Verbindung der allgemeinen Formel R⁴-C≡C-C(=O)-A, worin R⁴ die vorstehend genannte Bedeutung hat und A für eine Abgangsgruppe, vorzugsweise für einen Halogen-Rest, besonders bevorzugt für Chlor oder Brom steht, in einem Reaktionsmedium, ggf. in Gegenwart wenigstens einer Base, bei einer Temperatur von - 70 °C bis 100 °C, in wenigstens eine entsprechende Verbindung der allgemeinen Formel I, ggf. in Form eines entsprechenden Salzes überführt wird, 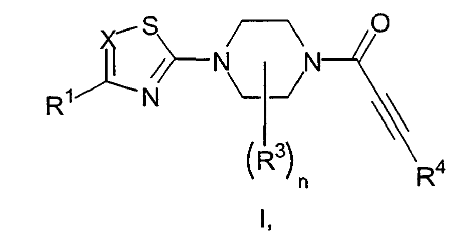 worin X und R¹, R³, R⁴ und n die vorstehend genannte Bedeutung haben, und diese ggf. gereinigt und/oder isoliert wird;
oder wenigstens eine Verbindung der allgemeinen Formel IV durch Umsetzung mit Propinsäure [HC≡C-C(=O)-OH] in einem Reaktionsmedium, ggf. in Gegenwart wenigstens eines geeigneten Kopplungsmittels, ggf. in Gegenwart wenigstens einer Base, vorzugsweise bei einer Temperatur von - 70°C bis 100 °C, oder durch Umsetzung mit wenigstens einer Verbindung der allgemeinen Formel HC≡C-C(=O)-A, worin A für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel VIII, ggf. in Form eines entsprechenden Salzes überführt wird, 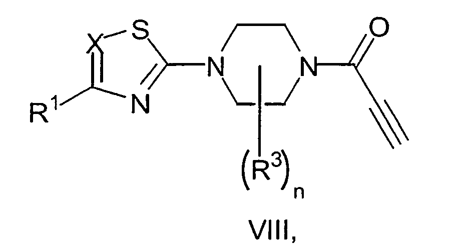 worin R¹, R³ X und n die vorstehend genannte Bedeutung haben, und diese ggf. gereinigt und/oder isoliert wird,
und wenigstens eine Verbindung der allgemeinen Formel VIII durch Umsetzung mit wenigstens einer Verbindung der allgemeinen Formel R⁴-A, worin R⁴ die vorstehend genannte Bedeutung mit Ausnahme von Wasserstoff hat und A für eine Abgangsgruppe, vorzugsweise für einen Halogen-Rest oder einen Sulfonsäureester, besonders bevorzugt für Iod, Brom oder Triflat, steht, in einem Reaktionsmedium, ggf. in Gegenwart wenigstens eines Katalysators, bevorzugt in Gegenwart eines Palladium-Katalysators ausgewählt aus der Gruppe bestehend aus Palladiumchlorid [PdCl₂], Palladiumacetat [Pd(OAc)₂], Tetrakistriphenylphosphinpalladium [Pd(PPh₃)₄], Bistriphenylphosphinpalladiumdichlorid [Pd(PPh₃)₂Cl₂] und Bistriphenylphosphinpalladiumacetat [Pd(PPh₃)₂(OAc)₂], ggf. in Gegenwart wenigstens eines Liganden, bevorzugt in Gegenwart wenigstens eines Liganden ausgewählt aus der Gruppe bestehend aus Triphenylphosphin, Triphenylarsin und Tri-2-furyl-phoshpin, ggf. in Gegenwart wenigstens eines anorganischen Salzes, bevorzugt in Gegenwart wenigstens eines anorganischen Salzes ausgewählt aus der Gruppe bestehend aus Lithiumchlorid und Zinkchlorid, ggf. in Gegenwart wenigstens eines Kupfersalzes, bevorzugt in Gegenwart von Kupferiodid, ggf. in Gegenwart wenigstens einer organischen oder anorganischen Base, vorzugsweise in Gegenwart wenigstens einer Base ausgewählt aus der Gruppe bestehend aus Triethylamin, [1,4]-Diazabicyclo-[2.2.2]-octan, Diisopropylamin, Diisopropylethylamin, Kaliumcarbonat und Natriumhydrogencarbonat, vorzugsweise bei einer Temperatur zwischen -70 °C bis 300 °C in wenigstens eine entsprechende Verbindung der allgemeinen Formel I, ggf. in Form eines entsprechenden Salzes überführt wird, und diese ggf. gereinigt und/oder isoliert wird.

Ein weiterer Gegenstand der vorliegenden Erfindung ist ein Verfahren zur Herstellung von Verbindungen der vorstehend angegebenen allgemeinen Formel I gemäß dem wenigstens eine Verbindung der allgemeinen Formel III, 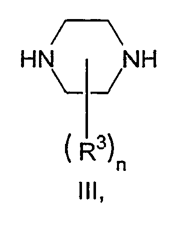 worin R³ und n die vorstehend genannte Bedeutung haben, durch Umsetzung mit wenigstens einer Verbindung der allgemeinen Formel R⁴-C≡C-C(=O)-OH, worin R⁴ die vorstehend genannte Bedeutung hat, in einem Reaktionsmedium, ggf. in Gegenwart wenigstens eines geeigneten Kopplungsmittels, ggf. in Gegenwart wenigstens einer Base, vorzugsweise bei einer Temperatur von - 70 °C bis 100 °C, oder durch Umsetzung mit wenigstens einer Verbindung der allgemeinen Formel R⁴-C≡C-C(=O)-A, worin R⁴ die vorstehend genannte Bedeutung hat und A für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel IX, ggf. in Form eines entsprechenden Salzes überführt wird, 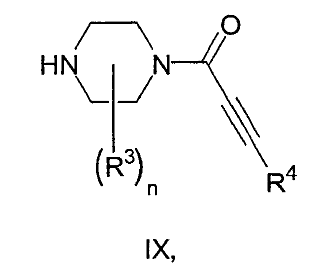 worin R³, R⁴ und n die vorstehend genannte Bedeutung haben, und diese ggf. gereinigt und/oder isoliert wird;
oder wenigstens eine Verbindung der allgemeinen Formel V, 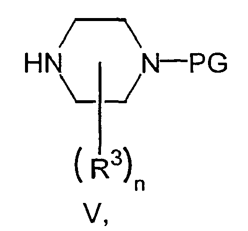 worin R³ und n die vorstehend genannte Bedeutung haben und PG für eine Schutzgruppe, bevorzugt eine Schutzgruppe ausgewählt aus der Gruppe bestehend aus tert-Butyloxy-carbonyl, Benzyl, Benzyloxycarbonyl und 9-Fluorenylmethyloxycarbonyl, steht, durch Umsetzung mit wenigstens einer Verbindung der allgemeinen Formel R⁴-C≡C-C(=O)-OH, worin R⁴ die vorstehend genannte Bedeutung hat, in einem Reaktionsmedium, ggf. in Gegenwart wenigstens eines geeigneten Kopplungsmittels, ggf. in Gegenwart wenigstens einer Base, vorzugsweise bei einer Temperatur von - 70 °C bis 100 °C, oder durch Umsetzung mit wenigstens einer Verbindung der allgemeinen Formel R⁴-C≡C-C(=O)-A, worin R⁴ die vorstehend genannte Bedeutung hat und A für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70°C bis 100°C, in wenigstens eine entsprechende Verbindung der allgemeinen Formel XI, ggf. in Form eines entsprechenden Salzes, überführt wird, 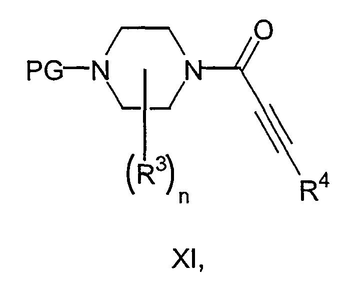 worin R³, R⁴, n und PG die vorstehend genannte Bedeutung haben, und diese ggf. gereinigt und/oder isoliert wird;
und wenigstens eine Verbindung der allgemeinen Formel XI für den Fall, dass PG für eine tert-Butoxycarbonyl- oder 9-Fluorenylmethyloxycarbonyl-Gruppe steht, in einem Reaktionsmedium, in Gegenwart wenigstens einer Säure, vorzugsweise in Gegenwart wenigstens einer Säure ausgewählt aus der Gruppe bestehend aus Salzsäure und Trifluoressigsäure, vorzugsweise bei einer Temperatur zwischen -70 °C bis 100 °C oder für den Fall, dass PG für eine Benzyl-Gruppe oder eine Benzyloxycarbonyl-Gruppe steht, in einem Reaktionsmedium, in Gegenwart von Wasserstoff und in Gegenwart wenigstens eines Katalysators, vorzugsweise in Gegenwart von Palladium auf Kohle, vorzugsweise bei einer Temperatur zwischen - 70 °C bis 100 °C in wenigstens eine entsprechende Verbindung der allgemeinen Formel IX, ggf. in Form eines entsprechenden Salzes überführt wird, worin R¹, R³ und n die vorstehend genannte Bedeutung haben, und diese ggf. gereinigt und/oder isoliert wird;
und wenigstens eine Verbindung der allgemeinen Formel IX durch Umsetzung mit wenigstens einer Verbindung der allgemeinen Formel II, 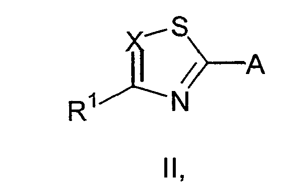 worin die Reste X und R¹ die vorstehend genannte Bedeutung haben und A für eine Abgangsgruppe, vorzugsweise für einen Halogen-Rest oder einen Sulfonsäureester, besonders bevorzugt für einen Chlor- oder Brom-Rest, steht, in einem Reaktionsmedium, ggf. in Gegenwart wenigstens einer Base und/oder wenigstens einer metallorganischen Verbindung und/oder wenigstens eines Metallhydrid-Reagenzes, vorzugsweise bei einer Temperatur von - 70 °C bis 300 °C in wenigstens eine entsprechende Verbindung der allgemeinen Formel I, ggf. in Form eines entsprechenden Salzes überführt wird, und diese ggf. gereinigt und/oder isoliert wird.

Ein erfindungsgemäßes Verfahren zur Herstellung substituierter 1-Propiolyl-piperazine der vorstehend angegebenen allgemeinen Formel I ist auch im folgenden Schema 1 angegeben.

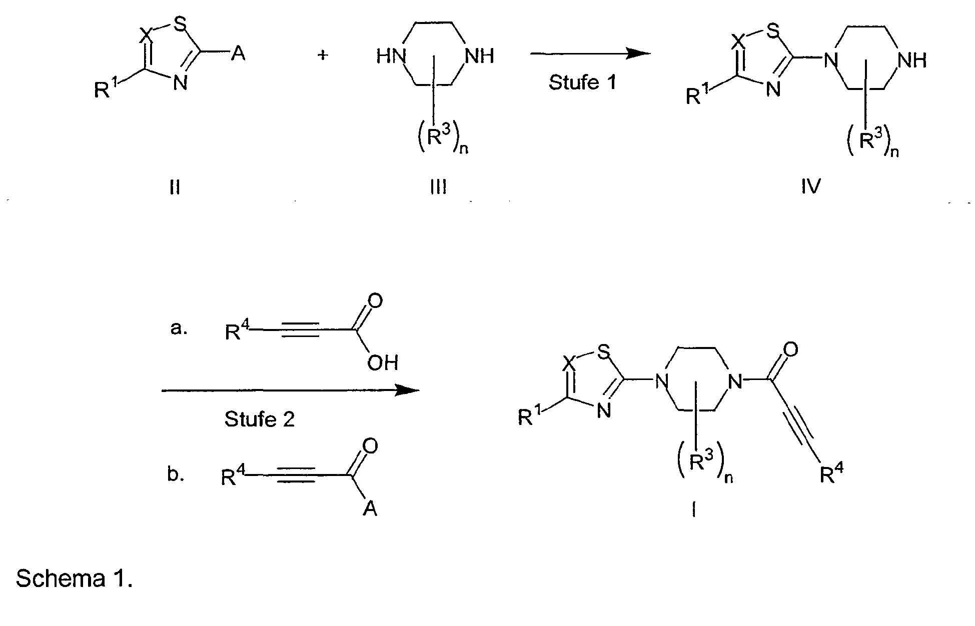

In Stufe 1 werden Thiazole oder Thiadiazole der vorstehend angegebenen allgemeinen Formel II, worin A für eine Abgangsgruppe, vorzugsweise für einen Halogen-Rest oder einen Sulfonsäureester ausgewählt aus der Gruppe bestehend aus Mesylat, Triflat und Tosylat, besonders bevorzugt für ein Chlor- oder Bromatom steht, mit Piperazinen der vorstehend angegebenen allgemeinen Formel III, ggf. in einem Reaktionsmedium, vorzugsweise ausgewählt aus der Gruppe bestehend aus Methanol, Ethanol, Isopropanol, n-Butanol, Diethylether, Tetrahydrofuran, Dichlormethan, Chloroform, Dimethylformamid, Acetonitril, Pyridin, Dioxan, Ethylacetat, Dimethylsulfoxid, Toluol und entsprechenden Mischungen, besonders bevorzugt in einem Reaktionsmedium ausgewählt aus der Gruppe bestehend aus Methanol, Ethanol und n-Butanol, ggf. in Gegenwart einer organischen oder anorganischen Base, vorzugsweise ausgewählt aus der Gruppe bestehend aus Triethylamin, Natriumhydrogencarbonat, Dimethylaminopyridin, Kaliumcarbonat und Natriumhydroxid, und/oder ggf. in Gegenwart einer metallorganischen Verbindung oder eines Metallhydrid-Reagenzes, vorzugsweise ausgewählt aus der Gruppe bestehend aus n-Butyllithium, Phenyllithium, Natriumhydrid, Kaliumhydrid und Natriumamid, vorzugsweise bei Temperaturen von -70°C bis 300 °C, besonders bevorzugt bei Temperaturen von -70°C bis 150 °C, zu Verbindungen der allgemeinen Formel IV umgesetzt.

In Stufe 2 werden Verbindungen der vorstehend angegebenen allgemeinen Formel IV mit Carbonsäuren der vorstehend angegebenen allgemeinen Formel R⁴-C≡C-(C=O)-OH in einem Reaktionsmedium, vorzugsweise ausgewählt aus der Gruppe bestehend aus Diethylether, Tetrahydrofuran, Acetonitril, Methanol, Ethanol, (1,2)-Dichlorethan, Dimethylformamid, Dichlormethan und entsprechenden Mischungen, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), Diisoproylcarbodiimid, 1,1'-Carbonyl-diimidazol (CDI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N`,N`-tetramethyluroniom hexafluorophosphat (HBTU), O-(Benzotriazol-1-yl)-N,N,N`,N`-tetramethyluronium-tetrafluoroborat (TBTU) und 1-Hydroxy-7-azabenzotriazol (HOAt), bevorzugt in Gegenwart von TBTU als Kopplungsreagenz, ggf. in Gegenwart wenigstens einer organischen Base, vorzugsweise ausgewählt aus der Gruppe bestehend aus Triethylamin, Pyridin, Dimethylaminopyridin, N-Methylmorpholin und Diisopropylethylamin, bevorzugt in Gegenwart von Diisopropylethylamin, vorzugsweise bei Temperaturen von -70°C bis 100°C zu Verbindungen der allgemeinen Formel I umgesetzt.

Alternativ werden Verbindungen der vorstehend angegebenen allgemeinen Formel IV mit Carbonsäurederivaten der vorstehend angegebenen allgemeinen Formel R⁴-C≡C-(C=O)-A, worin A für eine Abgangsgruppe, bevorzugt einen Halogen-Rest, besonders bevorzugt Chlor oder Brom, steht, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart einer organischen oder anorganischen Base, vorzugsweise ausgewählt aus der Gruppe bestehend aus Triethylamin, Dimethylaminopyridin, Pyridin und Diisopropylamin, bei Temperaturen von -70°C bis 100°C zu Verbindungen der allgemeinen Formel umgesetzt.

Ein weiteres erfindungsgemäßes Verfahren zur Herstellung substituierter 1-Propiolyl-piperazine der vorstehend angegebenen allgemeinen Formel I ist auch im folgenden Schema 2 angegeben.

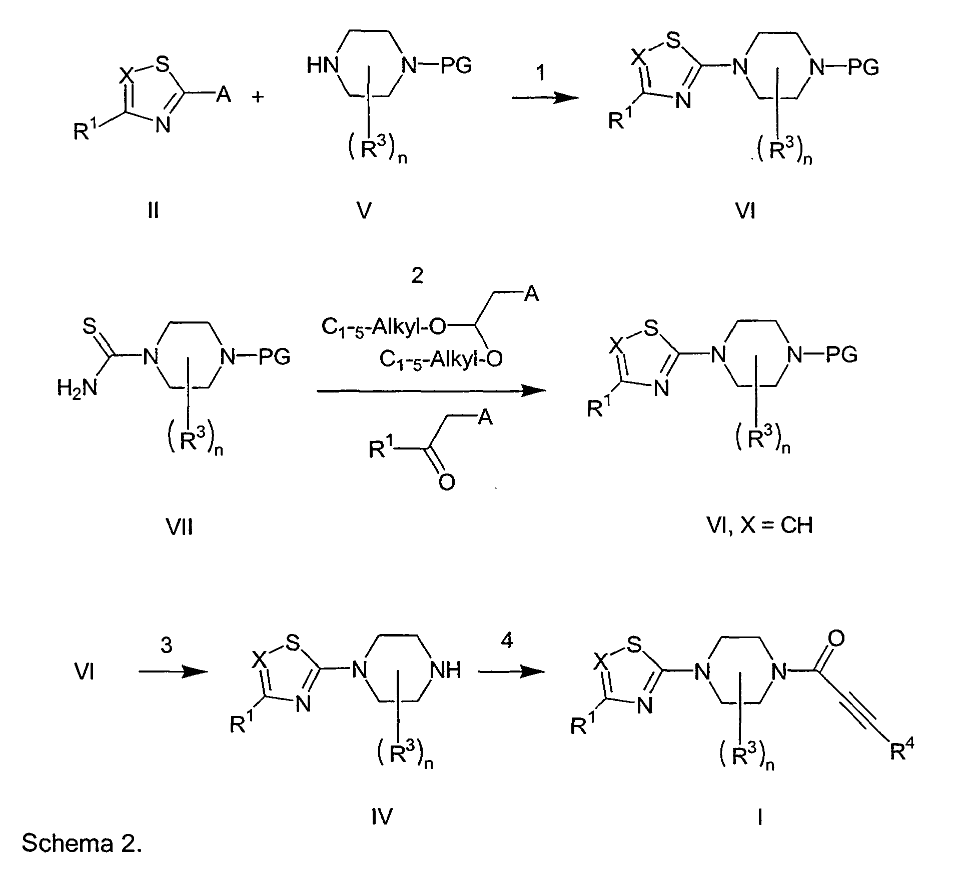

In Stufe 1 werden Thiazole oder Thiadiazole der vorstehend angegebenen allgemeinen Formel II, worin A für eine Abgangsgruppe, vorzugsweise für einen Halogen-Rest oder einen Sulfonsäureester ausgewählt aus der Gruppe bestehend aus Mesylat, Triflat und Tosylat, besonders bevorzugt für ein Chlor- oder Bromatom steht, mit Piperazinen der vorstehend angegebenen allgemeinen Formel V, worin PG für eine Schutzgruppe, bevorzugt für eine Schutzgruppe ausgewählt aus der Gruppe bestehend aus tert-Butyloxy-carbonyl, Benzyloxycarbonyl, Benzyl und 9-Fluorenylmethyloxycarbonyl steht zu Verbindungen der allgemeinen Formel VI umgesetzt.

Genaue Bedingungen lassen sich auch der Veröffentlichung Journal of Medicinal Chemistry 1972, 15(3), Seiten 295 bis 301 entnehmen. Die entsprechenden Teile der Veröffentlichung gelten hiermit als Teil der Offenbarung.

In Stufe 2 werden Verbindungen der vorstehend angegebenen allgemeinen Formel VII, worin PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht, mit wenigstens einer Verbindung der allgemeinen Formel R¹-C(=O)-CH₂-A oder (C₁₋₅-Alkyl-O)₂-CH-CH₂-A, bevorzugt mit wenigstens einer Verbindung der allgemeinen Formel R¹-C(=O)-CH₂-A oder (C₂H₅)₂-CH-CH₂-A, worin A für eine Abgangsgruppe, vorzugsweise für einen Halogen-Rest, besonders bevorzugt für ein Brom-Atom steht, in einem Reaktionsmedium, bevorzugt in einem Reaktionsmedium ausgewählt aus der Gruppe bestehend aus Methanol, Ethylacetat, Ethanol, Isopropanol, n-Butanol, Diethylether, Dioxan, Tetrahydrofuran, Chloroform, Dichlormethan, Dimethylformamid, Acetonitril, Pyridin, Dimethylsulfoxid, Toluol und entsprechenden Mischungen, besonders bevorzugt in Ethanol und/oder Dioxan, ggf. in Gegenwart wenigstens einer organischen Base oder in Gegenwart wenigstens einer Säure, vorzugsweise in Gegenwart wenigstens einer Base ausgewählt aus der Gruppe bestehend aus Triethylamin, Diisopropylethylamin, N-Methylmorpholin, Dimethylaminopyridin und Pyridin oder in Gegenwart wenigstens einer Säure ausgewählt aus der Gruppe bestehend aus Essigsäure, Trifluoressigsäure und Salzsäure, vorzugsweise bei einer Temperatur zwischen - 70 °C bis 300 °C in eine entsprechende Verbindung der allgemeinen Formel VI umgesetzt.

Genaue Bedingungen lassen sich auch der Veröffentlichung Journal of Medicinal Chemistry 1998, 41 (25), Seiten 5037 bis 5054 entnehmen. Die entsprechenden Teile der Veröffentlichung gelten hiermit als Teil der Offenbarung.

In Stufe 3 werden Verbindungen der allgemeinen Formel VI für den Fall, dass PG für eine tert-Butoxycarbonyl- oder 9-Fluorenylmethyloxycarbonyl-Gruppe steht, in einem Reaktionsmedium, vorzugsweise in einem Reaktionsmedium ausgewählt aus der Gruppe bestehend aus Methanol, Ethylacetat, Ethanol, Isopropanol, n-Butanol, Diethylether, Dioxan, Tetrahydrofuran, Chloroform, Dichlormethan, Dimethylformamid, Acetonitril, Pyridin, Dimethylsulfoxid, Toluol und entsprechenden Mischungen, in Gegenwart wenigstens einer Säure, vorzugsweise in Gegenwart wenigstens einer Säure ausgewählt aus der Gruppe bestehend aus Salzsäure und Trifluoressigsäure, vorzugsweise bei einer Temperatur zwischen -70 °C bis 100 °C oder für den Fall, dass PG für eine Benzyl-Gruppe oder Benzyloxycarbonyl-Gruppe steht, in einem Reaktionsmedium, vorzugsweise in einem Reaktionsmedium ausgewählt aus der Gruppe bestehend aus Methanol, Ethylacetat, Ethanol, Isopropanol, n-Butanol, Diethylether, Dioxan, Tetrahydrofuran, Chloroform, Dichlormethan, Dimethylformamid, Acetonitril, Pyridin, Dimethylsulfoxid, Toluol und entsprechenden Mischungen, in Gegenwart von Wasserstoff und in Gegenwart wenigstens eines Katalysators, vorzugsweise in Gegenwart von Palladium auf Kohle, vorzugsweise bei einer Temperatur zwischen -70 °C bis 100 °C in eine entsprechende Verbindung der allgemeinen Formel IV überführt.

Geeignete Methoden zum Entfernen der vorstehend genannten Schutzgruppen lassen sich auch den Monografien Protective Groups in Organic Synthesis, T. W. Greene et al., 3rd edition, 1999, Wiley, New York und Protecting Groups, P. J. Kocienski, 3rd edition, 2004, Georg Thieme Verlag, Stuttgart 2004 entnehmen. Die entsprechenden Teile der Referenzen gelten hiermit als Teil der Offenbarung.

In Stufe 4 werden Verbindungen der vorstehend angegebenen allgemeinen Formel IV mit Carbonsäuren der vorstehend angegebenen allgemeinen Formel R⁴-C≡C-(C=O)-OH oder mit Carbonsäurederivaten der vorstehend angegebenen allgemeinen Formel R⁴-C=C-(C=O)-A wie in Schema 1, Stufe 2, beschrieben zu Verbindungen der allgemeinen Formel I umgesetzt.

Ein weiteres erfindungsgemäßes Verfahren zur Herstellung substituierter 1-Propiolyl-piperazine der vorstehend angegebenen allgemeinen Formel I ist auch im folgenden Schema 3 angegeben.

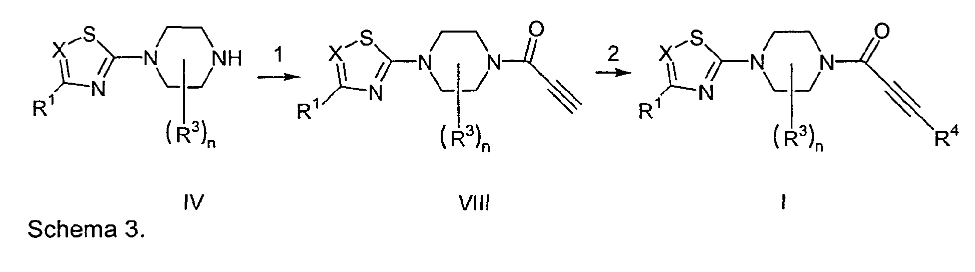

In Stufe 1 werden Verbindungen der vorstehend angegebenen allgemeinen Formel IV mit Propinsäure H-C≡C-(C=O)-OH oder mit Carbonsäurederivaten der allgemeinen Formel H-C≡C-(C=O)-A, worin A für eine Abgangsgruppe, bevorzugt einen Halogen-Rest, besonders bevorzugt Chlor oder Brom, steht, wie in Schema 1, Stufe 2, beschrieben, zu Verbindungen der allgemeinen Formel VIII umgesetzt.

In Stufe 2 werden Verbindungen der vorstehend angegebenen allgemeinen Formel VIII mit Verbindungen der allgemeinen Formel R⁴-A, worin R⁴ die vorstehend genannte Bedeutung mit Ausnahme von Wasserstoff hat und A für eine Abgangsgruppe, vorzugsweise für einen Halogen-Rest oder einen Sulfonsäureester, besonders bevorzugt für Iod, Brom oder Triflat, steht, in einem Reaktionsmedium, bevorzugt in einem Reaktionsmedium ausgewählt aus der Gruppe bestehend aus Methanol, Ethylacetat, Ethanol, Isopropanol, n-Butanol, Diethylether, Dioxan, Tetrahydrofuran, Chloroform, Dichlormethan, Dimethylformamid, Acetonitril, Pyridin, Dimethylsulfoxid, Wasser, Toluol und entsprechenden Mischungen, bevorzugt in Dimethylformamid, Wasser, Ethylacetat, Tetrahydrofuran und entsprechenden Mischungen, ggf. in Gegenwart wenigstens eines Katalysators, bevorzugt in Gegenwart eines Palladium-Katalysators ausgewählt aus der Gruppe bestehend aus Palladiumchlorid [PdCl₂], Palladiumacetat [Pd(OAc)₂], Tetrakistriphenylphosphinpalladium [Pd(PPh₃)₄], Bistriphenylphosphinpalladiumdichlorid [Pd(PPh₃)₂Cl₂] und Bistriphenylphosphinpalladiumacetat [Pd(PPh₃)₂(OAc)₂], bevorzugt in Gegenwart von Pd(PPh₃)₄, Pd(PPh₃)₂Cl₂ und Pd(PPh₃)₂(OAc)₂, ggf. in Gegenwart wenigstens eines Liganden, bevorzugt in Gegenwart wenigstens eines Liganden ausgewählt aus der Gruppe bestehend aus Triphenylphosphin, Triphenylarsin und Tri-2-furyl-phoshpin, bevorzugt in Gegenwart von Triphenylphosphin, ggf. in Gegenwart wenigstens eines anorganischen Salzes, bevorzugt in Gegenwart wenigstens eines anorganischen Salzes ausgewählt aus der Gruppe bestehend aus Lithiumchlorid und Zinkchlorid, ggf. in Gegenwart wenigstens eines Kupfersalzes, bevorzugt in Gegenwart von Kupferiodid, ggf. in Gegenwart wenigstens einer organischen oder anorganischen Base, vorzugsweise in Gegenwart wenigstens einer Base ausgewählt aus der Gruppe bestehend aus Triethylamin, [1,4]-Diazabicyclo-[2.2.2]-octan, Diisopropylamin, Diisopropylethylamin, Kaliumcarbonat und Natriumhydrogencarbonat, vorzugsweise bei einer Temperatur zwischen -70°C bis 300 °C in eine Verbindung der allgemeinen Formel I umgsetzt. Besonders bevorzugt werden Verbindungen der allgemeinen Formel R⁴-I oder R⁴-Br mit Verbindungen der allgemeinen Formel VIII in Dimethylformamid in Gegenwart von Pd(PPh₃)₂Cl₂, Kupfer(I)iodid und Diisopropylamin oder Triethylamin umgesetzt.

Ein weiteres erfindungsgemäßes Verfahren zur Herstellung substituierter 1-Propiolyl-piperazine der vorstehend angegebenen allgemeinen Formel I ist auch im folgenden Schema 4 angegeben

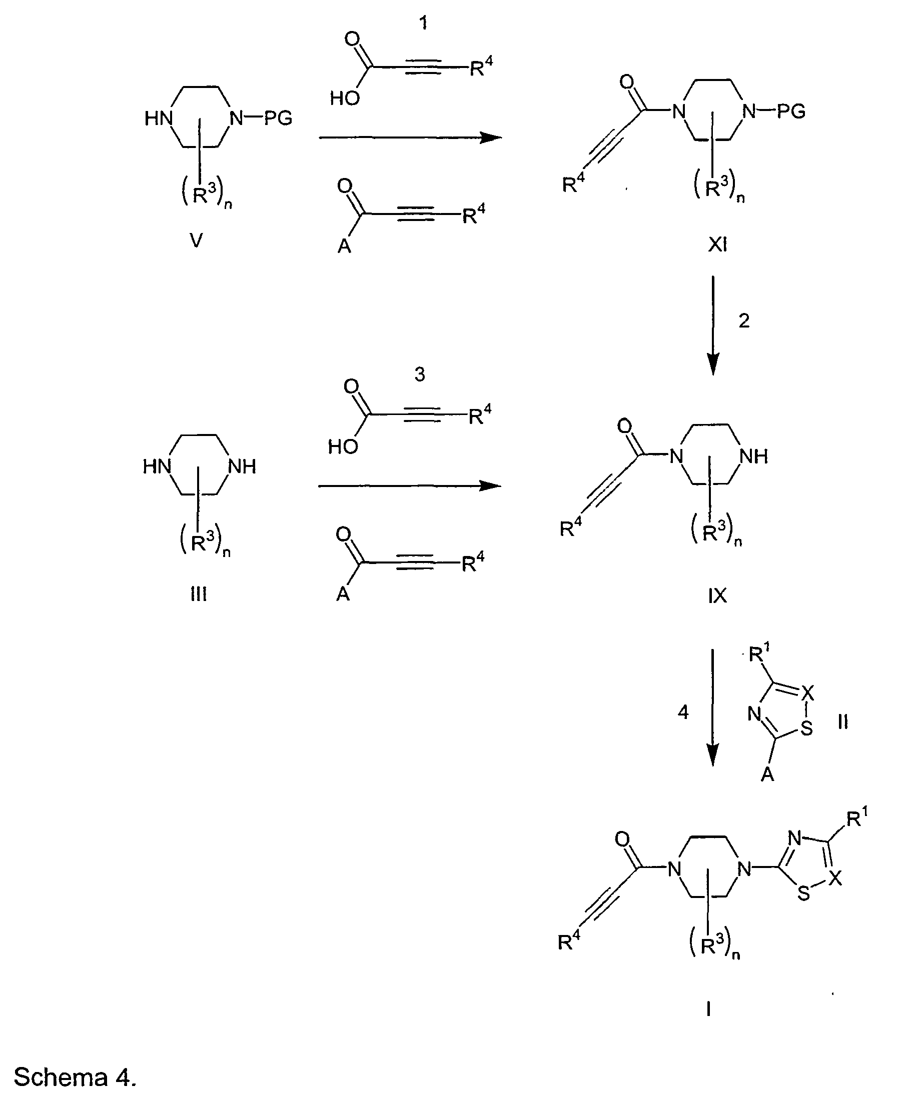

In Stufe 1 werden Verbindungen der allgemeinen Formel V, worin PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht, mit Verbindungen der allgemeinen Formel R⁴-C≡C-(C=O)-OH oder R⁴-C=C-(C=O)-A, worin A für eine Abgangsgruppe, bevorzugt einen Halogen-Rest, besonders bevorzugt Chlor oder Brom, steht, wie in Schema 1, Stufe 2, zu Verbindungen der allgemeinen Formel XI umgesetzt.

In Stufe 2 werden Verbindungen der allgemeinen Formel XI, worin PG für eine Schutzgruppe, bevorzugt für eine Schutzgruppe ausgewählt aus der Gruppe bestehend aus tert-Butyloxy-carbonyl, Benzyl, Benzyloxycarbonyl und 9-Fluorenylmethyloxycarbonyl steht, wie in Schema 2, Stufe 3, beschrieben zu Verbindungen der allgemeinen Formel IX umgesetzt.

In Stufe 3 werden Verbindungen der vorstehend angegebenen allgemeinen Formel III mit Propinsäure H-C≡C-(C=O)-OH oder mit Carbonsäurederivaten der vorstehend angegebenen allgemeinen Formel H-C≡C-(C=O)-A, worin A für eine Abgangsgruppe, bevorzugt einen Halogen-Rest, besonders bevorzugt Chlor oder Brom, steht, wie in Schema 1, Stufe 2, beschrieben, zu Verbindungen der allgemeinen Formel IX umgesetzt.

In Stufe 4 werden Verbindungen der allgemeinen Formel IX mit Verbindungen der allegemeinen Formel II wie in Schema 1, Stufe 1, zu Verbindungen der allgemeinen Formel I umgesetzt.

Die Verbindungen der vorstehend angegebenen Formeln II, III, V und VII, sowie der allgemeinen Formeln R⁴-C≡C-(C=O)-OH, R⁴-C≡C-(C=O)-A und H-C≡C-C(=O)-A sind jeweils am Markt käuflich erhältlich und/oder können nach den üblichen, dem Fachmann bekannten Verfahren hergestellt werden.

Die vorstehend beschriebenen Umsetzungen können jeweils unter üblichen, dem Fachmann geläufigen Bedingungen, beispielsweise in Hinblick auf Druck oder Reihenfolge der Zugabe der Komponenten durchgeführt werden. Ggf. kann die gemäß den jeweiligen Bedingungen optimale Verfahrensführung vom Fachmann durch einfache Vorversuche ermittelt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie.

Sämtliche der vorstehend beschriebenen Verfahrensschritte sowie jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Sofern die erfindungsgemäßen substituierten 1-Propiolyl-piperazine der vorstehend angegebenen allgemeinen Formeln I, Ia, Ib, Ic, Id, Ie, If, Ig, Ih und Ii, im Folgenden nur als erfindungsgemäße substituierte 1-Propiolyl-piperazine der allgemeinen Formel I bezeichnet, nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeits-chromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomere, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten 1-Propiolyl-piperazine der vorstehend angegebenen allgemeinen Formel I sowie ggf. jeweils entsprechende Stereoisomere können nach üblichen, dem Fachmann bekannten Verfahren in Form entsprechender Salze, bevorzugt in Form entsprechender Hydrochloride, insbesondere in Form entsprechender physiologisch verträglicher Salze erhalten werden, erhalten werden, wobei das erfindungsgemäße Arzneimittel eines oder mehrere Salze einer oder mehrerer dieser Verbindungen aufweisen kann.

Die jeweiligen Salze der erfindungsgemäßen substituierten 1-Propiolyl-piperazine der vorstehend angegebenen allgemeinen Formel I sowie entsprechender Stereoisomeren können beispielsweise durch Umsetzung mit einer oder mehreren anorganischen Säuren und/oder einer oder mehreren organischen Säuren erhalten werden. Geeignete Säuren können bevorzugt ausgewählt werden aus der Gruppe bestehend aus Perchlorsäure, Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Sacharinsäure, Cyclohexansulfamidsäure, Aspartam, Monomethylsebacinsäure, 5-Oxo-prolin, Hexan-1-sulfonsäure, Nicotinsäure, 2-Aminobenzoesäure, 3-Aminobenzoesäure oder 4-Aminobenzoesäure, 2,4,6-Trimethylbenzoesäure, α-Liponsäure, Acetylglycin, Hippursäure, Phosphorsäure, Maleinsäure, Malonsäure und Asparaginsäure.

Die erfindungsgemäßen 1-Propiolyl-piperazine der vorstehend angegebenen allgemeinen Formel I sowie ggf. entsprechende Stereoisomere und jeweils deren physiologisch verträgliche Salze können nach üblichen, dem Fachmann bekannten Verfahren auch in Form ihrer Solvate, insbesondere in Form ihrer Hydrate erhalten werden.

Es wurde überraschenderweise gefunden, dass sich die erfindungsgemäßen substituierten 1-Propiolyl-piperazine der vorstehend angegebenen allgemeinen Formel I zur mGluR5-Rezeptor-Regulation eignen und daher insbesondere als pharmazeutische Wirkstoffe in Arzneimitteln zur Prophylaxe und/oder Behandlung von mit diesen Rezeptoren bzw. Prozessen in Verbindung stehenden Störungen oder Erkrankungen eingesetzt werden können.

Die erfindungsgemäßen substituierten 1-Propiolyl-piperazine der vorstehend angegebenen allgemeinen Formel I und ggf. entsprechende Stereoisomere sowie jeweils die entsprechenden Salze und Solvate erscheinen toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens ein erfindungsgemäßes 1-Propiolyl-piperazin der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Das erfindungsgemäße Arzneimittel eignet sich zur mGluR5-Rezeptor-Regulation, insbesondere zur Inhibierung des mGluR5-Rezeptors.

Bevorzugt eignet sich das erfindungsgemäße Arzneimittel zur Prophylaxe und/oder Behandlung von Störungen und/oder Krankheiten, die zumindest teilweise durch mGluR5-Rezeptoren vermittelt werden.

Besonders bevorzugt eignet sich das erfindungsgemäße Arzneimittel daher zur Prophylaxe und/oder Behandlung von Schmerz, vorzugsweise ausgewählt aus der Gruppe bestehend aus akutem Schmerz, chronischem Schmerz, neuropathischem Schmerz und visceralem Schmerz; von Migräne; Depressionen; neurodegenerativen Erkrankungen, vorzugsweise ausgewählt aus der Gruppe bestehend aus Morbus Parkinson, Morbus Alzheimer, Morbus Huntington und Multipler Sklerose; kognitiven Erkrankungen, vorzugsweise kognitiven Mangelzuständen, besonders bevorzugt des Aufmerksamkeit-Defizit-Syndroms (ADS); Panikattacken; Angstzuständen; Epilepsie; Husten; Harninkontinenz; Diarrhöe; Pruritus; Schizophrenie; cerebralen Ischämien; Muskelspasmen; Krämpfen; Störungen der Nahrungsaufnahme, vorzugsweise ausgewählt aus der Gruppe bestehend aus Bulimie, Kachexie, Anorexie und Fettleibigkeit; Alkohol- und/oder Drogen (insbesondere Nikotin- und/oder Kokain-) und/oder Medikamentenmißbrauch; Alkohol- und/oder Drogen (insbesondere Nikotin-und/oder Kokain-) und/oder Medikamentenabhängigkeit vorzugsweise zur Prophylaxe und/oder Verminderung von Entzugserscheinungen bei Alkohol-und/oder Drogen (insbesondere Nikotin- und/oder Kokain-) und/oder Medikamentenabhängigkeit; Entwicklung von Toleranzerscheinungen gegenüber Medikamenten; insbesondere gegenüber Opioiden; des Magen-Ösaphagus-Reflux-Syndroms; zur Diurese; zur Antinatriurese; zur Beeinflussung des kardiovaskulären Systems; zur Anxiolyse; zur Vigilanzsteigerung; zur Libidosteigerung, zur Modulation der Bewegungsaktivität und zur Lokalanästhesie.

Ganz besonders bevorzugt eignet sich das erfindungsgemäße Arzneimittel zur Prophylaxe und/oder Behandlung von akuten Schmerzen, chronischen Schmerzen, neuropathischen Schmerzen oder visceralen Schmerzen; Panikattacken, Angstzuständen, Depressionen; Epilepsie;Morbus Parkinson; Störungen der Nahrungsaufnahme, vorzugsweise ausgewählt aus der Gruppe bestehend aus Bulimie, Kachexie, Anorexie und Fettleibigkeit; Alkohol- und/oder Drogen (insbesondere Nikotin- und/oder Kokain-) und/oder Medikamentenmißbrauch; Alkohol- und/oder Drogen (insbesondere Nikotin- und/oder Kokain-) und/oder Medikamentenabhängigkeit;vorzugsweise zur Prophylaxe und/oder Verminderung von Entzugserscheinungen bei Alkohol- und/oder Drogen (insbesondere Nikotin-und/oder Kokain-) und/oder Medikamentenabhängigkeit; der Entwicklung von Toleranzerscheinungen gegenüber Medikamenten, insbesondere gegenüber Opioiden, oder zur Anxiolyse.

Noch weiter bevorzugt eignet sich das erfindungsgemäße Arzneimittel zur Prophylaxe und/oder Behandlung von Schmerzen, vorzugsweise von akuten Schmerzen, chronischen Schmerzen, neuropathischen Schmerzen oder visceralen Schmerzen, Störungen der Nahrungsaufnahme, vorzugsweise ausgewählt aus der Gruppe bestehend aus Bulimie, Kachexie, Anorexie und Fettleibigkeit; Panikattacken, Angstzuständen oder zur Anxiolyse.

Am weitesten bevorzugt eignet sich das erfindungsgemäße Arzneimittel zur Prophylaxe und/oder Behandlung von Schmerzen, vorzugsweise von akuten Schmerzen, chronischen Schmerzen, neuropathischen Schmerzen oder visceralen Schmerzen, Panikattacken, Angstzuständen oder zur Anxiolyse.

Am allerweitesten bevorzugt eignet sich das erfindungsgemäße Arzneimittel zur Prophylaxe und/oder Behandlung von Schmerzen, vorzugsweise von akuten Schmerzen, chronischen Schmerzen, neuropathischen Schmerzen oder visceralen Schmerzen.

Ein weiterer Gegenstand der vorliegenden Erfindung ist die Verwendung wenigstens eines erfindungsgemäßen substituierten 1-Propiolyl-piperazins der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur mGluR5-Rezeptor-Regulation, vorzugsweise zur Inhibierung des mGluR5-Rezeptors.

Bevorzugt ist die Verwendung wenigstens eines erfindungsgemäßen substituierten 1-Propiolyl-piperazins der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Störungen und/oder Krankheiten, die zumindest teilweise durch mGluR5-Rezeptoren vermittelt werden.

Besonders bevorzugt ist die Verwendung wenigstens eines erfindungsgemäßen substituierten 1-Propiolyl-piperazins der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerz, vorzugsweise ausgewählt aus der Gruppe bestehend aus akutem Schmerz, chronischem Schmerz, neuropathischem Schmerz und visceralem Schmerz, von Migräne, Depressionen, neurodegenerativen Erkrankungen, vorzugsweise ausgewählt aus der Gruppe bestehend aus Morbus Parkinson, Morbus Alzheimer, Morbus Huntington und Multipler Sklerose, kognitiven Erkrankungen, vorzugsweise kognitiven Mangelzuständen, besonders bevorzugt des Aufmerksamkeit-Defizit-Syndroms (ADS), Panikattacken, Angstzuständen, Epilepsie, Husten, Harninkontinenz, Diarrhöe, Pruritus, Schizophrenie, cerebralen Ischämien, Muskelspasmen, Krämpfen, Störungen der Nahrungsaufnahme, vorzugsweise ausgewählt aus der Gruppe bestehend aus Bulimie, Kachexie, Anorexie und Fettleibigkeit, Alkohol- und/oder Drogen (insbesondere Nikotin- und/oder Kokain-) und/oder Medikamentenmißbrauch, Alkohol- und/oder Drogen (insbesondere Nikotin- und/oder Kokain-) und/oder Medikamentenabhängigkeit, vorzugsweise zur Prophylaxe und/oder Verminderung von Entzugserscheinungen bei Alkohol- und/oder Drogen (insbesondere Nikotin- und/oder Kokain-) und/oder Medikamentenabhängigkeit, Entwicklung von Toleranzerscheinungen gegenüber Drogen- und/oder Medikamenten, insbesondere gegenüber Opioiden, des Magen-Ösaphagus-Reflux-Syndroms, zur Diurese, zur Antinatriurese, zur Beeinflussung des kardiovaskulären Systems, zur Anxiolyse, zur Vigilanzsteigerung, zur Libidosteigerung, zur Modulation der Bewegungsaktivität und zur Lokalanästhesie.

Ganz besonders bevorzugt ist die Verwendung wenigstens eines erfindungsgemäßen substituierten 1-Propiolyl-piperazins der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerzen, vorzugsweise von akuten Schmerzen, chronischen Schmerzen, neuropathischen Schmerzen oder visceralen Schmerzen, Störungen der Nahrungsaufnahme, vorzugsweise ausgewählt aus der Gruppe bestehend aus Bulimie, Kachexie, Anorexie und Fettleibigkeit, Panikattacken, Angstzuständen oder zur Anxiolyse.

Noch weiter bevorzugt ist die Verwendung wenigstens eines erfindungsgemäßen substituierten 1-Propiolyl-piperazins der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerzen, vorzugsweise von akuten Schmerzen, chronischen Schmerzen, neuropathischen Schmerzen oder visceralen Schmerzen, Panikattacken, Angstzuständen oder zur Anxiolyse.

Am weitesten bevorzugt ist die Verwendung wenigstens eines erfindungsgemäßen substituierten 1-Propiolyl-piperazins der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerzen, vorzugsweise von akuten Schmerzen, chronischen Schmerzen, neuropathischen Schmerzen oder visceralen Schmerzen.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einem erfindungsgemäßen substituierten 1-Propiolyl-piperazin der vorstehend angegebenen allgemeinen Formel I, ggf. in Form seiner reinen Stereoisomeren, insbesondere Enantiomeren oder Diastereomeren, seines Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden substituierten 1-Propiolyl-piperazine der vorstehend angegebenen allgemeinen Formel I sind in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen substituierten 1-Propiolyl-piperazine der vorstehend angegebenen allgemeinen Formel I auch verzögert freisetzen.

Die Herstellung der erfindungsgemäßen Arzneimittel erfolgt mittels üblichen, aus dem Stande der Technik wohl bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge des jeweiligen substituierten 1-Propiolyl-piperazins der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,005 bis 5000 mg/kg, vorzugsweise 0,05 bis 500 mg/kg, besonders bevorzugt 0,05 bis 100 mg/kg, ganz besonders bevorzugt 0,05 bis 10 mg/kg, Körpergewicht des Patienten wenigstens einer solchen Verbindung appliziert.

### Pharmakologische Methoden:

### I. Methode zur Bestimmung der Hemmung der [³H]-MPEP-Bindung im mGluR5-Rezeptor-Bindungsassay

Schweinehirnhomogenat wird durch Homogenisieren (Polytron PT 3000, Kinematica AG, 10.000 Umdrehungen pro Minute für 90 Sekunden) von Schweinehirnhälften ohne Medulla, Cerebellum und Pons in Puffer pH 8.0 (30mM Hepes, Sigma, Bestellnr.H3375 + 1 Tablette Complete auf 100ml, Roche Diagnostics, Bestellnr. 1836145) im Verhältnis 1:20 (Hirngewicht/Volumen) und Differentialzentrifugation bei 900 x g und 40.000 x g hergestellt. In 250 µl Inkubationsansätzen in 96 Well Mikrotiterplatten werden jeweils 450 µg Protein aus Hirnhomogenat mit 5nM ³[H]-MPEP (Tocris, Bestellnr. R1212) (MPEP = 2-Methyl-6-(3-methoxyphenyl)-ethinylpyridin) und die zu untersuchenden Verbindungen (10 µM im Test) in Puffer (wie oben) bei Raumtemperatur für 60 min inkubiert.

Danach werden die Ansätze mit Hilfe eines Brandel Cell Harvesters (Brandel, TYP Robotic 9600) auf Unifilterplates mit Glasfaserfiltermatten (Perkin Elmer, Bestellnr. 6005177) filtriert und anschließend mit Puffer (wie oben) 3 mal mit je 250 µl pro Probe nach gewaschen. Die Filterplatten werden anschließend für 60 min bei 55°C getrocknet. Anschließend werden pro Well 30 µL Ultima Gold™ Szintillator (Packard BioScience, Bestellnr.6013159) zugegeben und nach 3 Stunden werden die Proben am ß-Counter (Mikrobeta, Perkin Eimer) gemessen. Die unspezifische Bindung wird durch Zugabe von 10 µM MPEP (Tocris, Bestellnr.1212) bestimmt.

### II. Methode zur Bestimmung des Ca²⁺-Influx im mGluR5-Rezeptor-Assay

Eine agonistische und/oder antagonistische Wirkung von Substanzen kann am mGluR5-Rezeptor der Spezies Ratte mit folgendem Assay bestimmt werden. Gemäß diesem Assay wird die intrazelluläre Ca²⁺-Freisetzung nach Aktivierung des mGluR5-Rezeptors mit Hilfe eines Ca²⁺-sensitiven Farbstoffs (Typ Fluo-4, Molecular Probes Europe BV, Leiden Niederlande) in der FlexStation (Molecular Devices, Sunnyvale, USA) quantifiziert.

### Präparation kortikaler Neurone:

Kortikale Neuronen werden unter sterilen Bedingungen aus postnatalen Ratten (P2-6) präpariert. Hierzu wird der Kortex entnommen und direkt in Kollagenase-Lösung (PAA Laboratories GmbH, Cölbe, Deutschland) überführt und 45 Minuten im Heizschüttler (37°C, 300 Umdrehungen pro Minute) inkubiert. Anschließend wird die Kollagenase-Lösung entnommen und das Gewebe mit Kulturmedium versetzt.
Kulturmedium (100 ml):
Neurobasalmedium (Gibco Invitrogen GmbH, Karlsruhe, Deutschland)
2 mM L-Glutamin (Sigma, Taufkirchen, Deutschland)
1 Vol-% Antibiotika/Antimycotika-Lösung (PAA Laboratories GmbH, Cölbe, Deutschland)
15 ng/ml NGF (Gibco Invitrogen GmbH, Karlsruhe, Deutschland)
1 ml B27 Supplement (Gibco Invitrogen GmbH, Karlsruhe, Deutschland)
1 ml ITS Supplement (Sigma, Taufkirchen, Deutschland)

Die Zellen werden durch Resuspendieren vereinzelt und nach Zugabe von 15 ml Neurobasalmedium durch einen 70 µm Filtereinsatz (BD Biosciences, Heidelberg, Deutschland) zentrifugiert. Das resultierende Zellpellet wird in Kulturmedium aufgenommen. Anschließend werden die Zellen auf Poly-D-Lysin-beschichtete, schwarze 96-Loch-Platten mit klarem Boden (BD Biosciences, Heidelberg, Deutschland), die zuvor zusätzlich mit Laminin (2 µg/cm², Gibco Invitrogen GmbH, Karlsruhe, Deutschland) beschichtet wurden, ausplattiert. Die Zelldichte beträgt 15.000 Zellen/Loch. Die Zellen werden bei 37 °C und 5 % CO₂ inkubiert und am 2. oder 3. Tag nach Präparation erfolgt ein Mediumwechsel. Je nach Zellwachstum kann die funktionelle Untersuchung am 3.-7. Tag nach Präparation durchgeführt werden.

### FlexStation-Assay:

Zur funktionellen Untersuchung werden die Zellen mit 2 µM Fluo-4 und 0,01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37°C beladen. Anschließend werden die Platten 2 x mit HBSS-Puffer gewaschen und nach einer weiteren Inkubation von 15 Minuten bei Raumtemperatur zur Ca²⁺-Messung an der FlexStation eingesetzt. Die Ca²⁺-abhängige Fluoreszenz wird dabei vor und nach Zugabe von Substanzen gemessen (λₑₓ = 485 nm, λₑₘ = 525 nm). Die Quantifizierung erfolgt durch die Messung der höchsten Fluoreszenzintensität über die Zeit. Das FlexStation-Protokoll besteht aus 2 Substanzzugaben. Zunächst werden Testsubstanzen in unterschiedlichen Konzentrationen (0,024 µM - 100 µM) auf die Zellen pipettiert. Nach 3 Minuten Inkubation werden 10 µM DHPG ((S)-3,5-Dihydroxyphenylglycin, Tocris Biotrend Chemikalien GmbH, Köln, Deutschland) appliziert und simultan der Einstrom von Ca²⁺ ermittelt. mGluR5-Antagonisten führten zu einer Unterdrückung des Ca²⁺-Einstroms.

Zur Bestimmung des IC₅₀-Wertes wurden die Substanzen in verschiedenen Konzentrationen zugegeben. Es wurden Dreifach-Bestimmungen (n=3) durchgeführt und diese in mindestens 2 unabhängigen Experimenten wiederholt (N=2).

### III. Formalintest an der Ratte:

Der Formalintest (Dubuisson, D. and Dennis, S.G., 1977, Pain, 4,161 -174) stellt ein Modell für den akuten sowie den chronischen Schmerz dar. Durch eine einmalige Formalin-Injektion in die dorsale Seite einer Hinterpfote wird bei freibeweglichen Versuchstieren eine biphasische nozizeptive Reaktion induziert, die durch Beobachtung von drei deutlich voneinander unterscheidbaren Verhaltensmustern erfasst wird. Die Reaktion ist zweiphasisch: Phase 1 = Sofortreaktion (Dauer bis 10 min; Pfotenschütteln, Lecken), Phase 2 = Spätreaktion (nach einer Ruhephase; ebenfalls Pfotenschütteln, Lecken; Dauer bis 60 min). Die 1. Phase reflektiert eine direkte Stimulation der peripheren Nozisensoren mit hohem spinalen nozizeptiven Input bzw. Glutamatfreisetzung (akute Schmerzphase); die 2. Phase reflektiert eine spinale und periphere Hypersensibilisierung (chronische Schmerzphase). In den hier vorgestellten Untersuchungen wurde die chronische Schmerzkomponente (Phase 2) ausgewertet.

Formalin wird mit dem Volumen von 50µl und einer Konzentration von 5 % subkutan in die dorsale Seite der rechten Hinterpfote jedes Tieres appliziert. Die zu testenden Substanzen werden 15 min vor der Formalininjektion intraperitoneal appliziert (i.p.). Die spezifischen Verhaltensänderungen, wie Anheben und Schütteln der Pfote, Gewichtsverlagerungen des Tieres sowie Beiss- und Leckreaktionen werden im Beobachtungszeitraum von 21 bis 27 min nach Formalininjektion beobachtet und registriert. Die Zusammenfassung der verschiedenen Verhaltensweisen erfolgt in der sogenannten Pain-Rate (PR), die, auf die Teilintervalle von 3 min bezogen, die Berechnung einer mittleren Nozizeptionsreaktion darstellt. Die Berechnung der PR erfolgt aufgrund einer numerischen Gewichtung (= jeweils Faktor 1, 2, 3) der beobachteten Verhaltensweisen (entsprechend Verhaltensscore 1, 2, 3) und wird mit folgender Formel berechnet: PR = [(T₀ x 0) + (T₁ x 1) + (T₂ x 2) + (T₃ x 3)] / 180
wobei T₀, T₁, T₂, und T₃ jeweils der Zeit in Sekunden entspricht, in der das Tier die Verhaltensweisen 0, 1, 2 oder 3 zeigt. Die Gruppengröße beträgt 10 Tiere (n=10).

Im folgenden wird die Erfindung anhand einiger Beispiele erläutert. Diese Erläuterungen sind lediglich beispielhaft und schränken den allgemeinen Erfindungsgedanken nicht ein.

### Beispiele:

Die Ausbeuten der hergestellten Verbindungen wurden nicht optimiert.

Alle Temperaturen sind unkorrigiert.

Die Angabe "Äquivalente" bedeutet Stoffmengenäquivalente, "RT" Raumtemperatur, 3 "konz." konzentriert, "d" Tage, "min" Minuten, "h" Stunden, "M" ist eine Konzentrationsangabe in mol/l, "aq." wäßrig, "ges." gesättigt, "Lsg." Lösung, "SC" Säulenchromatographie

Weitere Abkürzungen:
- CDI: 1,1'-Carbonyl-diimidazol
- DCC: Dicyclohexylcarbodiimid
- DIC: N,N'-Diisopropylcarbodiimid
- DCE: Dichlorethan
- DCM: Dichlormethan
- DMF: N,N-Dimethylformamid
- DIPE: Diisopropylether
- DIPEA: Diisopropylethylamin
- EDCI: N-Ethyl-N'-(3-dimethylaminopropyl)-carbodiimid Hydrochlorid
- EE: Essigsäureethylester
- H₂O: Wasser
- HOBt: 1-Hydroxy-benzotriazol
- MeCN: Acetonitril
- MeOH: Methanol
- TEA: Triethylamin
- TBTU: O-(Benzotriazol-1-yl)-N,N,N',N'-tetramethyluronium-tetrafluoroborat
- TMSCI: Trimethylchlorsilan
- THF: Tetrahydrofuran

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den üblichen dem Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Lösungsmitteln, Laufmitteln oder für chromatographische Untersuchungen sind stets in Volumen/Volumen angegeben.

Die Analytik erfolgte durch Massenspektroskopie und/oder NMR.

### Manuelle Synthese:

### Beispiel 1: 1-(3-Phenyl-propiolyl)-4-(thiazol-2-yl)-piperazin

### a) Synthese von 1-Thiazol-2-yl-piperazin [Vorstufe BBB1]

3.61 g (22.0 mmol) 2-Bromthiazol wurden zusammen mit 1.90 g (22.0 mmol) Piperazin in n-Butanol (20 ml) gelöst. Die Reaktionslösung wurde 3 h auf 120 °C erhitzt und weitere 16 h bei RT gerührt. Anschließend wurde der entstandene Niederschlag abfiltriert und das Filtrat im Vakuum eingeengt. Der Rückstand wurde mit DCM aufgenommen und mit einer 1 M aq. Natriumcarbonat-Lösung versetzt. Nach Trennen der Phasen wurde die organische Phase über Magnesiumsulfat getrocknet, filtriert und im Vakuum eingeengt. Mit dem Rückstand wurde eine Säulenchromatographie (MeOH:DIPE 1:1) durchgeführt, wobei 1.06 g (6.3 mmol, 28% der Theorie) 1-Thiazol-2-yl-piperazin erhalten wurden.

### b) Synthese von 1-(3-Phenyl-propiolyl)-4-(thiazol-2-yl)-piperazin

0.818 g (5.60 mmol) Phenylpropiolsäure wurden zusammen mit 0.947 g (5.60 mmol) 1-Thiazol-2-yl-piperazin [BBB1] in DMF (5 ml) gelöst. Nach Zugabe von 0.756 g (5.69 mmol) 1-Hydroxybenzotriazol und 0.706 g (5.60 mmol) DCC wurde die Reaktionslösung 16 h bei RT gerührt. Anschließend wurde mit einer 1 M aq. Natriumcarbonat-Lösung verdünnt und nacheinander mit EE und THF extrahiert. Die vereinigten organischen Phasen wurden über Magnesiumsulfat getrocknet, filtriert und im Vakuum eingeengt. Mit dem Rückstand wurde eine Säulenchromatographie (MeOH:DIPE 1:1) durchgeführt, wobei 1.32 g (4.44 mmol, 79% der Theorie) 1-(3-Phenyl-propiolyl)-4-(thiazol-2-yl)-piperazin erhalten wurden.

Im Folgenden wird die Synthese der nicht kommerziell erhältlichen Ausgangsverbindungen detailliert beschrieben:

### Vorstufe BBB2: Synthese von 4-(Thiazol-2-yl)-1-propiolyl-piperazin

16.9 g (100 mmol) 1-Thiazol-2-yl-piperazin [Vorstufe BBB1], 6.2 ml (100 mmol) Propiolsäure, 17.5 ml (100 mmol) DIPEA und 32.1 g (100 mmol) TBTU wurden in MeCN (1000 ml) gelöst und 3 h bei RT gerührt. Anschließend wurde die Reaktionsmischung im Vakuum eingeengt. Der Rückstand wurde in Chloroform aufgenommen und nacheinander mit einer 10%-igen aq. NaOH-Lsg., Wasser und einer ges. aq. NaCl-Lsg. gewaschen. Die organische Phase wurde über MgSO₄ getrocknet, filtriert und im Vakuum eingeengt. Durch Kristallisation aus EE wurden aus dem Rohprodukt 7.5 g (34 mmol, 34%) 4-(Thiazol-2-yl)-1-propiolyl-piperazin erhalten.

### Vorstufe BBB3: Synthese von 6-Brom-chinolin

Zu einer Mischung aus 24 g (140.0 mmol) 4-Brom-anilin, 8.2 ml (80.0 mmol) Nitrobenzol, 38.7 ml (530.0 mmol) Glycerin, 4.7 g (17 mmol) Eisensulfatheptahydrat und 8.65 g (140 mmol) Borsäure wurden bei- 10 °C 22.5 ml konz. H₂SO₄ gegeben. Anschließend wurde die Reaktionsmischung 10 h unter Rückfluß erhitzt. Nach Einstellen von einem pH-Wert >12 mit einer 50%-igen aq. NaOH-Lsg. wurden aus dem Rückstand 3,69 g (17.7 mmol, 13%) 6-Brom-chinolin durch Wasserdampfdestillation erhalten.

### Vorstufe BBB4: Synthese von 5-Brom-1-methyl-indol

Zu einer Lösung von 420 mg (7.5 mmol) gepulvertem KOH in DMSO (15 ml) wurden nach 15 min Rühren bei RT 980 mg (5.0 mmol) 5-Brom-indol gegeben. Nach weiteren 5 min Rühren bei RT wurden 342 µl (5.5 mmol) lodmethan zugegeben und anschließend wurde 1 h bei RT gerührt. Nach Neutralisation mit 5%-iger aq. Salzsäure wurde die Reaktionsmischung mit EE extrahiert. Die organische Phase wurde nacheinander mit Wasser und einer ges. aq. NaCl-Lsg. gewaschen und über MgSO₄ getrocknet. Nach Filtration und Entfernen des Lösungsmittels im Vakuum wurden 980 mg (4.7 mmol, 93%) 5-Brom-1-methyl-indol erhalten.

### Vorstufe BBB5: Synthese von 3-Methoxymethyl-iodbenzol

Zu einer Lösung von 508 µl (4.0 mmol) 3-lod-benzylalkohol und 1.25 ml (20.0 mmol) Iodmethan in MeCN (20 ml) wurden 829 mg (6.0 mmol) Kaliumcarbonat gegeben. Nach 3 h Rühren bei 30°C wurden 320 mg (8.00 mmol) NaH zugefügt und es wurde weitere 3 h bei RT gerührt. Anschließend wurde die Reaktion mit Wasser versetzt und mit Chloroform extrahiert. Die organische Phase wurde nacheinander mit Wasser und einer ges. aq. NaCl-Lsg. gewaschen und über MgSO₄ getrocknet. Nach Filtration und Entfernen des Lösungsmittels im Vakuum wurden 863 mg (3.5 mmol, 87%) 3-Methoxymethyl-iodbenzol erhalten.

### Vorstufe BBB6: Synthese von (4-Chlor-phenyl)-propiolsäure

Ausgehend von 4-Chlor-benzaldehyd wurde gemäß dem nachfolgend bei Vorstufe BBB7 beschriebenen Verfahren (4-Chlor-phenyl)-propiolsäure erhalten.

### Vorstufe BBB7: (4-Fluor-phenyl)-propiolsäure

### a) Synthese von 3-(4-Fluor-phenyl)-acrylsäureethylester

Aus 11.67 g (507.6 mmol) Natrium wurde in Toluol (160 ml) Natriumsand hergestellt. Nach Abdekantieren des Toluols wurden nacheinander EE (185 ml) und Ethanol (2 ml) vorsichtig zugegeben. Anschließend wurde auf 0 °C abgekühlt und 43.2 ml (402.8 mmol) 4-Fluor-benzaldehyd wurden über einen Zeitraum von 90 min so langsam hinzu getropft, dass die Temperatur der Reaktionsmischung 5 °C nicht überstieg. Danach wurde noch 1 h bei RT gerührt. Nach Zugabe von Essigsäure (40 ml) und Wasser (40 ml) wurden die Phasen getrennt. Die wässrige Phase wurde mit EE extrahiert und die vereinigten organischen Phasen wurde mit einer 6N HCl-Lsg. gewaschen und über MgSO₄ getrocknet. Nach Filtration und Entfernen des Lösungsmittels im Vakuum wurde mit dem Rückstand eine Vakuumdestillation durchgeführt. Dabei wurden 37.0 g (190 mmol, 47%) 3-(4-Fluor-phenyl)-acrylsäureethylester erhalten.

### b) Synthese von 2,3-Dibrom-3-(4-fluor-phenyl)-propionsäureethylester

Zu einer Lösung von 37.0 g (0.19 mol) 3-(4-Fluor-phenyl)-acrylsäureethylester in Tetrachlormethan (23 ml) wurden bei 0 °C 9.72 ml (0.19 mol) Brom getropft. Beim Einengen im Vakuum kristallisierten 46.1 g (0.13 mol, 69%) 2,3-Dibrom-3-(4-fluor-phenyl)-propionsäureethylester aus, die abgesaugt und mit Hexan gewaschen wurden.

### c) Synthese von (4-Fluor-phenyl)-propiolsäure

Unter Erwärmen auf 70 °C wurden 21.9 g (390.7 mmol) KOH in Ethanol (170 ml) gelöst. Nach Abkühlen auf 40 °C wurden 46.1 g (0.13 mmol) 2,3-Dibrom-3-(4-fluor-phenyl)-propionsäureethylester zugegeben. Anschließend wurde 3 h unter Rückfluß erhitzt. Nach Abkühlen auf RT wurde der entstandene Niederschlag abfiltriert. Die Reaktionslösung wurde mit konz. HCl neutralisiert und im Vakuum eingeengt. Der Rückstand wurde zusammen mit dem zuvor abgesaugten Niederschlag in Wasser (100 ml) gelöst. Die Lösung wurde mit 20-%iger Schwefelsäure unter Kühlung (Eisbad) auf pH = 1 eingestellt. Nach 20 min Rühren bei RT wurde der entstandene Niederschlag abgesaugt und mit 2%-iger Schwefelsäure gewaschen. Nach Kristallisation aus MeCN und erneuter Kristallisation des Rückstandes aus Ethanol wurden 1.37 g (8.4 mmol, 6%) (4-Fluor-phenyl)-propiolsäure erhalten.

### Vorstufe BBB8: Tol-2-yl-propiolsäure

### a) Synthese von 2,3-Dibromo-3-tol-2-yl-propionsäure

Zu einer Lösung von 30 g (185.2 mmol) 3-Tol-2-yl-acrylsäure in Tetrachlormethan wurden bei 0 °C 15.2 ml (296.7 mmol) Brom so langsam hinzu getropft, dass die Temperatur der Reaktionsmischung 5 °C nicht überstieg. Die Reaktionslösung wurde dann 2 h unter Rückfluß erhitzt und anschließend 12 h bei RT stehen gelassen. Der entstandene Niederschlag wurde abfiltriert und mit Tetrachlormethan gewaschen,
wobei 51.0 g (158.9 mmol, 86%) 2,3-Dibromo-3-tol-2-yl-propionsäure erhalten wurden.

### b) Synthese von Tol-2-yl-propiolsäure

Zu einer Suspension von 35.5 g KOH in Ethanol (250 ml) wurden 51.0 g (158.9 mmol) 2,3-Dibromo-3-tol-2-yl-propionsäure portionsweise über einen Zeitraum von 30 min gegeben. Anschließend wurde 4 h unter Rückfluß erhitzt. Nach Entfernen des Lösungsmittels im Vakuum wurde der Rückstand in 10%-iger Salzsäure aufgenommen. Die Lösung wurde mit EE gewaschen und anschließend mit einer 6N aq. NaOH-Lsg. auf pH >10 eingestellt. Anschließende wurde mit EE extrahiert und die organische Phase wurde über MgSO₄ getrocknet. Nach Filtration und Entfernen des Lösungsmittels im Vakuum wurden 13.8 g (86.2 mmol, 54%) Tol-2-yl-propiolsäure erhalten.

### Vorstufe BBB9: Synthese von Tol-3-yl-propiolsäure

Ausgehend von 3-Tol-3-yl-acrylsäure wurde gemäß dem vorstehend bei Vorstufe BBB8 beschriebenen Verfahren Tol-3-yl-propiolsäure erhalten.

### Vorstufe BBB10: Synthese von Tol-4-yl-propiolsäure

Ausgehend von 3-Tol-4-yl-acrylsäure wurde gemäß dem vorstehend bei Vorstufe BBB8 beschriebenen Verfahren Tol-4-yl-propiolsäure erhalten.

### Vorstufe BBB11: Synthese von (2-Trifluormethyl-phenyl)-propiolsäure

Ausgehend von 3-(2-Trifluoromethyl-phenyl)-acrylsäure wurde gemäß dem vorstehend bei Vorstufe BBB8 beschriebenen Verfahren (2-Trifluormethyl-phenyl)-propiolsäure erhalten.

### Vorstufe BBB12: Synthese von (3-Trifluormethyl-phenyl)-propiolsäure

Ausgehend von 3-(3-Trifluoromethyl-phenyl)-acrylsäure wurde gemäß dem vorstehend bei Vorstufe BBB8 beschriebenen Verfahren (3-Trifluormethyl-phenyl)-propiolsäure erhalten.

### Vorstufe BBB13: Synthese von (4-Trifluormethyl-phenyl)-propiolsäure

Ausgehend von 3-(4-Trifluoromethyl-phenyl)-acrylsäure wurde gemäß dem vorstehend bei Vorstufe BBB8 beschriebenen Verfahren (4-Trifluormethyl-phenyl)-propiolsäure erhalten.

### Vorstufe BBB14: Synthese von 3-Methyl-1-thiazol-2-yl-piperazin

30.06 g (300.0 mmol) 2-Methylpiperazin wurde mit 4.51 ml (50.0 mmol) 2-Bromthiazol vermischt. Diese Mischung wurde geschmolzen und 5 min unter Rückfluß erhitzt. Nach Abkühlen wurde die Reaktionsmischung in 10%-iger Salzsäure aufgenommen und mit EE gewaschen. Die wässrige Phase wurde mit einer 10%-igen aq. NaOH-Lsg. auf einen pH-Wert > 12 eingestellt und dann mit EE extrahiert. Die organische Phase wurde über MgSO₄ getrocknet und im Vakuum eingeengt. Es wurden 8.26g (45.1 mmol, 90%) 3-Methyl-1-thiazol-2-yl-piperazin erhalten.

### Vorstufe BBB15: Synthese von (S)-3-Methyl-1-thiazol-2-yl-piperazin

Durch die Umsetzung von (S)-2-Methylpiperazin mit 2-Bromthiazol gemäß den vorstehend bei Vorstufe BBB14 beschriebenen Bedingungen wurde (S)-3-Methyl-1-thiazol-2-yl-piperazin erhalten.

### Vorstufe BBB16: Synthese von (R)-3-Methyl-1-thiazol-2-yl-piperazin

Durch die Umsetzung von 2-Methylpiperazin mit 2-Bromthiazol gemäß den vorstehend bei Vorstufe BBB14 beschriebenen Bedingungen wurde (R)-3-Methyl-1-thiazol-2-yl-piperazin erhalten.

### Vorstufe BBB17: Cyclohexyl-propiolsäure

### a) Synthese von 3-Cyclohexyl-3-oxo-2-(triphenyl-λ5-phosphanyliden)-propionsäureethylester

Zu einer Lösung von 4.74 g (13.6 mmol) (Triphenyl-λ5-phosphanyliden)-essigsäureethylester und 1.9 ml (13.6 mmol) TEA in Toluol (75 ml) wurden 2.0 g (13.6 mmol) Cyclohexylcarbonsäurechlorid gegeben. Nach 2 h Rühren bei RT wurde die Reaktionslösung nacheinander mit Wasser und einer ges. aq. NaCl-Lösung gewaschen. Die organische Phase wurde über MgSO₄ getrocknet, filtriert und im Vakuum eingeengt. Aus dem Rückstand wurden 2.85 g (6.2 mmol, 46%) 3-Cyclohexyl-3-oxo-2-(triphenyl-λ5-phosphanyliden)-propionsäureethylester aus Ethanol kristallisiert.

### b) Synthese von Cyclohexyl-propiolsäure

2.0 g (4.36 mmol) 3-Cyclohexyl-3-oxo-2-(triphenyl-λ5-phosphanyliden)-propionsäureethylester wurden zusammen mit ca. 15 g Glassplitter in ein Pyrex-Rohr gefüllt, das anschließend mit einer Kühlfalle verbunden wurde. Der oberer Teil des Pyrex-Rohrs wurde mit einer offenen Gasflamme solange erhitzt, bis das gesamte Ausgangsmaterial in die Kühlfalle überdestilliert war. Das entstandene, überdestillierte Gemisch wurde mit Ethanol (25 ml) aufgenommen und mit 2 ml einer 50%-igen aq. KOH-Lsg. versetzt. Anschließend wurde 16 h bei RT gerührt. Dann wurde das Lösungsmittel im Vakuum entfernt und der Rückstand mit Wasser aufgenommen. Der ausgefallene Niederschlag wurde abgesaugt und das Filtrat mit Salzsäure auf einen pH-Wert < 2 eingestellt und mit Chloroform extrahiert. Die organische Phase wurde nacheinander mit Wasser und einer ges. aq. NaCl-Lsg. gewaschen und über M_{g}SO₄ getrocknet. Nach Filtration und Entfernen des Lösungsmittels im Vakuum wurden aus dem Rückstand 500 mg (3.3 mmol, 75%) Cyclohexyl-propiolsäure aus Diethylether kristallisiert.

### Vorstufe BBB18: Synthese von Trifluor-methansulfonsäurechinolin-7-yl ester

Zu einer Lösung von 2.9 g (20.0 mmol) 7-Hydroxy-chinolin und 4.45 ml (32.0 mmol) Triethylamin in DCE (200 ml) wurde eine Lösung von 4.71 ml (28.0 mmol) Trifluormethansulfonsäureanhydrid in DCE (40 ml) getropft. Anschließend wurde über Nacht bei RT gerührt. Die Lösung wurde dann nacheinander mit Wasser und einer ges. aq. NaCl-Lsg. gewaschen und über M_{g}SO₄ getrocknet. Nach Filtration und Einengen im Vakuum wurde mit dem Rückstand eine SC (DCE) durchgeführt, wobei 3.86 g (13.9 mmol, 70%) Trifluor-methansulfonsäurechinolin-7-yl ester erhalten wurden.

### Vorstufe BBB19: Synthese von 3-Ethyl-1-thiazol-2-yl-piperazin

Durch die Umsetzung von 2-Ethyl-piperazin mit 2-Bromthiazol gemäß den vorstehend bei Vorstufe BBB14 beschriebenen Bedingungen wurde 3-Ethyl-1-thiazol-2-yl-piperazin erhalten.

### Vorstufe BBB20: Synthese von 3-Phenyl-1-thiazol-2-yl-piperazin

Durch die Umsetzung von 2-Phenyl-piperazin mit 2-Bromthiazol gemäß den vorstehend bei Vorstufe BBB1 beschriebenen Bedingungen wurde 3-Phenyl-1-thiazol-2-yl-piperazin erhalten.

### Vorstufe BBB21: Synthese von Furan-2-yl-propiolsäure

Ausgehend von Furan-2-carbonsäurechlorid wurde gemäß den vorstehend bei Vorstufe BBB17 beschriebenen Bedingungen Furan-2-yl-propiolsäure erhalten.

### Vorstufe BBB22: Synthese von Furan-3-yl-propiolsäure

Ausgehend von Furan-3-carbonsäurechlorid wurde gemäß den vorstehend bei Vorstufe BBB17 beschriebenen Bedingungen Furan-3-yl-propiolsäure erhalten.

### Vorstufe BBB23: Synthese von cis-3,5-Dimethyl-1-thiazol-2-yl-piperazin

Durch die Umsetzung von cis-2,6-Dimethylpiperazin mit 2-Bromthiazol unter de vorstehend bei Vorstufe BBB14 beschriebenen Bedingungen wurde cis-3,5-Dimethyl-1-thiazol-2-yl-piperazin erhalten.

### Vorstufe BBB24: Synthese von 2-Piperazin-1-yl-thiazol-5-carbonsäureethylester

1.02 g (5.0 mmol) 2-Brom-thiazol-5-carbonsäureethylester und 4.31 g (50 mmol) Piperazin wurden in Ethanol (20 ml) gelöst und 4 h unter Rückfluß erhitzt. Danach wurde die Lösung im Vakuum eingeengt, in EE aufgenommen und nacheinander mit Wasser und einer ges. aq. NaCl-Lsg. gewaschen. Nach Trocknen der organischen Phase über MgSO₄, Filtration und Entfernen des Lösungsmittels im Vakuum wurden 1.12 g (4.6 mmol, 93%) 2-Piperazin-1-yl-thiazol-5-carbonsäureethylester erhalten.

### Vorstufe BBB25: Synthese von 2-Piperazin-1-yl-thiazol-4-carbonsäure ethylester

Durch die Umsetzung von 2-Brom-thiazol-4-carbonsäureethylester mit Piperazin gemäß den vorstehend bei Vorstufe BBB24 beschriebenen Bedingungen wurde 2-Piperazin-1-yl-thiazol-4-carbonsäureethylester erhalten.

### Vorstufe BBB27: Synthese von 1-[3-(4-Fluor-benzyl)-[1,2,4]-thiadiazol-5-yl]-piperazin

Durch die Umsetzung von Piperazin mit 5-Chlor-3-(4-fluor-benzyl)-[1,2,4]-thiadiazol gemäß den vorstehend bei Vorstufe BBB1 beschriebenen Bedingungen wurde 1-[3-(4-Fluor-benzyl)-[1,2,4]-thiadiazol-5-yl]-piperazin erhalten.

### Vorstufe BBB28: Synthese von (2,4-Difluor-phenyl)-propiolsäure

4.95 ml (41.4 mmol) 2,4-Difluor-iodbenzol und 2.56 ml (41.4 mmol) Propiolsäure wurden in DMF (16 ml) gelöst. Nach Abkühlen der Reaktionsmischung auf 0 °C (EisWasser-Bad) wurden 578 mg (0.83 mmol) Pd(PPh₃)₂Cl₂ und 308 mg (1.66 mmol) Cul zugegeben. Die Reaktionslösung wurde dann auf-10°C (Eis-Methanol-Bad) abgekühlt und bei dieser Temperatur wurden 14.5 ml (103.4 mmol) Diisopropylamin hinzu getropft. Anschließend wurde das Kühlbad entfernt und die Reaktion wurde nach Erreichen von RT noch 16 h gerührt. Die Reaktionslösung wurde dann mit EE verdünnt und nacheinander mit einer 2N HCl-Lsg. und einer ges. aq.NaCl-Lsg. gewaschen. Nach Trocknen über MgSO₄, Filtration und Entfernen des Lösungsmittels im Vakuum wurde der Rückstand mit Hexan aufgekocht und nach Abkühlen auf RT filtriert. Der Rückstand wurde bei 30 °C in Diethylether gelöst und bei dieser Temperatur filtriert. Durch Entfernen der Lösungsmittel im Vakuum wurden aus dem Filtrat 6.43 g (35.3 mmol, 85%) (2,4-Difluor-phenyl)-propiolsäure erhalten.

### Vorstufe BBB29: Synthese von 1-(5-Nitro-thiazol-2-yl)-piperazin

Durch die Umsetzung von 2-Brom-5-nitro-thiazol mit Piperazin gemäß den vorstehend bei Vorstufe BBB24 beschriebenen Bedingungen wurde 1-(5-Nitrothiazol-2-yl)-piperazin erhalten.

### Vorstufe BBB30: Synthese von 2-Brom-4-tert-butyl-thiazol

85%-ige Phosphorsäure (50 ml) und 65%-ige Salpetersäure (20 ml) wurden bei 0 °C miteinander vermischt. Zu dieser Lösung wurden 3.12 g (20.0 mmol) 4-tert-Butylthiazol-2-ylamin gegeben und anschließend wurde bei 0 °C eine Lösung von 1.38 g (20 mmol) NaNO₂ in Wasser (10 ml) innerhalb von 30 min hinzu getropft. Die Reaktionslösung wurde 1 h bei 0 °C gerührt und dann in eine auf 0 °C gekühlte Suspension aus 20.0 g NaBr und 5.8 g CuBr in Wasser (20 ml) getropft. Die Mischung wurde so lange gerührt, bis keine Gasentwicklung mehr zu erkennen war. Anschließend wurde mit einer 50%-igen aq. KOH Lösung auf einen pH-Wert > 10 eingestellt und das Produkt durch Wasserdampfdestillation abgetrennt. Das Destillat wurde mit EE extrahiert. Die organische Phase wurde über MgSO₄ getrocknet, filtriert und im Vakuum eingeengt. Als Rückstand wurden 3.03 g (13.8 mmol, 69%) 2-Brom-4-tert-butyl-thiazol erhalten.

### Vorstufe BBB31: Synthese von 1-(4-tert-Butyl-thiazol-2-yl)-piperazin

Durch die Umsetzung von 2-Brom-4-tert-butyl-thiazol [Vorstufe BBB30] mit Piperazin gemäß den vorstehend bei Vorstufe BBB1 beschriebenen Bedingungen wurde 1-(4-tert-Butyl-thiazol-2-yl)-piperazin erhalten.

### Vorstufe BBB32: Synthese von 2-Brom-5-methyl-thiazol

Ausgehend von 5-Methyl-thiazol-2-ylamin wurde nach dem vorstehend unter Vorstufe BBB30 beschriebenen Verfahren 2-Brom-5-methyl-thiazol erhalten.

### Vorstufe BBB33: Synthese von 1-(5-Methyl-thiazol-2-yl)-piperazin

Durch die Umsetzung von 2-Brom-5-methyl-thiazol [Vorstufe BBB32] mit Piperazin gemäß den vorstehend bei Vorstufe BBB1 beschriebenen Bedingungen wurde 1-(5-Methyl-thiazol-2-yl)-piperazin erhalten.

### Vorstufe BBB34: Synthese von 2-Brom-4,5-dimethyl-thiazol

Ausgehend von 4,5-Dimethyl-thiazol-2-ylamin wurde nach dem vorstehend unter Vorstufe BBB30 beschriebenen Verfahren 2-Brom-4,5-dimethyl-thiazol erhalten.

### Vorstufe BBB35: Synthese von 1-(4,5-Dimethyl-thiazol-2-yl)-piperazin

Durch die Umsetzung von 2-Brom-4,5-dimethyl-thiazol [Vorstufe BBB34] mit Piperazin gemäß den vorstehend bei Vorstufe BBB1 beschriebenen Bedingungen wurde 1-(4,5-Dimethyl-thiazol-2-yl)-piperazin erhalten.

### Vorstufe BBB36: Synthese von 2,5-Dibrom-4-phenyl-thiazol

Ausgehend von 4-Phenyl-thiazol-2-ylamin wurde nach dem vorstehend unter Vorstufe BBB30 beschriebenen Verfahren 2,5-Dibrom-4-phenyl-thiazol erhalten.

### Vorstufe BBB37: Synthese von 1-(5-Brom-4-phenyl-thiazol-2-yl)-piperazin

Durch die Umsetzung von 2,5-Dibrom-4-phenyl-thiazol [Vorstufe BBB36] mit Piperazin gemäß den vorstehend bei Vorstufe BBB1 beschriebenen Bedingungen wurde 1-(5-Brom-4-phenyl-thiazol-2-yl)-piperazin erhalten.

### Vorstufe BBB38: Synthese von 2,5-Dibrom-4-methyl-thiazol

Ausgehend von 4-Methyl-thiazol-2-ylamin wurde nach dem vorstehend unter Vorstufe BBB30 beschriebenen Verfahren 2,5-Dibrom-4-methyl-thiazol erhalten.

### Vorstufe BBB39: Synthese von 1-(5-Brom-4-methyl-thiazol-2-yl)-piperazin

Durch die Umsetzung von 2,5-Dibrom-4-methyl-thiazol [Vorstufe BBB38] mit Piperazin gemäß den vorstehend bei Vorstufe BBB1 beschriebenen Bedingungen wurde 1-(5-Brom-4-methyl-thiazol-2-yl)-piperazin erhalten.

### Vorstufe BBB40: Synthese von 2-Chlor-4-methyl-thiazol

Zu einer Lösung von 2.28 g (20.0 mmol) 4-Methyl-thiazol-2-ylamin und 2.68 g (20.0 mmol) CuCl₂ in MeCN (200 ml) wurden bei 0 °C 4.02 ml (30.0 mmol) Isoamylnitrit gegeben. Anschließend wurde die Reaktionslösung 16 h bei RT gerührt und im Vakuum eingeengt. Der Rückstand wurde mit Chloroform aufgenommen und durch Kieselgur filtriert. Das Filtrat wurde im Vakuum eingeengt. Durch SC des Rückstands mit einem DCE-Ethanol Gemisch wurden 1.27 g (9.6 mmol, 48%) 2-Chlor-4-methylthiazol erhalten.

### Vorstufe BBB41: Synthese von 1-(4-Methyl-thiazol-2-yl)-piperazin

Durch die Umsetzung von 2-Chlor-4-methyl-thiazol [Vorstufe BBB40] mit Piperazin gemäß den vorstehend bei Vorstufe BBB1 beschriebenen Bedingungen wurde 1-(4-Methyl-thiazol-2-yl)-piperazin erhalten.

### Vorstufe BBB42: Synthese von 2-Chlor-4-trifluormethyl-thiazol

Ausgehend von 4-Trifluormethyl-thiazol-2-ylamin wurde nach dem vorstehend unter Vorstufe BBB40 beschriebenen Verfahren 2-Chlor-4-trifluormethyl-thiazol erhalten.

### Vorstufe BBB43: Synthese von 1-(4-Trifluormethyl-thiazol-2-yl)-piperazin

Durch die Umsetzung von 2-Chlor-4-trifluormethyl-thiazol [Vorstufe BBB42] mit Piperazin gemäß den vorstehend bei Vorstufe BBB1 beschriebenen Bedingungen wurde 1-(4-Trifluormethyl-thiazol-2-yl)-piperazin erhalten.

### Vorstufe BBB44: Synthese von 2,4-Dichlor-thiazol

Zu 13.3 ml (145.0 mmol) POCl₃ wurden unter Kühlen (Eisbad) 1.67 ml (21.0 mmol) Pyridin gegeben. Anschließend wurden 2.5 g (21.0 mmol) Thiazolidin-2,4-dion hinzu gegeben und die Reaktionslösung wurde 5 h unter Rückfluß erhitzt. Nach Einengen im Vakuum wurde der Rückstand in DCE (20 ml) aufgenommen und anschließend erneut im Vakuum eingeengt. Der Rückstand wurde auf Eis gegossen und mit Ether extrahiert. Die organische Phase wurde nacheinander mit einer 5%-igen aq. NaOH-Lsg. und einer ges. aq. Nacl-Lsg gewaschen und über MgSO₄ getrocknet. Nach Filtration und Einengen im Vakuum wurden 1.41 g 2,4-Dichlor-thiazol erhalten, das ohne weitere Aufreinigung für weitere Reaktion eingesetzt wurde.

### Vorstufe BBB45: Synthese von 1-(4-Chlor-thiazol-2-yl)-piperazin

Durch die Umsetzung von 2,4-Dichlor-thiazol mit Piperazin gemäß den vorstehend bei Vorstufe BBB24 beschriebenen Bedingungen wurde 1-(4-Chlor-thiazol-2-yl)-piperazin erhalten.

### Vorstufe BBB46: Synthese von 2-Brom-5-chloro-thiazol

Ausgehend von 5-Chlor-thiazol-2-ylamin wurde nach dem vorstehend unter Vorstufe BBB30 beschriebenen Verfahren 2-Bromo-5-chloro-thiazol erhalten.

### Vorstufe BBB47: Synthese von 1-(5-Chlor-thiazol-2-yl)-piperazin

Durch die Umsetzung von 2-Bromo-5-chloro-thiazol mit Piperazin gemäß den vorstehend bei Vorstufe BBB1 beschriebenen Bedingungen wurde 1-(5-Chlor-thiazol-2-yl)-piperazin erhalten.

### Vorstufe BBB48: Synthese von 1-(5-Brom-thiazol-2-yt)-piperazin

Durch die Umsetzung von 2,5-Dibrom-thiazol mit Piperazin gemäß den vorstehend bei Vorstufe BBB1 beschriebenen Bedingungen wurde 1-(5-Brom-thiazol-2-yl)-piperazin erhalten.

### Vorstufe BBB49: Synthese von 1-(4-Brom-thiazol-2-yl)-piperazin

Durch die Umsetzung von 2,4-Dibrom-thiazol mit Piperazin gemäß den vorstehend bei Vorstufe BBB1 beschriebenen Bedingungen wurde 1-(4-Brom-thiazol-2-yl)-piperazin erhalten.

### Vorstufe BBB50: Synthese von 1-(3-Methylsulfanyl-[1,2,4]-thiadiazol-5-yl)-piperazin

Durch die Umsetzung von 5-Chlor-3-methylsulfanyl-[1,2,4]-thiadiazol mit Piperazin gemäß den vorstehend bei Vorstufe BBB24 beschriebenen Bedingungen wurde 1-(3-Methyl-sulfanyl-[1,2,4]-thiadiazol-5-yl)-piperazin erhalten.

### Vorstufe BBB51: Synthese von 1-(3-Methansulfonyl-[1,2,4]-thiadiazol-5-yl)-piperazin

Durch die Umsetzung von 5-Chlor-3-methansulfonyl-[1,2,4]-thiadiazol mit Piperazin gemäß den vorstehend bei Vorstufe BBB24 beschriebenen Bedingungen wurde 1-(3-Methan-sulfonyl-[1,2,4]-thiadiazol-5-yl)-piperazin erhalten.

### Vorstufe BBB52: Synthese von 1-(3-Methoxy-[1,2,4]-thiadiazol-5-yl)-piperazin

100 mg (4.0 mmol) Natrium wurde in Ethanol (20 ml) gegeben. Nach vollständiger Reaktion des Natriums wurden 500 mg (2.0 mmol) 1-(3-Methan-sulfonyl-[1,2,4]-thiadiazol-5-yl)-piperazin zugegeben und anschließend wurde 2 h bei 50 °C gerührt. Die abgekühlte Reaktionslösung wurde in eine auf 0 °C abgekühlte ges. aq. NaCl-Lsg. gegossen. Dieses Gemisch wurde mit EE extrahiert. Die organische Phase wurde über MgSO₄ getrocknet, filtriert und im Vakuum eingeengt. Dabei wurden 310 mg (1.54 mmol, 77%) 1-(3-Methoxy-[1,2,4]-thiadiazol-5-yl)-piperazin erhalten.

### Vorstufe BBB53: Synthese von 5-Chlor-[1,2,4]-thiadiazol

Zu einer Lösung von 20.1 g (0.25 mol) Formamidin Hydrochlorid in DCM (250 ml) wurden 41.8 g (0.225 mol) Perchlormethylchlormercaptan gegeben. Nach Abkühlen auf - 14 °C wurde eine Lösung von 50 g NaOH in 75 ml Wasser so zugegeben, dass die Temperatur der Reaktionsmischung -5 °C Grad nicht überstieg. Es wurde 1 h weiter gerührt, wobei sich die Reaktionslösung auf RT erwärmt hat. Der entstandene Niederschlag wurde abfiltriert und mit DCM gewaschen. Die wässrige Phase wurde vom Filtrat abgetrennt und die organische Phase wurde über Na₂SO₄ getrocknet. Nach Filtration und Einengen im Vakuum wurde mit dem Rückstand eine Vakuumdestillation (Vigreux-Kolonne) durchgeführt. Es wurden 1.7 g (15 mmol, 6%) 5-Chlor-[1,2,4]-thiadiazol erhalten.

### Vorstufe BBB54: Synthese von 5-Chlor-3-methyl-[1,2,4]-thiadiazol

Ausgehend von Acetamidin Hydrochlorid wurde nach dem vorstehend unter Vorstufe BBB53 beschriebenen Verfahren 5-Chlor-3-methyl-[1,2,4]-thiadiazol erhalten.

### Vorstufe BBB55: Synthese von 5-Chlor-3-trichlormethyl-[1,2,4]-thiadiazol

Ausgehend von Trichlor-acetamidin Hydrochlorid wurde nach dem vorstehend unter Vorstufe BBB53 beschriebenen Verfahren 5-Chlor-3-trichlormethyl-[1,2,4]-thiadiazol erhalten.

### Vorstufe BBB56: Synthese von 5-Chlor-3-trifluormethyl-[1,2,4]-thiadiazol

In eine Mischung aus 103.4 g wasserfreiem SbF₃ und 18.2 g SbCl₅ wurde bei 150 °C Chlorgas eingeleitet, bis die Gewichtszunahme 2.4 g betrug. Anschließend wurden bei der gleichen Temperatur 40.0 g (168.1 mmol) 5-Chlor-3-trichlormethyl-[1,2,4]-thiadiazol über einen Zeitraum von 30 min hinzu getropft. Danach wurde noch 4 h unter Rückfluß erhitzt. Nach Einengen im Vakuum wurde mit dem Rückstand eine Vakuumdestillation (Vigreux-Kolonne) durchgeführt, wobei 22.4 g (118.8 mmol, 71%) 5-Chlor-3-trifluormethyl-[1,2,4]-thiadiazol erhalten wurden.

### Vorstufe BBB57: Synthese von 1-[1,2,4]-thiadiazol-5-yl-piperazin

Durch die Umsetzung von 5-Chlor-[1,2,4]-thiadiazol [Vorstufe BBB53] mit Piperazin gemäß den vorstehend bei Vorstufe BBB24 beschriebenen Bedingungen wurde 1-[1,2,4]-thiadiazol-5-yl-piperazin erhalten.

### Vorstufe BBB58: Synthese von 1-(3-Methyl-[1,2,4]-thiadiazol-5-yl)-piperazin

Durch die Umsetzung von 5-Chlor-3-methyl-[1,2,4]-thiadiazol [Vorstufe BBB54] mit Piperazin gemäß den vorstehend bei Vorstufe BBB24 beschriebenen Bedingungen wurde 1-(3-Methyl-[1,2,4]-thiadiazol-5-yl)-piperazin erhalten.

### Vorstufe BBB59: Synthese von 1-(3-Trifluormethyl-[1,2,4]-thiadiazol-5-yl)-piperazin

Durch die Umsetzung von 5-Chlor-3-trifluormethyl-[1,2,4]-thiadiazol [Vorstufe BBB56] mit Piperazin gemäß den vorstehend bei Vorstufe BBB24 beschriebenen Bedingungen wurde 1-(3-Trifluormethyl-[1,2,4]-thiadiazol-5-yl)-piperazin erhalten.

### Vorstufe BBB60: Synthese von 4-(5-Ethoxycarbonyl-thiazol-2-yl)-piperazin-1-carbonsäure tert.-butyl-ester

Zu einer Lösung von 3.69 g (16.0 mmol) 2-Piperazin-1-yl-thiazol-5-carbonsäureethylester [Vorstufe BBB24] in MeOH (50 ml) wurde bei 10 °C eine Lösung von 3.65 g (16.7 mmol) Pyrokohlensäure-di-tert.-butylester in MeOH (25 ml) gegeben. Anschießend wurde die Reaktionslösung 2 h bei 40 °C gerührt. Nach Entfernen des Lösungsmittels im Vakuum wurden aus dem Rückstand 5.33 g (15.6 mmol, 98%) 4-(5-Ethoxycarbonyl-thiazol-2-yl)-piperazin-1-carbonsäure tert.-butylester aus Wasser kristallisiert.

### Vorstufe BBB61: Synthese von 4-(5-Carboxy-thiazol-2-yl)-piperazin-1-carbonsäure tert.-butylester

5.33 g (15.6 mmol) 4-(5-Ethoxycarbonyl-thiazol-2-yl)-piperazin-1-carbonsäure tert-butyl-ester [Vorstufe BBB60] wurden zusammen mit 0.96 g (17.1 mmol) KOH in Ethanol (100 ml) gelöst und 4 h bei 40 °C gerührt. Der entstandene Niederschlag wurde abfiltriert und in Wasser gelöst. Danach wurde mit 10%-iger Salzsäure auf einen pH-Wert <2 eingestellt und mit Chloroform extrahiert. Die organische Phase wurde mit Wasser und einer ges. aq. Nacl-Lsg gewaschen und über MgSO₄ getrocknet. Nach Filtration und Einengen im Vakuum wurden aus dem Rückstand durch Kristallisation aus Diethylether 2.77 g (8.8 mmol, 56%) 4-(5-Carboxy-thiazol-2-yl)-piperazin-1-carbonsäure-tert.-butylester erhalten.

### Vorstufe BBB62: Synthese von 4-[5-(3-Methyl-[1,2,4]-oxadiazol-5-yl)-thiazol-2-yl]-piperazin-1-carbonsäure-tert.-butylester

939 mg (3.0 mmol) 4-(5-Carboxy-thiazol-2-yl)-piperazin-1-carbonsäure-tert.-butylester [Vorstufe BBB61] wurden zusammen mit 534 mg (3.3 mmol) CDI in DMF (15 ml) gelöst und 4 h bei RT gerührt. Anschließend wurden 243 mg (3.3 mmol) N-Hydroxy-acetamidin hinzu gegeben und es wurde weitere 4 h bei 120 °C gerührt. Die Reaktionslösung wurde dann auf Wasser gegossen und der erstandene Niederschlag wurde abfiltriert und mit Wasser gewaschen. Durch SC (DCE/Ethanol Gemisch) mit dem Rückstand wurden 220 mg (0.63 mmol, 21 %) 4-[5-(3-Methyl-[1,2,4]-oxadiazol-5-yl)-thiazol-2-yl]-piperazin-1-carbonsäure-tert.-butylester erhalten.

### Vorstufe BBB63: Synthese von 1-[5-(3-Methyl-[1,2,4]-oxadiazol-5-yl)-thiazol-2-yl]-piperazin

Zu einer Lösung von 220 mg (0.63 mmol) 4-[5-(3-Methyl-[1,2,4]-oxadiazol-5-yl)-thiazol-2-yl]-piperazin-1-carbonsäure-tert.-butylester [Vorstufe BBB62] in DCE (10 ml) wurden eine ges. ethanolische HCl-Lsg. (4 ml) gegeben. Nach 5 min Rühren bei RT wurde die Reaktionslösung im Vakuum eingeengt. Der Rückstand wurde in DCM aufgenommen und nacheinander mit einer 1 %-igen aq. NaOH-Lsg. und Wasser gewaschen. Die organische Phase wurde über MgSO₄ getrocknet, filtriert und im Vakuum eingeengt. Dabei wurden 152 mg (0.61 mmol, 96%) 1-[5-(3-Methyl-[1,2,4]-oxadiazol-5-yl)-thiazol-2-yl]-piperazin erhalten.

### Vorstufe BBB65: Synthese von 4-(4-tert-Butyl-thiazol-2-yl)-1-propiolyl-piperazin

Durch die Umsetzung von 1-(4-tert-Butyl-thiazol-2-yl)-piperazin [Vorstufe BBB31] mit Propiolsäure gemäß den vorstehend bei Vorstufe BBB2 beschriebenen Bedingungen wurde 4-(4-tert-Butyl-thiazol-2-yl)-1-propiolyl-piperazin erhalten.
gemäß den

### Vorstufe BBB67: Synthese von (4-Methyl-thiophen-2-yl)-propiolsäure

Ausgehend von 4-Methyl-thiophen-2-carbonyl-chlorid wurde gemäß den bei Vorstufe BBB17 beschriebenen Bedingungen (4-Methyl-thiophen-2-yl)-propiolsäure erhalten.

### Vorstufe BBB68: Synthese von (3-Chlor-phenyl)-propiolsäure

Ausgehend von 3-Chlor-benzaldehyd wurde nach dem bei Vorstufe BBB7 beschriebenen Verfahren (3-Chlor-phenyl)-propiolsäure erhalten.

### Vorstufe BBB69: Synthese von 3-tert-Butyl-l-thiazol-2-yl-piperazin

Durch die Umsetzung von 2-tert-Butyl-piperazin mit 2-Bromthiazol gemäß den bei Vorstufe BBB14 beschriebenen Bedingungen wurde 3-tert-Butyl-1-thiazol-2-yl-piperazin erhalten.

### Vorstufe BBB70: Synthese von 3-Isopropyl-1-thiazol-2-yl-piperazin

Durch die Umsetzung von 2-Isopropyl-piperazin mit 2-Bromthiazol gemäß den bei Vorstufe BBB14 beschriebenen Bedingungen wurde 3-Isopropyl-1-thiazol-2-yl-piperazin erhalten.

### Beispiel AAA1: Synthese von 4-(Thiazol-2-yl)-1-(3-(2-carboxy-phenyl)-propiolyl)-piperazinmethylester

664 mg (3 mmol) 4-(Thiazol-2-yl)-1-propiolyl-piperazin [Vorstufe BBB2], 786 mg (3 mmol), 2-lod-benzoesäuremethylester, 39 mg (0.054 mmol) Pd(PPh₃)₂Cl₂ und 21 mg (0.111 mmol) Cul wurden in DMF (15 ml) gelöst und mit 1.05 ml (7.5 mmol) Diisopropylamin versetzt. Anschließend wurde die Reaktionslösung 1 h bei 60 °C gerührt. Die abgekühlte Lösung wurde in Wasser gegossen und anschließend wurde mit DCM extrahiert. Die organische Phase wurde nacheinander mit Wasser und einer ges. aq. NaCl-Lsg gewaschen und über MgSO₄ getrocknet. Nach Abfiltrieren und Entfernen des Lösungsmittels im Vakuum wurde mit dem Rückstand eine SC mit einem Gemisch aus Chloroform und EE durchgeführt, wobei 621 mg (1.8 mmol, 58%) 4-(Thiazol-2-yl)-1-(3-(2-carboxy-phenyl)-propiolyl)-piperazin methylester isoliert wurden.

### Beispiel AAA2: Synthese von 4-(Thiazol-2-yl)-1-(3-(4-carboxy-phenyl)-propiolyl)-piperazinmethylester

Durch die Umsetzung von 4-(Thiazol-2-yl)-1-propiolyl-piperazin [Vorstufe BBB2] mit 4-lodbenzoesäuremethylester gemäß den bei Beispiel AAA1 beschriebenen Bedingungen wurde 4-(Thiazol-2-yl)-1-(3-(4-carboxy-phenyl)-propiolyl)-piperazin methylester erhalten.

### Beispiel AAA3: Synthese von 4-(Thiazol-2-yl)-1-(3-(3-carboxy-phenyl)-propiolyl)-piperazinethylester

Durch die Umsetzung von 4-(Thiazol-2-yl)-1-propiolyl-piperazin [Vorstufe BBB2] mit 3-lodbenzoesäureethylester gemäß den bei Beispiel AAA1 beschriebenen Bedingungen wurde 4-(Thiazol-2-yl)-1-(3-(3-carboxy-phenyl)-propiolyl)-piperazinethylester erhalten.

### Beispiel AAA4: Synthese von 4-(Thiazol-2-yl)-1-(3-(2-hydroxy-phenyl)-propiolyl)-piperazin

Durch die Umsetzung von 4-(Thiazol-2-yl)-1-propiolyl-piperazin [Vorstufe BBB2] mit 2-lodphenol gemäß den bei Beispiel AAA1 beschriebenen Bedingungen wurde 4-(Thiazol-2-yl)-1-(3-(2-hydroxy-phenyl)-propiolyl)-piperazin erhalten.

### Beispiel AAA5: Synthese von 4-(Thiazol-2-yl)-1-(3-(4-hydroxy-phenyl)-propiolyl)-piperazin

Durch die Umsetzung von 4-(Thiazol-2-yl)-1-propiolyl-piperazin [Vorstufe BBB2] mit 4-lodphenol gemäß den bei Beispiel AAA1 beschriebenen Bedingungen wurde 4-(Thiazol-2-yl)-1-(3-(4-hydroxy-phenyl)-propiolyl)-piperazin erhalten.

### Beispiel AAA6: Synthese von 4-(Thiazol-2-yl)-1-(3-(2-amino-phenyl)-propiolyl)-piperazin

Durch die Umsetzung von 4-(Thiazol-2-yl)-1-propiolyl-piperazin [Vorstufe BBB2] mit 2-lodanilin gemäß den bei Beispiel AAA1 beschriebenen Bedingungen wurde 4-(Thiazol-2-yl)-1-(3-(2-amino-phenyl)-propiolyl)-piperazin erhalten.

### Beispiel AAA7: Synthese von 4-(Thiazol-2-yl)-1-(3-(3-amino-phenyl)-propiolyl)-piperazin

Durch die Umsetzung von 4-(Thiazol-2-yl)-1-propiolyl-piperazin [Vorstufe BBB2] mit 3-lodanilin gemäß den bei Beispiel AAA1 beschriebenen Bedingungen wurde 4-(Thiazol-2-yl)-1-(3-(3-amino-phenyl)-propiolyl)-piperazin erhalten.

### Beispiel AAA8: Synthese von 4-(Thiazol-2-yl)-1-(3-(4-amino-phenyl)-propiolyl)-piperazin

Durch die Umsetzung von 4-(Thiazol-2-yl)-1-propiolyl-piperazin [Vorstufe BBB2] mit 4-lodanilin gemäß den bei Beispiel AAA1 beschriebenen Bedingungen wurde 4-(Thiazol-2-yl)-1-(3-(4-amino-phenyl)-propiolyl)-piperazin erhalten.

### Beispiel AAA9: Synthese von 4-(Thiazol-2-yl)-1-(3-(indol-5-yl)-propiolyl)-piperazin

Durch die Umsetzung von 4-(Thiazol-2-yl)-1-propiolyl-piperazin [Vorstufe BBB2] mit 5-lodindol gemäß den bei Beispiel AAA1 beschriebenen Bedingungen wurde 4-(Thiazol-2-yl)-1-(3-(indol-5-yl)-propiolyl)-piperazin erhalten.

### Beispiel AAA10: Synthese von 4-(Thiazol-2-yl)-1-(3-(2-thienyl)-propiolyl)-piperazin

Durch die Umsetzung von 4-(Thiazol-2-yl)-1-propiolyl-piperazin [Vorstufe BBB2] mit 2-lodthiophen gemäß den bei Beispiel AAA1 beschriebenen Bedingungen wurde 4-(Thiazol-2-yl)-1-(3-(2-thienyl)-propiolyl)-piperazin erhalten.

### Beispiel AAA11: Synthese von 4-(Thiazol-2-yl)-1-(3-(3-thienyl)-propiolyl)-piperazin

Durch die Umsetzung von 4-(Thiazol-2-yl)-1-propiolyl-piperazin [Vorstufe BBB2] mit 3-lodthiophen gemäß den bei Beispiel AAA1 beschriebenen Bedingungen wurde 4-(Thiazol-2-yl)-1-(3-(3-thienyl)-propiolyl)-piperazin erhalten.

### Beispiel AAA12: Synthese von 4-(Thiazol-2-yl)-1-(3-(2-methoxy-phenyl)-propiolyl)-piperazin

Durch die Umsetzung von 4-(Thiazol-2-yl)-1-propiolyl-piperazin [Vorstufe BBB2] mit 2-lodanisol gemäß den bei Beispiel AAA1 beschriebenen Bedingungen wurde 4-(Thiazol-2-yl)-1-(3-(2-methoxy-phenyl)-propiolyl)-piperazin erhalten.

### Beispiel AAA13: Synthese von 4-(Thiazol-2-yl)-1-(3-(3-methoxy-phenyl)-propiolyl)-piperazin

Durch die Umsetzung von 4-(Thiazol-2-yl)-1-propiolyl-piperazin [Vorstufe BBB2] mit 3-lodanisol gemäß den bei Beispiel AAA1 beschriebenen Bedingungen wurde 4-(Thiazol-2-yl)-1-(3-(3-methoxy-phenyl)-propiolyl)-piperazin erhalten.

### Beispiel AAA14: Synthese von 4-(Thiazol-2-yl)-1-(3-(4-methoxy-phenyl)-propiolyl)-piperazin

Durch die Umsetzung von 4-(Thiazol-2-yl)-1-propiolyl-piperazin [Vorstufe BBB2] mit 4-lodanisol gemäß den bei Beispiel AAA1 beschriebenen Bedingungen wurde 4-(Thiazol-2-yl)-1-(3-(4-methoxy-phenyl)-propiolyl)-piperazin erhalten.

### Beispiel AAA15: Synthese von 4-(Thiazol-2-yl)-1-(3-(2-cyano-phenyl)-propiolyl)-piperazin

Durch die Umsetzung von 4-(Thiazol-2-yl)-1-propiolyl-piperazin [Vorstufe BBB2] mit 2-lodbenzonitril gemäß den bei Beispiel AAA1 beschriebenen Bedingungen wurde 4-(Thiazol-2-yl)-1-(3-(2-cyano-phenyl)-propiolyl)-piperazin erhalten.

### Beispiel AAA16: Synthese von 4-(Thiazol-2-yl)-1-(3-(3-cyano-phenyl)-propiolyl)-piperazin

Durch die Umsetzung von 4-(Thiazol-2-yl)-1-propiolyl-piperazin [Vorstufe BBB2] mit 3-lodbenzonitril gemäß den bei Beispiel AAA1 beschriebenen Bedingungen wurde 4-(Thiazol-2-yl)-1-(3-(2-cyano-phenyl)-propiolyl)-piperazin erhalten.

### Beispiel AAA17: Synthese von 4-(Thiazol-2-yl)-1-(3-(4-cyano-phenyl)-propiolyl)-piperazin

Durch die Umsetzung von 4-(Thiazol-2-yl)-1-propiolyl-piperazin [Vorstufe BBB2] mit 4-lodbenzonitril gemäß den bei Beispiel AAA1 beschriebenen Bedingungen wurde 4-(Thiazol-2-yl)-1-(3-(2-cyano-phenyl)-propiolyl)-piperazin erhalten.

### Beispiel AAA18: Synthese von 4-(Thiazol-2-yl)-1-(3-(2,4-dimethyl-phenyl)-propiolyl)-piperazin

Durch die Umsetzung von 4-(Thiazol-2-yl)-1-propiolyl-piperazin [Vorstufe BBB2] mit 2,4-Dimethyl-lodbenzol gemäß den bei Beispiel AAA1 beschriebenen Bedingungen wurde 4-(Thiazol-2-yl)-1-(3-(2,4-dimethyl-phenyl)-propiolyl)-piperazin erhalten.

### Beispiel AAA19: Synthese von 4-(Thiazol-2-yl)-1-(3-(3,5-dimethyl-phenyl)-propiolyl)-piperazin

Durch die Umsetzung von 4-(Thiazol-2-yl)-1-propiolyl-piperazin [Vorstufe BBB2] mit 3,5-Dimethyl-lodbenzol gemäß den bei Beispiel AAA1 beschriebenen Bedingungen wurde 4-(Thiazol-2-yl)-1-(3-(3,5-dimethyl-phenyl)-propiolyl)-piperazin erhalten.

### Beispiel AAA20: Synthese von 4-(Thiazol-2-yl)-1-(3-(2,6-dimethyl-phenyl)-propiolyl)-piperazin

Durch die Umsetzung von 4-(Thiazol-2-yl)-1-propiolyl-piperazin [Vorstufe BBB2] mit 2,6-Dimethyl-iodbenzol gemäß den bei Beispiel AAA1 beschriebenen Bedingungen wurde 4-(Thiazol-2-yl)-1-(3-(2,6-dimethyl-phenyl)-propiolyl)-piperazin erhalten.

### Beispiel AAA21: Synthese von 4-(Thiazol-2-yl)-1-(3-(2-fluor-phenyl)-propiolyl)-piperazin

Durch die Umsetzung von 4-(Thiazol-2-yl)-1-propiolyl-piperazin [Vorstufe BBB2] mit 2-lod-fluorbenzol gemäß den bei Beispiel AAA1 beschriebenen Bedingungen wurde 4-(Thiazol-2-yl)-1-(3-(2-fluor-phenyl)-propiolyl)-piperazin erhalten.

### Beispiel AAA22: Synthese von 4-(Thiazol-2-yl)-1-(3-(3-fluor-phenyl)-propiolyl)-piperazin

Durch die Umsetzung von 4-(Thiazol-2-yl)-1-propiolyl-piperazin [Vorstufe BBB2] mit 3-lod-fluorbenzol gemäß den bei Beispiel AAA1 beschriebenen Bedingungen wurde 4-(Thiazol-2-yl)-1-(3-(3-fluor-phenyl)-propiolyl)-piperazin erhalten.

### Beispiel AAA23: Synthese von 4-(Thiazol-2-yl)-1-(3-(2-chlor-phenyl)-propiolyl)-piperazin

Durch die Umsetzung von 4-(Thiazol-2-yl)-1-propiolyl-piperazin [Vorstufe BBB2] mit 2-lod-chlorbenzol gemäß den bei Beispiel AAA1 beschriebenen Bedingungen wurde 4-(Thiazol-2-yl)-1-(3-(2-chlor-phenyl)-propiolyl)-piperazin erhalten.

### Beispiel AAA24: Synthese von 4-(Thiazol-2-yl)-1-(3-(3-chlor-phenyl)-propiolyl)-piperazin

Durch die Umsetzung von 4-(Thiazol-2-yl)-1-propiolyl-piperazin [Vorstufe BBB2] mit 3-lod-chlorbenzol gemäß den bei Beispiel AAA1 beschriebenen Bedingungen wurde 4-(Thiazol-2-yl)-1-(3-(3-chlor-phenyl)-propiolyl)-piperazin erhalten.

### Beispiel AAA25: Synthese von 4-(Thiazol-2-yl)-1-(3-naphthyl-propiolyl)-piperazin

Durch die Umsetzung von 4-(Thiazol-2-yl)-1-propiolyl-piperazin [Vorstufe BBB2] mit 1-lod-Naphthalin-chlorbenzol gemäß den bei Beispiel AAA1 beschriebenen Bedingungen wurde 4-(Thiazol-2-yl)-1-(3-naphthyl-propiolyl)-piperazin erhalten.

### Beispiel AAA26: Synthese von 4-(Thiazol-2-yl)-1-(3-(2,3-dimethyl-phenyl)-propiolyl)-piperazin

Durch die Umsetzung von 4-(Thiazol-2-yl)-1-propiolyl-piperazin [Vorstufe BBB2] mit 2,3-Dimethyl-iodbenzol gemäß den bei Beispiel AAA1 beschriebenen Bedingungen wurde 4-(Thiazol-2-yl)-1-(3-(2,3-dimethyl-phenyl)-propiolyl)-piperazin erhalten.

### Beispiel AAA27: Synthese von 4-(Thiazol-2-yl)-1-(3-(3,4-dimethyl-phenyl)-propiolyl)-piperazin

Durch die Umsetzung von 4-(Thiazol-2-yl)-1-propiolyl-piperazin [Vorstufe BBB2] mit 3,4-Dimethyl-iodbenzol gemäß den bei Beispiel AAA1 beschriebenen Bedingungen wurde 4-(Thiazol-2-yl)-1-(3-(3,4-dimethyl-phenyl)-propiolyl)-piperazin erhalten.

### Beispiel AAA28: Synthese von 4-(Thiazol-2-yl)-1-(3-(3-nitro-phenyl)-propiolyl)-piperazin

Durch die Umsetzung von 4-(Thiazol-2-yl)-1-propiolyl-piperazin [Vorstufe BBB2] mit 3-Nitro-iodbenzol gemäß den bei Beispiel AAA1 beschriebenen Bedingungen wurde 4-(Thiazol-2-yl)-1-(3-(3-nitro-phenyl)-propiolyl)-piperazin erhalten.

### Beispiel AAA29: Synthese von 4-(Thiazol-2-yl)-1-(3-(2-nitro-phenyl)-propiolyl)-piperazin

Durch die Umsetzung von 4-(Thiazol-2-yl)-1-propiolyl-piperazin [Vorstufe BBB2] mit 2-Nitro-iodbenzol gemäß den bei Beispiel AAA1 beschriebenen Bedingungen wurde 4-(Thiazol-2-yl)-1-(3-(2-nitro-phenyl)-propiolyl)-piperazin erhalten.

### Beispiel AAA30: Synthese von 4-(Thiazol-2-yl)-1-(3-(3-formyl-phenyl)-propiolyl)-piperazin

Durch die Umsetzung von 4-(Thiazol-2-yl)-1-propiolyl-piperazin [Vorstufe BBB2] mit 3-lod-benzaldehyd gemäß den bei Beispiel AAA1 beschriebenen Bedingungen wurde 4-(Thiazol-2-yl)-1-(3-(3-formyl-phenyl)-propiolyl)-piperazin erhalten.

### Beispiel AAA31: Synthese von 4-(Thiazol-2-yl)-1-(3-(3-ethenyl-phenyl)-propiolyl)-piperazin

Durch die Umsetzung von 4-(Thiazol-2-yl)-1-propiolyl-piperazin [Vorstufe BBB2] mit 3-lod-styrol gemäß den bei Beispiel AAA1 beschriebenen Bedingungen wurde 4-(Thiazol-2-yl)-1-(3-(3-ethenyl-phenyl)-propiolyl)-piperazin erhalten.

### Beispiel AAA32: Synthese von 4-(Thiazol-2-yl)-1-(3-pyrid-2-yl-propiolyl)-piperazin

Durch die Umsetzung von 4-(Thiazol-2-yl)-1-propiolyl-piperazin [Vorstufe BBB2] mit 2-lod-pyridin gemäß den bei Beispiel AAA1 beschriebenen Bedingungen wurde 4-(Thiazol-2-yl)-1-(3-pyrid-2-yl-propiolyl)-piperazin erhalten.

### Beispiel AAA33: Synthese von 4-(Thiazol-2-yl)-1-(3-pyrid-3-yl-propiolyl)-piperazin

Durch die Umsetzung von 4-(Thiazol-2-yl)-1-propiolyl-piperazin [Vorstufe BBB2] mit 3-lod-pyridin gemäß den bei Beispiel AAA1 beschriebenen Bedingungen wurde 4-(Thiazol-2-yl)-1-(3-pyrid-3-yl-propiolyl)-piperazin erhalten.

### Beispiel AAA34: Synthese von 4-(Thiazol-2-yl)-1-(3-pyrid-4-yl-propiolyl)-piperazin

Durch die Umsetzung von 4-(Thiazol-2-yl)-1-propiolyl-piperazin [Vorstufe BBB2] mit 4-lod-pyridin gemäß den bei Beispiel AAA1 beschriebenen Bedingungen wurde 4-(Thiazol-2-yl)-1-(3-pyrid-4-yl-propiolyl)-piperazin erhalten.

### Beispiel AAA35: Synthese von 4-(Thiazol-2-yl)-1-(3-(chinolin-6-yl)-propiolyl)-piperazin

442 mg (2.0 mmol) 4-(Thiazol-2-yl)-1-propiolyl-piperazin [Vorstufe BBB2], 416 mg (2.0 mmol) 6-Brom-chinolin [Vorstufe BBB3], 25 mg (0.035 mmol) Pd(PPh₃)₂Cl₂ und 14 mg (0.074 mmol) Cul wurden in DMF (10 ml) gelöst und mit 0.7 ml (5.0 mmol) TEA versetzt. Anschließend wurde die Reaktionslösung in der Mikrowelle (CEM Discover LabMate, Intellivent Pressure, monomode cavity, 50W) 20 min auf 110 °C erhitzt. Die abgekühlte Lösung wurde in Wasser gegossen und anschließend wurde mit Chloroform extrahiert. Die organische Phase wurde nacheinander mit Wasser und einer ges. aq. NaCl-Lsg gewaschen und über MgSO₄ getrocknet. Nach Abfiltrieren und Entfernen der Lösungsmittel im Vakuum wurden aus dem Rückstand durch SC mit einem Gemisch aus Hexan und EE 103 mg (1.8 mmol, 15%) 4-(Thiazol-2-yl)-1-(3-(chinolin-6-yl)-propiolyl)-piperazin erhalten.

### Beispiel AAA36: Synthese von 4-(Thiazol-2-yl)-1-(3-(3-isopropyl-phenyl)-propiolyl)-piperazin

Durch die Umsetzung von 4-(Thiazol-2-yl)-1-propiolyl-piperazin [Vorstufe BBB2] mit 3-Brom-isopropylbenzol gemäß den bei Beispiel AAA35 beschriebenen Bedingungen wurde 4-(Thiazol-2-yl)-1-(3-(3-isopropyl-phenyl)-propiolyl)-piperazin erhalten.

### Beispiel AAA37: Synthese von 4-(Thiazol-2-yl)-1-(3-biphenyl-3-yl-propiolyl)-piperazin

Durch die Umsetzung von 4-(Thiazol-2-yl)-1-propiolyl-piperazin [Vorstufe BBB2] mit 3-Brom-biphenyl gemäß den bei Beispiel AAA35 beschriebenen Bedingungen wurde 4-(Thiazol-2-yl)-1-(3-biphenyl-3-yl-propiolyl)-piperazin erhalten.

### Beispiel AAA38: Synthese von 4-(Thiazol-2-yl)-1-(3-naphth-2-yl-propiolyl)-piperazin

Durch die Umsetzung von 4-(Thiazol-2-yl)-1-propiolyl-piperazin [Vorstufe BBB2] mit 2-Brom-naphthalin gemäß den bei Beispiel AAA35 beschriebenen Bedingungen wurde 4-(Thiazol-2-yl)-1-(3-naphth-2-yl-propiolyl)-piperazin erhalten.

### Beispiel AAA39: Synthese von 4-(Thiazol-2-yl)-1-(3-(1-methyl-indol-5-yl)-propiolyl)-piperazin

Durch die Umsetzung von 4-(Thiazol-2-yl)-1-propiolyl-piperazin [Vorstufe BBB2] mit 5-Brom-1-methyl-indol [Vorstufe BBB4] gemäß den bei Beispiel AAA35 beschriebenen Bedingungen wurde 4-(Thiazol-2-yl)-1-(3-(1-methyl-indol-5-yl)-propiolyl)-piperazin erhalten.

### Beispiel AAA40: Synthese von 4-(Thiazol-2-yl)-1-(3-(3-methylmercapto-phenyl)-propiolyl)-piperazin

Durch die Umsetzung von 4-(Thiazol-2-yl)-1-propiolyl-piperazin [Vorstufe BBB2] mit 3-Brom-thioanisol gemäß den bei Beispiel AAA35 beschriebenen Bedingungen wurde von 4-(Thiazol-2-yl)-1-(3-(3-methylmercapto-phenyl)-propiolyl)-piperazin erhalten.

### Beispiel AAA41: Synthese von 4-(Thiazol-2-yl)-1-(3-(3-cyano-4-fluor-phenyl)-propiolyl)-piperazin

Durch die Umsetzung von 4-(Thiazol-2-yl)-1-propiolyl-piperazin [Vorstufe BBB2] mit 5-Brom-2-Fluor-benzonitril gemäß den bei Beispiel AAA35 beschriebenen Bedingungen wurde 4-(Thiazol-2-yl)-1-(3-(3-cyano-4-fluor-phenyl)-propiolyl)-piperazin erhalten.

### Beispiel AAA42: Synthese von 4-(Thiazol-2-yl)-1-(3-(3-methoxymethyl-phenyl)-propiolyl)-piperazin

Durch die Umsetzung von 4-(Thiazol-2-yl)-1-propiolyl-piperazin [Vorstufe BBB2] mit 3-Methoxymethyl-iodbenzol [Vorstufe BBB5] gemäß den bei Beispiel AAA1 beschriebenen Bedingungen wurde 4-(Thiazol-2-yl)-1-(3-(3-methoxymethyl-phenyl)-propiolyl)-piperazin erhalten.

### Beispiel AAA43: Synthese von 4-(Thiazol-2-yl)-1-(3-(3-hydroxy-phenyl)-propiolyl)-piperazin

Zu einer Lösung von 224 mg (0.72 mmol) 4-(Thiazol-2-yl)-1-(3-(3-amino-phenyl)-propiolyl)-piperazin [Beispiel AAA7] in 5%-iger Schwefelsäure (100 ml) wurde bei 40 °C eine Lösung von 57 mg (0.83 mmol) Natriumnitrit in Wasser (10 ml) getropft. Nach beendeter Zugabe wurde noch 2 h bei 80 °C gerührt. Anschließend wurde die Reaktionslösung mit einer ges. aq. NaHCO₃-Lsg. neutralisiert und mit Chloroform extrahiert. Die organische Phase wurde nacheinander mit Wasser und einer ges. aq. NaCl-Lsg. gewaschen und über MgSO₄ getrocknet. Nach Filtration und Entfernen des Lösungsmittels im Vakuum wurden aus dem Rückstand 120 mg (0.38 mmol, 53%) 4-(Thiazol-2-yl)-1-(3-(3-hydroxy-phenyl)-propiolyl)-piperazin aus Diethylether kristallisiert.

### Beispiel AAA44: Synthese von 4-(Thiazol-2-yl)-1-(3-(3-acetamino-phenyl)-propiolyl)-piperazin

209 mg (0.67 mmol) 4-(Thiazol-2-yl)-1-(3-(3-amino-phenyl)-propiolyl)-piperazin [Beispiel AAA7] und 193 µl (1.1 mmol) DIPEA wurden in Dichlorethan (5 ml) gelöst und mit 65 µl (1.0 mmol) Acetylchlorid versetzt. Nach 1 h Rühren bei RT wurde die Reaktionslösung nacheinander mit einer 3%-igen aq. Na₂Co₃-Lsg. und einer ges. aq.

NaCL-Lsg gewaschen und über MgSO₄ getrocknet. Nach Filtration und Entfernen des Lösungsmittels im Vakuum wurden aus dem Rückstand 161 mg (0.45 mmol, 68%) 4-(Thiazol-2-yl)-1-(3-(3-acetamino-phenyl)-propiolyl)-piperazin aus Diethylether kristallisiert.

### Beispiel AAA45: Synthese von 4-(Thiazol-2-yl)-1-(3-(4-acetamino-phenyl)-propiolyl)-piperazin

Durch die Umsetzung von 4-(Thiazol-2-yl)-1-(3-(4-amino-phenyl)-propiolyl)-piperazin [Beispiel AAA8] mit Acetylchlorid gemäß den bei Beispiel AAA44 beschriebenen Bedingungen wurde 4-(Thiazol-2-yl)-1-(3-(4-acetamino-phenyl)-propiolyl)-piperazin erhalten.

### Beispiel AAA46: Synthese von 4-(Thiazol-2-yl)-1-(3-(2-carboxy-phenyl)-propiolyl)-piperazin

257 mg (0.72 mmol) 4-(Thiazol-2-yl)-1-(3-(2-carboxy-phenyl)-propiolyl)-piperazinmethylester [Beispiel AAA1] wurden in Ethanol (10 ml) gelöst und mit 0.32 ml einer 10%-igen aq. NaOH-Lsg versetzt. Die Reaktionslösung wurde 30 min bei 70 °C gerührt. Anschließend wurde das Ethanol ab destilliert und der Rückstand in Wasser aufgenommen. Die erhaltene Lösung wurde mit EE gewaschen und anschließend mit einer 3%-igen HCl-Lsg auf einen pH-Wert <3 eingestellt. Danach wurde mit Chloroform extrahiert und die organische Phase wurde über MgSO₄ getrocknet, filtriert und im Vakuum eingeengt. Durch Kristallisation des Rückstands aus Diethylether, gefolgt von einer zweiten Kristallisation aus Ethanol wurden 72 mg (21 mmol, 29%) 4-(Thiazol-2-yl)-1-(3-(2-carboxy-phenyl)-propiolyl)-piperazin erhalten.

### Beispiel AAA47: Synthese von 4-(Thiazol-2-yl)-1-(3-(3-carboxy-phenyl)-propiolyl)-piperazin

Durch die Umsetzung von 4-(Thiazol-2-yl)-1-(3-(3-carboxy-phenyl)-propiolyl)-piperazin ethylester [Beispiel AAA3] mit NaOH gemäß den bei Beispiel AAA46 beschriebenen Bedingungen wurde 4-(Thiazol-2-yl)-1-(3-(3-carboxy-phenyl)-propiolyl)-piperazin erhalten.

### Beispiel AAA48: Synthese von 4-(Thiazol-2-yl)-1-(3-(4-carboxy-phenyl)-propiolyl)-piperazin

Durch die Umsetzung von 4-(Thiazol-2-yl)-1-(3-(4-carboxy-phenyl)-propiolyl)-piperazinmethylester [Beispiel AAA2] mit NaOH gemäß den bei Beispiel AAA46 beschriebenen Bedingungen wurde 4-(Thiazol-2-yl)-1-(3-(4-carboxy-phenyl)-propiolyl)-piperazin erhalten.

### Beispiel AAA49: Synthese von 4-(Thiazol-2-yl)-1-(3-(3-carboxy-phenyl)-propiolyl)-piperazin methylester

347 mg (1.00 mmol) 4-(Thiazol-2-yl)-1-(3-(3-carboxy-phenyl)-propiolyl)-piperazin [Beispiel AAA47] wurden in DCE (20 ml) gelöst und mit 170 mg (1.05 mmol) CDI versetzt. Nach 45 min Rühren bei RT wurde MeOH (80 ml) zugegeben und 16 h bei 50 °C gerührt. Anschließend wurde im Vakuum eingeengt und der Rückstand wurde in Chloroform aufgenommen. Die erhaltene Lösung wurde nacheinander mit einer 3%-igen Na₂CO₃-Lsg., Wasser und einer ges. aq. NaCl-Lsg gewaschen, über MgSO₄ getrocknet, filtriert und im Vakuum eingeengt. Durch Kristallisation des Rückstands aus Ether wurden 127 mg (0.36 mmol, 36%) 4-(Thiazol-2-yl)-1-(3-(3-carboxy-phenyl)-propiolyl)-piperazinmethylester erhalten.

### Beispiel AAA50: Synthese von 4-(Thiazol-2-yl)-1-(3-(3-aminocarbonyl-phenyl)-propiolyl)-piperazin

172 mg (0.50 mmol) 4-(Thiazol-2-yl)-1-(3-(3-carboxy-phenyl)-propiolyl)-piperazin [Beispiel AAA47] wurden in THF (5 ml) gelöst und mit 86 mg (0.53 mmol) CDI versetzt. Nach 60 min Rühren bei RT wurde über einen Zeitraum von 5 min NH₃ durch die Lösung geleitet. Anschließend wurde weitere 16 h bei RT gerührt und im Vakuum eingeengt. Der Rückstand wurde in MeCN suspendiert, abfiltriert und mit Ether gewaschen. Dabei wurden 107 mg (0.32 mmol, 63%) 4-(Thiazol-2-yl)-1-(3-(3-aminocarbonyl-phenyl)-propiolyl)-piperazin erhalten.

### Beispiel AAA51: Synthese von 4-(Thiazol-2-yl)-1-(3-(3-methylaminocarbonylphenyl)-propiolyl)-piperazin

480 mg (1.40 mmol) 4-(Thiazol-2-yl)-1-(3-(3-carboxy-phenyl)-propiolyl)-piperazin [Beispiel AAA47] wurden in DCE (10 ml) gelöst und mit 239 mg (1.47 mmol) CDI versetzt. Nach 45 min Rühren bei RT wurden 4 ml einer ca.15%-igen Lösung von Methylamin in Dioxan zugegeben. Anschließend wurde weitere 2 h bei RT gerührt. Der entstandene Niederschlag wurde abfiltriert und mit DCE gewaschen. Durch Kristallisation des Rückstands aus Ethanol wurden 337 mg (0.95 mmol, 68%) 4-(Thiazol-2-yl)-1-(3-(3-methylaminocarbonyl-phenyl)-propiolyl)-piperazin erhalten.

### Beispiel AAA52: Synthese von 4-(Thiazol-2-yl)-1-(3-(3-dimethylaminocarbonylphenyl)-propiolyl)-piperazin

480 mg (1.40 mmol) 4-(Thiazol-2-yl)-1-(3-(3-carboxy-phenyl)-propiolyl)-piperazin [Beispiel AAA47] wurden in DCE (10 ml) gelöst und mit 239 mg (1.47 mmol) CDI versetzt. Nach 45 min Rühren bei RT wurden 5 ml einer ca.8%-igen Lösung von Dimethylamin in Dioxan zugegeben. Anschließend wurde weitere 2 h bei RT gerührt. Danach wurde die Reaktionslösung nacheinander mit einer 5%-igen aq. Na₂CO₃-Lsg., Wasser und einer ges. aq. NaCl-Lsg gewaschen und über MgSO₄ getrocknet. Nach Filtration und Entfernen des Lösungsmittels im Vakuum wurden durch Kristallisation des Rückstands aus Diethylether 283 mg (0.77 mmol, 55%) 4-(Thiazol-2-yl)-1-(3-(3-dimethylaminocarbonyl-phenyl)-propiolyl)-piperazin erhalten.

### Beispiel AAA53: Synthese von 4-(Thiazol-2-yl)-1-(3-(2-tolyl)-propiolyl)-piperazin Hydrochlorid

Zu 320 mg (2.0 mmol) Tol-2-yl-propiolsäure [Vorstufe BBB8] wurde eine Lösung von 338 mg (2.0 mmol) 1-Thiazol-2-yl-piperazin [Vorstufe BBB1] in DMF (1.5 ml) gegeben. Die Reaktionslösung wurde anschließend mit 0.31 ml (2.0 mmol) DIC und 270 mg (2.0 mmol) HOBt versetzt und 16 h bei RT gerührt. Danach wurde mit einer 1 M aq. Na₂CO₃-Lsg verdünnt und nacheinander mit EE und THF extrahiert. Die vereinigten organischen Phasen wurden über MgSO₄ getrocknet, filtriert und im Vakuum eingeengt. Der Rückstand wurde mit Aceton (20 ml) aufgenommen und nacheinander mit 40 µl Wasser und 280 µl TMSCl versetzt und 30 min bei RT gerührt. Der entstandene Niederschlag wurde abfiltriert, wobei 453 mg (1.3 mmol, 65%) 4-(Thiazol-2-yl)-1-(3-(2-tolyl)-propiolyl)-piperazin Hydrochlorid erhalten wurden.

### Beispiel AAA54: Synthese von 4-(Thiazol-2-yl)-1-(3-(3-tolyl)-propiolyl)-piperazin Hydrochlorid

Durch die Umsetzung Tol-3-yl-propiolsäure [Vorstufe BBB9] mit 1-Thiazol-2-yl-piperazin [Vorstufe BBB1] gemäß den bei Beispiel AAA53 beschriebenen Bedingungen wurde 4-(Thiazol-2-yl)-1-(3-(3-tolyl)-propiolyl)-piperazin Hydrochlorid erhalten.

### Beispiel AAA55: Synthese von 4-(Thiazol-2-yl)-1-(3-(4-tolyl)-propiolyl)-piperazin Hydrochlorid

Durch die Umsetzung Tol-4-yl-propiolsäure [Vorstufe BBB10] mit 1-Thiazol-2-yl-piperazin [Vorstufe BBB1] gemäß den bei Beispiel AAA53 beschriebenen Bedingungen wurde 4-(Thiazol-2-yl)-1-(3-(4-tolyl)-propiolyl)-piperazin Hydrochlorid erhalten.

### Beispiel AAA56: Synthese von 4-(Thiazol-2-yl)-1-(3-phenyl-propiolyl)-piperazin Hydrochlorid

Durch die Umsetzung von Phenylpropiolsäure mit 1-Thiazol-2-yl-piperazin [Vorstufe BBB1] gemäß den bei Beispiel AAA53 beschriebenen Bedingungen wurde 4-(Thiazol-2-yl)-1-(3-phenyl-propiolyl)-piperazin Hydrochlorid erhalten.

### Beispiel AAA57: Synthese von 4-(Thiazol-2-yl)-1-(3-(2-trifluormethyl-phenyl)-propiolyl)-piperazin Hydrochlorid

Durch die Umsetzung von (2-Trifluormethyl-phenyl)-propiolsäure [Vorstufe BBB11] mit 1-Thiazol-2-yl-piperazin [Vorstufe BBB1] gemäß den bei Beispiel AAA53 beschriebenen Bedingungen wurde 4-(Thiazol-2-yl)-1-(3-(2-trifluormethyl-phenyl)-propiolyl)-piperazin Hydrochlorid erhalten.

### Beispiel AAA58: Synthese von 4-(Thiazol-2-yl)-1-(3-(3-trifluormethyl-phenyl)-propiolyl)-piperazin Hydrochlorid

Durch die Umsetzung von (3-Trifluormethyl-phenyl)-propiolsäure [Vorstufe BBB12] mit 1-Thiazol-2-yl-piperazin [Vorstufe BBB1] gemäß den bei Beispiel AAA53 beschriebenen Bedingungen wurde 4-(Thiazol-2-yl)-1-(3-(3-trifluormethyl-phenyl)-propiolyl)-piperazin Hydrochlorid erhalten.

### Beispiel AAA59: Synthese von 4-(Thiazol-2-yl)-1-(3-(4-trifluormethyl-phenyl)-propiolyl)-piperazin Hydrochlorid

Durch die Umsetzung von (4-Trifluormethyl-phenyl)-propiolsäure [Vorstufe BBB13] mit 1-Thiazol-2-yl-piperazin [Vorstufe BBB1] gemäß den bei Beispiel AAA53 beschriebenen Bedingungen wurde 4-(Thiazol-2-yl)-1-(3-(4-trifluormethyl-phenyl)-propiolyl)-piperazin Hydrochlorid erhalten.

### Beispiel AAA60: Synthese von 4-(Thiazol-2-yl)-1-(3-pentyl-propiolyl)-piperazin

Durch die Umsetzung von 2-Octinsäure mit 1-Thiazol-2-yl-piperazin [Vorstufe BBB1] gemäß den bei Beispiel 1 beschriebenen Bedingungen wurde 4-(Thiazol-2-yl)-1-(3-pentyl-propiolyl)-piperazin erhalten.

### Beispiel AAA61: Synthese von 4-(Thiazol-2-yl)-1-(3-(4-fluor-phenyl)-propiolyl)-piperazin

Eine Lösung von 1.0 g (6.1 mmol) (4-Fluor-phenyl)-propiolsäure [Vorstufe BBB7] in DCE (70 ml) wurde mit 1.0 g (6.4 mmol) CDI versetzt und 100 min bei RT gerührt. Nach Abkühlen auf 10 °C wurden 1.0 g (6.0 mmol) 1-Thiazol-2-yl-piperazin [Vorstufe BBB1] hinzu gegeben und es wurde weitere 2 h bei RT gerührt. Anschließend wurde die Reaktionslösung nacheinander mit Wasser, einer 3%-igen HCl-Lsg. und einer ges. aq. NaCl-Lsg gewaschen und über MgSO₄ getrocknet. Nach Filtration und Entfernen des Lösungsmittels im Vakuum wurden aus dem Rückstand 785 mg (2.5 mmol, 42%) 4-(Thiazol-2-yl)-1-(3-(4-fluor-phenyl)-propiolyl)-piperazin durch Kristallisation aus Diethylether erhalten.

### Beispiel AAA62: Synthese von 4-(Thiazol-2-yl)-1-(3-(4-chlor-phenyl)-propiolyl)-piperazin

Eine Lösung von 722 mg (4.0 mmol) (4-Chlor-phenyl)-propiolsäure [Vorstufe BBB6] in DCE (25 ml) wurde mit 681 mg (4.2 mmol) CDI versetzt und 60 min bei RT gerührt. Nach Abkühlen auf 10 °C wurden 677 mg (4.0 mmol) 1-Thiazol-2-yl-piperazin [Vorstufe BBB1] zugegeben und es wurde 1 h bei RT gerührt. Nach Entfernen des Lösungsmittels im Vakuum wurden aus dem Rückstand durch Kristallisation aus Ethanol 607 mg (1.8 mmol, 46%) 4-(Thiazol-2-yl)-1-(3-(4-chlor-phenyl)-propiolyl)-piperazin erhalten.

### Beispiel AAA63: Synthese von 2-Methyl-4-(thiazol-2-yl)-1-(3-phenyl-propiolyl)-piperazin

Zu einer Lösung von 1.40 g (8.6 mmol) Phenylpropiolsäure in MeCN (90 ml) wurden 1.42 ml (8.6 mmol) DIPEA, 1.58 g (8.6 mmol) 3-Methyl-1-thiazol-2-yl-piperazin [Vorstufe BBB14] und 2.76 g (8.6 mmol) TBTU gegeben. Nach 2 h Rühren bei RT wurde im Vakuum eingeengt. Der Rückstand wurde in Chloroform aufgenommen, nacheinander mit einer 10%-igen aq. NaOH-Lsg, Wasser und einer ges. aq. NaCl-Lsg gewaschen und über MgSO₄ getrocknet. Nach Filtration und Einengen im Vakuum wurde mit dem Rückstand eine SC mit einem Gemisch aus Hexan und EE durchgeführt. Durch Kristallisation des erhaltenen Rohprodukts aus DIPE wurden 1.67 g (5.4 mmol, 62%) 2-Methyl-4-(thiazol-2-yl)-1-(3-phenyl-propiolyl)-piperazin erhalten.

### Beispiel AAA64: Synthese von (S)-2-Methyl-4-(thiazol-2-yl)-1-(3-phenylpropiolyl)-piperazin

Durch die Umsetzung von (S)-3-Methyl-1-thiazol-2-yl-piperazin [Vorstufe BBB15] mit Phenylpropiolsäure gemäß den bei Beispiel AAA63 beschriebenen Bedingungen wurde (S)-2-Methyl-4-(thiazol-2-yl)-1-(3-phenyl-propiolyl)-piperazin erhalten.

### Beispiel AAA65: Synthese von (R)-2-Methyl-4-(thiazol-2-yl)-1-(3-phenylpropiolyl)-piperazin

Durch die Umsetzung von (R)-3-Methyl-1-thiazol-2-yl-piperazin [Vorstufe BBB16] mit Phenylpropiolsäure gemäß den bei Beispiel AAA63 beschriebenen Bedingungen wurde (R)-2-Methyl-4-(thiazol-2-yl)-1-(3-phenyl-propiolyl)-piperazin erhalten.

### Beispiel AAA66: Synthese von 2-Methyl-4-(thiazol-2-yl)-1-(3-phenyl-propiolyl)-piperazin Hydrochlorid

1.67 g (5.4 mmol) 2-Methyl-4-(thiazol-2-yl)-1-(3-phenyl-propiolyl)-piperazin (AAA63) wurden in Diethylether gelöst und mit einer Lösung von HCl in Diethylether versetzt. Der entstandene Niederschlag wurde abgesaugt und mit Diethylether gewaschen. Es wurden 1.75 g (5.0 mmol, 93%) 2-Methyl-4-(thiazol-2-yl)-1-(3-phenyl-propiolyl)-piperazin Hydrochlorid erhalten.

### Beispiel AAA68: Synthese von 2-Methyl-4-(thiazol-2-yl)-1-(3-(3-tolyl)-propiolyl)-piperazin Hydrochlorid

Durch die Umsetzung von Tol-3-yl-propiolsäure [Vorstufe BBB9] mit 3-Methyl-1-thiazol-2-yl-piperazin [Vorstufe BBB14] gemäß den bei Beispiel AAA53 beschriebenen Bedingungen wurde 2-Methyl-4-(thiazol-2-yl)-1-(3-(3-tolyl)-propiolyl)-piperazin Hydrochlorid erhalten.

### Beispiel AAA67: Synthese von 2-Methyl-4-(thiazol-2-yl)-1-(3-(3-tolyl)-propiolyl)-piperazin

Durch die Umsetzung von Tol-3-yl-propiolsäure [Vorstufe BBB9] mit 3-Methyl-1-thiazol-2-yl-piperazin [Vorstufe BBB14] gemäß den bei Beispiel 1 beschriebenen Bedingungen wurde 2-Methyl-4-(thiazol-2-yl)-1-(3-(3-tolyl)-propiolyl)-piperazin erhalten.

### Beispiel AAA69: Synthese von 4-(Thiazol-2-yl)-1-(3-cyclohexyl-propiolyl)-piperazin

Durch die Umsetzung von Cyclohexylpropiolsäure [Vorstufe BBB17] mit 1-Thiazol-2-yl-piperazin [Vorstufe BBB1] gemäß den bei Beispiel AAA62 genannten Bedingungen wurde 4-(Thiazol-2-yl)-1-(3-methyl-propiolyl)-piperazin erhalten.

### Beispiel AAA70: Synthese von 4-(Thiazol-2-yl)-1-(3-methyl-propiolyl)-piperazin

Durch die Umsetzung von 2-Butinsäure mit 1-Thiazol-2-yl-piperazin [Vorstufe BBB1] gemäß den bei Beispiel 1 beschriebenen Bedingungen wurde 4-(Thiazol-2-yl)-1-(3-methyl-propiolyl)-piperazin erhalten.

### Beispiel AAA71: Synthese von 2-Ethyl-4-(thiazol-2-yl)-1-(3-phenyl-propiolyl)-piperazin

Durch die Umsetzung von 3-Ethyl-1-thiazol-2-yl-piperazin [Vorstufe BBB19] mit Phenylpropiolsäure gemäß den bei Beispiel AAA63 beschriebenen Bedingungen wurde 2-Ethyl-4-(thiazol-2-yl)-1-(3-phenyl-propiolyl)-piperazin erhalten.

### Beispiel AAA72: Synthese von 2-Phenyl-4-(thiazol-2-yl)-1-(3-phenyl-propiolyl)-piperazin

Durch die Umsetzung von 3-Phenyl-1-thiazol-2-yl-piperazin [Vorstufe BBB20] mit Phenylpropiolsäure gemäß den bei Beispiel AAA63 beschriebenen Bedingungen wurde 2-Phenyl-4-(thiazol-2-yl)-1-(3-phenyl-propiolyl)-piperazin erhalten.

### Beispiel AAA73: Synthese von 4-(Thiazol-2-yl)-1-(3-(2-furyl)-propiolyl)-piperazin

Durch die Umsetzung von Furan-2-yl-propiolsäure [Vorstufe BBB21] mit Phenylpropiolsäure gemäß den bei Beispiel AAA62 beschriebenen Bedingungen wurde 4-(Thiazol-2-yl)-1-(3-(2-furyl)-propiolyl)-piperazin erhalten.

### Beispiel AAA74: Synthese von 4-(Thiazol-2-yl)-1-(3-(3-furyl)-propiolyl)-piperazin

Durch die Umsetzung von Furan-3-yl-propiolsäure [Vorstufe BBB22] mit Phenylpropiolsäure gemäß den bei Beispiel AAA62 beschriebenen Bedingungen wurde 4-(Thiazol-2-yl)-1-(3-(3-furyl)-propiolyl)-piperazin erhalten.

### Beispiel AAA75: Synthese von cis-2,6-Dimethyl-4-(thiazol-2-yl)-1-(3-phenylpropiolyl)-piperazin

Durch die Umsetzung von cis-3,5-Dimethyl-1-thiazol-2-yl-piperazin [Vorstufe BBB23] mit Phenylpropiolsäure gemäß den bei Beispiel AAA63 beschriebenen Bedingungen wurde cis-2,6-Dimethyl-4-(thiazol-2-yl)-1-(3-phenyl-propiolyl)-piperazin erhalten.

### Beispiel AAA76: Synthese von 4-(5-Carboxy-thiazol-2-yl)-1-(3-phenyl-propiolyl)-piperazinethylester

Eine Lösung von 678 mg (4.64 mmol) Phenylpropiolsäure in DCE (20 ml) wurde mit 790 mg (4.87 mmol) CDI versetzt und 50 min bei RT gerührt. Nach Abkühlen auf 10 °C wurden 1.12 g (4.64 mmol) 2-Piperazin-1-yl-thiazol-5-carbonsäureethylester [Vorstufe BBB24] hinzu gegeben und es wurde 2 h bei RT gerührt. Die Reaktionslösung wurde mit Wasser und einer ges. aq. NaCl-Lsg. gewaschen und über MgSO₄ getrocknet. Nach Filtration und Einengen im Vakuum wurden aus dem Rückstand 890 mg (2.41 mmol, 52%) 4-(5-Carboxy-thiazol-2-yl)-1-(3-phenylpropiolyl)-piperazinethylester durch Kristallisation aus Ethanol erhalten.

### Beispiel AAA77: Synthese von 4-(4-Carboxy-thiazol-2-yl)-1-(3-phenyl-propiolyl)-piperazin ethylester

Durch die Umsetzung von 2-Piperazin-1-yl-thiazol-4-carbonsäureethylester [Vorstufe BBB25] mit Phenylpropiolsäure gemäß den bei Beispiel AAA76 beschriebenen Bedingungen wurde 4-(4-Carboxy-thiazol-2-yl)-1-(3-phenyl-propiolyl)-piperazinethylester erhalten.

### Beispiel AAA78: Synthese von 4-(5-Carboxy-thiazol-2-yl)-1-(3-phenyl-propiolyl)-piperazin

Durch die Umsetzung 4-(5-Carboxy-thiazol-2-yl)-1-(3-phenyl-propiolyl)-piperazin [Beispiel AAA76] mit NaOH gemäß den bei Beispiel AAA46 beschriebenen Bedingungen wurde 4-(5-Carboxy-thiazol-2-yl)-1-(3-phenyl-propiolyl)-piperazin erhalten.

### Beispiel AAA79: Synthese von 4-(4-Carboxy-thiazol-2-yl)-1-(3-phenyl-propiolyl)-piperazin

Durch die Umsetzung 4-(4-Carboxy-thiazol-2-yl)-1-(3-phenyl-propiolyl)-piperazin [Beispiel AAA77] mit NaOH gemäß den bei Beispiel AAA46 beschriebenen Bedingungen wurde 4-(4-Carboxy-thiazol-2-yl)-1-(3-phenyl-propiolyl)-piperazin erhalten.

### Beispiel AAA80: Synthese von 4-(5-Methylaminocarbonyl-thiazol-2-yl)-1-(3-phenyl-propiolyl)-piperazin

Ausgehend von 4-(5-Carboxy-thiazol-2-yl)-1-(3-phenyl-propiolyl)-piperazin [Beispiel AAA78] wurde nach dem unter AAA51 beschriebenen Verfahren 4-(5-Methylaminocarbonyl-thiazol-2-yl)-1-(3-phenyl-propiolyl)-piperazin erhalten.

### Beispiel AAA81: Synthese von 4-(5-Dimethylaminocarbonyl-thiazol-2-yl)-1-(3-phenyl-propiolyl)-piperazin

Ausgehend von 4-(5-Carboxy-thiazol-2-yl)-1-(3-phenyl-propiolyl)-piperazin [Beispiel AAA78] wurde nach dem unter AAA52 beschriebenen Verfahren 4-(5-Dimethylaminocarbonyl-thiazol-2-yl)-1-(3-phenyl-propiolyl)-piperazin erhalten.

### Beispiel AAA82: Synthese von 4-(3-Phenyl-1,2,4-thiadiazol-5-yl)-1-(3-phenylpropiolyl)-piperazin

Durch die Umsetzung 1-(3-Phenyl-[1,2,4]-thiadiazol-5-yl)-piperazin mit Phenylpropiolsäure gemäß den bei Beispiel AAA76 beschriebenen Bedingungen wurde 4-(3-Phenyl-1,2,4-thiadiazol-5-yl)-1-(3-phenyl-propiolyl)-piperazin erhalten.

### Beispiel AAA83: Synthese von 4-(Thiazol-2-yl)-1-(3-(chinol-7-yl)-propiolyl)-piperazin

Durch die Umsetzung von 4-(Thiazol-2-yl)-1-propiolyl-piperazin [Vorstufe BBB2] mit Trifluor-methansulfonsäurechinolin-7-yl ester [Vorstufe BBB19] gemäß den bei Beispiel AAA1 beschriebenen Bedingungen wurde 4-(Thiazol-2-yl)-1-(3-(chinol-7-yl)-propiolyl)-piperazin erhalten.

### Beispiel AAA85: Synthese 4-(3-(4-Fluorbenzyl)-1,2,4-thiadiazol-5-yl)-1-(3-phenyl-propiolyl)-piperazin

Durch die Umsetzung von 1-[3-(4-Fluor-benzyl)-[1,2,4]-thiadiazol-5-yl]-piperazin [Vorstufe BBB27] mit Phenylpropiolsäure gemäß den bei Beispiel AAA76 beschriebenen Bedingungen wurde 4-(3-(4-Fluorbenzyl)-1,2,4-thiadiazol-5-yl)-1-(3-phenyl-propiolyl)-piperazin erhalten.

### Beispiel AAA86: Synthese von 4-(5-Nitro-thiazol-2-yl)-1-(3-phenyl-propiolyl)-piperazin

Durch die Umsetzung von 1-(5-Nitro-thiazol-2-yl)-piperazin [Vorstufe BBB29] mit Phenylpropiolsäure gemäß den bei Beispiel AAA76 beschriebenen Bedingungen wurde 4-(5-Nitro-thiazol-2-yl)-1-(3-phenyl-propiolyl)-piperazin erhalten.

### Beispiel AAA87: Synthese von 4-(4-tert-Butyl-thiazol-2-yl)-1-(3-phenylpropiolyl)-piperazin

Durch die Umsetzung von 1-(4-tert-Butyl-thiazol-2-yl)-piperazin [Vorstufe BBB31] mit Phenylpropiolsäure gemäß den bei Beispiel AAA76 beschriebenen Bedingungen wurde 4-(4-tert-Butyl-thiazol-2-yl)-1-(3-phenyl-propiolyl)-piperazin erhalten.

### Beispiel AAA88: Synthese von 4-(5-Methyl-thiazol-2-yl)-1-(3-phenyl-propiolyl)-piperazin

Durch die Umsetzung von 1-(5-Methyl-thiazol-2-yl)-piperazin [Vorstufe BBB33] mit Phenylpropiolsäure gemäß den bei Beispiel AAA76 beschriebenen Bedingungen wurde 4-(5-Methyl-thiazol-2-yl)-1-(3-phenyl-propiolyl)-piperazin erhalten.

### Beispiel AAA89: Synthese von 4-(4,5-Dimethyl-thiazol-2-yl)-1-(3-phenylpropiolyl)-piperazin

Durch die Umsetzung von 1-(4,5-Dimethyl-thiazol-2-yl)-piperazin [Vorstufe BBB35] mit Phenylpropiolsäure gemäß den bei Beispiel AAA76 beschriebenen Bedingungen wurde 4-(4,5-Dimethyl-thiazol-2-yl)-1-(3-phenyl-propiolyl)-piperazin erhalten.

### Beispiel AAA90: Synthese von 4-(5-Brom-4-phenyl-thiazol-2-yl)-1-(3-phenylpropiolyl)-piperazin

Durch die Umsetzung von 1-(5-Brom-4-phenyl-thiazol-2-yl)-piperazin [Vorstufe BBB37] mit Phenylpropiolsäure gemäß den bei Beispiel AAA76 beschriebenen Bedingungen wurde 4-(5-Brom-4-phenyl-thiazol-2-yl)-1-(3-phenyl-propiolyl)-piperazin erhalten.

### Beispiel AAA91: Synthese von 4-(5-Brom-4-methyl-thiazol-2-yl)-1-(3-phenylpropiolyl)-piperazin

Durch die Umsetzung von 1-(5-Brom-4-methyl-thiazol-2-yl)-piperazin [Vorstufe BBB39] mit Phenylpropiolsäure gemäß den bei Beispiel AAA76 beschriebenen Bedingungen wurde 4-(5-Brom-4-phenyl-thiazol-2-yl)-1 -(3-phenyl-propiolyl)-piperazin erhalten.

### Beispiel AAA92: Synthese von 4-(4-Methyl-thiazol-2-yl)-1-(3-phenyl-propiolyl)-piperazin

Durch die Umsetzung von 1-(4-Methyl-thiazol-2-yl)-piperazin [Vorstufe BBB41] mit Phenylpropiolsäure gemäß den bei Beispiel AAA76 beschriebenen Bedingungen wurde 4-(4-Methyl-thiazol-2-yl)-1-(3-phenyl-propiolyl)-piperazin erhalten.

### Beispiel AAA93: Synthese von 4-(4-Trifluormethyl-thiazol-2-yl)-1-(3-phenylpropiolyl)-piperazin

Durch die Umsetzung von 1-(4-Trifluormethyl-thiazol-2-yl)-piperazin [Vorstufe BBB43] mit Phenylpropiolsäure gemäß den bei Beispiel AAA76 beschriebenen Bedingungen wurde 4-(4-Trifluormethyl-thiazol-2-yl)-1-(3-phenyl-propiolyl)-piperazin erhalten.

### Beispiel AAA94: Synthese von 4-(4-Chlor-thiazol-2-yl)-1-(3-phenyl-propiolyl)-piperazin

Durch die Umsetzung von 1-(4-Chlor-thiazol-2-yl)-piperazin [Vorstufe BBB45] mit Phenylpropiolsäure gemäß den bei Beispiel AAA76 beschriebenen Bedingungen wurde 4-(4-Chlor-thiazol-2-yl)-1-(3-phenyl-propiolyl)-piperazin erhalten.

### Beispiel AAA95: Synthese von 4-(5-Chlor-thiazol-2-yl)-1-(3-phenyl-propiolyl)-piperazin

Durch die Umsetzung von 1-(5-Chlor-thiazol-2-yl)-piperazin [Vorstufe BBB47] mit Phenylpropiolsäure gemäß den bei Beispiel AAA76 beschriebenen Bedingungen wurde 4-(5-Chlor-thiazol-2-yl)-1-(3-phenyl-propiolyl)-piperazin erhalten.

### Beispiel AAA96: Synthese von 4-(5-Brom-thiazol-2-yl)-1-(3-phenyl-propiolyl)-piperazin

Durch die Umsetzung von 1-(5-Brom-thiazol-2-yl)-piperazin [Vorstufe BBB48] mit Phenylpropiolsäure gemäß den bei Beispiel AAA76 beschriebenen Bedingungen wurde 4-(5-Brom-thiazol-2-yl)-1-(3-phenyl-propiolyl)-piperazin erhalten.

### Beispiel AAA97: Synthese von 4-(4-Brom-thiazol-2-yl)-1-(3-phenyl-propiolyl)-piperazin

Durch die Umsetzung von 1-(4-Brom-thiazol-2-yl)-piperazin [Vorstufe BBB49] mit Phenylpropiolsäure gemäß den bei Beispiel AAA76 beschriebenen Bedingungen wurde 4-(4-Brom-thiazol-2-yl)-1-(3-phenyl-propiolyl)-piperazin erhalten.

### Beispiel AAA98: Synthese von 4-(5-Phenyl-thiazol-2-yl)-1-(3-phenyl-propiolyl)-piperazin

Zu einer Lösung von 376 mg (1.0 mmol) 4-(5-Brom-thiazol-2-yl)-1-(3-phenylpropiolyl)-piperazin [Beispiel AAA96], 171 mg (1.4 mmol) Phenylboronsäure und 30 mg Pd(PPh₃)₂Cl₂ in DCE (4 ml) wurde eine Lösung von 265 mg (2.5 mmol) Na₂CO₃ in Wasser (1.6 ml) gegeben. Anschließend wurde 3 h unter Rückfluß erhitzt. Die abgekühlte Reaktionslösung wurde mit Wasser versetzt und mit EE extrahiert. Die organische Phase wurde mit Wasser und einer ges. aq. NaCl-Lsg gewaschen, über MgSO₄ getrocknet, filtriert und im Vakuum eingeengt. Der Rückstand wurde aus Diethylether kristallisiert. Durch SC (Chloroform) mit dem Kristallisationsprodukt wurden 160 mg (0.43 mmol, 43%) 4-(5-Phenyl-thiazol-2-yl)-1-(3-phenyl-propiolyl)-piperazin erhalten.

### Beispiel AAA99: Synthese von Synthese von 4-(4-Phenyl-thiazol-2-yl)-1-(3-phenyl-propiolyl)-piperazin

Ausgehend von 4-(4-Brom-thiazol-2-yl)-1-(3-phenyl-propiolyl)-piperazin [Beispiel AAA97] wurde nach dem unter AAA98 beschriebenen Verfahren 4-(4-Phenyl-thiazol-2-yl)-1-(3-phenyl-propiolyl)-piperazin erhalten.

### Beispiel AAA00100: Synthese von 4-(3-Methylmercapto-1,2,4-thiadiazol-5-yl)-1-(3-phenyl-propiolyl)-piperazin

Durch die Umsetzung von 1-(3-Methyl-sulfanyl-[1,2,4]-thiadiazol-5-yl)-piperazin [Vorstufe BBB50] mit Phenylpropiolsäure gemäß den bei Beispiel AAA76 beschriebenen Bedingungen wurde 4-(3-Methylmercapto-1,2,4-thiadiazol-5-yl)-1-(3-phenyl-propiolyl)-piperazin erhalten.

### Beispiel AAA00101: Synthese von 4-(3-Methansulfonyl-1,2,4-thiadiazol-5-yl)-1-(3-phenyl-propiolyl)-piperazin

Durch die Umsetzung von 1-(3-Methan-sulfonyl-[1,2,4]-thiadiazol-5-yl)-piperazin [Vorstufe BBB51] mit Phenylpropiolsäure gemäß den bei Beispiel AAA76 beschriebenen Bedingungen wurde 4-(3-Methansulfonyl-1,2,4-thiadiazol-5-yl)-1-(3-phenyl-propiolyl)-piperazin erhalten.

### Beispiel AAA00102: Synthese von 4-(3-Methoxy-1,2,4-thiadiazol-5-yl)-1-(3-phenyl-propiolyl)-piperazin

Durch die Umsetzung von 1-(3-Methoxy-[1,2,4]-thiadiazol-5-yl)-piperazin [Vorstufe BBB52] mit Phenylpropiolsäure gemäß den bei Beispiel AAA76 beschriebenen Bedingungen wurde 4-(3-Methoxy-1,2,4-thiadiazol-5-yl)-1-(3-phenyl-propiolyl)-piperazin erhalten.

### Beispiel AAA00103: Synthese von 4-(1,2,4-Thiadiazol-5-yl)-1-(3-phenylpropiolyl)-piperazin

Durch die Umsetzung von 1-[1,2,4]-thiadiazol-5-yl-piperazin [Vorstufe BBB57] mit Phenylpropiolsäure gemäß den bei Beispiel AAA76 beschriebenen Bedingungen wurde 4-(1,2,4-Thiadiazol-5-yl)-1-(3-phenyl-propiolyl)-piperazin erhalten.

### Beispiel AAA00104: Synthese von 4-(3-Methyl-1,2,4-thiadiazol-5-yl)-1-(3-phenylpropiolyl)-piperazin

Durch die Umsetzung von 1-(3-Methyl-[1,2,4]-thiadiazol-5-yl)-piperazin [Vorstufe BBB58] mit Phenylpropiolsäure gemäß den bei Beispiel AAA76 beschriebenen Bedingungen wurde 4-(3-Methyl-1,2,4-thiadiazol-5-yl)-1-(3-phenyl-propiolyl)-piperazin erhalten.

### Beispiel AAA00105: Synthese von 4-(3-Trifluormethyl-1,2,4-thiadiazol-5-yl)-1-(3-phenyl-propiolyl)-piperazin

Durch die Umsetzung von 1-(3-Trifluormethyl-[1,2,4]-thiadiazol-5-yl)-piperazin [Vorstufe BBB59] mit Phenylpropiolsäure gemäß den bei Beispiel AAA76 beschriebenen Bedingungen wurde 4-(3-Trifluormethyl-1,2,4-thiadiazol-5-yl)-1-(3-phenyl-propiolyl)-piperazin erhalten.

### Beispiel AAA00106: Synthese von 4-(5-(3-Methyl-1,2,4-oxadiazol-5-yl)-thiazol-2-yl)-1-(3-phenyl-propiolyl)-piperazin

Durch die Umsetzung von 1-[5-(3-Methyl-[1,2,4]-oxadiazol-5-yl)-thiazol-2-yl]-piperazin [Vorstufe BBB63] mit Phenylpropiolsäure gemäß den bei Beispiel AAA76 beschriebenen Bedingungen wurde 4-(5-(3-Methyl-1,2,4-oxadiazol-5-yl)-thiazol-2-yl)-1-(3-phenyl-propiolyl)-piperazin erhalten.

### Beispiel AAA00107: Synthese von 4-(4-tert-Butyl-thiazol-2-yl)-1-(3-(4-aminophenyl)-propiolyl)-piperazin

Durch die Umsetzung von 4-(4-tert-Butyl-thiazol-2-yl)-1-propiolyl-piperazin [Vorstufe BBB65] mit 4-lod-anilin gemäß den bei Beispiel AAA1 beschriebenen Bedingungen wurde von 4-(4-tert-Butyl-thiazol-2-yl)-1-(3-(4-amino-phenyl)-propiolyl)-piperazin erhalten.

### Beispiel AAA00108: Synthese von 4-(Thiazol-2-yl)-1-(3-(1-Methyl-indol-6-yl)-propiolyl)-piperazin

Durch die Umsetzung von 4-(Thiazol-2-yl)-1-propiolyl-piperazin [Vorstufe BBB2] mit 6-Brom-1-Methyl-indol unter den bei Beispiel AAA35 beschriebenen Bedingungen wurde 4-(Thiazol-2-yl)-1-(3-(1-Methyl-indol-6-yl)-propiolyl)-piperazin erhalten.

### Beispiel AAA00109: Synthese von 4-(Thiazol-2-yl)-1-(3-(3-acetyl-phenyl)-propiolyl)-piperazin

Durch die Umsetzung von 4-(Thiazol-2-yl)-1-propiolyl-piperazin [Vorstufe BBB2] mit 3-Acetyl-iodbenzol unter den bei Beispiel AAA1 beschriebenen Bedingungen wurde 4-(Thiazol-2-yl)-1-(3-(3-acetyl-phenyl)-propiolyl)-piperazin erhalten.

### Beispiel AAA00110: Synthese von 4-(Thiazol-2-yl)-1-(3-(3-fluor-5-methylphenyl)-propiolyl)-piperazin

Durch die Umsetzung von 4-(Thiazol-2-yl)-1-propiolyl-piperazin [Vorstufe BBB2] mit 3-Brom-5-fluor-toluol unter den bei Beispiel AAA35 beschriebenen Bedingungen wurde 4-(Thiazol-2-yl)-1-(3-(3-fluor-5-methyl-phenyl)-propiolyl)-piperazin erhalten.

### Beispiel AAA00111: Synthese von 4-(Thiazol-2-yl)-1-(3-(2-fluor-3-methylphenyl)-propiolyl)-piperazin

Durch die Umsetzung von 4-(Thiazol-2-yl)-1-propiolyl-piperazin [Vorstufe BBB2] mit 3-Brom-2-fluor-toluol unter den bei Beispiel AAA35 beschriebenen Bedingungen wurde 4-(Thiazol-2-yl)-1-(3-(2-fluor-3-methyl-phenyl)-propiolyl)-piperazin erhalten.

### Beispiel AAA00112: Synthese von 4-(Thiazol-2-yl)-1-(3-(3-methylamino-phenyl)-propiolyl)-piperazin

Durch die Umsetzung von 4-(Thiazol-2-yl)-1-propiolyl-piperazin [Vorstufe BBB2] mit 3-lod-N-methylanilin unter den bei Beispiel AAA1 beschriebenen Bedingungen wurde 4-(Thiazol-2-yl)-1-(3-(3-methylamino-phenyl)-propiolyl)-piperazin erhalten.

### Beispiel AAA00113: Synthese von 4-(Thiazol-2-yl)-1-(3-(3-dimethylaminophenyl)-propiolyl)-piperazin

Durch die Umsetzung von 4-(Thiazol-2-yl)-1-propiolyl-piperazin [Vorstufe BBB2] mit 3-lod-N,N-dimethylanilin unter den bei Beispiel AAA1 beschriebenen Bedingungen wurde 4-(Thiazol-2-yl)-1-(3-(3-dimethylamino-phenyl)-propiolyl)-piperazin erhalten.

### Beispiel AAA00114: Synthese von 4-(Thiazol-2-yl)-1-(3-(3-dimethylaminophenyl)-propiolyl)-piperazin

Zu einer Lösung von 885 mg (4.0 mmol) 4-(Thiazol-2-yl)-1-propiolyl-piperazin [Vorstufe BBB2] in MeCN (10 ml) wurden unter Kühlen (Eisbad) 240 mg (6.0 mmol) Natriumhydrid zugegeben. Nach 15 min Rühren bei RT wurde mit 479 µl (5.2 mmol) Dimethylcarbamoylchlorid versetzt und weitere 16 h bei RT gerührt. Anschließend wurde mit Wasser und Toluol verdünnt und die Phasen wurden getrennt. Die wässrige Phase wurde mit Toluol extrahiert und die vereinigten organischen Phasen wurden über MgSO₄ getrocknet, filtriert und im Vakuum eingeengt. Mit dem Rückstand wurde eine SC mit einem Gemisch aus Chloroform und EE durchgeführt, wobei 137 mg (0.5 mmol, 12%) 4-(Thiazol-2-yl)-1-(3-(3-dimethylamino-phenyl)-propiolyl)-piperazin isoliert wurden.

### Beispiel AAA00115: Synthese von 4-(Thiazol-2-yl)-1-(3-(3-methylsulfinylphenyl)-propiolyl)-piperazin

Durch die Umsetzung von 4-(Thiazol-2-yl)-1-propiolyl-piperazin [Vorstufe BBB2] mit 3-Methylsulfinyl-brombenzol unter den bei Beispiel AAA35 beschriebenen Bedingungen wurde 4-(Thiazol-2-yl)-1-(3-(3-methylsulfinyl-phenyl)-propiolyl)-piperazin erhalten.

### Beispiel AAA00116: Synthese von 4-(Thiazol-2-yl)-1-(3-(3-methylsulfonylphenyl)-propiolyl)-piperazin

Durch die Umsetzung von 4-(Thiazol-2-yl)-1-propiolyl-piperazin [Vorstufe BBB2] mit 3-Methylsulfonyl-brombenzol unter den bei Beispiel AAA35 beschriebenen Bedingungen wurde 4-(Thiazol-2-yl)-1-(3-(3-methylsulfonyl-phenyl)-propiolyl)-piperazin erhalten.

### Beispiel AAA00117: Synthese von 4-(Thiazol-2-yl)-1-(3-(3-ethinyl-phenyl)-propiotyl)-piperazin

Eine Mischung aus 387 mg (0.98 mmol) 4-(Thiazol-2-yl)-1-(3-(3-trimethylsilanylethinyl-phenyl)-propiolyl)-piperazin [Beispiel AAA00125] und 39 mg Tetrabutylammoniumfluorid Trihydrat in MeCN (20 ml) wurde 120 min bei RT gerührt. Anschliessend wurde im Vakuum eingeengt. Der Rückstand wurde mit DCM aufgenommen und mit Wasser gewaschen. Die organische Phase wurde über MgSO₄ getrocknet, filtriert und im Vakuum eingeengt. Mit dem Rückstand wurde eine SC mit DCE durchgeführt, wobei 214 mg (0.67 mmol, 68%) 4-(Thiazol-2-yl)-1-(3-(3-ethinyl-phenyl)-propiolyl)-piperazin erhalten.

### Beispiel AAA118: Synthese von 4-(Thiazol-2-yl)-1-(3-(4-methyl-thiophen-2-yl)-propiolyl)-piperazin

Durch die Umsetzung von 1-Thiazol-2-yl-piperazin [Vorstufe BBB1] mit (4-Methylthiophen-2-yl)-propiolsäure [Vorstufe BBB67] unter den bei Beispiel AAA63 beschriebenen Bedingungen wurde 4-(Thiazol-2-yl)-1-(3-(4-methyl-thiophen-2-yl)-propiolyl)-piperazin erhalten.

### Beispiel AAA119: Synthese von 2-Methyl-4-(thiazol-2-yl)-1-(3-(3-chlor-phenyl)-propiolyl)-piperazin

Durch die Umsetzung von 3-Methyl-1-thiazol-2-yl-piperazin [Vorstufe BBB14] mit (3-Chlor-phenyl)-propiolsäure [Vorstufe BBB68] unter den bei Beispiel AAA63 beschriebenen Bedingungen wurde 2-Methyl-4-(thiazol-2-yl)-1-(3-(3-chlor-phenyl)-propiolyl)-piperazin erhalten.

### Beispiel AAA00120: Synthese von 4-(Thiazol-2-yl)-1-(3-(3-ethyl-phenyl)-propiolyl)-piperazin

Durch die Umsetzung von 4-(Thiazol-2-yl)-1-propiolyl-piperazin [Vorstufe BBB2] mit 3-lod-ethylbenzol unter den bei Beispiel AAA1 beschriebenen Bedingungen wurde 4-(Thiazol-2-yl)-1-(3-(3-ethyl-phenyl)-propiolyl)-piperazin erhalten.

### Beispiel AAA121: Synthese von 2-tert-Butyl-4-(thiazol-2-yl)-1-(3-phenylpropiolyl)-piperazin

Durch die Umsetzung von 3-tert-Butyl-1-thiazol-2-yl-piperazin [Vorstufe BBB69] mit Phenyl-propiolsäure unter den bei Beispiel AAA63 beschriebenen Bedingungen wurde 2-tert-Butyl-4-(thiazol-2-yl)-1-(3-phenyl-propiolyl)-piperazin erhalten.

### Beispiel AAA00122: Synthese von 4-(Thiazol-2-yl)-1-(3-(3-difluormethyl-phenyl)-propiolyl)-piperazin

Durch die Umsetzung von 4-(Thiazol-2-yl)-1-propiolyl-piperazin [Vorstufe BBB2] mit 3-Difluormethyl-iodbenzol unter den bei Beispiel AAA1 beschriebenen Bedingungen wurde 4-(Thiazol-2-yl)-1-(3-(3-difluormethyl-phenyl)-propiolyl)-piperazin erhalten.

### Beispiel AAA123: Synthese von 2-Methyl-4-(thiazol-2-yl)-1-(3-(3-cyano-phenyl)-propiolyl)-piperazin

Durch die Umsetzung von 3-Methyl-1-thiazol-2-yl-piperazin [Vorstufe BBB14] mit (3-Cyano-phenyl)-propiolsäure unter den bei Beispiel AAA63 beschriebenen Bedingungen wurde 2-Methyl-4-(thiazol-2-yl)-1-(3-(3-cyano-phenyl)-propiolyl)-piperazin erhalten.

### Beispiel AAA124: Synthese von 2-Isopropyl-4-(thiazol-2-yl)-1-(3-phenylpropiolyl)-piperazin

Durch die Umsetzung von 3-Isopropyl-1-thiazol-2-yl-piperazin [Vorstufe BBB70] mit Phenyl-propiolsäure unter den bei Beispiel AAA63 beschriebenen Bedingungen wurde 2-Isopropyl-4-(thiazol-2-yl)-1=(3-phenyl-propiolyl)-piperazin erhalten.

### Beispiel AAA00125: Synthese von 4-(Thiazol-2-yl)-1-(3-(3-trimethylsilanylethinyl-phenyl)-propiolyl)-piperazin

Durch die Umsetzung von 4-(Thiazol-2-yl)-1-propiolyl-piperazin [Vorstufe BBB2] mit 3-Trimethylsilanylethinyl-iodbenzol unter den bei Beispiel AAA1 beschriebenen Bedingungen wurde 4-(Thiazol-2-yl)-1-(3-(3-trimethylsilanylethinyl-phenyl)-propiolyl)-piperazin erhalten.

Die erfindungsgemäßen Beispiele sind in der folgenden Übersicht zuammengefasst:

| | |
|---|---|
| AAA00100 | 4-(3-Methylmercapto-1,2,4-thiadiazol-5-yl)-1-(3-phenyl-propiolyl)-piperazin |
| AAA00101 | 4-(3-Methansulfonyl-1,2,4-thiadiazol-5-yl)-1-(3-phenyl-propiolyl)-piperazin |
| AAA00102 | 4-(3-Methoxy-1,2,4-thiadiazol-5-yl)-1-(3-phenyl-propiolyl)-piperazin |
| AAA00103 | 4-(1,2,4-Thiadiazol-5-yl)-1-(3-phenyl-propiolyl)-piperazin |
| AAA00104 | 4-(3-Methyl-1,2,4-thiadiazol-5-yl)-1-(3-phenyl-propiolyl)-piperazin |
| AAA00105 | 4-(3-Trifluormethyl-1,2,4-thiadiazol-5-yl)-1-(3-phenyl-propiolyl)-piperazin |
| AAA00106 | 4-(5-(3-Methyl-1,2,4-oxadiazol-5-yl)-thiazol-2-yl)-1 -(3-phenyl-propiolyl)-piperazin |
| AAA00107 | 4-(4-tert-Butyl-thiazol-2-yl)-1-(3-(4-amino-phenyl)-propiolyl)-piperazin |
| AAA1 | 4-(Thiazol-2-yl)-1-(3-(2-carboxy-phenyl)-propiolyl)-piperazin methylester |
| AAA10 | 4-(Thiazol-2-yl)-1-(3-(2-thienyl)-propiolyl)-piperazin |
| AAA11 | 4-(Thiazol-2-yl)-1-(3-(3-thienyl)-propiolyl)-piperazin |
| AAA12 | 4-(Thiazol-2-yl)-1-(3-(2-methoxy-phenyl)-propiolyl)-piperazin |
| AAA13 | 4-(Thiazol-2-yl)-1-(3-(3-methoxy-phenyl)-propiolyl)-piperazin |
| AAA14 | 4-(Thiazol-2-yl)-1-(3-(4-methoxy-phenyl)-propiolyl)-piperazin |
| AAA15 | 4-(Thiazol-2-yl)-1-(3-(2-cyano-phenyl)-propiolyl)-piperazin |
| AAA16 | 4-(Thiazol-2-yl)-1-(3-(3-cyano-phenyl)-propiolyl)-piperazin |
| AAA17 | 4-(Thiazol-2-yl)-1-(3-(4-cyano-phenyl)-propiolyl)-piperazin |
| AAA18 | 4-(Thiazol-2-yl)-1-(3-(2,4-dimethyl-phenyl)-propiolyl)-piperazin |
| AAA19 | 4-(Thiazol-2-yl)-1-(3-(3,5-dimethyl-phenyl)-propiolyl)-piperazin |
| AAA2 | 4-(Thiazol-2-yl)-1-(3-(4-carboxy-phenyl)-propiolyl)-piperazin methylester |
| AAA20 | 4-(Thiazol-2-yl)-1-(3-(2,6-dimethyl-phenyl)-propiolyl)-piperazin |
| AAA21 | 4-(Thiazol-2-yl)-1-(3-(2-fluor-phenyl)-propiolyl)-piperazin |
| AAA22 | 4-(Thiazol-2-yl)-1-(3-(3-fluor -phenyl)-propiolyl)-piperazin |
| AAA23 | 4-(Thiazol-2-yl)-1-(3-(2-chlor-phenyl)-propiolyl)-piperazin |
| AAA24 | 4-(Thiazol-2-yl)-1-(3-(3-chlor-phenyl)-propiolyl)-piperazin |
| AAA25 | 4-(Thiazol-2-yl)-1-(3-naphthyl-propiolyl)-piperazin |
| AAA26 | 4-(Thiazol-2-yl)-1-(3-(2,3-dimethyl-phenyl)-propiolyl)-piperazin |
| AAA27 | 4-(Thiazol-2-yl)-1-(3-(3,4-dimethyl-phenyl)-propiolyl)-piperazin |
| AAA28 | 4-(Thiazol-2-yl)-1-(3-(3-nitro-phenyl)-propiolyl)-piperazin |
| AAA29 | 4-(Thiazol-2-yl)-1-(3-(2-nitro-phenyl)-propioly!)-piperazin |
| AAA3 | 4-(Thiazol-2-yl)-1-(3-(3-carboxy-phenyl)-propiolyl)-piperazin ethylester |
| AAA30 | 4-(Thiazol-2-yl)-1-(3-(3-formyl-phenyl)-propiolyl)-piperazin |
| AAA31 | 4-(Thiazol-2-yl)-1-(3-(3-ethenyl-phenyl)-propiolyl)-piperazin |
| AAA32 | 4-(Thiazol-2-yl)-1-(3-pyrid-2-yl-propiolyl)-piperazin |
| AAA33 | 4-(Thiazol-2-yl)-1-(3-pyrid-3-yl-propiolyl)-piperazin |
| AAA24 | 4-(Thiazol-2-yl)-1-(3-pyrid-4-yl-propiolyl)-piperazin |
| AAA35 | 4-(Thiazol-2-yl)-1-(3-(chinolin-6-yl)-propiolyl)-piperazin |
| AAA36 | 4-(Thiazol-2-yl)-1-(3-(3-isopropyl-phenyl)-propiolyl)-piperazin |
| AAA37 | 4-(Thiazol-2-yl)-1-(3-biphenyl-3-yl-propiolyl)-piperazin |
| AAA38 | 4-(Thiazol-2-yl)-1-(3-naphth-2-yl-propiolyl)-piperazin |
| AAA39 | 4-(Thiazol-2-yl)-1 -(3-(1 -methyl-indol-5-yl)-propiolyl)-piperazin |
| AAA4 | 4-(Thiazol-2-yl)-1-(3-(2-hydroxy-phenyl)-propiolyl)-piperazin |
| AAA40 | 4-(Thiazol-2-yl)-1-(3-(3-methylmercapto-phenyl)-propiolyl)-piperazin |
| AAA41 | 4-(Thiazol-2-yl)-1-(3-(3-cyano-4-fluor-phenyl)-propiolyl)-piperazin |
| AAA42 | 4-(Thiazol-2-yl)-1-(3-(3-methoxymethyl-phenyl)-propiolyl)-piperazin |
| AAA43 | 4-(Thiazol-2-yl)-1-(3-(3-hydroxy-phenyl)-propiolyl)-piperazin |
| AAA44 | 4-(Thiazol-2-yl)-1-(3-(3-acetamino-phenyl)-propiolyl)-piperazin |
| AAA45 | 4-(Thiazol-2-yl)-1-(3-(4-acetamino-phenyl)-propiolyl)-piperazin |
| AAA46 | 4-(Thiazol-2-yl)-1-(3-(2-carboxy-phenyl)-propiolyl)-piperazin |
| AAA47 | 4-(Thiazol-2-yl)-1-(3-(3-carboxy-phenyl)-propiolyl)-piperazin |
| AAA48 | 4-(Thiazol-2-yl)-1-(3-(4-carboxy-phenyl)-propiolyl)-piperazin |
| AAA49 | 4-(Thiazol-2-yl)-1-(3-(3-carboxy-phenyl)-propiolyl)-piperazin methylester |
| AAA5 | 4-(Thiazol-2-yl)-1-(3-(4-hydroxy-phenyl)-propiolyl)-piperazin |
| AAA50 | 4-(Thiazol-2-yl)-1-(3-(3-aminocarbonyl-phenyl)-propiolyl)-piperazin |
| AAA51 | 4-(Thiazol-2-yl)-1-(3-(3-methylaminocarbonyl-phenyl)-propiolyl)-piperazin |
| AAA52 | 4-(Thiazol-2-yl)-1-(3-(3-dimethylaminocarbonyl-phenyl)-propiolyl)-piperazin |
| AAA53 | 4-(Thiazol-2-yl)-1-(3-(2-tolyl)-propiolyl)-piperazin Hydrochlorid |
| AAA54 | 4-(Thiazol-2-yl)-1-(3-(3-tolyi)-propiolyl)-piperazin Hydrochlorid |
| AAA55 | 4-(Thiazol-2-yl)-1-(3-(4-tolyl)-propiolyl)-piperazin Hydrochlorid |
| AAA56 | 4-(Thiazol-2-yl)-1-(3-phenyl-propiolyl)-piperazin Hydrochlorid |
| AAA57 | 4-(Thiazol-2-yl)-1-(3-(2-trifluormethyl-phenyl)-propiolyl)-piperazin Hydrochlorid |
| AAA58 | 4-(Thiazol-2-yl)-1-(3-(3-trifluormethyl-phenyl)-propiolyl)-piperazin Hydrochlorid |
| AAA59 | 4-(Thiazol-2-yl)-1-(3-(4-trifluormethyl-phenyl)-propiolyl)-piperazin Hydrochlorid |
| AAA6 | 4-(Thiazol-2-yl)-1-(3-(2-amino-phenyl)-propiolyl)-piperazin |
| AAA60 | 4-(Thiazol-2-yl)-1-(3-pentyl-propiolyl)-piperazin |
| AAA61 | 4-(Thiazol-2-yl)-1-(3-(4-fluor-phenyl)-propiolyl)-piperazin |
| AAA62 | 4-(Thiazol-2-yl)-1-(3-(4-chlor-phenyl)-propiolyl)-piperazin |
| AAA63 | 2-Methyl-4-(thiazol-2-yl)-1-(3-phenyl-propiolyl)-piperazin |
| AAA64 | (S)-2-Methyl-4-(thiazol-2-yl)-1-(3-phenyl-propiolyl)-piperazin |
| AAA65 | (R)-2-Methyl-4-(thiazol-2-yl)-1-(3-phenyl-propiolyl)-piperazin |
| AAA66 | 2-Methyl-4-(thiazol-2-yl)-1-(3-phenyl-propiolyl)-piperazin Hydrochlorid |
| AAA67 | 2-Methyl-4-(thiazol-2-yl)-1-(3-(3-tolyl)-propiolyl)-piperazin |
| AAA68 | 2-Methyl-4-(thiazol-2-yl)-1-(3-(3-tolyl)-propiolyl)-piperazin Hydrochlorid |
| AAA69 | 4-(Thiazol-2-yl)-1-(3-cyclohexyl-propiolyl)-piperazin |
| AAA7 | 4-(Thiazol-2-yl)-1-(3-(3-amino-phenyl)-propiolyl)-piperazin |
| AAA70 | 4-(Thiazol-2-yl)-1-(3-methyl-propiolyl)-piperazin |
| AAA71 | 2-Ethyl-4-(thiazol-2-yl)-1-(3-phenyl-propiolyl)-piperazin |
| AAA72 | 2-Phenyl-4-(thiazol-2-yl)-1-(3-phenyl-propiolyl)-piperazin |
| AAA73 | 4-(Thiazol-2-yl)-1-(3-(2-furyl)-propiolyl)-piperazin |
| AAA74 | 4-(Thiazol-2-yl)-1-(3-(3-furyl)-propiolyl)-piperazin |
| AAA75 | cis-2,6-Dimethyl-4-(thiazol-2-yl)-1-(3-phenyl-propiolyl)-piperazin |
| AAA76 | 4-(5-Carboxy-thiazol-2-yl)-1-(3-phenyl-propiolyl)-piperazin ethylester |
| AAA77 | 4-(4-Carboxy-thiazol-2-yl)-1-(3-phenyl-propiolyl)-piperazin ethylester |
| AAA78 | 4-(5-Carboxy-thiazol-2-yl)-1-(3-phenyl-propiolyl)-piperazin |
| AAA79 | 4-(4-Carboxy-thiazol-2-yl)-1-(3-phenyl-propiolyl)-piperazin |
| AAA8 | 4-(Thiazol-2-yl)-1-(3-(4-amino-phenyl)-propiolyl)-piperazin |
| AAA80 | 4-(5-Methylaminocarbonyl-thiazol-2-yl)-1-(3-phenyl-propiolyl)-piperazin |
| AAA81 | 4-(5-Dimethylaminocarbonyl-thiazol-2-yl)-1-(3-phenyl-propiolyl)-piperazin |
| AAA82 | 4-(3-Phenyl-1,2,4-thiadiazol-5-yl)-1-(3-phenyl-propiolyl)-piperazin |
| AAA83 | 4-(Thiazol-2-yl)-1-(3-(chinol-7-yl)-propiolyl)-piperazin |
| AAA84 | 4-(Thiazol-2-yl)-1-(3-phenyl-propiolyl)-piperazin-3-on |
| AAA85 | 4-(3-(4-Fluorbenzyl)-1,2,4-thiadiazol-5-yl)-1-(3-phenyl-propiolyi)-piperazin |
| AAA86 | 4-(5-Nitro-thiazol-2-yl)-1-(3-phenyl-propiolyl)-piperazin |
| AAA87 | 4-(4-tert-Butyl-thiazol-2-yl)-1-(3-phenyl-propiolyl)-piperazin |
| AAA88 | 4-(5-Methyl-thiazol-2-yl)-1-(3-phenyl-propiolyi)-piperazin |
| AAA89 | 4-(4,5-Dimethyl-thiazol-2-yl)-1-(3-phenyl-propiolyl)-piperazin |
| AAA9 | 4-(Thiazol-2-yl)-1-(3-(indol-5-yl)-propiolyl)-piperazin |
| AAA90 | 4-(5-Brom-4-phenyl-thiazol-2-yl)-1-(3-phenyl-propiolyl)-piperazin |
| AAA91 | 4-(5-Brom-4-methyl-thiazol-2-yl)-1-(3-phenyl-propiolyl)-piperazin |
| AAA92 | 4-(4-Methyl-thiazol-2-yl)-1-(3-phenyl-propiolyl)-piperazin |
| AAA93 | 4-(4-Trifluormethyl-thiazol-2-yl)-1-(3-phenyl-propiolyl)-piperazin |
| AAA94 | 4-(4-Chlor-thiazol-2-yl)-1-(3-phenyl-propiolyl)-piperazin |
| AAA95 | 4-(5-Chlor-thiazol-2-yl)-1-(3-phenyl-propiolyi)-piperazin |
| AAA96 | 4-(4-Brom-thiazol-2-yl)-1-(3-phenyl-propiolyl)-piperazin |
| AAA97 | 4-(5-Brom-thiazol-2-yl)-1-(3-phenyl-propiotyl)-piperazin |
| AAA98 | 4-(5-Phenyl-thiazol-2-yl)-1-(3-phenyl-propiolyl)-piperazin |
| AAA99 | 4-(4-Phenyl-thiazol-2-yl)-1-(3-phenyl-propiolyl)-piperazin |
| AAA00108 | 4-(Thiazol-2-yl)-1-(3-(1-Methyl-indol-6-yl)-propiolyl)-piperazin |
| AAA001 09 | 4-(Thiazol-2-yl)-1-(3-(3-acetyl-phenyl)-propiolyl)-piperazin |
| AAA00110 | 4-(Thiazol-2-yl)-1-(3-(3-fluor-5-methyl-phenyl)-propiolyl)-piperazin |
| AAA00111 | 4-(Thiazol-2-yl)-1-(3-(2-fluor-3-methyl-phenyl)-propiolyl)-piperazin |
| AAA00112 | 4-(Thiazol-2-yl)-1-(3-(3-methylamino-phenyl)-propiolyl)-piperazin |
| AAA00113 | 4-(Thiazol-2-yl)-1-(3-(3-dimethylamino-phenyl)-propiolyl)-piperazin |
| AAA00114 | 4-(Thiazol-2-yl)-1-(dimethylcarbamoyl-propiolyl)-piperazin |
| AAA00115 | 4-(Thiazol-2-yl)-1-(3-(3-methylsulfinyl-phenyl)-propiolyl)-piperazin |
| AAA00116 | 4-(Thiazol-2-yl)-1-(3-(3-methylsulfonyl-phenyl)-propiolyl)-piperazin |
| AAA00117 | 4-(Thiazol-2-yl)-1-(3-(3-ethinyl-phenyl)-propiolyl)-piperazin |
| AAA00118 | 4-(Thiazol-2-yl)-1-(3-(4-methyl-thiophen-2-yl)-propiolyl)-piperazin |
| AAA00119 | 2-Methyl-4-(thiazol-2-yl)-1-(3-(3-chlor-phenyl)-propiolyl)-piperazin |
| AAA00120 | 4-(Thiazol-2-yl)-1-(3-(3-ethyl-phenyl)-propiolyl)-piperazin |
| AAA00121 | 2-tert-Butyl-4-(thiazol-2-yl)-1-(3-phenyl-propiolyl)-piperazin |
| AAA00122 | 4-(Thiazol-2-yl)-1-(3-(3-difluormethyl-phenyl)-propiolyl)-piperazin |
| AAA00123 | 2-Methyl-4-(thiazol-2-yl)-1-(3-(3-cyano-phenyl)-propiolyl)-piperazin |
| AAA00124 | 2-Isopropyl-4-(thiazol-2-yl)-1-(3-phenyl-propiolyl)-piperazin |
| AAA00125 | 4-(Thiazol-2-yl)-1-(3-(3-trimethylsilanylethinyl-phenyl)-propiolyl)-piperazin |

### Kombinatorische Synthese:

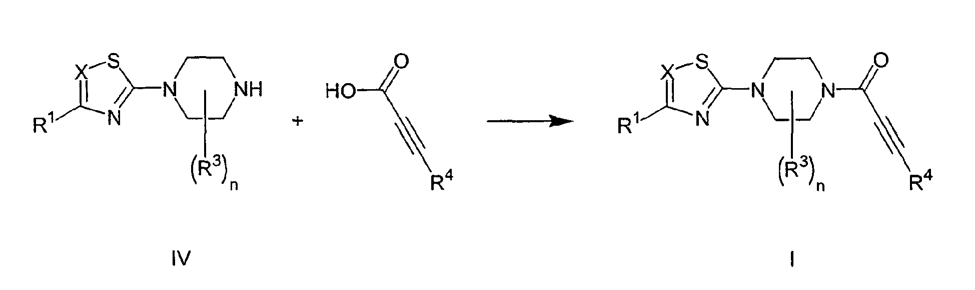

Zu einer Lösung einer Säure der allgemeinen Formel R⁴-C≡C-(C=O)-OH (100 µmol) in DCM (2 ml) wurde eine Lösung von Carbonyldiimidazol (105 µmol) in DCM (1.05 ml) hinzu gegeben und die Reaktionsmischung wurde für eine Stunde bei 20°C gerührt. Anschließend wurde eine Lösung der Verbindung der allgemeinen Formel IV (100 µmol) in DCM (1 ml) hinzu gegeben und weitere 16h bei 20°C gerührt. Die Reaktionslösung wurde mit Wasser (3 ml) versetzt und extrahiert. Nach Abtrennen der wäßrigen Phase wurde die organische Phase mit Wasser (3 ml) und mit ges. aq. NaCl-Lsg. (3 ml) extrahiert. Die separierte organische Phase wurde über MgSO₄ getrocknet, abfiltriert und das Lösungsmittel abdestilliert. Die Substanzen wurden über präparative HPLC aufgereinigt.

Folgende Verbindungen wurden ausgehend von kommerziell erhältlichen oder nach dem Fachmann bekannten Methoden hergestellten Säuren R⁴-C≡C-(C=O)-OH und Aminen der allgemeinen Formel IV, worin n jeweils gleich 0 ist, hergestellt:

| **Verbindung gemäß Beispiel** | **X** | **R¹** | **R⁴** |
|---|---|---|---|
| CCC1 | N | Phenyl | 2-Methoxy-phenyl |
| CCC2 | N | Phenyl | 4-Methoxy-phenyl |
| CCC3 | N | Phenyl | 2-Fluor-phenyl |
| CCC4 | N | Phenyl | 3-Fluor-phenyl |
| CCC5 | N | Phenyl | 4-Fluor-phenyl |
| CCC6 | N | Phenyl | 2,4-Dichlor-phenyl |
| CCC7 | N | Phenyl | 3,5-Dichlor-phenyl |
| CCC8 | N | 4-Fluor-phenyl | Tol-2-yl |
| CCC9 | N | 4-Fluor-phenyl | 3-Methoxy-phenyl |
| CCC10 | N | 4-Fluor-phenyl | 3-Fluor-4-methyl-phenyl |
| CCC11 | N | 4-Fluor-phenyl | 2,4-Difluor-phenyl |
| CCC12 | N | 4-Fluor-phenyl | Tol-3-yl |
| CCC13 | N | 4-Fluor-phenyl | 4-Trifluormethyl-phenyl |
| CCC14 | N | 4-Fluor-phenyl | 3-Trifluormethyl-phenyl |
| CCC15 | N | 4-Fluor-phenyl | 2-Chlor-5-trifluormethyl-phenyl |
| CCC16 | N | tert-Butyl | Thiophen-2-yl |
| CCC17 | N | tert-Butyl | 3-Trifluormethyl-phenyl |
| CCC18 | N | tert-Butyl | 2-Methoxy-phenyl |
| CCC19 | N | tert-Butyl | Tol-2-yl |
| CCC20 | N | tert-Butyl | 3-Fluor-4-methyl-phenyl |
| CCC21 | N | tert-Butyl | 2-Chlor-5-trifluormethyl-phenyl |
| CCC22 | CH | H | 2,4-Difluor-phenyl |
| CCC23 | CH | H | 2-Brom-5-methoxy-phenyl |
| CCC24 | CH | H | 3-Brom-4-methoxy-phenyl |
| CCC25 | CH | H | 3,5-Dichlor-phenyl |
| CCC26 | CH | H | 4-Fluor-3-methyl-phenyl |
| CCC27 | CH | tert-Butyl | 3-Cyano-phenyl |
| CCC28 | CH | tert-Butyl | 2-Trifluormethyl-phenyl |
| CCC29 | CH | Phenyl | 2,3-Dimethyl-phenyl |
| CCC30 | CH | Phenyl | 4-Fluor-phenyl |
| CCC31 | CH | Methyl | Tol-3-yl |
| CCC32 | CH | Methyl | Thiophen-2-yl |
| CCC33 | C-Methyl | H | Tol-4-yl |
| CCC34 | C-Methyl | H | 2-Cyano-phenyl |
| CCC35 | C-Methyl | Methyl | 3-Cyano-phenyl |
| CCC36 | C-Methyl | Methyl | 3,4-Dimethyl-phenyl |
| CCC37 | CH | 4-Methoxy-phenyl | 2,4-Dimethyl-phenyl |
| CCC38 | CH | 4-Methoxy-phenyl | 4-Trifluormethyl-phenyl |
| CCC39 | CH | 4-Fluor-phenyl | 2-Cyano-phenyl |
| CCC40 | CH | 4-Chlor-phenyl | 4-Cyano-phenyl |

### Pharmakologische Daten:

Die Affinität der erfindungsgemäßen substituierten 1-Propiolyl-piperazine für den mGluR5-Rezeptor wurde wie vorstehend beschrieben bestimmt.

Die erfindungsgemäßen substituierten 1-Propiolyl-piperazine zeigen eine ausgezeichnete Affinität für den mGluR5-Rezeptor.

In der nachfolgenden Tabelle I sind die pharmakologischen Daten für die substituierte 1-Propiolyl-piperazin-Verbindung gemäß Beispiel 1 wiedergegeben:

**Tabelle I.**

| **Verbindung gemäß Beispiel** | **IC₅₀ mGluR5-Rezeptor [³H]-MPEP-Bindung Schwein** | **IC₅₀ mGluR5-Rezeptor Ca²⁺-Influx Ratte** |
|---|---|---|
| 1 | 100 nM | 190 nM |

In der nachfolgenden Tabelle II sind die pharmakologischen Daten für weitere substituierte 1-Propiolyl-piperazin-Verbindungen gemäß den Beispielen AAA1 bis AAA0025 und CCC1 bis CCC40 wiedergegeben.

**Tabelle II.**

| **Verbindung gemäß Beispiel** | **mGluR5-Rezeptor (% Hemung 10 µM) Schwein** | **IC50 mGluR5-Rezeptor [³H]-MPEP-Bindung** |
|---|---|---|
| AAA00101 | 30 | |
| AAA00102 | 61 | |
| AAA00103 | 83 | |
| AAA00104 | 91 | |
| AAA00105 | 65 | |
| AAA00106 | 68 | |
| AAA00107 | 30 | |
| AAA10 | | 0,98 |
| AAA11 | 52 | |
| AAA12 | 34 | |
| AAA13 | 87 | |
| AAA14 | 34 | |
| AAA15 | 54 | |
| AAA16 | 93 | |
| AAA21 | | 0,83 |
| AAA22 | 86 | |
| AAA23 | 70 | |
| AAA24 | 88 | |
| AAA28 | 82 | |
| AAA3 | 40 | |
| AAA30 | 91 | |
| AAA31 | | 0,038 |
| AAA32 | 57 | |
| AAA33 | 50 | |
| AAA34 | 71 | |
| AAA36 | 40 | |
| AAA37 | 45 | |
| AAA38 | 82 | |
| AAA39 | 74 | |
| AAA4 | | 0,45 |
| AAA40 | 80 | |
| AAA41 | 78 | |
| AAA42 | 88 | |
| AAA43 | 83 | |
| AAA44 | 30 | |
| AAA49 | 43 | |
| AAA5 | 35 | |
| AAA53 | 33 | |
| AAA54 | 97 | |
| AAA55 | 41 | |
| AAA56 | | 0,15 |
| AAA57 | 81 | |
| AAA58 | | 0,24 |
| AAA6 | 54 | |
| AAA60 | | 1,85 |
| AAA61 | 76 | |
| AAA63 | 89 | |
| AAA64 | 72 | |
| AAA65 | 93 | |
| AAA66 | 93 | |
| AAA67 | 94 | |
| AAA68 | | 0,013 |
| AAA69 | 72 | |
| AAA7 | 91 | |
| AAA71 | | 0,038 |
| AAA72 | 81 | |
| AAA73 | 74 | |
| AAA74 | | 3,19 |
| AAA75 | 97 | |
| AAA76 | | 0,17 |
| AAA77 | 89 | |
| AAA8 | 65 | |
| AAA80 | 76 | |
| AAA81 | 72 | |
| AAA82 | 37 | |
| AAA85 | 93 | |
| AAA86 | 31 | |
| AAA88 | 94 | |
| AAA89 | 86 | |
| AAA9 | | 0,7 |
| AAA92 | 87 | |
| AAA93 | 52 | |
| AAA94 | 91 | |
| AAA95 | 58 | |
| AAA96 | | 0,36 |
| AAA97 | | 0,72 |
| AAA99 | 87 | |
| AAA00108 | | 1,24 |
| AAA00110 | | 1,39 |
| AAA00111 | 31 | |
| AAA00112 | | 0,56 |
| AAA00113 | 27 | |
| AAA00114 | 14 | |
| AAA00115 | 17 | |
| AAA00116 | 31 | |
| AAA00117 | | 0,026 |
| AAA00118 | | 0,093 |
| AAA00119 | | 0,009 |
| AAA00120 | | 0,26 |
| AAA00121 | | 0,11 |
| AAA00122 | | 0,062 |
| AAA00123 | | 0,019 |
| CCC3 | 28 | |
| CCC4 | 59 | |
| CCC5 | 25 | |
| CCC8 | 25 | |
| CCC16 | 33 | |
| CCC22 | | 10,50 |
| CCC23 | 30 | |
| CCC31 | 93 | |
| CCC33 | 43 | |

Die erfindungsgemäßen substituierte 1-Propiolyl-piperazin-Verbindungen der vorstehend angegebenen allgemeinen Formel I führen ebenfalls zu einer ausgezeichneten Reduzierung des nozizeptiven Verhaltens im Formalintest an Ratten wie in der nachfolgenden Tabelle III beschrieben.

**Tabelle III.**

| **Verbindung gemäß Beispiel** | **Dosis** **[mg/kg]** | **Reduzierung des nozizeptiven Verhaltens gegenüber Kontrollen [%]** |
|---|---|---|
| AAA24 | 46.4 (i.p.) | 43 |
| AAA54 | 10.0 (i.v.) | 72 |
| AAA56 | 46.4 (i.p.) | 85 |
| AAA68 | 46.4 (p.o.) | 66 |

## Patentansprüche

1. Substituierte 1-Propiolyl-piperazine der allgemeinen Formel I, 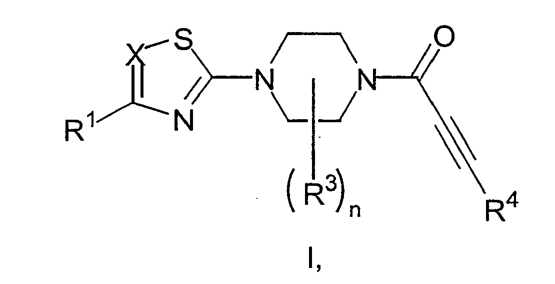 worin
X für N oder C-R² steht,
R¹ und R², unabhängig voneinander, jeweils für einen Wasserstoff-Rest, für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -NH-C(=O)-R¹⁴¹-Gruppe, für eine -NR¹²-C(=O)-R¹³-Gruppe, für eine -C(=O)-NH₂-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für eine -NH-C(=O)-NH-R²¹-Gruppe, für eine-NH-C(=S)-NH-R²²-Gruppe, für eine -NH-S(=O)2-R²³-Gruppe, für eine -NR²⁴-S(=O)2-R²⁵-Gruppe, für einen linearen oder verzweigten, gesättigten oder ungesättigten, unsubstituierten oder wenigstens einfach substituierten, ggf. wenigstens ein Heteroatom als Kettenglied aufweisenden aliphatischen Rest, für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, stehen,
R³ für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo-Gruppe (=O), für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -NH-C(=O)-R¹¹-Gruppe, für eine -NR¹²-C(=O)-R¹³-Gruppe, für eine -C(=O)-NH₂-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für eine-NH-C(=O)-NH-R²¹-Gruppe, für eine -NH-C(=S)-NH-R²²-Gruppe, für eine -NH-S(=O)2-R²³-Gruppe, für eine -NR²⁴-S(=O)₂-R²⁵-Gruppe, für einen linearen oder verzweigten, gesättigten oder ungesättigten, unsubstituierten oder wenigstens einfach substituierten, ggf. wenigstens ein Heteroatom als Kettenglied aufweisenden aliphatischen Rest, für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, steht,
R⁴ für einen Wasserstoff-Rest, für ein Fluoratom, für ein Chloratom, für ein Bromatom, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine -NH-R⁵-Gruppe, für eine -NR⁶R⁷-Gruppe, für eine -C(=O)-R⁸-Gruppe, für eine-C(=O)-O-R⁹-Gruppe, für eine -O-C(=O)-R¹⁰-Gruppe, für eine -NH-C(=O)-R¹¹-Gruppe, für eine -NR¹²-C(=O)-R¹³-Gruppe, für eine -C(=O)-NH₂-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine-S(=O)2-R²⁰-Gruppe, für eine -NH-C(=O)-NH-R²¹-Gruppe, für eine -NH-C(=S)-NH-R²²-Gruppe, für eine -NH-S(=O)2-R²³-Gruppe, für eine -NR²⁴-S(=O)₂-R²⁵-Gruppe, für einen linearen oder verzweigten, gesättigten oder ungesättigten, unsubstituierten oder wenigstens einfach substituierten, ggf. wenigstens ein Heteroatom als Kettenglied aufweisenden aliphatischen Rest, für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfache substituierten mono- oder polyzyklischen Ringsystem kondensiert sein kann, steht,
R⁵ bis R²⁵, unabhängig voneinander, jeweils für einen linearen oder verzweigten, gesättigten oder ungesättigten, unsubstituierten oder wenigstens einfach substituierten, ggf. wenigstens ein Heteroatom als Kettenglied aufweisenden aliphatischen Rest, für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden sein kann, stehen,
und n gleich 0, 1, 2, 3, 4, 5, 6, 7 oder 8 ist,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

2. Verbindungen gemäß Anspruch 1, **dadurch gekennzeichnet, dass**
X für N oder C-R² steht,
n gleich 0, 1, 2, 3, 4, 5, 6, 7 oder 8 ist,
R¹ und R², unabhängig voneinander, jeweils für einen Wasserstoff-Rest, für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -NH-C(=O)-R¹¹-Gruppe, für eine -NR¹²-C(=O)-R¹³-Gruppe, für eine -C(=O)-NH₂-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für eine -NH-C(=O)-NH-R²¹-Gruppe, für eine-NH-C(=S)-NH-R²²-Gruppe, für eine -NH-S(=O)₂-R²³-Gruppe, für eine -NR²⁴-S(=O)2-R²⁵-Gruppe, für einen linearen oder verzweigten, gesättigten oder ungesättigten, unsubstituierten oder wenigstens einfach substituierten, ggf. 1, 2 oder 3 unabhängig voneinander aus der Gruppe bestehend aus Sauerstoff, Schwefel und Stickstoff ausgewählte Heteroatome als Kettenglied(er) aufweisenden aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, stehen,
R³ für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo-Gruppe (=O), für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -NH-C(=O)-R¹¹-Gruppe, für -NR¹²-C(=O)-R¹³-Gruppe, für eine -C(=O)-NH₂-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine-S(=O)-R¹⁹-Gruppe, für eine -S(=O)₂-R²⁰-Gruppe, für eine -NH-C(=O)-NH-R²¹-Gruppe, für eine -NH-C(=S)-NH-R²²-Gruppe, für eine -NH-S(=O)2-R²³-Gruppe, für eine -NR²⁴-S(=O)₂-R²⁵-Gruppe, für einen linearen oder verzweigten, gesättigten oder ungesättigten, unsubstituierten oder wenigstens einfach substituierten, ggf. 1, 2 oder 3 unabhängig voneinander aus der Gruppe bestehend aus Sauerstoff, Schwefel und Stickstoff ausgewählte Heteroatome als Kettenglied(er) aufweisenden aliphatischen C₁₋₅-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5-bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, steht,
R⁴ für einen Wasserstoff-Rest, für eine -C(=O)-NH₂-Gruppe, für eine -C(=O)-NH-R¹⁴-Gruppe, für eine -C(=O)-NR¹⁵R¹⁶-Gruppe, für einen linearen oder verzweigten, gesättigten oder ungesättigten, unsubstituierten oder wenigstens einfach substituierten aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, steht,
und
R⁵ bis R²⁵, unabhängig voneinander, jeweils für einen linearen oder verzweigten, gesättigten oder ungesättigten, unsubstituierten oder wenigstens einfach substituierten aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, stehen;
wobei
die vorstehend genannten cycloaliphatischen Reste ggf. 1, 2, 3, 4 oder 5 Heteroatom(e) als Ringglied(er) aufweisen können, die jeweils unabhängig voneinander, ausgewählt werden können aus der Gruppe bestehend aus Stickstoff, Sauerstoff und Schwefel,
die Ringe der vorstehend genannten mono- oder bizyklischen Ringsysteme jeweils 4-, 5- oder 6-gliedrig sind und jeweils ggf. 0, 1, 2, 3, 4 oder 5 Heteroatom(e) als Ringglied(er) aufweisen, die unabhängig voneinander aus der Gruppe bestehend aus Sauerstoff, Stickstoff und Schwefel ausgewählt sind;
und
die vorstehend genannten Heteroaryl-Reste ggf. 1, 2, 3, 4 oder 5 Heteroatom(e) als Ringglied(er) aufweisen können, die jeweils unabhängig voneinander, ausgewählt werden können aus der Gruppe bestehend aus Sauerstoff, Schwefel und Stickstoff;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

3. Verbindungen gemäß Anspruch 1 oder 2, **dadurch gekennzeichnet, dass** R¹ für einen Wasserstoff-Rest, für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine -NH-R⁵-Gruppe, für eine -NR⁶R⁷-Gruppe, für eine-C(=O)-R⁸-Gruppe, für eine -C(=O)-O-R⁹-Gruppe, für eine -O-C(=O)-R¹⁰-Gruppe, für eine -NH-C(=O)-R¹¹-Gruppe, für eine -NR¹²-C(=O)-R¹³-Gruppe, für eine -C(=O)-NH₂-Gruppe, für eine -C(=O)-NH-R¹⁴-Gruppe, für eine-C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für eine -NH-C(=O)-NH-R²¹-Gruppe, für eine -NH-C(=S)-NH-R²²-Gruppe, für eine -NH-S(=O)₂-R²³-Gruppe, für eine -NR²⁴-S(=O)₂-R²⁵-Gruppe, für einen linearen oder verzweigten, gesättigten oder ungesättigten, unsubstituierten oder wenigstens einfach substituierten, ggf. 1, 2 oder 3 unabhängig voneinander aus der Gruppe bestehend aus Sauerstoff, Schwefel und Stickstoff ausgewählte Heteroatome als Kettenglied(er) aufweisenden aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5-bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, steht,
bevorzugt für einen Wasserstoff-Rest, für einen Halogen-Rest ausgewählt aus der Gruppe bestehend aus F, Cl, Br und I, für eine Nitro-Gruppe, für eine CF₃-Gruppe, für eine Cyano-Gruppe, für eine Amino-Grüppe, für eine Hydroxy-Gruppe, für eine Thiol-Gruppe, für eine Carboxy-Gruppe, für eine -NH-R⁵-Gruppe, für eine -NR⁶R⁷-Gruppe, für eine -C(=O)-R⁸-Gruppe, für eine-C(=O)-O-R⁹-Gruppe, für eine -O-C(=O)-R¹⁰-Gruppe, für eine -C(=O)-NH₂-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für einen linearen oder verzweigten C₁₋₈-Alkyl-Rest, für einen linearen oder verzweigten C₂₋₈-Alkenyl-Rest, für einen linearen oder verzweigten C₂₋₈-Alkinyl-Rest, für einen unsubstituierten oder wenigstens einfach substituierten, ungesättigten oder gesättigten, ggf. 1, 2 oder 3 unabhängig voneinander aus der Gruppe bestehend aus Sauerstoff, Schwefel und Stickstoff ausgewählte Heteroatome als Kettenglied(er) aufweisenden 3-, 4-, 5-, 6- oder 7-gliedrigen cycloaliphatischen Rest, der über eine lineare oder verzweigte, unsubstituierte oder wenigstens einfach substituierte C₁₋₅-Alkylen-. C₂₋₅-Alkenylen- oder C₂₋₅-Alkinylen-Gruppe gebunden sein kann oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte C₁₋₅-Alkylen-, C₂₋₅-Alkenylen- oder C₂₋₅-Alkinylen-Gruppe gebunden sein kann, steht,
besonders bevorzugt für einen Wasserstoff-Rest, für einen Halogen-Rest ausgewählt aus der Gruppe bestehend aus F, Cl und Br, für eine CF₃-Gruppe, eine Nitro-Gruppe, für eine Cyano-Gruppe, für eine Amino-Gruppe, für eine Hydroxy-Gruppe, für eine Thiol-Gruppe, für eine Carboxy-Gruppe, für eine - NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für einen linearen oder verzweigten C₁₋₄-Alkyl-Rest, für einen linearen oder verzweigten C₂₋₄-Alkenyl-Rest, für einen linearen oder verzweigten C₂₋₄-Alkinyl-Rest, oder für einen Rest ausgewählt aus der Gruppe bestehend aus Cyclopropyl-, Cyclobutyl-, Cyclopentyl-, Cyclohexyl-, Pyrrolidinyl-, Tetrahydrofuranyl-, Tetrahydrothiophenyl- Piperidinyl-, Tetrahydropyranyl-, Piperazinyl-, Morpholinyl-, Thiomorpholinyl-, Dioxolanyl-, Azepanyl-, Diazepanyl-, Imidazolidinyl-, Phenyl-, Naphthyl-, Anthracenyl-, Pyrrolyl-, Indolyl-, Furanyl-, Benzo[b]furanyl-, Thiophenyl-, Benzo[b]thiophenyl-, Pyrazolyl-, Imidazolyl-, Thiazolyl-, Dithiazolyl-, Thiadiazolyl-, Triazolyl-, Oxazolyl-, Oxadiazolyl-, Isoxazolyl-, Pyridinyl-, Pyridazinyl-, Pyrimidinyl-, Pyrazinyl-, Pyranyl-, Indazolyl-, Purinyl-, Indolizinyl-, Chinolinyl-, Isochinolinyl- und Chinazolinyl-Rest, der jeweils über eine C₁₋₃-Alkylen-, C₂₋₃-Alkenylen- oder C₂₋₃-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅, -O-C₃H₇, -NH₂,-N(CH₃)₂, -N(C₂H₅)₂, -NH-CH₃, -NH-C₂H₅, -NO₂, -CF₃, -O-CF₃, -S-CF₃, -SH,-NH-S(=O)₂-CH₃, -C(=O)-OH, -C(=O)-CH₃, -C(=O)-C₂H₅, -C(=O)-N(CH₃)₂,-C(=O)-NH-CH₃, -NH-C(=O)-CH₃, -NH-C(=O)-C₂H₅, -C(=O)-O-CH₃ und-C(=O)-O-C₂H₅ substituiert ist, steht,
ganz besonders bevorzugt für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Monomethylamino-Gruppe, für eine Monoethylamino-Gruppe, für eine Hydroxy-Gruppe, für eine Thiol-Gruppe, für eine Carboxy-Gruppe, für eine-C(=O)-O-CH₃-Gruppe, für eine -C(=O)-O-C₂H₅-Gruppe, für eine -C(=O)-NH-R¹⁴ -Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht.

4. Verbindungen gemäß einem oder mehreren der Ansprüche 1 bis 3, **dadurch gekennzeichnet, dass** R² für einen Wasserstoff-Rest, für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -NH-C(=O)-R¹¹-Gruppe, für eine -NR¹²-C(=O)-R¹³-Gruppe, für eine -C(=O)-NH₂-Gruppe, für eine -C(=O)-NH-R¹⁴ -Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für eine -NH-C(=O)-NH-R²¹-Gruppe, für eine -NH-C(=S)-NH-R²²-Gruppe, für eine -NH-S(=O)₂-R²³-Gruppe, für eine -NR²⁴-S(=O)₂-R²⁵-Gruppe, für einen linearen oder verzweigten, gesättigten oder ungesättigten, unsubstituierten oder wenigstens einfach substituierten, ggf. 1, 2 oder 3 unabhängig voneinander aus der Gruppe bestehend aus Sauerstoff, Schwefel und Stickstoff ausgewählte Heteroatome als Kettenglied(er) aufweisenden aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, steht,
bevorzugt für einen Wasserstoff-Rest, für einen Halogen-Rest ausgewählt aus der Gruppe bestehend aus F, Cl, Br und I, für eine Nitro-Gruppe, für eine CF₃-Gruppe, für eine Cyano-Gruppe, für eine Amino-Gruppe, für eine Hydroxy-Gruppe, für eine Thiol-Gruppe, für eine Carboxy-Gruppe, für eine -NH-R⁵-Gruppe, für eine -NR⁶R⁷-Gruppe, für eine -C(=O)-R⁸-Gruppe, für eine-C(=O)-O-R⁹-Gruppe, für eine -O-C(=O)-R¹⁰-Gruppe, für eine -C(=O)-NH₂-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für einen linearen oder verzweigten C₁₋₈-Alkyl-Rest, für einen linearen oder verzweigten C₂₋₈-Alkenyl-Rest oder für einen linearen oder verzweigten C₂₋₈-Alkinyl-Rest, für einen unsubstituierten oder wenigstens einfach substituierten, ungesättigten oder gesättigten, ggf. 1, 2 oder 3 unabhängig voneinander aus der Gruppe bestehend aus Sauerstoff, Schwefel und Stickstoff ausgewählte Heteroatome als Ringglied(er) aufweisenden, 3-, 4-, 5-, 6- oder 7-gliedrigen cycloaliphatischen Rest, der über eine lineare oder verzweigte, unsubstituierte oder wenigstens einfach substituierte C₁₋₅-Alkylen-, C₂₋₅-Alkenylen- oder C₂₋₅-Alkinylen-Gruppe gebunden sein kann oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte C₁₋₅-Alkylen-, C₂₋₅-Alkenylen- oder C₂₋₅-Alkinylen-Gruppe gebunden sein kann, steht,
besonders bevorzugt für einen Wasserstoff-Rest, für einen Halogen-Rest ausgewählt aus der Gruppe bestehend aus F, Cl und Br, für eine CF₃-Gruppe, eine Nitro-Gruppe, für eine Cyano-Gruppe, für eine Amino-Gruppe, für eine Hydroxy-Gruppe, für eine Thiol-Gruppe, für eine Carboxy-Gruppe, für eine-NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -C(=O)-NH-R¹⁴-Gruppe, für eine -C(=O)-NR¹⁵R¹⁶-Gruppe, für eine -S(=O)-R¹⁹-Gruppe, für eine -S(=O)₂-R²⁰-Gruppe, für eine -O-R¹⁷-Gruppe, für eine -S-R¹⁸-Gruppe, für einen linearen oder verzweigten C₁₋₄-Alkyl-Rest, für einen linearen oder verzweigten C₂₋₄-Alkenyl-Rest, für einen linearen oder verzweigten C₂₋₄-Alkinyl-Rest, oder für einen Rest ausgewählt aus der Gruppe bestehend aus Cyclopropyl-, Cyclobutyl-, Cyclopentyl-, Cyclohexyl-, Pyrrolidinyl-, Tetrahydrofuranyl-, Tetrahydrothiophenyl- Piperidinyl-, Tetrahydropyranyl-, Piperazinyl-, Morpholinyl-, Thiomorpholinyl-, Dioxolanyl-, Azepanyl-, Diazepanyl-, Imidazolidinyl-, Phenyl-, Naphthyl-, Anthracenyl-, Pyrrolyl-, Indolyl-, Furanyl-, Benzo[b]furanyl-, Thiophenyl-, Benzo[b]thiophenyl-, Pyrazolyl-, Imidazolyl-, Thiazolyl-, Dithiazolyl-, Thiadiazolyl-, Triazolyl-, Oxazolyl-, Oxadiazolyl-, Isoxazolyl-, Pyridinyl-, Pyridazinyl-, Pyrimidinyl-, Pyrazinyl-, Pyranyl-, Indazolyl-, Purinyl-, Indolizinyl-, Chinolinyl-, Isochinolinyl- und Chinazolinyl-Rest, der jeweils über eine C₁₋₃-Alkylen-, C₂₋₃-Alkenylen- oder C₂₋₃-Alkinylen-Gruppe gebunden sein kann und/oder unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅, -O-C₃H₇, -NH₂,-N(CH₃)₂, -N(C₂H₅)₂, -NH-CH₃, -NH-C₂H₅, -NO₂, -CF₃, -O-CF₃, -S-CF₃, -SH,-NH-S(=O)₂-CH₃, -C(=O)-OH, -C(=O)-CH₃, -C(=O)-C₂H₅, -C(=O)-N(CH₃)₂,-C(=O)-NH-CH₃, -NH-C(=O)-CH₃, -NH-C(=O)-C₂H₅, -C(=O)-O-CH₃ und-C(=O)-O-C₂H₅ substituiert ist, steht,
ganz besonders bevorzugt für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine
Monomethylamino-Gruppe, für eine Monoethylamino-Gruppe, für eine Hydroxy-Gruppe, für eine Thiol-Gruppe, für eine Carboxy-Gruppe, für eine-C(=O)-O-CH₃-Gruppe, für eine -C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine -S(=O)-R¹⁹-Gruppe, für eine -S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht.

5. Verbindungen gemäß einem oder mehreren der Ansprüche 1 bis 4, **dadurch gekennzeichnet, dass** R³ für einen Halogen-Rest, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Formyl-Gruppe, für eine -NH-C(=O)-H-Gruppe, für eine Oxo-Gruppe (=O), für eine -NH-R⁵-Gruppe, für eine -NR⁶R⁷-Gruppe, für eine -C(=O)-R⁸-Gruppe, für eine -C(=O)-O-R⁹-Gruppe, für eine -O-C(=O)-R¹⁰-Gruppe, für eine -NH-C(=O)-R¹¹-Gruppe, für -NR¹²-C(=O)-R¹³-Gruppe, für eine -C(=O)-NH₂-Gruppe für eine -C(=O)-NH-R¹⁴-Gruppe, für eine -C(=O)-NR¹⁵R¹⁶-Gruppe, für eine -O-R¹⁷-Gruppe, für eine-S-R¹⁸-Gruppe, für eine -S(=O)-R¹⁹-Gruppe, für eine -S(=O)₂-R²⁰-Gruppe, für eine -NH-C(=O)-NH-R²¹-Gruppe, für eine -NH-C(=S)-NH-R²²-Gruppe, für eine -NH-S(=O)₂-R²³-Gruppe, für eine -NR²⁴-S(=O)₂-R²⁵-Gruppe, für einen linearen oder verzweigten, gesättigten oder ungesättigten, unsubstituierten oder wenigstens einfach substituierten, ggf. 1, 2 oder 3 unabhängig voneinander aus der Gruppe bestehend aus Sauerstoff, Schwefel und Stickstoff ausgewählte Heteroatome als Kettenglied(er) aufweisenden aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, steht,
bevorzugt für einen Halogen-Rest ausgewählt aus der Gruppe bestehend aus F, Cl und Br, für eine Nitro-Gruppe, für eine Cyano-Gruppe, für eine Amino-Gruppe, für eine Hydroxy-Gruppe, für eine Thiol-Gruppe, für eine Carboxy-Gruppe, für eine Oxo-Gruppe (=O), oder für einen linearen oder verzweigten C₁₋₄-Alkyl-Rest, oder für einen Rest ausgewählt aus der Gruppe bestehend aus Cyclopropyl-, Cyclobutyl-, Cyclopentyl-, Cyclohexyl-, Pyrrolidinyl-, Tetrahydrofuranyl-, Tetrahydrothiophenyl- Piperidinyl-, Tetrahydropyranyl-, Piperazinyl-, Morpholinyl-, Thiomorpholinyl-, Dioxolanyl-, Azepanyl-, Diazepanyl-, Imidazolidinyl-, Phenyl-, Naphthyl-, Anthracenyl-, Pyrrolyl-, Indolyl-, Furanyl-, Benzo[b]furanyl-, Thiophenyl-, Benzo[b]thiophenyl-, Pyrazolyl-, Imidazolyl-, Thiazolyl-, Dithiazolyl-, Thiadiazolyl-, Triazolyl-, Oxazolyl-, Oxadiazolyl-, Isoxazolyl-, Pyridinyl-, Pyridazinyl-, Pyrimidinyl-, Pyrazinyl-, Pyranyl-, Indazolyl-, Purinyl-, Indolizinyl-, Chinolinyl-, Isochinolinyl- und Chinazolinyl-Rest, der unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht,
besonders bevorzugt für eine Oxo-Gruppe (=O), für einen Alkyl-Rest ausgewählt aus der Gruppe bestehend aus Methyl, Ethyl, n-Propyl, Isopropyl, n-Butyl, sec-Butyl, Isobutyl und tert-Butyl oder für einen Phenyl-Rest, der unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht.

6. Verbindungen gemäß einem oder mehreren der Ansprüche 1 bis 5, **dadurch gekennzeichnet, dass** R⁴ für einen Wasserstoff-Rest, für eine -C(=O)-NH₂-Gruppe, für eine -C(=O)-NH-R¹⁴-Gruppe, für eine -C(=O)-NR¹⁵R¹⁶-Gruppe, für einen linearen oder verzweigten, gesättigten oder ungesättigten, unsubstituierten oder wenigstens einfach substituierten aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, steht,
bevorzugt für einen Wasserstoff-Rest, für eine -C(=O)-NH₂-Gruppe, für eine-C(=O)-NH-R¹⁴-Gruppe, für eine -C(=O)-NR¹⁵R¹⁶-Gruppe, für einen linearen oder verzweigten, unsubstituierten oder wenigstens einfach substituierten C₁₋₈-Alkyl-Rest, für einen linearen oder verzweigten, unsubstituierten oder wenigstens einfach substituierten C₂₋₈-Alkenyl-Rest, für einen linearen oder verzweigten, unsubstituierten oder wenigstens einfach substituierten C₂₋₈-Alkinyl-Rest, für einen unsubstituierten oder wenigstens einfach substituierten, ungesättigten oder gesättigten 4-, 5-, 6- oder 7-gliedrigen cycloaliphatischen Rest, der mit einem ungesättigten, gesättigten oder aromatischen, unsubstituierten oder wenigstens einfach substituierten, mono- oder bizyklischen Ringsystem kondensiert sein kann, wobei jeder der Ringe 1, 2 oder 3 Heteroatome aufweisen kann, die unabhängig voneinander ausgewählt werden können aus der Gruppe bestehend aus Sauerstoff, Stickstoff und Schwefel und wobei die Ringe des mono- oder bizyklischen Ringsystems jeweils 4-, 5- oder 6-gliedrig sind, oder für einen unsubstituierten oder wenigstens einfach substituierten 5- oder 6-gliedrigen Aryl- oder Heteroaryl-Rest, der mit einem gesättigten, ungesättigten oder aromatischen, unsubstituierten oder wenigstens einfach substituierten, mono- oder bizyklischen Ringsystem kondensiert sein kann, wobei der Heteroaryl-Rest und ggf. einer oder beide Ringe des mono- oder bizyklischen Ringsystems jeweils 1, 2 oder 3 Heteroatome aufweisen, die unabhängig voneinander ausgewählt werden können aus der Gruppe bestehend aus Sauerstoff, Stickstoff und Schwefel und wobei die Ringe des mono- oder bizyklischen Ringsystems jeweils 4-, 5- oder 6-gliedrig sind, steht,
besonders bevorzugt für einen Wasserstoff-Rest, für eine -C(=O)-NH₂-Gruppe, für eine -C(=O)-NH-R¹⁴-Gruppe, für eine -C(=O)-NR¹⁵R¹⁶-Gruppe, für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, n-Heptyl und n-Octyl, oder für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Pyrrolidinyl, Tetrahydrofuranyl, Tetrahydrothiophenyl, Piperidinyl, Tetrahydropyranyl, Piperazinyl, Morpholinyl, Thiomorpholinyl, Dioxolanyl, Azepanyl, Diazepanyl, Imidazolidinyl, Phenyl, Naphthyl, Anthracenyl, Furanyl, Thiophenyl, Pyrazolyl, Pyrazinyl, Pyrimidinyl, Pyridinyl, Pyrrolyl, Oxazolyl, Isoxazolyl, Thiazolyl, Dithiazolyl, Oxadiazolyl, Triazolyl, Imidazolyl, Indolyl, Benzo[b]thiophenyl, Benzo[b]furanyl, Chinolinyl, Isochinolinyl und Chinazolyl steht, der jeweils ggf. mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, Ethenyl, Allyl, Ethinyl, Propinyl, -C≡C-Si(CH₃)₃, -C≡C-Si(C₂H₅)₃, -CH₂-O-CH₃,CH₂-O-C₂H₅, -OH, -O-CH₃, -O-C₂H₅, -O-C₃H₇, -S-CH₃, -S-C₂H₅, -S(=O)-CH₃,S(=O)₂-CH₃, -S(=O)-C₂H₅, -S(=O)₂-C₂H₅, -NH₂, -N(CH₃)₂, -N(C₂H₅)₂, -NH-CH₃, -NH-C₂H₅, -NO₂, -CF₃, -CH₂F, -CHF₂, -O-CF₃, -S-CF₃, -SH, -NH-S(=O)₂-CH₃,C(=O)-OH, -C(=O)-H; -C(=O)-CH₃, -C(=O)-C₂H₅, -C(=O)-NH₂, -C(=O)-N(CH₃)₂, -C(=O)-NH-CH₃, -NH-C(=O)-CH₃, -NH-C(=O)-C₂H₅, -C(=O)-O-CH₃, -C(=O)-O-C₂H₅, -C(=O)-O-C(CH₃)₃ und Phenyl substituiert sein kann, steht,
ganz besonders bevorzugt für einen Wasserstoff-Rest, für eine -C(=O)-NH₂-Gruppe, für eine -C(=O)-NH-R¹⁴-Gruppe, für eine -C(=O)-NR¹⁵R¹⁶-Gruppe, für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, neo-Pentyl, n-Hexyl, n-Heptyl und n-Octyl, oder für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Phenyl, Pyrid-2-yl, Pyrid-3-yl, Pyrid-4-yl, Pyrrol-2-yl, Pyrrol-3-yl, Thiophen-2-yl, Thiophen-3-yl, 4-Methyl-thiophen-2-yl, 3-Methyl-thiophen-2-yl, 5-Methylthiophen-2-yl, Furan-2-yl, Furan-3-yl, Imidazol-2-yl, Imidazol-4-yl, Thiazol-2-yl, Thiazol-4-yl, Thiazol-5-yl, Oxazol-2-yl, Oxazol-4-yl, Oxazol-5-yl, (1,2,4)-Thiadiazol-5-yl, (1,2,4)-Oxadiazol-5-yl, Napht-1-yl, Napht-2-yl, Anthracen-1-yl, Anthracen-2-yl, Anthracen-9-yl, Indol-2-yl, Indol-3-yl, Indol-4-yl, Indol-5-yl, Indol-6-yl, Indol-7-yl, 1-Methyl-indol-2-yl, 1-Methyl-indol-3-yl, 1-Methyl-indol-4-yl, 1-Methyl-indol-5-yl, 1-Methyl-indol-6-yl, 1-Methyl-indol-7-yl, Chinolin-3-yl, Chinolin-4-yl, Chinolin-2-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, Isochinolin-8-yl, 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Hydroxy-phenyl, 3-Hydroxy-phenyl, 4-Hydroxy-phenyl, 2-Amino-phenyl, 3-Amino-phenyl, 4-Amino-phenyl, 2-Dimethylamino-phenyl, 3-Dimethylaminophenyl, 4-Dimethylamino-phenyl, 2-Methylamino-phenyl, 3-Methylaminophenyl, 4-Methylamino-phenyl, 2-Acetyl-phenyl, 3-Acetyl-phenyl, 4-Acetylphenyl, 2-Methylsulfinyl-phenyl, 3-Methylsulfinyl-phenyl, 4-Methylsulfinyl-phenyl, 2-Methylsulfonyl-phenyl, 3-Methylsulfonyl-phenyl, 4-Methylsulfonyl-phenyl, 2-Methoxy-phenyl, 3-Methoxy-phenyl, 4-Methoxy-phenyl, 2-Chlor-phenyl, 3-Chlor-phenyl, 4-Chlor-phenyl, 2-Ethoxy-phenyl, 3-Ethoxy-phenyl, 4-Ethoxyphenyl, 2-Trifluormethyl-phenyl, 3-Trifluormethyl-phenyl, 4-Trifluormethyl-phenyl, 2-Difluormethyl-phenyl, 3-Difluormethyl-phenyl, 4-Difluormethyl-phenyl, 2-Fluormethyl-phenyl, 3-Fluormethyl-phenyl, 4-Fluormethyl-phenyl, 2-Nitro-phenyl, 3-Nitro-phenyl, 4-Nitro-phenyl, 2-Ethyl-phenyl, 3-Ethyl-phenyl, 4-Ethyl-phenyl, 2-Propyl-phenyl, 3-Propylphenyl, 4-Propyl-phenyl, 2-Isopropyl-phenyl, 3-Isopropyl-phenyl, 4-Isopropylphenyl, 2-tert-Butyl-phenyl, 3-tert-Butyl-phenyl, 4-tert-Butyl-phenyl, 2-Carboxyphenyl, 3-Carboxy-phenyl, 4-Carboxyphenyl, 2-Ethenyl-phenyl, 3-Ethenyl-phenyl, 4-Ethenyl-phenyl, 2-Ethinyl-phenyl, 3-Ethinyl-phenyl, 4-Ethinyl-phenyl, 2-Allyl-phenyl, 3-Allyl-phenyl, 4-Allyl-phenyl, 2-Trimethylsilanylethinyl-phenyl, 3-Trimethylsilanylethinyl-phenyl, 4-Trimethylsilanylethinyl-phenyl, 2-Formyl-phenyl, 3-Formyl-phenyl, 4-Formylphenyl, 2-Acetamino-phenyl, 3-Acetamino-phenyl, 4-Acetamino-phenyl, 2-Dimethylaminocarbonyl-phenyl, 3-Dimethylaminocarbonyl-phenyl, 4-Dimethylaminocarbonyl-phenyl, 2-Methoxymethyl-phenyl, 3-Methoxymethylphenyl, 4-Methoxymethyl-phenyl, 2-Ethoxymethyl-phenyl, 3-Ethoxymethylphenyl, 4-Ethoxymethyl-phenyl, 2-Aminocarbonyl-phenyl, 3-Aminocarbonylphenyl, 4-Aminocarbonyl-phenyl, 2-Methylaminocarbonyl-phenyl, 3-Methylaminocarbonyl-phenyl, 4-Methylaminocarbonyl-phenyl, 2-Carboxymethylester-phenyl, 3-Carboxymethylester-phenyl, 4-Carboxymethylester-phenyl, 2-Carboxyethylester-phenyl, 3-Carboxyethylesterphenyl, 4-Carboxyethylester-phenyl, 2-Carboxy-tert-butylester-phenyl, 3-Carboxy-tert-butylester-phenyl, 4-Carboxy-tert-butylester-phenyl, 2-Methylmercapto-phenyl, 3-Methylmercapto-phenyl, 4-Methylmercapto-phenyl, 2-Ethylmercapto-phenyl, 3-Ethylmercapto-phenyl, 4-Ethylmercaptophenyl, 2-Biphenyl, 3-Biphenyl, 4-Biphenyl, 2-Bromphenyl, 3-Bromphenyl, 4-Bromphenyl, 2-lod-phenyl, 3-lodphenyl, 4-lodphenyl, 2-Trifluormethoxy-phenyl, 3-Trilfuormethoxy-phenyl, 4-Trifluormethoxy-phenyl, 2-Fluor-3-trifluormethylphenyl, 2-Fluor-4-methyl-phenyl, (2,3)-Difluorphenyl, (2,3)-Dimethyl-phenyl, (2,3)-Dichlorphenyl, 3-Fluor-2-trifluormethylphenyl, (2,4)-Dichlor-phenyl, (2,4)-Difluorphenyl, 4-Fluor-2-trifluormethyl-phenyl, (2,4)-Dimethoxyphenyl, 2-Chlor-4-fluor-phenyl, 2-Chlor-4-nitro-phenyl, 2-Chlor-4-methyl-phenyl, 2-Chlor-5-trifluormethyl-phenyl, 2-Chlor-5-methoxy-phenyl, 2-Brom-5-trifluormethyl-phenyl, 2-Brom-5-methoxy-phenyl, (2,4)-Dibrom-phenyl, (2,4)-Dimethyl-phenyl, 2-Fluor-4-trifluormethyl-phenyl, (2,5)-Difluor-phenyl, 2-Fluor-5-trifluormethyl-phenyl, 5-Fluor-2-trifluormethyl-phenyl, 5-Chlor-2-trifluormethyl-phenyl, 5-Brom-2-trifluormethyl-phenyl, (2,5)-Dimethoxy-phenyl, (2,5)-Bis-trifluormethyl-phenyl, (2,5)-Dichlor-phenyl, (2,5)-Dibrom-phenyl, 2-Methoxy-5-nitro-phenyl, 2-Fluor-6-trifluormethyl-phenyl, (2,6)-Dimethoxyphenyl, (2,6)-Dimethyl-phenyl, (2,6)-Dichlor-phenyl, 2-Chlor-6-fluor-phenyl, 2-Brom-6-chlor-phenyl, 2-Brom-6-fluor-phenyl, (2,6)-Difluor-phenyl, (2,6)-Difluor-3-methyl-phenyl, (2,6)-Dibrom-phenyl, (2,6)-Dichlorphenyl, 3-Chlor-2-fluor-phenyl, 3-Chlor-5-methyl-phenyl, (3,4)-Dichlorphenyl, (3,4)-Dimethyl-phenyl, 3-Methyl-4-methoxy-phenyl, 4-Chlor-3-nitro-phenyl, (3,4)-Dimethoxy-phenyl, 4-Fluor-3-trifluormethylphenyl, 3-Fluor-4-trifluormethyl-phenyl, (3,4)-Difluorphenyl, 3-Cyano-4-fluor-phenyl, 3-Cyano-4-methyl-phenyl, 3-Cyano-4-methoxy-phenyl, 3-Brom-4-fluor-phenyl, 3-Brom-4-methyl-phenyl, 3-Brom-4-methoxy-phenyl, 4-Chlor-2-fluor-phenyl, 4-Chlor-3-trifluormethyl, 4-Brom-3-methyl-phenyl, 4-Brom-5-methyl-phenyl, 3-Chlor-4-fluor-phenyl, 4-Fluor-3-nitro-phenyl, 4-Brom-3-nitro-phenyl, (3,4)-Dibrom-phenyl, 4-Chlor-3-methylphenyl, 4-Brom-3-methyl-phenyl, 4-Fluor-3-methyl-phenyl, 3-Fluor-4-methylphenyl, 3-Fluor-5-methyl-phenyl, 2-Fluor-3-methyl-phenyl, 4-Methyl-3-nitrophenyl, (3,5)-Dimethoxy-phenyl, (3,5)-Dimethyl-phenyl, (3,5)-Bis-trifluormethyl-phenyl, (3,5)-Difluor-phenyl, (3,5)-Dinitro-phenyl, (3,5)-Dichlor-phenyl, 3-Fluor-5-trifluormethyl-phenyl, 5-Fluor-3-trifluormethyl-phenyl, (3,5)-Dibrom-phenyl, 5-Chlor-4-fluor-phenyl, 5-Chlor-4-fluor-phenyl, 5-Brom-4-methyl-phenyl, (2,3,4)-Trifluorphenyl, (2,3,4)-Trichlorphenyl, (2,3,6)-Trifluor-phenyl, 5-Chlor-2-methoxy-phenyl, (2,3)-Difluor-4-methyl, (2,4,5)-Trifluor-phenyl, (2,4,5)-Trichlorphenyl, (2,4)-Dichlor-5-fluor-phenyl, (2,4,6)-Trichlor-phenyl, (2,4,6)-Trimethylphenyl, (2,4,6)-Trifluor-phenyl, (2,4,6)-Trimethoxy-phenyl, (3,4,5)-Trimethoxy-phenyl, (2,3,4,5)-Tetrafluor-phenyl, 4-Methoxy-(2,3,6)-trimethylphenyl, 4-Methoxy-(2,3,6)-trimethyl-phenyl, 4-Chlor-2,5-dimethyl-phenyl, 2-Chlor-6-fluor-3-methyl-phenyl, 6-Chlor-2-fluor-3-methyl, (2,4,6)-Trimethylphenyl, (2,3,4,5,6)-Pentafluor-phenyl, 3-Methyl-pyrid-2-yl, 4-Methyl-pyrid-2-yl, 5-Methyl-pyrid-2-yl, 6-Methyl-pyrid-2-yl, 2-Methyl-pyrid-3-yl, 4-Methyl-pyrid-3-yl, 5-Methyl-pyrid-3-yl, 6-Methyl-pyrid-3-yl, 2-Methyl-pyrid-4-yl, 3-Methyl-pyrid-4-yl, 3-Fluor-pyrid-2-yl, 4-Fluor-pyrid-2-yl, 5-Fluor-pyrid-2-yl, 6-Fluor-pyrid-2-yl, 3-Chlor-pyrid-2-yl, 4-Chlor-pyrid-2-yl, 5-Chlor-pyrid-2-yl, 6-Chlor-pyrid-2-yl, 3-Trifluormethyl-pyrid-2-yl, 4-Trifluormethyl-pyrid-2-yl, 5-Trifluormethyl-pyrid-2-yl, 6-Trifluormethyl-pyrid-2-yl, 3-Methoxy-pyrid-2-yl, 4-Methoxy-pyrid-2-yl, 5-Methoxy-pyrid-2-yl, 6-Methoxypyrid-2-yl, 4-Methyl-thiazol-2-yl, 5-Methyl-thiazol-2-yl, 4-Trifluormethyl-thiazol-2-yl, 5-Trifluormethyl-thiazol-2-yl, 4-Chlor-thiazol-2-yl, 5-Chlor-thiazol-2-yl, 4-Brom-thiazol-2-yl, 5-Brom-thiazol-2-yl, 4-Fluor-thiazol-2-yl, 5-Fluor-thiazol-2-yl, 4-Cyano-thiazol-2-yl, 5-Cyano-thiazol-2-yl, 4-Methoxy-thiazol-2-yl, 5-Methoxythiazol-2-yl, 4-Methyl-oxazol-2-yl, 5-Methyl-oxazol-2-yl, 4-Trifluormethyloxazol-2-yl, 5-Trifluormethyl-oxazol-2-yl, 4-Chlor-oxazol-2-yl, 5-Chlor-oxazol-2-yl, 4-Brom-oxazol-2-yl, 5-Brom-oxazol-2-yl, 4-Fluor-oxazol-2-yl, 5-Fluoroxazol-2-yl, 4-Cyano-oxazol-2-yl, 5-Cyano-oxazol-2-yl, 4-Methoxy-oxazol-2-yl, 5-Methoxy-oxazol-2-yl, 2-Methyl-(1,2,4)-thiadiazol-5-yl, 2-Trifluormethyl-(1,2,4)-thiadiazol-5-yl, 2-Chlor-(1,2,4)-thiadiazol-5-yl, 2-Fluor-(1,2,4)-thiadiazol-5-yl, 2-Methoxy-(1,2,4)-thiadiazol-5-yl, 2-Cyano-(1,2,4)-thiadiazol-5-yl, 2-Methyl-(1,2,4)-oxadiazol-5-yl, 2-Trifluormethyl-(1,2,4)-oxadiazol-5-yl, 2-Chlor-(1,2,4)-oxadiazol-5-yl, 2-Fluor-(1,2,4)-oxadiazol-5-yl, 2-Methoxy-(1,2,4)-oxadiazol-5-yl und 2-Cyano-(1,2,4)-oxadiazol-5-yl steht.

7. Verbindungen gemäß einem oder mehreren der Ansprüche 1 bis 6, **dadurch gekennzeichnet, dass** n gleich 0, 1, 2, 3 oder 4 ist,
bevorzugt gleich 0, 1 oder 2 ist.

8. Verbindungen gemäß einem oder mehreren der Ansprüche 1 bis 7, **dadurch gekennzeichnet, dass** R⁵ bis R²⁵, unabhängig voneinander, jeweils für einen linearen oder verzweigten, gesättigten oder ungesättigten, unsubstituierten oder wenigstens einfach substituierten aliphatischen C₁₋₈-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden und/oder mit einem unsubstituierten oder wenigstens einfach substituierten mono- oder polyzyklischen Ringsystem kondensiert sein kann, stehen,
bevorzugt für einen linearen oder verzweigten, unsubstituierten oder wenigstens einfach substituierten C₁₋₈-Alkyl-Rest, für einen linearen oder verzweigten, unsubstituierten oder wenigstens einfach substituierten C₂₋₈-Alkenyl-Rest, für einen linearen oder verzweigten, unsubstituierten oder wenigstens einfach substituierten C₂₋₈-Alkinyl-Rest, für einen unsubstituierten oder wenigstens einfach substituierten, ungesättigten oder gesättigten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden sein kann, oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. 1, 2 oder 3 unabhängig voneinander aus der Gruppe bestehend aus Sauerstoff, Schwefel und Stickstoff ausgewählte Heteroatome als Kettenglied(er) aufweisende C₁₋₅-Alkylen-, C₂₋₅-Alkenylen- oder C₂₋₅-Alkinylen-Gruppe gebunden sein kann, stehen,
besonders bevorzugt für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl, oder für einen Rest ausgewählt aus der Gruppe bestehend aus Cyclopropyl-, Cyclobutyl-, Cyclopentyl-, Cyclohexyl-, Pyrrolidinyl-, Tetrahydrofuranyl-, Tetrahydrothiophenyl- Piperidinyl-, Tetrahydropyranyl-, Piperazinyl-, Morpholinyl-, Thiomorpholinyl-, Dioxolanyl-, Azepanyl-, Diazepanyl-, Imidazolidinyl-, Phenyl-, Naphthyl-, Anthracenyl-, Pyrrolyl-, Indolyl-, Furanyl-, Benzo[b]furanyl-, Thiophenyl-, Benzo[b]thiophenyl-, Pyrazolyl-, Imidazolyl-, Thiazolyl-, Dithiazolyl-, Thiadiazolyl-, Triazolyl-, Oxazolyl-, Oxadiazolyl-, Isoxazolyl-, Pyridinyl-, Pyridazinyl-, Pyrimidinyl-, Pyrazinyl-, Pyranyl-, Indazolyl-, Purinyl-, Indolizinyl-, Chinolinyl-, Isochinolinyl- und Chinazolinyl-, wobei der jeweilige zyklische Rest über eine, ggf. 1 oder 2 Heteroatome ausgewählt aus der Gruppe bestehend aus Sauerstoff, Schwefel und Stickstoff als Kettenglied(er) aufweisende C₁₋₃-Alkylen-, C₂₋₃-Alkenylen- oder C₂₋₃-Alkinylen-Gruppe gebunden sein kann und/oder unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅, -O-C₃H₇, -NH₂, -N(CH₃)₂, -N(C₂H₅)₂, -NH-CH₃, -NH-C₂H₅, -NO₂, -CF₃, -O-CF₃, -S-CF₃, -SH, -NH-S(=O)₂-CH₃, -C(=O)-OH, -C(=O)-CH₃, -C(=O)-C₂H₅, -C(=O)-N(CH₃)₂, -C(=O)-NH-CH₃, -NH-C(=O)-CH₃, -NH-C(=O)-C₂H₅, -C(=O)-O-CH₃ und -C(=O)-O-C₂H₅ substituiert ist, steht.

9. Verbindungen gemäß einem oder mehreren der Ansprüche 1 bis 8, **dadurch gekennzeichnet, dass**
n = 0, 1, 2, 3 oder 4 ist,
X für N oder C-R² steht,
R¹ für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine-S-R¹⁸-Gruppe, für eine -S(=O)-R¹⁹-Gruppe, für eine -S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht,
R² für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine-S-R¹⁸-Gruppe, für eine -S(=O)-R¹⁹-Gruppe, für eine -S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht,
R³ für eine Oxo-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl oder für einen Phenyl-Rest, der unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht,
R⁴ für einen Wasserstoff-Rest, für eine -C(=O)-NH₂-Gruppe, für eine -C(=O)-NH-R¹⁴ -Gruppe, für eine -C(=O)-NR¹⁵R¹⁶-Gruppe, für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, neo-Pentyl, n-Hexyl, n-Heptyl und n-Octyl, oder für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Pyrrolidinyl, Tetrahydrofuranyl, Tetrahydrothiophenyl, Piperidinyl, Tetrahydropyranyl, Piperazinyl, Morpholinyl, Thiomorpholinyl, Dioxolanyl, Azepanyl, Diazepanyl, Imidazolidinyl, Phenyl, Naphthyl, Anthracenyl, Furanyl, Thiophenyl, Pyrazolyl, Pyrazinyl, Pyrimidinyl, Pyridinyl, Pyrrolyl, Oxazolyl, Isoxazolyl, Thiazolyl, Dithiazolyl, Oxadiazolyl, Triazolyl, Imidazolyl, Indolyl, Benzo[b]thiophenyl, Benzo[b]furanyl, Chinolinyl, Isochinolinyl und Chinazolyl steht, der jeweils ggf. mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, Ethenyl, Allyl, Ethinyl, Propinyl, -C≡C-Si(CH3)₃,-C≡C-Si(C₂H₅)₃, -CH₂-O-CH₃, -CH₂-O-C₂H₅, -OH, -O-CH₃, -O-C₂H₅, -O-C₃H₇,-S-CH₃, -S-C₂H₅, -S(=O)-CH₃, -S(=O)₂-CH₃, -S(=O)-C₂H₅, -S(=O)₂-C₂H₅, -NH₂, -N(CH₃)₂, -N(C₂H₅)₂, -NH-CH₃, -NH-C₂H₅, -NO₂, -CF₃, -CHF₂, -CH₂F, -O-CF₃,-S-CF₃, -SH, -NH-S(=O)₂-CH₃, -C(=O)-OH, -C(=O)-H; -C(=O)-CH₃, -C(=O)-C₂H₅, -C(=O)-NH₂, -C(=O)-N(CH₃)₂, -C(=O)-NH-CH₃, -NH-C(=O)-CH₃, -NH-C(=O)-C₂H₅, -C(=O)-O-CH₃ , -C(=O)-O-C₂H₅, -C(=O)-O-C(CH₃)₃ und Phenyl substituiert sein kann, steht;
und
R¹⁴, R¹⁵, R¹⁶, R¹⁷, R¹⁸, R¹⁹ und R²⁰, unabhängig voneinander, jeweils für einen Alkyl-Rest ausgewählt aus der Gruppe bestehend aus Methyl, Ethyl, n-Propyl, Isopropyl, n-Butyl, sec-Butyl, Isobutyl und tert-Butyl stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

10. Verbindungen gemäß einem oder mehreren der Ansprüche 1 bis 9, **dadurch gekennzeichnet, dass**
n = 0, 1, 2, 3 oder 4 ist,
X für N oder C-R² steht,
R¹ für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine-S-R¹⁸-Gruppe, für eine -S(=O)-R¹⁹-Gruppe, für eine -S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht,
R² für einen Wasserstoff-Rest, für einen Halogen-Rest ausgewählt aus der Gruppe bestehend aus F, Cl und Br, für eine CF₃-Gruppe, für eine Nitro-Gruppe, für eine Cyano-Gruppe, für eine Amino-Gruppe, für eine Dimethylamino-Gruppe, für eine Diethylamino-Gruppe, für eine Hydroxy-Gruppe, für eine Thiol-Gruppe, für eine Carboxy-Gruppe, für eine -C(=O)-O-CH₃-Gruppe, für eine -C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eineS-R¹⁸-Gruppe, für eine -S(=O)-R¹⁹-Gruppe, für eine -S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, für einen Alkenyl-Rest ausgewählt aus der Gruppe bestehend aus Ethenyl, 1-Propenyl, 2-Propenyl, 1-Butenyl, 2-Butenyl und 3-Butenyl, für einen Alkinyl-Rest ausgewählt aus der Gruppe bestehend aus Ethinyl, 1-Propinyl, 2-Propinyl, 1-Butinyl, 2-Butinyl und 3-Butinyl oder für einen Rest ausgewählt aus der Gruppe bestehend aus (1,3)-Dioxolan-2-yl, Phenyl, Benzyl, Phenethyl, Oxadiazolyl, 2-Pyridyl, 3-Pyridyl, 4-Pyridyl, 2-Thienyl, 3-Thienyl, 2-Furyl und 3-Furyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht,
R³ für eine Oxo-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl oder für einen Phenyl-Rest, der unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, sec-Butyl, Isobutyl, tert-Butyl, -OH, -O-CH₃, -O-C₂H₅ und -O-C₃H₇ substituiert ist, steht,
R⁴ für einen Wasserstoff-Rest, für eine -C(=O)-NH₂-Gruppe, für eine -C(=O)-NH-R¹⁴-Gruppe, für eine -C(=O)-NR¹⁵R¹⁶-Gruppe, für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, neo-Pentyl, n-Hexyl, n-Heptyl und n-Octyl, oder für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Phenyl, Pyrid-2-yl, Pyrid-3-yl, Pyrid-4-yl, Pyrrol-2-yl, Pyrrol-3-yl, Thiophen-2-yl, Thiophen-3-yl, 4-Methyl-thiophen-2-yl, 3-Methyl-thiophen-2-yl, 5-Methyl-thiophen-2-yl, Furan-2-yl, Furan-3-yl, Imidazol-2-yl, Imidazol-4-yl, Thiazol-2-yl, Thiazol-4-yl, Thiazol-5-yl, Oxazol-2-yl, Oxazol-4-yl, Oxazol-5-yl, (1,2,4)-Thiadiazol-5-yl, (1,2,4)-Oxadiazol-5-yl, Napht-1-yl, Napht-2-yl, Anthracen-1-yl, Anthracen-2-yl, Anthracen-9-yl, Indol-2-yl, Indol-3-yl, Indol-4-yl, Indol-5-yl, Indol-6-yl, Indol-7-yl, 1-Methyl-indol-2-yl, 1-Methylindol-3-yl, 1-Methyl-indol-4-yl, 1-Methyl-indol-5-yl, 1-Methyl-indol-6-yl, 1-Methyl-indol-7-yl, Chinolin-3-yl, Chinolin-4-yl, Chinolin-2-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, Isochinolin-8-yl, 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Hydroxy-phenyl, 3-Hydroxy-phenyl, 4-Hydroxy-phenyl, 2-Amino-phenyl, 3-Amino-phenyl, 4-Amino-phenyl, 2-Dimethylamino-phenyl, 3-Dimethylaminophenyl, 4-Dimethylamino-phenyl, 2-Methylamino-phenyl, 3-Methylaminophenyl, 4-Methylamino-phenyl, 2-Acetyl-phenyl, 3-Acetyl-phenyl, 4-Acetylphenyl, 2-Methylsulfinyl-phenyl, 3-Methylsulfinyl-phenyl, 4-Methylsulfinyl-phenyl, 2-Methylsulfonyl-phenyl, 3-Methylsulfonyl-phenyl, 4-Methylsulfonyl-phenyl, 2-Methoxy-phenyl, 3-Methoxy-phenyl, 4-Methoxy-phenyl, 2-Chlor-phenyl, 3-Chlor-phenyl, 4-Chlor-phenyl, 2-Ethoxy-phenyl, 3-Ethoxy-phenyl, 4-Ethoxyphenyl, 2-Trifluormethyl-phenyl, 3-Trifluormethyl-phenyl, 4-Trifluormethyl-phenyl, 2-Difluormethyl-phenyl, 3-Difluormethyl-phenyl, 4-Difluormethyl-phenyl, 2-Fluormethyl-phenyl, 3-Fluormethyl-phenyl, 4-Fluormethyl-phenyl, 2-Nitro-phenyl, 3-Nitro-phenyl, 4-Nitro-phenyl, 2-Ethylphenyl, 3-Ethyl-phenyl, 4-Ethyl-phenyl, 2-Propyl-phenyl, 3-Propyl-phenyl, 4-Propyl-phenyl, 2-Isopropyl-phenyl, 3-Isopropyl-phenyl, 4-Isopropyl-phenyl, 2-tert-Butyl-phenyl, 3-tert-Butyl-phenyl, 4-tert-Butyl-phenyl, 2-Carboxyphenyl, 3-Carboxy-phenyl, 4-Carboxyphenyl, 2-Ethenyl-phenyl, 3-Ethenyl-phenyl, 4-Ethenyl-phenyl, 2-Ethinyl-phenyl, 3-Ethinyl-phenyl, 4-Ethinyl-phenyl, 2-Allylphenyl, 3-Allyl-phenyl, 4-Allyl-phenyl, 2-Trimethylsilanylethinyl-phenyl, 3-Trimethylsilanylethinyl-phenyl, 4-Trimethylsilanylethinyl-phenyl, 2-Formylphenyl, 3-Formyl-phenyl, 4-Formyl-phenyl, 2-Acetamino-phenyl, 3-Acetaminophenyl, 4-Acetamino-phenyl, 2-Dimethylaminocarbonyl-phenyl, 3-Dimethylaminocarbonyl-phenyl, 4-Dimethylaminocarbonyl-phenyl, 2-Methoxymethyl-phenyl, 3-Methoxymethyl-phenyl, 4-Methoxymethyl-phenyl, 2-Ethoxymethyl-phenyl, 3-Ethoxymethyl-phenyl, 4-Ethoxymethyl-phenyl, 2-Aminocarbonyl-phenyl, 3-Aminocarbonyl-phenyl, 4-Aminocarbonyl-phenyl, 2-Methylaminocarbonyl-phenyl, 3-Methylaminocarbonyl-phenyl, 4-Methylaminocarbonyl-phenyl, 2-Carboxymethylester-phenyl, 3-Carboxymethylester-phenyl, 4-Carboxymethylester-phenyl, 2-Carboxyethylester-phenyl, 3-Carboxyethylester-phenyl, 4-Carboxyethylesterphenyl, 2-Carboxy-tert-butylester-phenyl, 3-Carboxy-tert-butylester-phenyl, 4-Carboxy-tert-butylester-phenyl, 2-Methylmercapto-phenyl, 3-Methylmercaptophenyl, 4-Methylmercapto-phenyl, 2-Ethylmercapto-phenyl, 3-Ethylmercaptophenyl, 4-Ethylmercaptophenyl, 2-Biphenyl, 3-Biphenyl, 4-Biphenyl, 2-Bromphenyl, 3-Bromphenyl, 4-Bromphenyl, 2-lod-phenyl, 3-lodphenyl, 4-Iodphenyl, 2-Trifluormethoxy-phenyl, 3-Trilfuormethoxy-phenyl, 4-Trifluormethoxy-phenyl, 2-Fluor-3-trifluormethylphenyl, 2-Fluor-4-methylphenyl, (2,3)-Difluorphenyl, (2,3)-Dimethyl-phenyl, (2,3)-Dichlorphenyl, 3-Fluor-2-trifluormethylphenyl, (2,4)-Dichlor-phenyl, (2,4)-Difluorphenyl, 4-Fluor-2-trifluormethyl-phenyl, (2,4)-Dimethoxyphenyl, 2-Chlor-4-fluor-phenyl, 2-Chlor-4-nitro-pheriyl, 2-Chlor-4-methyl-phenyl, 2-Chlor-5-trifluormethyl-phenyl, 2-Chlor-5-methoxy-phenyl, 2-Brom-5-trifluormethyl-phenyl, 2-Brom-5-methoxyphenyl, (2,4)-Dibrom-phenyl, (2,4)-Dimethyl-phenyl, 2-Fluor-4-trifluormethyl-phenyl, (2,5)-Difluor-phenyl, 2-Fluor-5-trifluormethyl-phenyl, 5-Fluor-2-trifluormethyl-phenyl, 5-Chlor-2-trifluormethyl-phenyl, 5-Brom-2-trifluormethyl-phenyl, (2,5)-Dimethoxy-phenyl, (2,5)-Bis-trifluormethyl-phenyl, (2,5)-Dichlorphenyl, (2,5)-Dibrom-phenyl, 2-Methoxy-5-nitro-phenyl, 2-Fluor-6-trifluormethyl-phenyl, (2,6)-Dimethoxy-phenyl, (2,6)-Dimethyl-phenyl, (2,6)-Dichlor-phenyl, 2-Chlor-6-fluor-phenyl, 2-Brom-6-chlor-phenyl, 2-Brom-6-fluor-phenyl, (2,6)-Difluor-phenyl, (2,6)-Difluor-3-methyl-phenyl, (2,6)-Dibrom-phenyl, (2,6)-Dichlorphenyl, 3-Chlor-2-fluor-phenyl, 3-Chlor-5-methyl-phenyl, (3,4)-Dichlorphenyl, (3,4)-Dimethyl-phenyl, 3-Methyl-4-methoxy-phenyl, 4-Chlor-3-nitro-phenyl, (3,4)-Dimethoxy-phenyl, 4-Fluor-3-trifluormethylphenyl, 3-Fluor-4-trifluormethyl-phenyl, (3,4)-Difluor-phenyl, 3-Cyano-4-fluor-phenyl, 3-Cyano-4-methyl-phenyl, 3-Cyano-4-methoxy-phenyl, 3-Brom-4-fluor-phenyl, 3-Brom-4-methyl-phenyl, 3-Brom-4-methoxy-phenyl, 4-Chlor-2-fluor-phenyl, 4-Chlor-3-trifluormethyl, 4-Brom-3-methyl-phenyl, 4-Brom-5-methyl-phenyl, 3-Chlor-4-fluor-phenyl, 4-Fluor-3-nitro-phenyl, 4-Brom-3-nitro-phenyl, (3,4)-Dibrom-phenyl, 4-Chlor-3-methyl-phenyl, 4-Brom-3-methyl-phenyl, 4-Fluor-3-methyl-phenyl, 3-Fluor-4-methyl-phenyl, 3-Fluor-5-methyl-phenyl, 2-Fluor-3-methyl-phenyl, 4-Methyl-3-nitro-phenyl, (3,5)-Dimethoxy-phenyl, (3,5)-Dimethyl-phenyl, (3,5)-Bis-trifluormethyl-phenyl, (3,5)-Difluor-phenyl, (3,5)-Dinitro-phenyl, (3,5)-Dichlor-phenyl, 3-Fluor-5-trifluormethyl-phenyl, 5-Fluor-3-trifluormethyl-phenyl, (3,5)-Dibrom-phenyl, 5-Chlor-4-fluor-phenyl, 5-Chlor-4-fluor-phenyl, 5-Brom-4-methyl-phenyl, (2,3,4)-Trifluorphenyl, (2,3,4)-Trichlorphenyl, (2,3,6)-Trifluor-phenyl, 5-Chlor-2-methoxy-phenyl, (2,3)-Difluor-4-methyl, (2,4,5)-Trifluor-phenyl, (2,4,5)-Trichlor-phenyl, (2,4)-Dichlor-5-fluor-phenyl, (2,4,6)-Trichlor-phenyl, (2,4,6)-Trimethylphenyl, (2,4,6)-Trifluor-phenyl, (2,4,6)-Trimethoxy-phenyl, (3,4,5)-Trimethoxy-phenyl, (2,3,4,5)-Tetrafluor-phenyl, 4-Methoxy-(2,3,6)-trimethyl-phenyl, 4-Methoxy-(2,3,6)-trimethylphenyl, 4-Chlor-2,5-dimethyl-phenyl, 2-Chlor-6-fluor-3-methyl-phenyl, 6-Chlor-2-fluor-3-methyl, (2,4,6)-Trimethylphenyl, (2,3,4,5,6)-Pentafluor-phenyl, 3-Methyl-pyrid-2-yl, 4-Methyl-pyrid-2-yl, 5-Methyl-pyrid-2-yl, 6-Methyl-pyrid-2-yl, 2-Methyl-pyrid-3-yl, 4-Methyl-pyrid-3-yl, 5-Methyl-pyrid-3-yl, 6-Methyl-pyrid-3-yl, 2-Methyl-pyrid-4-yl, 3-Methyl-pyrid-4-yl, 3-Fluor-pyrid-2-yl, 4-Fluor-pyrid-2-yl, 5-Fluor-pyrid-2-yl, 6-Fluor-pyrid-2-yl, 3-Chlor-pyrid-2-yl, 4-Chlor-pyrid-2-yl, 5-Chlor-pyrid-2-yl, 6-Chlor-pyrid-2-yl, 3-Trifluormethyl-pyrid-2-yl, 4-Trifluormethyl-pyrid-2-yl, 5-Trifluormethyl-pyrid-2-yl, 6-Trifluormethyl-pyrid-2-yl, 3-Methoxy-pyrid-2-yl, 4-Methoxy-pyrid-2-yl, 5-Methoxy-pyrid-2-yl, 6-Methoxypyrid-2-yl, 4-Methyl-thiazol-2-yl, 5-Methyl-thiazol-2-yl, 4-Trifluormethyl-thiazol-2-yl, 5-Trifluormethyl-thiazol-2-yl, 4-Chlor-thiazol-2-yl, 5-Chlor-thiazol-2-yl, 4-Brom-thiazol-2-yl, 5-Brom-thiazol-2-yl, 4-Fluor-thiazol-2-yl, 5-Fluor-thiazol-2-yl, 4-Cyano-thiazol-2-yl, 5-Cyano-thiazol-2-yl, 4-Methoxy-thiazol-2-yl, 5-Methoxythiazol-2-yl, 4-Methyl-oxazol-2-yl, 5-Methyl-oxazol-2-yl, 4-Trifluormethyloxazol-2-yl, 5-Trifluormethyl-oxazol-2-yl, 4-Chlor-oxazol-2-yl, 5-Chlor-oxazol-2-yl, 4-Brom-oxazol-2-yl, 5-Brom-oxazol-2-yl, 4-Fluor-oxazol-2-yl, 5-Fluoroxazol-2-yl, 4-Cyano-oxazol-2-yl, 5-Cyano-oxazol-2-yl, , 4-Methoxy-oxazol-2-yl, 5-Methoxy-oxazol-2-yl, 2-Methyl-(1,2,4)-thiadiazol-5-yl, 2-Trifluormethyl-(1,2,4)-thiadiazol-5-yl, 2-Chlor-(1,2,4)-thiadiazol-5-yl, 2-Fluor-(1,2,4)-thiadiazol-5-yl, 2-Methoxy-(1,2,4)-thiadiazol-5-yl, 2-Cyano-(1,2,4)-thiadiazol-5-yl, 2-Methyl-(1,2,4)-oxadiazol-5-yl, 2-Trifluormethyl-(1,2,4)-oxadiazol-5-yl, 2-Chlor-(1,2,4)-oxadiazol-5-yl, 2-Fluor-(1,2,4)-oxadiazol-5-yl, 2-Methoxy-(1,2,4)-oxadiazol-5-yl und 2-Cyano-(1,2,4)-oxadiazol-5-yl steht,
und
R¹⁴, R¹⁵, R¹⁶, R¹⁷, R¹⁸, R¹⁹, und R²⁰, unabhängig voneinander, jeweils für einen Alkyl-Rest ausgewählt aus der Gruppe bestehend aus Methyl, Ethyl, n-Propyl, Isopropyl, n-Butyl, sec-Butyl, Isobutyl und tert-Butyl stehen; jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

11. Verbindungen gemäß einem oder mehreren der Ansprüche 1 bis 10, **dadurch gekennzeichnet, dass**
n = 0, 1 oder 2 ist,
X für N oder C-R² steht,
R¹ für einen Wasserstoff-Rest, für einen Halogen-Rest ausgewählt aus der Gruppe bestehend aus F, Cl und Br, für eine CF₃-Gruppe, für eine Nitro-Gruppe, für eine Carboxy-Gruppe, für eine -C(=O)-O-CH₃-Gruppe, für eine-C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine-S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, oder für einen Rest ausgewählt aus der Gruppe bestehend aus Phenyl und Oxadiazolyl, der jeweils unsubstituiert oder mit ggf. 1, 2, 3, 4 oder 5 Substituenten unabhängig voneinander ausgewählt aus der Gruppe bestehend aus F, Cl, Br, I, -O-CH₃, -O-C₂H₅ und -O-C₃H₇ substituiert ist, steht,
R² für einen Wasserstoff-Rest, für einen Halogen-Rest ausgewählt aus der Gruppe bestehend aus F, Cl und Br, für eine CF₃-Gruppe, für eine Nitro-Gruppe, für eine Carboxy-Gruppe, für eine -C(=O)-O-CH₃-Gruppe, für eine-C(=O)-O-C₂H₅-Gruppe, für eine -C(=O)-NH-R¹⁴-Gruppe, für eine -C(=O)-NR¹⁵R¹⁶-Gruppe, für eine -O-R¹⁷-Gruppe, für eine -S-R¹⁸-Gruppe, für eine-S(=O)₂-R²⁰-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl, oder für einen Rest ausgewählt aus der Gruppe bestehend aus Phenyl und Oxadiazolyl, der jeweils unsubstituiert oder mit ggf. 1, 2, 3, 4 oder 5 Substituenten unabhängig voneinander ausgewählt aus der Gruppe bestehend aus F, Cl, Br, I, -O-CH₃, -O-C₂H₅ und -O-C₃H₇ substituiert ist, steht,
R³ für eine Oxo-Gruppe, für einen Alkyl-Rest ausgewählt aus der Gruppe bestehend aus Methyl, Ethyl, n-Propyl, Isopropyl, n-Butyl, sec-Butyl, Isobutyl und tert-Butyl oder für einen unsubstituierten Phenyl-Rest steht,
R⁴ für einen Wasserstoff-Rest, für eine -C(=O)-NH₂-Gruppe, für eine -C(=O)-NH-R¹⁴-Gruppe, für eine -C(=O)-NR¹⁵R¹⁶-Gruppe, für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, neo-Pentyl, n-Hexyl, n-Heptyl und n-Octyl oder für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Phenyl, Pyrid-2-yl, Pyrid-3-yl, Pyrid-4-yl, Pyrrol-2-yl, Pyrrol-3-yl, Thiophen-2-yl, Thiophen-3-yl, 4-Methyl-thiophen-2-yl, 3-Methyl-thiophen-2-yl, 5-Methyl-thiophen-2-yl, Furan-2-yl, Furan-3-yl, Imidazol-2-yl, Imidazol-4-yl, Thiazol-2-yl, Thiazol-4-yl, Thiazol-5-yl, Oxazol-2-yl, Oxazol-4-yl, Oxazol-5-yl, (1,2,4)-Thiadiazol-5-yl, (1,2,4)-Oxadiazol-5-yl, Napht-1-yl, Napht-2-yl, Indol-3-yl, Indol-4-yl, Indol-5-yl, Indol-6-yl, Indol-7-yl, 1-Methylindol-2-yl, 1-Methyl-indol-3-yl, 1-Methyl-indol-4-yl, 1-Methyl-indol-5-yl, 1-Methyl-indol-6-yl, 1-Methyl-indol-7-yl, Chinolin-3-yl, Chinolin-4-yl, Chinolin-2-yl, Chinolin-5-yl, Chinolin-6-yl, Chinolin-7-yl, Chinolin-8-yl, Isochinolin-1-yl, lsochinolin-3-yl, Isochinolin-4-yl, Isochinolin-5-yl, Isochinolin-6-yl, Isochinolin-7-yl, Isochinolin-8-yl, 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyanophenyl, 4-Cyano-phenyl, 2-Hydroxy-phenyl, 3-Hydroxy-phenyl, 4-Hydroxyphenyl, 2-Amino-phenyl, 3-Amino-phenyl, 4-Amino-phenyl, 2-Dimethylaminophenyl, 3-Dimethylamino-phenyl, 4-Dimethylamino-phenyl, 2-Methylaminophenyl, 3-Methylamino-phenyl, 4-Methylamino-phenyl, 2-Acetyl-phenyl, 3-Acetyl-phenyl, 4-Acetyl-phenyl, 2-Methylsulfinyl-phenyl, 3-Methylsulfinyl-phenyl, 4-Methylsulfinyl-phenyl, 2-Methylsulfonyl-phenyl, 3-Methylsulfonyl-phenyl, 4-Methylsulfonyl-phenyl, 2-Methoxy-phenyl, 3-Methoxy-phenyl, 4-Methoxy-phenyl, 2-Chlor-phenyl, 3-Chlor-phenyl, 4-Chlor-phenyl, 2-Ethoxyphenyl, 3-Ethoxy-phenyl, 4-Ethoxyphenyl, 2-Trifluormethyl-phenyl, 3-Trifluormethyl-phenyl, 4-Trifluormethyl-phenyl, 2-Difluormethyl-phenyl, 3-Difluormethyl-phenyl, 4-Difluormethyl-phenyl, 2-Fluormethyl-phenyl, 3-Fluormethyl-phenyl, 4-Fluormethyl-phenyl, 2-Nitro-phenyl, 3-Nitro-phenyl, 4-Nitro-phenyl, 2-Ethyl-phenyl, 3-Ethyl-phenyl, 4-Ethyl-phenyl, 2-Propyl-phenyl, 3-Propyl-phenyl, 4-Propyl-phenyl, 2-Isopropyl-phenyl, 3-Isopropyl-phenyl, 4-Isopropyl-phenyl, 2-tert-Butyl-phenyl, 3-tert-Butyl-phenyl, 4-tert-Butyl-phenyl, 2-Carboxyphenyl, 3-Carboxy-phenyl, 4-Carboxyphenyl, 2-Ethenyl-phenyl, 3-Ethenyl-phenyl, 4-Ethenyl-phenyl, 2-Ethinyl-phenyl, 3-Ethinyl-phenyl, 4-Ethinyl-phenyl, 2-Allyl-phenyl, 3-Allyl-phenyl, 4-Allyl-phenyl, 2-Trimethylsilanylethinyl-phenyl, 3-Trimethylsilanylethinyl-phenyl, 4-Trimethylsilanylethinyl-phenyl, 2-Formyl-phenyl, 3-Formyl-phenyl, 4-Formylphenyl, 2-Acetamino-phenyl, 3-Acetamino-phenyl, 4-Acetamino-phenyl, 2-Dimethylaminocarbonyl-phenyl, 3-Dimethylaminocarbonyl-phenyl, 4-Dimethylaminocarbonyl-phenyl, 2-Methoxymethyl-phenyl, 3-Methoxymethylphenyl, 4-Methoxymethyl-phenyl, 2-Ethoxymethyl-phenyl, 3-Ethoxymethylphenyl, 4-Ethoxymethyl-phenyl, 2-Aminocarbonyl-phenyl, 3-Aminocarbonylphenyl, 4-Aminocarbonyl-phenyl, 2-Methylaminocarbonyl-phenyl, 3-Methylaminocarbonyl-phenyl, 4-Methylaminocarbonyl-phenyl, 2-Carboxymethylester-phenyl, 3-Carboxymethylester-phenyl, 4-Carboxymethylester-phenyl, 2-Carboxyethylester-phenyl, 3-Carboxyethylesterphenyl, 4-Carboxyethylester-phenyl, 2-Carboxy-tert-butylester-phenyl, 3-Carboxy-tert-butylester-phenyl, 4-Carboxy-tert-butylester-phenyl, 2-Methylmercapto-phenyl, 3-Methylmercapto-phenyl, 4-Methylmercapto-phenyl, 2-Ethylmercapto-phenyl, 3-Ethylmercapto-phenyl, 4-Ethylmercaptophenyl, 2-Biphenyl, 3-Biphenyl, 4-Biphenyl, 2-Bromphenyl, 3-Bromphenyl, 4-Bromphenyl, 2-lod-phenyl, 3-lodphenyl, 4-lodphenyl, 2-Trifluormethoxy-phenyl, 3-Trilfuormethoxy-phenyl, 4-Trifluormethoxy-phenyl, 2-Fluor-4-methyl-phenyl, (2,3)-Dimethyl-phenyl, (2,4)-Dichlor-phenyl, (2,4)-Difluorphenyl, 2-Chlor-4-methyl-phenyl, 2-Chlor-5-trifluormethyl-phenyl, 2-Chlor-5-methoxy-phenyl, 2-Brom-5-trifluormethyl-phenyl, 2-Brom-5-methoxyphenyl, (2,4)-Dimethyl-phenyl, (2,6)-Dimethyl-phenyl, 3-Chlor-5-methyl-phenyl, (3,4)-Dimethyl-phenyl, 3-Methyl-4-methoxy-phenyl, (3,4)-Difluor-phenyl, 3-Cyano-4-fluor-phenyl, 3-Cyano-4-methyl-phenyl, 3-Cyano-4-methoxy-phenyl, 3-Brom-4-fluor-phenyl, 3-Brom-4-methyl-phenyl, 3-Brom-4-methoxy-phenyl, 4-Chlor-2-fluor-phenyl, 4-Fluor-3-methyl-phenyl, 3-Fluor-4-methyl-phenyl, 3-Fluor-5-methyl-phenyl, 2-Fluor-3-methyl-phenyl, (3,5)-Dimethyl-phenyl und (3,5)-Dichlor-phenyl,
und
R¹⁴, R¹⁵, R¹⁶, R¹⁷, R¹⁸ und R²⁰, unabhängig voneinander, jeweils für einen Alkyl-Rest ausgewählt aus der Gruppe bestehend aus Methyl, Ethyl, n-Propyl, Isopropyl, n-Butyl, sec-Butyl, Isobutyl und tert-Butyl stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

12. Verbindungen gemäß einem oder mehreren der Ansprüche 1 bis 11 ausgewählt aus der Gruppe bestehend aus
| | |
|---|---|
| [1] | 1-(3-Phenyl-propiolyl)-4-(thiazol-2-yl)-piperazin, |
| [AAA00100] | 4-(3-Methylmercapto-1,2,4-thiadiazol-5-yl)-1-(3-phenyl-propiolyl)-piperazin |
| [AAA00101] | 4-(3-Methansulfonyl-1,2,4-thiadiazol-5-yl)-1-(3-phenyl-propiolyl)-piperazin |
| [AAA00102] | 4-(3-Methoxy-1,2,4-thiadiazol-5-yl)-1-(3-phenyl-propiolyl)-piperazin |
| [AAA00103] | 4-(1,2,4-Thiadiazol-5-yl)-1-(3-phenyl-propiolyl)-piperazin |
| [AAA00104] | 4-(3-Methyl-1,2,4-thiadiazol-5-yl)-1-(3-phenyl-propiolyl)-piperazin |
| [AAA00105] | 4-(3-Trifluormethyl-1,2,4-thiadiazol-5-yl)-1-(3-phenyl-propiolyl)-piperazin |
| [AAA00106] | 4-(5-(3-Methyl-1,2,4-oxadiazol-5-yl)-thiazol-2-yl)-1-(3-phenyl-propiolyl)-piperazin |
| [AAA00107] | 4-(4-tert-Butyl-thiazol-2-yl)-1-(3-(4-amino-phenyl)-propiolyl)-piperazin |
| [AAA1] | 4-(Thiazol-2-yl)-1-(3-(2-carboxy-phenyl)-propiolyl)-piperazin methylester |
| [AAA10] | 4-(Thiazol-2-yl)-1-(3-(2-thienyl)-propiolyl)-piperazin |
| [AAA11] | 4-(Thiazol-2-yl)-1-(3-(3-thienyl)-propiolyl)-piperazin |
| [AAA12] | 4-(Thiazol-2-yl)-1-(3-(2-methoxy-phenyl)-propiolyl)-piperazin |
| [AAA13] | 4-(Thiazol-2-yl)-1-(3-(3-methoxy-phenyl)-propiolyl)-piperazin |
| [AAA14] | 4-(Thiazol-2-yl)-1 -(3-(4-methoxy-phenyl)-propiolyl)-piperazin |
| [AAA15] | 4-(Thiazol-2-yl)-1-(3-(2-cyano-phenyl)-propiolyl)-piperazin |
| [AAA16] | 4-(Thiazol-2-yl)-1-(3-(3-cyano-phenyl)-propiolyl)-piperazin |
| [AAA17] | 4-(Thiazol-2-yl)-1-(3-(4-cyano-phenyl)-propiolyl)-piperazin |
| [AAA18] | 4-(Thiazol-2-yl)-1-(3-(2,4-dimethyl-phenyl)-propiolyl)-piperazin |
| [AAA19] | 4-(Thiazol-2-yl)-1-(3-(3,5-dimethyl-phenyl)-propiolyl)-piperazin |
| [AAA2] | 4-(Thiazol-2-yl)-1-(3-(4-carboxy-phenyl)-propiolyl)-piperazin methylester |
| [AAA20] | 4-(Thiazol-2-yl)-1-(3-(2,6-dimethyl-phenyl)-propiolyl)-piperazin |
| [AAA21] | 4-(Thiazol-2-yl)-1-(3-(2-fluor-phenyl)-propiolyl)-piperazin |
| [AAA22] | 4-(Thiazol-2-yl)-1-(3-(3-fluor-phenyl)-propiolyl)-piperazin |
| [AAA23] | 4-(Thiazol-2-yl)-1-(3-(2-chlor-phenyl)-propiolyl)-piperazin |
| [AAA24] | 4-(Thiazol-2-yl)-1-(3-(3-chlor-phenyl)-propiolyl)-piperazin |
| [AAA25] | 4-(Thiazol-2-yl)-1-(3-naphthyl-propiolyl)-piperazin |
| [AAA26] | 4-(Thiazol-2-yl)-1-(3-(2,3-dimethyl-phenyl)-propiolyl)-piperazin |
| [AAA27] | 4-(Thiazol-2-yl)-1-(3-(3,4-dimethyl-phenyl)-propiolyl)-piperazin |
| [AAA28] | 4-(Thiazol-2-yl)-1-(3-(3-nitro-phenyl)-propiolyl)-piperazin |
| [AAA29] | 4-(Thiazol-2-yl)-1-(3-(2-nitro-phenyl)-propiolyl)-piperazin |
| [AAA3] | 4-(Thiazol-2-yl)-1-(3-(3-carboxy-phenyl)-propiolyl)-piperazin ethylester |
| [AAA30] | 4-(Thiazol-2-yl)-1-(3-(3-formyl-phenyl)-propiolyl)-piperazin |
| [AAA31] | 4-(Thiazol-2-yl)-1-(3-(3-ethenyl-phenyl)-propiolyl)-piperazin |
| [AAA32] | 4-(Thiazol-2-yl)-1-(3-pyrid-2-yl-propiolyl)-piperazin |
| [AAA33] | 4-(Thiazol-2-yl)-1-(3-pyrid-3-yl-propiolyl)-piperazin |
| [AAA34] | 4-(Thiazol-2-yl)-1-(3-pyrid-4-yl-propiolyl)-piperazin |
| [AAA35] | 4-(Thiazol-2-yl)-1-(3-(chinolin-6-yl)-propiolyl)-piperazin |
| [AAA36] | 4-(Thiazol-2-yl)-1-(3-(3-isopropyl-phenyl)-propiolyl)-piperazin |
| [AAA37] | 4-(Thiazol-2-yl)-1-(3-biphenyl-3-yl-propiolyl)-piperazin |
| [AAA38] | 4-(Thiazol-2-yl)-1-(3-naphth-2-yl-propiolyl)-piperazin |
| [AAA39] | 4-(Thiazol-2-yl)-1-(3-(1-methyl-indol-5-yl)-propiolyl)-piperazin |
| [AAA4] | 4-(Thiazol-2-yl)-1-(3-(2-hydroxy-phenyl)-propiolyl)-piperazin |
| [AAA40] | 4-(Thiazol-2-yl)-1-(3-(3-methylmercapto-phenyl)-propiolyl)-piperazin |
| [AAA41] | 4-(Thiazol-2-yl)-1-(3-(3-cyano-4-fluor-phenyl)-propiolyl)-piperazin |
| [AAA42] | 4-(Thiazol-2-yl)-1-(3-(3-methoxymethyl-phenyl)-propiolyl)-piperazin |
| [AAA43] | 4-(Thiazol-2-yl)-1-(3-(3-hydroxy-phenyl)-propiolyl)-piperazin |
| [AAA44] | 4-(Thiazol-2-yl)-1-(3-(3-acetamino-phenyl)-propiolyl)-piperazin |
| [AAA45] | 4-(Thiazol-2-yl)-1-(3-(4-acetamino-phenyl)-propiolyl)-piperazin |
| [AAA46] | 4-(Thiazol-2-yl)-1-(3-(2-carboxy-phenyl)-propiolyl)-piperazin |
| [AAA47] | 4-(Thiazol-2-yl)-1-(3-(3-carboxy-phenyl)-propiolyl)-piperazin |
| [AAA48] | 4-(Thiazol-2-yl)-1-(3-(4-carboxy-phenyl)-propiolyl)-piperazin |
| [AAA49] | 4-(Thiazol-2-yl)-1-(3-(3-carboxy-phenyl)-propiolyl)-piperazin methylester |
| [AAA5] | 4-(Thiazol-2-yl)-1-(3-(4-hydroxy-phenyl)-propiolyl)-piperazin |
| [AAA50] | 4-(Thiazol-2-yl)-1-(3-(3-aminocarbonyl-phenyl)-propiolyl)-piperazin |
| [AAA51] | 4-(Thiazol-2-yl)-1-(3-(3-methylaminocarbonyl-phenyl)-propiolyl)-piperazin |
| [AAA52] | 4-(Thiazol-2-y1)-1-(3-(3-dimethylaminocarbonyl-phenyl)-propiolyl)-piperazin |
| [AAA53] | 4-(Thiazol-2-yl)-1-(3-(2-tolyl)-propiolyl)-piperazin Hydrochlorid |
| [AAA54] | 4-(Thiazol-2-yl)-1-(3-(3-tolyl)-propiolyl)-piperazin Hydrochlorid |
| [AAA55] | 4-(Thiazol-2-yl)-1-(3-(4-tolyl)-propiolyl)-piperazin Hydrochlorid |
| [AAA56] | 4-(Thiazol-2-yl)-1-(3-phenyl-propiolyl)-piperazin Hydrochlorid |
| [AAA57] | 4-(Thiazol-2-yl)-1-(3-(2-trifluormethyl-phenyl)-propiolyl)-piperazin Hydrochlorid |
| [AAA58] | 4-(Thiazol-2-yl)-1-(3-(3-trifluormethyl-phenyl)-propiolyl)-piperazin Hydrochlorid |
| [AAA59] | 4-(Thiazol-2-yl)-1-(3-(4-trifluormethyl-phenyl)-propiolyl)-piperazin Hydrochlorid |
| [AAA6] | 4-(Thiazol-2-yl)-1-(3-(2-amino-phenyl)-propiolyl)-piperazin |
| [AAA60] | 4-(Thiazol-2-yl)-1-(3-pentyl-propiolyl)-piperazin |
| [AAA61] | 4-(Thiazol-2-yl)-1-(3-(4-fluor-phenyl)-propiolyl)-piperazin |
| [AAA62] | 4-(Thiazol-2-yl)-1-(3-(4-chlor-phenyl)-propiolyl)-piperazin |
| [AAA63] | 2-Methyl-4-(thiazol-2-yl)-1-(3-phenyl-propiolyl)-piperazin |
| [AAA64] | (S)-2-Methyl-4-(thiazol-2-yl)-1 -(3-phenyl-propiolyl)-piperazin |
| [AAA65] | (R)-2-Methyl-4-(thiazol-2-yl)-1-(3-phenyl-propiolyl)-piperazin |
| [AAA66] | 2-Methyl-4-(thiazol-2-yl)-1-(3-phenyl-propiolyl)-piperazin Hydrochlorid |
| [AAA67] | 2-Methyl-4-(thiazol-2-yl)-1 -(3-(3-tolyl)-propiolyl)-piperazin |
| [AAA68] | 2-Methyl-4-(thiazol-2-yl)-1-(3-(3-tolyl)-propiolyl)-piperazin Hydrochlorid |
| [AAA69] | 4-(Thiazol-2-yl)-1-(3-cyclohexyl-propiolyl)-piperazin |
| [AAA7] | 4-(Thiazol-2-yl)-1-(3-(3-amino-phenyl)-propiolyl)-piperazin |
| [AAA70] | 4-(Thiazol-2-yl)-1-(3-methyl-propiolyl)-piperazin |
| [AAA71] | 2-Ethyl-4-(thiazol-2-yl)-1-(3-phenyl-propiolyl)-piperazin |
| [AAA72] | 2-Phenyl-4-(thiazol-2-yl)-1-(3-phenyl-propiolyl)-piperazin |
| [AAA73] | 4-(Thiazol-2-yl)-1-(3-(2-furyl)-propiolyl)-piperazin |
| [AAA74] | 4-(Thiazol-2-yl)-1-(3-(3-furyl)-propiolyl)-piperazin |
| [AAA75] | cis-2,6-Dimethyl-4-(thiazol-2-yl)-1-(3-phenyl-propiolyl)-piperazin |
| [AAA76] | 4-(5-Carboxy-thiazol-2-yl)-1-(3-phenyl-propiolyl)-piperazin ethylester |
| [AAA77] | 4-(4-Carboxy-thiazol-2-yl)-1-(3-phenyl-propiolyl)-piperazin ethylester |
| [AAA78] | 4-(5-Carboxy-thiazol-2-yl)-1-(3-phenyl-propiolyl)-piperazin |
| [AAA79] | 4-(4-Carboxy-thiazol-2-yl)-1-(3-phenyl-propiolyl)-piperazin |
| [AAA8] | 4-(Thiazol-2-yl)-1-(3-(4-amino-phenyl)-propiolyl)-piperazin |
| [AAA80] | 4-(5-Methylaminocarbonyl-thiazol-2-y1)-1-(3-phenyl-propiolyl)-piperazin |
| [AAA81] | 4-(5-Dimethylaminocarbonyl-thiazol-2-yl)-1-(3-phenyl-propiolyl)-piperazin |
| [AAA82] | 4-(3-Phenyl-1,2,4-thiadiazol-5-yl)-1-(3-phenyl-propiolyl)-piperazin |
| [AAA83] | 4-(Thiazol-2-yl)-1-(3-(chinol-7-yl)-propiolyl)-piperazin |
| [AAA84] | 4-(Thiazol-2-yl)-1 -(3-phenyl-propiolyi)-piperazin-3-on |
| [AAA85] | 4-(3-(4-Fluorbenzyl)-1,2,4-thiadiazol-5-yl)-1-(3-phenyl-propiolyl)-piperazin |
| [AAA86] | 4-(5-Nitro-thiazol-2-yl)-1-(3-phenyl-propiolyl)-piperazin |
| [AAA87] | 4-(4-tert-Butyl-thiazol-2-yl)-1-(3-phenyl-propiolyl)-piperazin |
| [AAA88] | 4-(5-Methyl-thiazol-2-yl)-1-(3-phenyl-propiolyl)-piperazin |
| [AAA89] | 4-(4,5-Dimethyl-thiazol-2-yl)-1-(3-phenyl-propiolyl)-piperazin |
| [AAA9] | 4-(Thiazol-2-yl)-1-(3-(indol-5-yl)-propiolyl)-piperazin |
| [AAA90] | 4-(5-Brom-4-phenyl-thiazol-2-yl)-1-(3-phenyl-propiolyl)-piperazin |
| [AAA91] | 4-(5-Brom-4-methyl-thiazol-2-yl)-1-(3-phenyl-propiolyl)-piperazin |
| [AAA92] | 4-(4-Methyl-thiazol-2-yl)-1-(3-phenyl-propiolyl)-piperazin |
| [AAA93] | 4-(4-Trifluormethyl-thiazol-2-yl)-1-(3-phenyl-propiolyl)-piperazin |
| [AAA94] | 4-(4-Chlor-thiazol-2-yl)-1-(3-phenyl-propiolyl)-piperazin |
| [AAA95] | 4-(5-Chlor-thiazol-2-yl)-1-(3-phenyl-propiolyl)-piperazin |
| [AAA96] | 4-(4-Brom-thiazol-2-yl)-1 -(3-phenyl-propiolyl)-piperazin |
| [AAA97] | 4-(5-Brom-thiazol-2-yl)-1-(3-phenyl-propiolyl)-piperazin |
| [AAA98] | 4-(5-Phenyl-thiazol-2-yl)-1-(3-phenyl-propiolyl)-piperazin |
| [AAA99] | 4-(4-Phenyl-thiazol-2-yl)-1-(3-phenyl-propiolyl)-piperazin |
| [BBB2] | 4-(Thiazol-2-yl)-1-propiolyl-piperazin; |
| [CCC1] | 4-(3-Phenyl-1,2,4-thiadiazol-5-yl)-1-(3-(2-methoxy-phenyl)-propiolyl)-piperazin |
| [CCC2] | 4-(3-Phenyl-1,2,4-thiadiazol-5-yl)-1-(3-(4-methoxy-phenyl)-propiolyl)-piperazin |
| [CCC3] | 4-(3-Phenyl-1,2,4-thiadiazol-5-yl)-1-(3-(2-fluor-phenyl)-propiolyl)-piperazin |
| [CCC4] | 4-(3-Phenyl-1,2,4-thiadiazol-5-yl)-1 -(3-(3-fluor-phenyl)-propiolyl)-piperazin |
| [CCC5] | 4-(3-Phenyl-1,2,4-thiadiazol-5-yl)-1-(3-(4-fluor-phenyl)-propiolyl)-piperazin |
| [CCC6] | 4-(3-Phenyl-1,2,4-thiadiazol-5-yl)-1-(3-(2,4-dichlor-phenyl)-propiolyl)-piperazin |
| [CCC7] | 4-(3-Phenyl-1,2,4-thiadiazol-5-yl)-1-(3-(3,5-dichlor-phenyl)-propiolyl)-piperazin |
| [CCC8] | 4-(3-(4-Fluor-phenyl)-1,2,4-thiadiazol-5-yl)-1-(3-(tol-2-yl)-propiolyl)-piperazin |
| [CCC9] | 4-(3-(4-Fluor-phenyl)-1,2,4-thiadiazol-5-yl)-1 -(3-(3-methoxy-phenyl)-propiolyl)-piperazin |
| [CCC10] | 4-(3-(4-Fluor-phenyl)-1,2,4-thiadiazol-5-yl)-1-(3-(3-fluor-4-methyl-phenyl)-propiolyl)-piperazin |
| [CCC11] | 4-(3-(4-Fluor-phenyl)-1,2,4-thiadiazol-5-yl)-1-(3-(2,4-difluor-phenyl)-propiolyl)-piperazin |
| [CCC12] | 4-(3-(4-Fluor-phenyl)-1,2,4-thiadiazol-5-yl)-1-(3-(tol-3-yl)-propiolyl)-piperazin |
| [CCC13] | 4-(3-(4-Fluor-phenyl)-1,2,4-thiadiazol-5-yl)-1-(3-(4-trifluormethyl-phenyl)-propiolyl)-piperazin |
| [CCC14] | 4-(3-(4-Fluor-phenyl)-1,2,4-thiadiazol-5-yl)-1-(3-(3-trifluormethylphenyl)-propiolyl)-piperazin |
| [CCC15] | 4-(3-(4-Fluor-phenyl)-1,2,4-thiadiazol-5-yl)-1-(3-(2-chlor-5-trifluormethyl-phenyl)-propiolyl)-piperazin |
| [CCC16] | 4-(3-tert-Butyl-1,2,4-thiadiazol-5-yl)-1-(3-(thiophen-2-yl)-propiolyl)-piperazin |
| [CCC17] | 4-(3-tert-Butyl-1,2,4-thiadiazol-5-yl)-1-(3-(3-trifluormethyl-phenyl)-propiolyl)-piperazin |
| [CCC18] | 4-(3-tert-Butyl-1,2,4-thiadiazol-5-yl)-1-(3-(2-methoxy-phenyl)-propiolyl)-piperazin |
| [CCC19] | 4-(3-tert-Butyl-1,2,4-thiadiazol-5-yl)-1 -(3-(tol-2-yl)-propiolyl)-piperazin |
| [CCC20] | 4-(3-tert-Butyl-1,2,4-thiadiazol-5-yl)-1 -(3-(3-fluor-4-methyl-phenyl)-propiolyl)-piperazin |
| [CCC21] | 4-(3-tert-Butyl-1,2,4-thiadiazol-5-yl)-1-(3-(2-chlor-5-trifluormethylphenyl)-propiolyl)-piperazin |
| [CCC22] | 4-(Thiazol-2-yl)-1-(3-(2,4-difluor-phenyl)-propiolyl)-piperazin |
| [CCC23] | 4-(Thiazol-2-yl)-1-(3-(2-brom-5-methoxy-phenyl)-propiolyl)-piperazin |
| [CCC24] | 4-(Thiazol-2-yl)-1-(3-(3-brom-4-methoxy-phenyl)-propiolyl)-piperazin |
| [CCC25] | 4-(Thiazol-2-yl)-1-(3-(3,5-dichlor-phenyl)-propiolyl)-piperazin |
| [CCC26] | 4-(Thiazol-2-yl)-1-(3-(4-fluor-3-methyl-phenyl)-propiolyl)-piperazin |
| [CCC27] | 4-(4-tert-Butyl-thiazol-2-yl)-1-(3-(3-cyano-phenyl)-propiolyl)-piperazin |
| [CCC28] | 4-(4-tert-Butyl-thiazol-2-yl)-1-(3-(2-trifluormethyl-phenyl)-propiolyl)-piperazin |
| [CCC29] | 4-(4-Phenyl-thiazol-2-yl)-1-(3-(2,3-dimethyl-phenyl)-propiolyl)-piperazin |
| [CCC30] | 4-(4-Phenyl-thiazol-2-yl)-1-(3-(4-fluor-phenyl)-propiolyl)-piperazin |
| [CCC31] | 4-(4-Methyl-thiazol-2-yl)-1-(3-(tol-3-yl)-propiolyl)-piperazin |
| [CCC32] | 4-(4-Methyl-thiazol-2-yl)-1-(3-(thiophen-2-yl)-propiolyl)-piperazin |
| [CCC33] | 4-(5-Methyl-thiazol-2-yl)-1-(3-(tol-4-yl)-propiolyl)-piperazin |
| [CCC34] | 4-(5-Methyl-thiazol-2-yl)-1-(3-(2-cyano-phenyl)-propiolyl)-piperazin |
| [CCC35] | 4-(4,5-Dimethyl-thiazol-2-yl)-1-(3-(3-cyano-phenyl)-propiolyl)-piperazin |
| [CCC36] | 4-(4,5-Dimethyl-thiazol-2-yl)-1-(3-(3,4-dimethyl-phenyl)-propiolyl)-piperazin |
| [CCC37] | 4-(4-(4-Methoxy-phenyl)-thiazol-2-yl)-1-(3-(2,4-dimethyl-phenyl)-propiolyl)-piperazin |
| [CCC38] | 4-(4-(4-Methoxy-phenyl)-thiazol-2-yl)-1-(3-(4-trifluormethyl-phenyl)-propiolyl)-piperazin |
| [CCC39] | 4-(4-(4-Fluor-phenyl)-thiazol-2-yl)-1-(3-(2-cyano-phenyl)-propiolyl)-piperazin |
| [CCC40] | 4-(4-(4-Chlor-phenyl)-thiazol-2-yl)-1-(3-(4-cyano-phenyl)-propiolyl)-piperazin |
| [AAA00108] | 4-(Thiazol-2-yl)-1-(3-(1-Methyl-indol-6-yl)-propiolyl)-piperazin |
| [AAA00109] | 4-(Thiazol-2-yl)-1-(3-(3-acetyl-phenyl)-propiolyl)-piperazin |
| [AAA00110] | 4-(Thiazol-2-yl)-1-(3-(3-fluor-5-methyl-phenyl)-propiolyl)-piperazin |
| [AAA00111] | 4-(Thiazol-2-yl)-1-(3-(2-fluor-3-methyl-phenyl)-propiolyl)-piperazin |
| [AAA00112] | 4-(Thiazol-2-yl)-1-(3-(3-methylamino-phenyl)-propiolyl)-piperazin |
| [AAA00113] | 4-(Thiazol-2-yl)-1-(3-(3-dimethylamino-phenyl)-propiolyl)-piperazin |
| [AAA00114] | 4-(Thiazol-2-yl)-1-(dimethylcarbamoyl-propiolyl)-piperazin |
| [AAA00115] | 4-(Thiazol-2-yl)-1-(3-(3-methylsulfinyl-phenyl)-propiolyl)-piperazin |
| [AAA00116] | 4-(Thiazol-2-yl)-1-(3-(3-methylsulfonyl-phenyl)-propiolyl)-piperazin |
| [AAA00117] | 4-(Thiazol-2-yl)-1-(3-(3-ethinyl-phenyl)-propiolyl)-piperazin |
| [AAA00118] | 4-(Thiazol-2-yl)-1-(3-(4-methyl-thiophen-2-yl)-propiolyl)-piperazin |
| [AAA00119] | 2-Methyl-4-(thiazol-2-yl)-1-(3-(3-chlor-phenyl)-propiolyl)-piperazin |
| [AAA00120] | 4-(Thiazol-2-yl)-1-(3-(3-ethyl-phenyl)-propiolyl)-piperazin |
| [AAA00121] | 2-tert-Butyl-4-(thiazol-2-yl)-1-(3-phenyl-propiolyl)-piperazin |
| [AAA00122] | 4-(Thiazol-2-yl)-1-(3-(3-difluormethyl-phenyl)-propiolyl)-piperazin |
| [AAA00123] | 2-Methyl-4-(thiazol-2-yl)-1-(3-(3-cyano-phenyl)-propiolyl)-piperazin |
| [AAA00124] | 2-Isopropyl-4-(thiazol-2-yl)-1-(3-phenyl-propiolyl)-piperazin |
| [AAA00125] | 4-(Thiazol-2-yl)-1-(3-(3-trimethylsilanylethinyl-phenyl)-propiolyl)-piperazin; |
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, bevorzugt in Form entsprechender Hydrochloride, oder jeweils in Form entsprechender Solvate.

13. Verfahren zur Herstellung von Verbindungen der allgemeinen Formel I gemäß einem oder mehreren der Ansprüche 1 bis 12, **dadurch gekennzeichnet, dass** wenigstens eine Verbindung der allgemeinen Formel II, 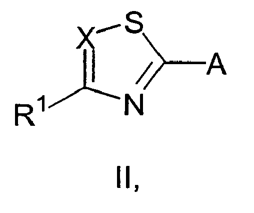 worin die Reste X und R¹ die Bedeutung gemäß einem oder mehreren der Ansprüche 1 bis 12 haben und A für eine Abgangsgruppe, vorzugsweise für einen Halogen-Rest oder einen Sulfonsäureester, besonders bevorzugt für einen Chlor- oder Brom-Rest, steht, mit wenigstens einer Verbindung der allgemeinen Formel III, 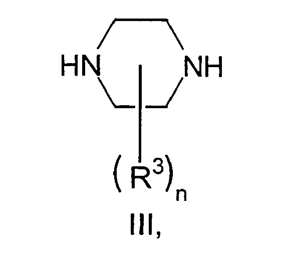 worin R³ und n die Bedeutung gemäß einem oder mehreren der Ansprüche 1 bis 12 haben, ggf. in einem Reaktionsmedium, ggf. in Gegenwart wenigstens einer Base und/oder wenigstens einer metallorganischen Verbindung und/oder wenigstens eines Metallhydrid-Reagenzes, vorzugsweise bei einer Temperatur von - 70 °C bis 300 °C, bevorzugt - 70 °C bis 150 °C, in wenigstens eine entsprechende Verbindung der allgemeinen Formel IV, ggf. in Form eines entsprechenden Salzes überführt wird, 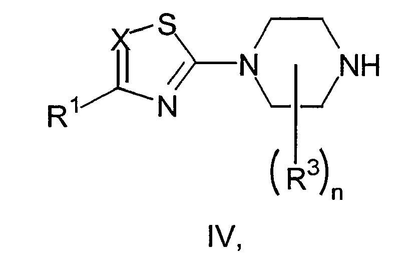 worin X, R¹, R³ und n die vorstehend genannte Bedeutung haben, und diese ggf. gereinigt und/oder isoliert wird;
oder wenigstens eine Verbindung der allgemeinen Formel II mit wenigstens einer Verbindung der allgemeinen Formel V, 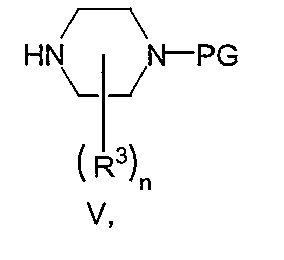 worin R³ und n die Bedeutung gemäß einem oder mehreren der Ansprüche 1 bis 12 haben und PG für eine Schutzgruppe, bevorzugt eine Schutzgruppe ausgewählt aus der Gruppe bestehend aus tert-Butyloxy-carbonyl, Benzyl, Benzyloxycarbonyl und 9-Fluorenylmethyloxycarbonyl, steht ggf. in einem Reaktionsmedium, ggf. in Gegenwart wenigstens einer Base und/oder wenigstens einer metallorganischen Verbindung und/oder wenigstens eines Metallhydrid-Reagenzes, vorzugsweise bei einer Temperatur von - 70 °C bis 300 °C in wenigstens eine entsprechende Verbindung der allgemeinen Formel VI überführt wird, 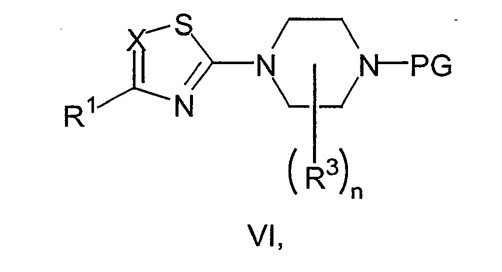 worin R¹, R³, X, n und PG die vorstehend genannte Bedeutung haben, und diese ggf. gereinigt und/oder isoliert wird;
oder wenigstens eine Verbindung der allgemeinen Formel VII, 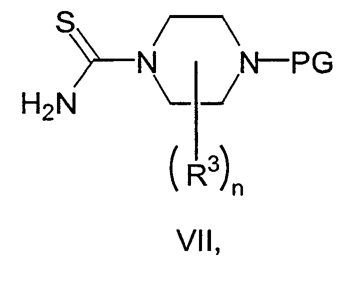 worin R³ und n die Bedeutung gemäß einem oder mehreren der Ansprüche 1 bis 12 haben und PG für eine Schutzgruppe, bevorzugt eine Schutzgruppe ausgewählt aus der Gruppe bestehend aus tert-Butyloxy-carbonyl, Benzyl, Benzyloxycarbonyl und 9-Fluorenylmethyloxycarbonyl, steht durch Umsetzung mit wenigstens einer Verbindung der allgemeinen Formel R¹-C(=O)-CH₂-A oder (C₁₋₅-Alkyl-O)₂-CH-CH₂-A, worin R¹ die Bedeutung gemäß einem oder mehreren der Ansprüche 1 bis 12 hat und A für eine Abgangsgruppe, vorzugsweise für einen Halogen-Rest, besonders bevorzugt für ein Brom-Atom steht, in einem Reaktionsmedium, ggf. in Gegenwart wenigstens einer organischen Base oder in Gegenwart wenigstens einer Säure, vorzugsweise in Gegenwart wenigstens einer Base ausgewählt aus der Gruppe bestehend aus Triethylamin, Diisopropylethylamin, N-Methylmorpholin, Dimethylaminopyridin und Pyridin oder in Gegenwart wenigstens einer Säure ausgewählt aus der Gruppe bestehend aus Essigsäure, Trifluoressigsäure und Salzsäure, vorzugsweise bei einer Temperatur zwischen - 70 °C bis 300 °C in wenigstens eine entsprechende Verbindung der allgemeinen Formel VI, ggf. in Form eines entsprechenden Salzes überführt wird, worin R¹, R³ und n die Bedeutung gemäß einem oder mehreren der Ansprüche 1 bis 12 haben, PG die vorstehend genannte Bedeutung hat und X für CH steht, und diese ggf. gereinigt und/oder isoliert wird;
und wenigstens eine Verbindung der allgemeinen Formel VI für den Fall, dass PG für eine tert-Butoxycarbonyl- oder 9-Fluorenylmethyloxycarbonyl-Gruppe steht, in einem Reaktionsmedium, in Gegenwart wenigstens einer Säure, vorzugsweise in Gegenwart wenigstens einer Säure ausgewählt aus der Gruppe bestehend aus Salzsäure und Trifluoressigsäure, vorzugsweise bei einer Temperatur zwischen -70 °C bis 100 °C oder für den Fall, dass PG für eine Benzyl-Gruppe oder Benzyloxycarbonyl-Gruppe steht, in einem Reaktionsmedium, in Gegenwart von Wasserstoff und in Gegenwart wenigstens eines Katalysators, vorzugsweise in Gegenwart von Palladium auf Kohle, vorzugsweise bei einer Temperatur zwischen -70 °C bis 100 °C in wenigstens eine entsprechende Verbindung der allgemeinen Formel IV, ggf. in Form eines entsprechenden Salzes überführt wird, worin X, R¹, R³ und n die Bedeutung gemäß einem oder mehreren der Ansprüche 1 bis 12 haben, und diese ggf. gereinigt und/oder isoliert wird;
und wenigstens eine Verbindung der allgemeinen Formel IV durch Umsetzung mit wenigstens einer Verbindung der allgemeinen Formel R⁴-C≡C-C(=O)-OH, worin R⁴ die Bedeutung gemäß einem oder mehreren der Ansprüche 1 bis 12 hat, in einem Reaktionsmedium, ggf. in Gegenwart wenigstens eines geeigneten Kopplungsmittels, ggf. in Gegenwart wenigstens einer Base, vorzugsweise bei einer Temperatur von - 70 °C bis 100 °C, oder durch Umsetzung mit wenigstens einer Verbindung der allgemeinen Formel R⁴-C≡C-C(=O)-A, worin R⁴ die vorstehend genannte Bedeutung hat und A für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel I, ggf. in Form eines entsprechenden Salzes überführt wird, 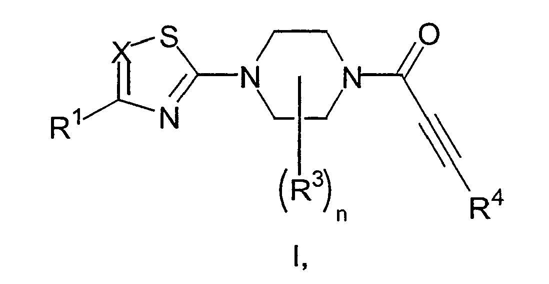 worin X und R¹, R³, R⁴ und n die vorstehend genannte Bedeutung haben, und diese ggf. gereinigt und/oder isoliert wird;
oder wenigstens eine Verbindung der allgemeinen Formel IV durch Umsetzung mit Propinsäure [HC≡C-C(=O)-OH] in einem Reaktionsmedium, ggf. in Gegenwart wenigstens eines geeigneten Kopplungsmittels, ggf. in Gegenwart wenigstens einer Base, vorzugsweise bei einer Temperatur von - 70 °C bis 100 °C, oder durch Umsetzung mit wenigstens einer Verbindung der allgemeinen Formel HC≡C-C(=O)-A, worin A für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel VIII, ggf. in Form eines entsprechenden Salzes überführt wird, 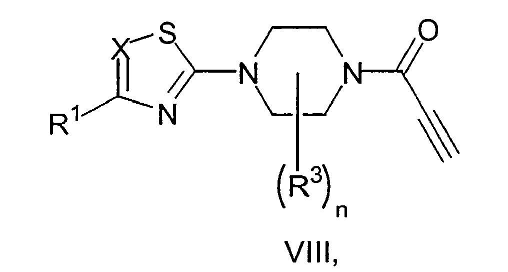 worin R¹ R³, X und n die vorstehend genannte Bedeutung haben, und diese ggf. gereinigt und/oder isoliert wird,
und wenigstens eine Verbindung der allgemeinen Formel VIII durch Umsetzung mit wenigstens einer Verbindung der allgemeinen Formel R⁴-A, worin R⁴ die Bedeutung gemäß einem oder mehreren der Ansprüche 10 bis 13 mit Ausnahme von Wasserstoff hat und A für eine Abgangsgruppe, vorzugsweise für einen Halogen-Rest oder einen Sulfonsäureester, besonders bevorzugt für Iod, Brom oder Triflat, steht, in einem Reaktionsmedium, ggf. in Gegenwart wenigstens eines Katalysators, bevorzugt in Gegenwart eines Palladium-Katalysators ausgewählt aus der Gruppe bestehend aus Palladiumchlorid [PdCl_{2]}, Palladiumacetat [Pd(OAc)₂], Tetrakistriphenylphosphinpalladium [Pd(PPh₃)₄], Bistriphenylphosphinpalladiumdichlorid [Pd(PPh₃)₂Cl₂] und Bistriphenylphosphinpalladiumacetat [Pd(PPh₃)₂(OAc)₂], ggf. in Gegenwart wenigstens eines Liganden, bevorzugt in Gegenwart wenigstens eines Liganden ausgewählt aus der Gruppe bestehend aus Triphenylphosphin, Triphenylarsin und Tri-2-furyl-phoshpin, ggf. in Gegenwart wenigstens eines anorganischen Salzes, bevorzugt in Gegenwart wenigstens eines anorganischen Salzes ausgewählt aus der Gruppe bestehend aus Lithiumchlorid und Zinkchlorid, ggf. in Gegenwart wenigstens eines Kupfersalzes, bevorzugt in Gegenwart von Kupferiodid, ggf. in Gegenwart wenigstens einer organischen oder anorganischen Base, vorzugsweise in Gegenwart wenigstens einer Base ausgewählt aus der Gruppe bestehend aus Triethylamin, [1,4]-Diazabicyclo-[2.2.2]-octan, Diisopropylamin, Diisopropylethylamin, Kaliumcarbonat und Natriumhydrogencarbonat, vorzugsweise bei einer Temperatur zwischen -70 °C bis 300 °C in wenigstens eine entsprechende Verbindung der allgemeinen Formel I, ggf. in Form eines entsprechenden Salzes überführt wird, und diese ggf. gereinigt und/oder isoliert wird.

14. Verfahren zur Herstellung von Verbindungen der allgemeinen Formel I gemäß einem oder mehreren der Ansprüche 1 bis 12, **dadurch gekennzeichnet, dass** wenigstens eine Verbindung der allgemeinen Formel III, 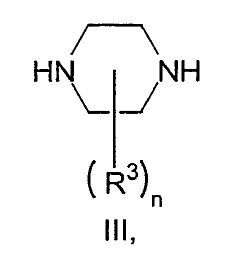 worin R³ und n die Bedeutung gemäß einem oder mehreren der Ansprüche 1 bis 12 haben, durch Umsetzung mit wenigstens einer Verbindung der allgemeinen Formel R⁴-C≡C-C(=O)-OH, worin R⁴ die Bedeutung gemäß einem oder mehreren der Ansprüche 1 bis 12 hat, in einem Reaktionsmedium, ggf. in Gegenwart wenigstens eines geeigneten Kopplungsmittels, ggf. in Gegenwart wenigstens einer Base, vorzugsweise bei einer Temperatur von - 70 °C bis 100 °C, oder durch Umsetzung mit wenigstens einer Verbindung der allgemeinen Formel R⁴-C≡C-C(=O)-A, worin R⁴ die vorstehend genannte Bedeutung hat und A für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel IX, ggf. in Form eines entsprechenden Salzes überführt wird, 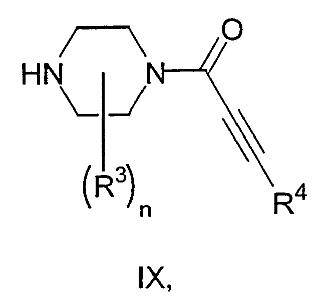 worin R³, R⁴ und n die vorstehend genannte Bedeutung haben, und diese ggf. gereinigt und/oder isoliert wird;
oder wenigstens eine Verbindung der allgemeinen Formel V, 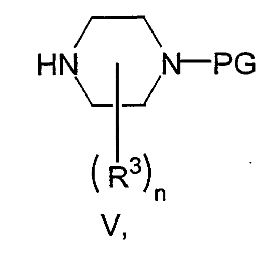 worin R³ und n die Bedeutung gemäß einem oder mehreren der Ansprüche 1 bis 12 haben und PG für eine Schutzgruppe, bevorzugt eine Schutzgruppe ausgewählt aus der Gruppe bestehend aus tert-Butyloxy-carbonyl, Benzyl, Benzyloxycarbonyl und 9-Fluorenylmethyloxycarbonyl, steht, durch Umsetzung mit wenigstens einer Verbindung der allgemeinen Formel R⁴-C≡C-C(=O)-OH, worin R⁴ die Bedeutung gemäß einem oder mehreren der Ansprüche 1 bis 12 hat, in einem Reaktionsmedium, ggf. in Gegenwart wenigstens eines geeigneten Kopplungsmittels, ggf. in Gegenwart wenigstens einer Base, vorzugsweise bei einer Temperatur von - 70 °C bis 100 °C, oder durch Umsetzung mit wenigstens einer Verbindung der allgemeinen Formel R⁴-C≡C-C(=O)-A, worin R⁴ die vorstehend genannte Bedeutung hat und A für eine Abgangsgruppe, vorzugsweise für einen Halogen-Rest, besonders bevorzugt für einen Chlor- oder Brom-Rest steht, in einem Reaktionsmedium, ggf. in Gegenwart wenigstens einer Base, vorzugsweise bei einer Temperatur von - 70 °C bis 100 °C, in wenigstens eine entsprechende Verbindung der allgemeinen Formel XI, ggf. in Form eines entsprechenden Salzes überführt wird, 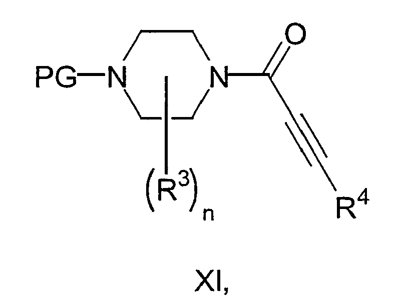 worin R³, R⁴, n und PG die vorstehend genannte Bedeutung haben, und diese ggf. gereinigt und/oder isoliert wird;
und wenigstens eine Verbindung der allgemeinen Formel XI für den Fall, dass PG für eine tert-Butoxycarbonyl- oder 9-Fluorenylmethyloxycarbonyl-Gruppe steht, in einem Reaktionsmedium, in Gegenwart wenigstens einer Säure, vorzugsweise in Gegenwart wenigstens einer Säure ausgewählt aus der Gruppe bestehend aus Salzsäure und Trifluoressigsäure, vorzugsweise bei einer Temperatur zwischen -70 °C bis 100 °C oder für den Fall, dass PG für eine Benzyl- oder Benzyloxycarbonyl-Gruppe steht, in einem Reaktionsmedium, in Gegenwart von Wasserstoff und in Gegenwart wenigstens eines Katalysators, vorzugsweise in Gegenwart von Palladium auf Kohle, vorzugsweise bei einer Temperatur zwischen -70 °C bis 100 °C in wenigstens eine entsprechende Verbindung der allgemeinen Formel IX, ggf. in Form eines entsprechenden Salzes überführt wird, worin R¹, R³ und n die vorstehend genannte Bedeutung haben, und diese ggf. gereinigt und/oder isoliert wird;
und wenigstens eine Verbindung der allgemeinen Formel IX durch Umsetzung mit wenigstens einer Verbindung der allgemeinen Formel II, 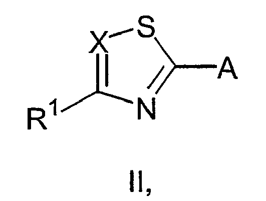 worin die Reste X und R¹ die Bedeutung gemäß einem oder mehreren der Ansprüche 1 bis 12 haben und A für eine Abgangsgruppe, vorzugsweise für einen Halogen-Rest oder einen Sulfonsäureester, besonders bevorzugt für einen Chlor- oder Brom-Rest, steht, in einem Reaktionsmedium, ggf. in Gegenwart wenigstens einer Base und/oder wenigstens einer metallorganischen Verbindung und/oder wenigstens eines Metallhydrid-Reagenzes, vorzugsweise bei einer Temperatur von - 70 °C bis 300 °C in wenigstens eine entsprechende Verbindung der allgemeinen Formel I, ggf. in Form eines entsprechenden Salzes überführt wird, und diese ggf. gereinigt und/oder isoliert wird.

15. Arzneimittel enthaltend wenigstens eine Verbindung gemäß einem oder mehreren der Ansprüche 1 bis 12 und ggf. einen oder mehrere physiologisch verträgliche Hilfsstoffe.

16. Arzneimittel gemäß Anspruch 15 zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Erkrankungen, vorzugsweise kognitiven Mangelzuständen, besonders bevorzugt des Aufmerksamkeit-Defizit-Syndroms (ADS); Angstzuständen; Panikattacken; Epilepsie; Husten; Harninkontinenz; Diarrhöe; Pruritus; Schizophrenie; cerebralen Ischämien; Muskelspasmen; Krämpfen; Störungen der Nahrungsaufnahme, vorzugsweise ausgewählt aus der Gruppe bestehend aus Bulimie, Kachexie, Anorexie und Fettleibigkeit; Alkoholabhängigkeit; Medikamentenabhängigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen- (insbesondere Nikotin- und/oder Kokain-) abhängigkeit; Toleranzentwicklung gegenüber Medikamenten, vorzugsweise gegenüber natürlichen oder synthetischen Opioiden; Magen-Ösaphagus-Reflux-Syndrom; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie.

17. Verwendung wenigstens einer Verbindung gemäß einem oder mehreren der Ansprüche 1 bis 12 zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Erkrankungen, vorzugsweise kognitiven Mangelzuständen, besonders bevorzugt des Aufmerksamkeit-Defizit-Syndroms (ADS): Angstzuständen; Panikattacken; Epilepsie; Husten; Harninkontinenz; Diarrhöe; Pruritus; Schizophrenie; cerebralen Ischämien; Muskelspasmen; Krämpfen; Störungen der Nahrungsaufnahme, vorzugsweise ausgewählt aus der Gruppe bestehend aus Bulimie, Kachexie, Anorexie und Fettleibigkeit; Alkoholabhängigkeit; Medikamentenabhängigkeit; Drogenabhängigkeit, vorzugsweise Nikotin- und/oder Kokainabhängigkeit; Alkoholmißbrauch, Medikamentenmißbrauch; Drogenmißbrauch; vorzugsweise Nikotin- und/oder Kokainmißbrauch; Entzugserscheinungen bei Alkohol-, Medikamenten- und/oder Drogen- (insbesondere Nikotin- und/oder Kokain-) abhängigkeit; Toleranzentwicklung gegenüber Medikamenten, insbesondere gegenüber natürlichen oder synthetischen Opioiden; Magen-Ösaphagus-Reflux-Syndrom; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie.

18. Verwendung gemäß Anspruch 17 zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz; Depressionen; Morbus Parkinson; Angstzuständen; Panikattacken; Epilepsie; Alkoholabhängigkeit; Medikamentenabhängigkeit; Störungen der Nahrungsaufnahme, vorzugsweise ausgewählt aus der Gruppe bestehend aus Bulimie, Kachexie, Anorexie und Fettleibigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen- (insbesondere Nikotin- und/oder Kokain-) abhängigkeit; Toleranzentwicklung gegenüber Medikamenten und/oder Drogen, insbesondere gegenüber natürlichen oder synthetischen Opioiden oder zur Lokalanästhesie.

19. Verwendung gemäß Anspruch 17 oder 18 zur Herstellung eines Arzneimittels zur Behandlung von Schmerz, vorzugsweise von Schmerz ausgewählt aus der Gruppe bestehend aus akutem Schmerz, chronischem Schmerz, neuropathischem Schmerz und visceralem Schmerz, Angstzuständen und Panickattacken.

## Claims

1. Substituted 1-propiolylpiperazines of the general formula I, 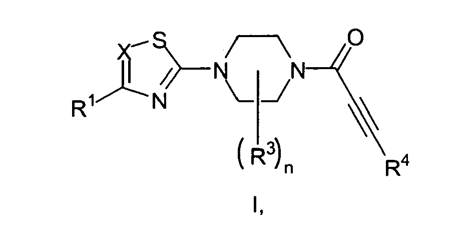 wherein
X stands for N or C-R ²
R¹ and R², independently of one another, stand for a hydrogen radical, a halogen radical, a nitro group, a cyano group, an amino group, a hydroxyl group, a thiol group, a carboxyl group, a formyl group, an -NH-C(=O)-H group, an -NH-R⁵ group, an-NR⁶ R⁷ group, a -C(=O)-R⁸ group, a -C(=O)-O-R⁹ group, an -O-C(=O)-R¹⁰ group, an-NH-C(=O)-R¹¹group, an -NR¹²-C(=O)-R¹³ group, a -C(=O)-NH₂ group, a -C(=O)-NH-R¹⁴ group, a -C(=O)-NR¹⁵R¹⁶ group, an -O-R¹⁷ group, an -S-R¹⁸ group, an-S(=O)-R¹⁹ group, an -S(=O)₂-R²⁰ group, an -NH-C(=O)-NH-R²¹ group, an -NH-C(=S)-NH-R²² group, an -NH-S(=O)₂-R²³ group, an -NR²⁴-S(=O)₂-R²⁵ group, for a linear or branched, saturated or unsaturated, unsubstituted or at least mono-substituted aliphatic radical which possibly contains at least one heteroatom as chain member, or for an unsubstituted or at least mono-substituted, unsaturated or saturated cycloaliphatic radical which possibly contains at least one heteroatom as ring member and which can be bonded via a linear or branched, unsubstituted or at least mono-substituted alkylene group, alkenylene group or alkinylene group possibly containing at least one heteroatom as chain member and/or possibly condensed with an unsubstituted or at least mono-substituted monocyclic or polycyclic ring system, or for an unsubstituted or at least mono-substituted aryl or heteroaryl radical which can be bonded via a linear or branched, unsubstituted or at least mono-substituted alkylene group, alkenylene group or alkinylene group possibly containing at least one heteroatom as chain member and/or possibly condensed with an unsubstituted or at least mono-substituted monocyclic or polycyclic ring system;
R³ stands for a halogen radical, a nitro group, a cyano group, an amino group, a hydroxyl group, a thiol group, a carboxyl group, a formyl group, an -NH-C(=O)-H group, an oxo group (=O), an -NH-R⁵ group, an -NR⁶R⁷ group, a -C(=O)-R⁸ group, a-C(=O)-O-R⁹ group, an -O-C(=O)-R¹⁰ group, an -NH-C(=O)-R¹¹group, an -NR¹² C(=O)-R¹³ group, a -C(=O)-NH₂ group, a -C(=O)-NH-R¹⁴ group, a -C(=O)-NR¹⁵R¹⁶ group, an -O-R¹⁷ group, an -S-R¹⁸ group, an -S(=O)-R¹⁹ group, an -S(=O)₂-R²⁰ group, an -NH-C(=O)-NH-R²¹ group, an -NH-C(=S)-NH-R²² group, an -NH-S(=O)₂-R²³ group, an -NR²⁴-S(=O)₂-R²⁵ group, for a linear or branched, saturated or unsaturated, unsubstituted or at least mono-substituted aliphatic radical which possibly contains at least one heteroatom as chain member, for an unsubstituted or at least mono-substituted, unsaturated or saturated cycloaliphatic radical which possibly contains at least one heteroatom as ring member and which can be bonded via a linear or branched, unsubstituted or at least mono-substituted alkylene group, alkenylene group or alkinylene group possibly containing at least one heteroatom as chain member and/or possibly condensed with an unsubstituted or at least mono-substituted monocyclic or polycyclic ring system, or for an unsubstituted or at least mono-substituted aryl or heteroaryl radical which can be bonded via a linear or branched, unsubstituted or at least mono-substituted alkylene group, alkenylene group or alkinylene group possibly containing at least one heteroatom as chain member and/or possibly condensed with an unsubstituted or at least mono-substituted monocyclic or polycyclic ring system;
R⁴ stands for a hydrogen radical, a fluorine atom, a chlorine atom, a bromine atom, a nitro group, a cyano group, an amino group, a hydroxyl group, a thiol group, a carboxyl group, a formyl group, an -NH-C(=O)-H group, an -NH-R⁵ group, an -NR⁶R⁷ group, a -C(=O)-R⁸ group, a -C(=O)-O-R⁹ group, an -O-C(=O)-R¹⁰ group, an -NH-C(=O)-R¹¹group, an -NR¹²-C(=O)-R¹³ group, a -C(=O)-NH₂ group, a -C(=O)-NH-R¹⁴ group, a -C(=O)-NR¹⁵R¹⁶ group, an -O-R¹⁷ group, an -S-R¹⁸ group, an -S(=O)-R¹⁹ group, an -S(=O)₂-R²⁰ group, an -NH-C(=O)-NH-R²¹ group, an -NH-C(=S)-NH-R²² group, an -NH-S(=O)₂-R²³ group, an -NR²⁴-S(=O)₂-R²⁵ group, a linear or branched, saturated or unsaturated, unsubstituted or at least mono-substituted aliphatic radical which possibly contains at least one heteroatom as chain member, for an unsubstituted or at least mono-substituted, unsaturated or saturated cycloaliphatic radical which possibly contains at least one heteroatom as ring member and which can be bonded via a linear or branched, unsubstituted or at least mono-substituted alkylene group, alkenylene group or alkinylene group possibly containing at least one heteroatom as chain member and/or possibly condensed with an unsubstituted or at least mono-substituted monocyclic or polycyclic ring system, or for an unsubstituted or at least mono-substituted aryl or heteroaryl radical which can be bonded via a linear or branched, unsubstituted or at least mono-substituted alkylene group, alkenylene group or alkinylene group possibly containing at least one heteroatom as chain member and/or possibly condensed with an unsubstituted or at least mono-substituted monocyclic or polycyclic ring system;
R⁵ to R²⁵, independently of one another, stand for a linear or branched, saturated or unsaturated, unsubstituted or at least mono-substituted aliphatic radical which possibly contains at least one heteroatom as chain member, for an unsubstituted or at least mono-substituted, unsaturated or saturated cycloaliphatic radical which possibly contains at least one heteroatom as ring member and which can be bonded via a linear or branched, unsubstituted or at least mono-substituted alkylene group, alkenylene group or alkinylene group possibly containing at least one heteroatom as chain member and/or possibly condensed with an unsubstituted or at least mono-substituted monocyclic or polycyclic ring system, or for an unsubstituted or at least mono-substituted aryl or heteroaryl radical which can be bonded via a linear or branched, unsubstituted or at least mono-substituted alkylene group, alkenylene group or alkinylene group possibly containing at least one heteroatom as chain member,
and n is equal to 0, 1, 2, 3, 4, 5, 6, 7, or 8;
in each case possibly in the form of one of the pure stereoisomers thereof, particularly enantiomers or diastereomers thereof, the racemates thereof or in the form of a mixture of stereoisomers and particularly of the enantiomers and/or diastereomers in any desired mixture ratio, or in each case in the form of appropriate salts, preferably in the form of corresponding hydrochlorides, or in each case in the form of appropriate solvates.

2. Compounds as defined in claim 1, **characterised in that**
X stands for N or C-R²;
n is equal to 0, 1, 2, 3, 4, S, 6, 7, or 8;
R¹ and R², independently of one another, stand for a hydrogen radical, a halogen radical, a nitro group, a cyano group, an amino group, a hydroxyl group, a thiol group, a carboxyl group, a formyl group, an -NH-C(=O)-H group, an -NH-R⁵ group, an-NR⁶R⁷ group, a -C(=O)-R⁸ group, a -C(=O)-O-R⁹ group, an -O-C(=O)-R¹⁰ group, an-NH-C(=O)-R¹¹ group, an -NR¹²-C(=O)-R¹³ group, a -C(=O)-NH₂ group, a -C(=O)-NH-R¹⁴ group, a -C(=O)-NR¹⁵R¹⁶ group, an -O-R¹⁷ group, an -S-R¹⁸ group, an-S(=O)-R¹⁹ group, an -S(=O)₂-R²⁰ group, an -NH-C(=O)-NH-R²¹ group, an -NH-C(=S)-NH-R²² group, an -NH-S(=O)₂-R²³ group, an -NR²⁴-S(=O)₂-R²⁵ group, for a linear or branched, saturated or unsaturated, unsubstituted or at least mono-substituted aliphatic C₁₋₈ radical which possibly contains 1, 2, or 3 heteroatoms selected independently of one another from the group comprising oxygen, sulphur and nitrogen, as chain member(s), for an unsubstituted or at least mono-substituted, unsaturated or saturated three-, four-, five-, six-, seven-, eight- or nine-membered cycloaliphatic radical possibly containing at least one heteroatom as ring member and which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or possibly condensed with an unsubstituted or at least mono-substituted monocyclic or polycyclic ring system, or for an unsubstituted or at least mono-substituted five- to fourteen-membered aryl or heteroaryl radical, which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or can be condensed with an unsubstituted or at least mono-substituted monocyclic or polycyclic ring system;
R³ stands for a halogen radical, a nitro group, a cyano group, an amino group, a hydroxyl group, a thiol group, or for a carboxyl group, a formyl group, an -NH-C(=O)-H group, an oxo group (=O), an -NH-R⁵ group, an -NR⁶R⁷ group, a -C(=O)-R⁸ group, a -C(=O)-O-R⁹ group, an -O-C(=O)-R¹⁰ group, an -NH-C(=O)-R¹¹ group,-NR¹²-C(=O)-R¹³ group, a -C(=O)-NH₂ group, a -C(=O)-NH-R¹⁴ group, a -C(=O)-NR¹⁵R¹⁶ group, an -O-R¹⁷ group, an -S-R¹⁸ group, an -S(=O)-R¹⁹ group, an -S(=O)₂-R²⁰ group, an -NH-C(=O)-NH-R²¹ group, an -NH-C(=S)-NH-R²² group, an -NH-S(=O)₂-R²³ group, an -NR²⁴-S(=O)2-R²⁵ group, a linear or branched, saturated or unsaturated, unsubstituted or at least mono-substituted aliphatic C₁₋₈ radical which possibly contains 1, 2, or 3 heteroatoms selected independently of one another from the group comprising oxygen, sulphur and nitrogen, as chain member(s), for an unsubstituted or at least mono-substituted, unsaturated or saturated three-, four-, five-, six-, seven-, eight- or nine-membered cycloaliphatic radical possibly containing at least one heteroatom as ring member and which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or possibly condensed with an unsubstituted or at least mono-substituted monocyclic or polycyclic ring system, or for an unsubstituted or at least mono-substituted five- to fourteen-membered aryl or heteroaryl radical, which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or can be condensed with an unsubstituted or at least mono-substituted monocyclic or polycyclic ring system;
R⁴ stands for a hydrogen radical, a -C(=O)-NH₂ group, a -C(=O)-NH-R¹⁴ group, a-C(=O)-NR¹⁵R¹⁶ group, a linear or branched, saturated or unsaturated, unsubstituted or at least mono-substituted aliphatic C₁₋₈ radical, for an unsubstituted or at least mono-substituted, unsaturated or saturated three-, four-, five-, six-, seven-, eight- or nine-membered cycloaliphatic radical which possibly contains at least one heteroatom as ring member and which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or possibly condensed with an unsubstituted or at least mono-substituted monocyclic or polycyclic ring system, or for an unsubstituted or at least mono-substituted five- to fourteen-membered aryl or heteroaryl radical, which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or can be condensed with an unsubstituted or at least mono-substituted monocyclic or polycyclic ring system;
and
R⁵ to R²⁵ independently respectively stand for a linear or branched, saturated or unsaturated, unsubstituted or at least mono-substituted aliphatic C₁₋₈ radical, for an unsubstituted or at least mono-substituted, unsaturated or saturated three-, four-, five-, six-, seven-, eight- or nine-membered cycloaliphatic radical which possibly contains at least one heteroatom as ring member and which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or possibly condensed with an unsubstituted or at least mono-substituted monocyclic or polycyclic ring system, or for an unsubstituted or at least mono-substituted five- to fourteen-membered aryl or heteroaryl radical, which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or can be condensed with an unsubstituted or at least mono-substituted monocyclic or polycyclic ring system;
wherein
the aforementioned cycloaliphatic radicals can possibly exhibit 1, 2, 3, 4, or 5 heteroatom(s) as ring member(s), which can be selected independently of one another from the group comprising nitrogen, oxygen and sulphur;
the rings of the aforementioned monocyclic or bicyclic ring systems are each four-, five- or six-membered and each has possibly 0, 1, 2, 3, 4, or 5 heteroatom(s) as ring member(s), which are selected independently of one another from the group comprising oxygen, nitrogen and sulphur;
and the aforementioned heteroaryl radicals can possibly have 1, 2, 3, 4, or 5 heteroatom(s) as ring member(s), which can be selected independently of one another from the group comprising oxygen, sulphur and nitrogen;
in each case possibly in the form of one of the pure stereoisomers thereof, particularly enantiomers or diastereomers thereof, the racemates thereof or in the form of a mixture of stereoisomers and particularly of the enantiomers and/or diastereomers in any desired mixture ratio, or in each case in the form of appropriate salts, preferably in the form of corresponding hydrochlorides, or in each case in the form of appropriate solvates.

3. Compounds as defined in claim 1 or claim 2, **characterised in that**
R¹ stands for a hydrogen radical, a halogen radical, a nitro group, a cyano group, an amino group, a hydroxyl group, a thiol group, a carboxyl group, a formyl group, an-NH-C(=O)-H group, an -NH-R⁵ group, an -NR⁶R⁷ group, a -C(=O)-R⁸ group, a-C(=O)-O-R⁹ group, an -O-C(=O)-R¹⁰ group, an -NH-C(=O)-R¹¹group, an -NR¹²-C(=O)-R¹³ group, a -C(=O)-NH₂ group, a -C(=O)-NH-R¹⁴ group, a -C(=O)-NR¹⁵R¹⁶ group, an -O-R¹⁷ group, an -S-R¹⁸ group, an -S(=O)-R¹⁹ group, an -S(=O)₂-R²⁰ group, an -NH-C(=O)-NH-R²¹ group, an -NH-C(=S)-NH-R²² group, an -NH-S(=O)₂-R²³ group, an -NR²⁴-S(=O)₂-R²⁵ group, for a linear or branched, saturated or unsaturated, unsubstituted or at least mono-substituted aliphatic C₁₋₈ radical which possibly contains 1, 2, or 3 heteroatoms selected independently of one another from the group comprising oxygen, sulphur and nitrogen, as chain member(s), for an unsubstituted or at least mono-substituted, unsaturated or saturated three-, four-, five-, six-, seven-, eight- or nine-membered cycloaliphatic radical possibly containing at least one heteroatom as ring member and which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or possibly condensed with an unsubstituted or at least mono-substituted monocyclic or polycyclic ring system, or for an unsubstituted or at least mono-substituted five- to fourteen-membered aryl or heteroaryl radical, which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or can be condensed with an unsubstituted or at least mono-substituted monocyclic or polycyclic ring system,
preferably stands for a hydrogen radical, a halogen radical selected from the group comprising F, Cl, Br and I, a nitro group, a CF₃ group, a cyano group, an amino group, a hydroxyl group, a thiol group, a carboxyl group, an -NH-R⁵ group, an -NR⁶R⁷group, a -C(=O)-R⁸ group, a -C(=O)-O-R⁹ group, an -O-C(=O)-R¹⁰ group, a -C(=O)-NH₂ group, a -C(=O)-NH-R¹⁴ group, a -C(=O)-NR¹⁵R¹⁶ group, an -O-R¹⁷ group, an -S-R¹⁸ group, an -S(=O)-R¹⁹ group, an -S(=O)₂-R²⁰ group, for a linear or branched C₁₋₈ alkyl radical, or for a linear or branched C₂₋₈ alkenyl radical, for a linear or branched C₂₋₈ alkinyl radical, stands for an unsubstituted or at least mono-substituted unsaturated or three-, four-, five-, six- or seven-membered saturated cycloaliphatic radical which possibly contains 1, 2, or 3 heteroatoms selected independently of one another from the group comprising oxygen, sulphur and nitrogen, as chain member(s) and which can be bonded via a linear or branched, unsubstituted or at least mono-substituted C₁₋₅ alkylene group, C₂₋₅ alkenylene group or C₂₋₅ alkinylene group or for an unsubstituted or at least mono-substituted five- to fourteen-membered aryl or heteroaryl radical, which can be bonded via a linear or branched, unsubstituted or at least mono-substituted C₁₋₅ alkylene group, C₂₋₅ alkenylene group or C₂₋₅ alkinylene group;
particularly preferred stands for a hydrogen radical, a halogen radical selected from the group comprising F, Cl and Br, a CF₃ group, a nitro group, a cyano group, an amino group, a hydroxyl group, a thiol group, for a carboxyl group, an -NH-R⁵ group, an NR⁶R⁷ group, a -C(=O)-R group, a -C(=O)-O-R⁹ group, an -O-C(=O)-R¹⁰ group, a -C(=O)-NH-R¹⁴ group, a -C(=O)-NR¹⁵R¹⁶ group, an -O-R¹⁷ group, an -S-R¹⁸ group, an -S(=O)-R¹⁹ group, an -S(=O)₂-R²⁰ group, a linear or branched C₁₋₄ alkyl radical, a linear or branched C₂₋₄ alkenyl radical, for a linear or branched C₂₋₄ alkinyl radical, or for a radical selected from the group comprising cyclopropyl, cyclobutyl, cyclopentyl, cyclohexyl, pyrrolidinyl, tetrahydrofuranyl, tetrahydrothiophenyl, piperidinyl, tetrahydropyranyl, piperazinyl, morpholinyl, thiomorpholinyl, dioxolanyl, azepanyl, diazepanyl, imidazolidinyl, phenyl, naphthyl, anthracenyl, pyrrolyl, indolyl, furanyl, benzo[b]furanyl, thiophenyl, benzo[b]thiophenyl, pyrazolyl, imidazolyl, thiazolyl, dithiazolyl, thiadiazolyl, triazolyl, oxazolyl, oxadiazolyl, isoxazolyl, pyridinyl, pyridazinyl, pyrimidinyl, pyrazinyl, pyranyl, indazolyl, purinyl, indolizinyl group, quinolinyl, isoquinolinyl and quinazolinyl radical, each of which can be bonded via a C₁₋₃ alkylene group, C₂₋₃ alkenylene group or C₂₋₃ alkinylene group and/or is unsubstituted or substituted with possibly 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl, -OH, -O-CH₃, -O-C₂H₅, -O-C₃H₇, -NH₂, -N(CH₃)₂, -N(C₂H₅)₂, -NH-CH₃, -NH-C₂H₅, -NO₂, -CF₃, -O-CF₃, -S-CF₃, -SH, -NH-S(=O)₂-CH₃, -C(=O)-OH, -C(=O)-CH₃, -C(=O)-C₂H₅, - C(=O)-N(CH₃)₂, -C(=O)-NH-CH₃, -NH-C(=O)-CH₃, -NH-C(=O)-C₂H₅, -C(=O)-O-CH₃ and -C(=O)-O-C₂H₅;
most preferred stands for a hydrogen radical, a halogen radical selected from the group comprising F, Cl and Br, a CF₃ group, a nitro group, a cyano group, an amino group, a dimethylamino group, a diethylamino group, a monomethylamino group, a monoethylamino group, a hydroxyl group, a thiol group, a carboxyl group, a -C(=O)-O-CH₃ group, a -C(=O)-O-C₂H₅ group, a -C(=O)-NH-R¹⁴ group, a -C(=O)-NR¹⁵R¹⁶ group, anO-R¹⁷ group, an -S-R¹⁸ group, an -S(=O)-R¹⁹ group, an -S(=O)₂-R²⁰ group, an alkyl radical selected from the group comprising methyl, ethyl, n-propyl, isopropyl, n-butyl, sec-butyl, isobutyl and tert-butyl, or for an alkenyl radical selected from the group comprising ethenyl, 1-propenyl, 2-propenyl, 1-butenyl, 2-butenyl and 3-butenyl, or for an alkinyl radical selected from the group comprising ethinyl, 1-propinyl, 2-propinyl, 1-butinyl, 2-butinyl and 3-butinyl or for a radical selected from the group comprising (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl, each of which is unsubstituted or substituted with possibly 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl, -OH, -O-CH₃, -O-C₂H₅ and -O-C₃H₇.

4. Compounds as defined in one or more of claims 1 to 3, **characterised in that** R² stands for a hydrogen radical, a halogen radical, a nitro group, a cyano group, an amino group, a hydroxyl group, a thiol group, a carboxyl group, a formyl group, an -NH-C(=O)-H group, an -NH-R⁵ group, an -NR⁶R group, a -C(=O)-R⁸ group, a -C(=O)-OR⁹ group, an -O-C(=O)-R¹⁰ group, an -NH-C(=O)-R¹¹ group, an -NR¹²-C(=O)-R¹³ group, a -C(=O)-NH₂ group, a -C(=O)-NH-R¹⁴ group, a -C(=O)-NR¹⁵R¹⁶ group, an-O-R¹⁷ group, an -S-R¹⁸ group, an -S(=O)-R¹⁹ group, an -S(=O)₂-R²⁰ group, an -NH-C(=O)-NH-R²¹ group, an -NH-C(=S)-NH-R²² group, an -NH-S(=O)₂-R²³ group, an-NR²⁴-S(=O)₂-R²⁵ group, for a linear or branched, saturated or unsaturated, unsubstituted or at least mono-substituted aliphatic C₁₋₈ radical which possibly contains 1, 2, or 3 heteroatoms selected independently of one another from the group comprising oxygen, sulphur and nitrogen, as chain member(s), for an unsubstituted or at least mono-substituted, unsaturated or saturated three-, four-, five-, six-, seven-, eight- or nine-membered cycloaliphatic radical possibly containing at least one heteroatom as ring member and which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or possibly condensed with an unsubstituted or at least mono-substituted monocyclic or polycyclic ring system, or for an unsubstituted or at least mono-substituted five- to fourteen-membered aryl or heteroaryl radical, which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or can be condensed with an unsubstituted or at least mono-substituted monocyclic or polycyclic ring system;
preferably stands for a hydrogen radical, a halogen radical selected from the group comprising F, Cl, Br and I, a nitro group, a CF₃ group, a cyano group, an amino group, a hydroxyl group, a thiol group, a carboxyl group, an -NH-R⁵ group, an -NR⁶R⁷ group, a -C(=O)-R⁸ group, a -C(=O)-O-R⁹ group, an -O-C(=O)-R¹⁰ group, a -C(=O)-NH₂ group, a -C(=O)-NH-R¹⁴ group, a -C(=O)-NR¹⁵SR¹⁶ group, an -O-R¹⁷ group, an -S-R¹⁸ group, an -S(=O)-R¹⁹ group, an -S(=O)₂-R²⁰ group, a linear or branched C₁₋₈ alkyl radical, a linear or branched C₂₋₈ alkenyl radical, for a linear or branched C₂₋₈ alkinyl radical, for an unsubstituted or at least mono-substituted unsaturated or saturated three-, four-, five-, six- or seven-membered cycloaliphatic radical which possibly contains 1, 2, or 3 heteroatoms selected independently of one another from the group comprising oxygen, sulphur and nitrogen, as ring member(s) and which can be bonded via a linear or branched, unsubstituted or at least mono-substituted C₁₋₅ alkylene group, C₂₋₅ alkenylene group or C₂₋₅ alkinylene group or for an unsubstituted or at least mono-substituted five- to fourteen-membered aryl or heteroaryl radical, which can be bonded via a linear or branched, unsubstituted or at least mono-substituted C₁₋₅ alkylene group, C₂₋₅ alkenylene group or C₂₋₅ alkinylene group;
particularly preferred stands for a hydrogen radical, a halogen radical selected from the group comprising F, Cl and Br, a CF₃ group, a nitro group, a cyano group, an amino group, a hydroxyl group, a thiol group, a carboxyl group, an -NH-R⁵ group, an-NR⁶R⁷ group, a -C(=O)-R⁸ group, a -C(=O)-O-R⁹ group, an -O-C(=O)-R¹⁰ group, a-C(=O)-NH-R¹⁴ group, a -C(=O)-NR¹⁵R¹⁶ group, an -S(=O)-R¹⁹ group, an -S(=O)₂-R²⁰ group, an -O-R¹⁷ group, an -S-R¹⁸ group, a linear or branched C₁₋₄ alkyl radical, a linear or branched C₂₋₄ alkenyl radical, a linear or branched C₂₋₄ alkinyl radical, a radical selected from the group comprising cyclopropyl, cyclobutyl, cyclopentyl, cyclohexyl, pyrrolidinyl, tetrahydrofuranyl, tetrahydrothiophenyl, piperidinyl, tetrahydropyranyl, piperazinyl, morpholinyl, thiomorpholinyl, dioxolanyl, azepanyl, diazepanyl, imidazolidinyl, phenyl, naphthyl, anthracenyl, pyrrolyl, indolyl, furanyl, benzo[b]furanyl, thiophenyl, benzo[b]thiophenyl, pyrazolyl, imidazolyl, thiazolyl, dithiazolyl, thiadiazolyl, triazolyl, oxazolyl, oxadiazolyl, isoxazolyl, pyridinyl, pyridazinyl, pyrimidinyl, pyrazinyl, pyranyl, indazolyl, purinyl, indolizinyl group, quinolinyl, isoquinolinyl and quinazolinyl radical, each of which can be bonded via a C₁₋₃ alkylene group, C₂₋₃ alkenylene group or C₂₋₃ alkinylene group and/or is unsubstituted or substituted with possibly 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl, -OH, -O-CH₃, -O-C₂H₅, -O-C₃H₇, -NH₂, -N(CH₃)₂, -N(C₂H₅)₂, -NH-CH₃, -NH-C₂H₅, -NOz, -CF₃, -O-CF₃, -S-CF₃, -SH, -NH-S(=O)₂-CH₃, -C(=O)-OH, -C(=O)-CH₃, -C(=O)-C₂H₅,-C(=O)-N(CH₃)₂, -C(=O)-NH-CH₃, -NH-C(=O)-CH₃, -NH-C(=O)-C₂H₅, -C(=O)-O-CH₃ and -C(=O)-O-C₂H₅;
most preferred stands for a hydrogen radical, a halogen radical selected from the group comprising F, Cl and Br, a CF₃ group, a nitro group, a cyano group, an amino group, a dimethylamino group, a diethylamino group, a monomethylamino group, a monoethylamino group, a hydroxyl group, a thiol group, a carboxyl group, a -C(=O)-O-CH₃ group, a -C(=O)-O-C₂H₅ group, a -C(=O)-NH-R¹⁴ group, a -C(=O)-NR¹⁵R¹⁶ group, an -O-R¹⁷ group, an -S-R¹⁸ group, an -S(=O)-R¹⁹ group, an -S(=O)₂-R²⁰ group, an alkyl radical selected from the group comprising methyl, ethyl, n-propyl, isopropyl, n-butyl, sec-butyl, isobutyl and tert-butyl, or for an alkenyl radical selected from the group comprising ethenyl, 1-propenyl, 2-propenyl, 1-butenyl, 2-butenyl and 3-butenyl, or for an alkinyl radical selected from the group comprising ethinyl, 1-propinyl, 2-propinyl, 1-butinyl, 2-butinyl and 3-butinyl or for a radical selected from the group comprising (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl, each of which is unsubstituted or substituted with possibly 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl, -OH, -O-CH₃, -O-C₂H₅ and -O-C₃H₇.

5. Compounds as defined in one or more of claims I to 4, **characterised in that** R³ stands for a halogen radical, a nitro group, a cyano group, an amino group, a hydroxyl group, a thiol group, a carboxyl group, a formyl group, an -NH-C(=O)-H group, an oxo group (=O), an -NH-R⁵ group, an -NR⁶R⁷ group, a -C(=O)-R⁸ group, a -C(=O)-O-R⁹ group, an -O-C(=O)-R¹⁰ group, an -NH-C(=O)-R¹¹ group, an -NR¹²-C(=O)-R¹³ group, a-C(=O)-NH₂ group, a -C(=O)-NH-R¹⁴ group, a -C(=O)-NR¹⁵R¹⁶ group, an -O-R¹⁷ group, an -S-R¹⁸ group, an -S(=O)-R¹⁹ group, an -S(=O)₂-R²⁰ group, an -NH-C(=O)-NH-R²¹ group, an -NH-C(=S)-NH-R²² group, an -NH-S(=O)₂-R²³ group, an -NR²⁴-S(=O)₂-R²⁵ group, for a linear or branched, saturated or unsaturated, unsubstituted or at least mono-substituted aliphatic C₁₋₈ radical which possibly contains 1, 2, or 3 heteroatoms selected independently of one another from the group comprising oxygen, sulphur and nitrogen, as chain member(s), for an unsubstituted or at least mono-substituted, unsaturated or saturated three-, four-, five-, six-, seven-, eight- or nine-membered cycloaliphatic radical possibly containing at least one heteroatom as ring member and which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or possibly condensed with an unsubstituted or at least mono-substituted monocyclic or polycyclic ring system, or for an unsubstituted or at least mono-substituted five- to fourteen-membered aryl or heteroaryl radical, which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or can be condensed with an unsubstituted or at least mono-substituted monocyclic or polycyclic ring system;
preferably stands for a halogen radical selected from the group comprising F, Cl and Br, a nitro group, a cyano group, an amino group, a hydroxyl group, a thiol group, a carboxyl group, an oxo group (=O), a linear or branched C₁₋₄ alkyl radical, a radical selected from the group comprising cyclopropyl, cyclobutyl, cyclopentyl, cyclohexyl, pyrrolidinyl, tetrahydrofuranyl, tetrahydrothiophenyl, piperidinyl, tetrahydropyranyl, piperazinyl, morpholinyl, thiomorpholinyl, dioxolanyl, azepanyl, diazepanyl, imidazolidinyl, phenyl, naphthyl, anthracenyl, pyrrolyl, indolyl, furanyl, benzo[b]furanyl, thiophenyl, benzo[b]thiophenyl, pyrazolyl, imidazolyl, thiazolyl, dithiazolyl, thiadiazolyl, triazolyl, oxazolyl, oxadiazolyl, isoxazolyl, pyridinyl, pyridazinyl, pyrimidinyl, pyrazinyl, pyranyl, indazolyl, purinyl, indolizinyl group, quinolinyl, isoquinolinyl and quinazolinyl radical, which is unsubstituted or substituted with possibly 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl, -OH, -O-CH₃, -O-C₂Hs and -O-C₃H₇; particularly preferred stands for an oxo group (=O), an alkyl radical selected from the group comprising methyl, ethyl, n-propyl, isopropyl, n-butyl, sec-butyl, isobutyl and tert-butyl or for a phenyl radical, which is unsubstituted or substituted with 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl, -OH, -O-CH₃, -O-C₂H₅ and -O-C₃H₇.

6. Compounds as defined in one or more of claims 1 to 5, **characterised in that** R⁴ stands for a hydrogen radical, a -C(=O)-NH₂ group, a -C(=O)-NH-R¹⁴ group, a -C(=O)-NR¹⁵R¹⁶ group, a linear or branched, saturated or unsaturated, unsubstituted or at least mono-substituted aliphatic C₁₋₈ radical, an unsubstituted or at least mono-substituted, unsaturated or saturated three-, four-, five-, six-, seven-, eight- or nine-membered cycloaliphatic radical which possibly contains at least one heteroatom as ring member and which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from said group comprising oxygen, sulphur and nitrogen, as chain member(s) and/or possibly condensed with an unsubstituted or at least mono-substituted monocyclic or polycyclic ring system, or for an unsubstituted or at least mono-substituted five- to fourteen-membered aryl or heteroaryl radical, which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or can be condensed with an unsubstituted or at least mono-substituted monocyclic or polycyclic ring system;
preferably stands for a hydrogen radical, a -C(=O)-NH₂ group, a -C(=O)-NH-R¹⁴ group, a -C(=O)-NR¹⁵R¹⁶ group, a linear or branched, unsubstituted or at least mono-substituted C₁₋₈ alkyl radical, a linear or branched, unsubstituted or at least mono-substituted C₂₋₈ alkenyl radical, a linear or branched, unsubstituted or at least mono-substituted C₂₋₈ alkinyl radical, an unsubstituted or at least mono-substituted unsaturated or saturated four-, five-, six- or seven-membered cycloaliphatic radical, which can be condensed with an unsaturated, saturated or aromatic, unsubstituted or at least mono-substituted monocyclic or bicyclic ring system and each of the rings can contain 1, 2, or 3 heteroatoms, which can be selected independently of one another from the group comprising oxygen, nitrogen and sulphur and each of the rings of the monocyclic or bicyclic ring system is four-, five- or six-membered, or for an unsubstituted or at least mono-substituted five-membered, or six-membered aryl or heteroaryl radical, which can be condensed with a saturated, unsaturated or aromatic, unsubstituted or at least mono-substituted monocyclic or bicyclic ring system and the heteroaryl radical and possibly one or both rings of the monocyclic or bicyclic ring system each have 1, 2, or 3 heteroatoms, which can be selected independently of one another from the group comprising oxygen, nitrogen and sulphur and each of the rings of the monocyclic or bicyclic ring system is four-, five- or six-membered;
more preferred for a hydrogen radical, a -C(=O)-NH₂ group, a -C(=O)-NH-R¹⁴ group, a -C(=O)-NR¹⁵R¹⁶ group, an alkyl radical selected from the group comprising methyl, ethyl, n-propyl, isopropyl, n-butyl, sec-butyl, isobutyl, tert-butyl, n-pentyl, 2-pentyl, 3-pentyl, neopentyl, n-hexyl, n-heptyl and n-octyl, or for a radical selected from the group comprising cyclopropyl, cyclobutyl, cyclopentyl, cyclohexyl, cycloheptyl, cyclopentenyl, cyclohexenyl, pyrrolidinyl, tetrahydrofuranyl, tetrahydrothiophenyl, piperidinyl, tetrahydropyranyl, piperazinyl, morpholinyl, thiomorpholinyl, dioxolanyl, azepanyl, diazepanyl, imidazolidinyl, phenyl, naphthyl, anthracenyl, furanyl, thiophenyl, pyrazolyl, pyrazinyl, pyrimidinyl, pyridinyl, pyrrolyl, oxazolyl, isoxazolyl, thiazolyl, dithiazolyl, oxadiazolyl, triazolyl, imidazolyl, indolyl, benzo[b]thiophenyl, benzo[b]furanyl, quinolinyl, isoquinolinyl and quinazolyl, each of which can be possibly substituted with 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl, ethenyl, allyl, ethinyl, propinyl,-C≡C-Si(CH₃)₃, -C≡C-Si(C₂H₅)₃, -CH₂-O-CH₃, -CH₂-O-C₂H₅, -OH, -O-CH₃, -O-C₂H₅, -O-C₃H₇, -S-CH₃, -S-C₂H₅, -S(=O)-CH₃, -S(=O)₂-CH₃, -S(=O)-C₂H₅, -S(=O)₂-C₂H₅,-NH₂, -N(CH₃)₂, -N(C₂Hs)₂, -NH-CH₃, -NH-C₂H₅, -NO₂, -CF₃, -CH₂F, -CHF₂, -O-CF₃, -S-CF₃, -SH, -NH-S(=O)₂-CH₃, -C(=O)-OH, -C(=O)-H; -C(=O)-CH₃, -C(=O)-C₂H₅,-C(=O)-NH₂, -C(=O)-N(CH₃)₂, -C(=O)-NH-CH₃, -NH-C(=O)-CH₃, -NH-C(=O)-C₂H₅, -C(=O)-O-CH₃, -C(=O)-O-C₂H₅, -C(=O)-O-C(CH₃)₃ and phenyl;
particularly preferred stands for a hydrogen radical, a -C(=O)-NH₂ group, a -C(=O)-NH-R¹⁴ group, a -C(=O)-NR¹⁵R¹⁶ group, for an alkyl radical selected from the group comprising methyl, ethyl, n-propyl, isopropyl, n-butyl, sec-butyl, isobutyl, tert-butyl, n-pentyl, sec-pentyl, neopentyl, n-hexyl, n-heptyl and n-octyl, or for a radical selected from the group comprising cyclopropyl, cyclobutyl, cyclopentyl, cyclohexyl, cycloheptyl, cyclopentenyl, cyclohexenyl, phenyl, pyrid-2-yl, pyrid-3-yl, pyrid-4-yl, pyrrol-2-yl, pyrrol-3-yl, thiophen-2-yl, thiophen-3-yl, 4-methylthiophen-2-yl, 3-methylthiophen-2-yl, 5-methylthiophen-2-yl, furan-2-yl, furan-3-yl, imidazol-2-yl, imidazol-4-yl, thiazol-2-yl, thiazol-4-yl, thiazol-5-yl, oxazol-2-yl, oxazol-4-yl, oxazol-5-yl, (1,2,4)-thiadiazol-5-yl, (1,2,4)-oxadiazol-5-yl and naphth-1-yl, naphth-2-yl, anthracen-1-yl_{;} anthracen-2-yl, anthracen-9-yl, indol-2-yl, indol-3-yl, indol-4-yl, indol-5-yl, indol-6-yl, indol-7-yl, 1-methylindol-2-yl, 1-methylindol-3-yl, 1-methylindol-4-yl, 1-methylindol-5-yl, 1-methylindol-6-yl, 1-methylindol-7-yl, quinolin-3-yl, quinolin-4-yl, quinolin-2-yl, quinolin-5-yl, quinolin-6-yl, quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl and isoquinolin-8-yl, 2-methylphenyl, 3-methylphenyl, 4-methylphenyl, 2-fluorophenyl, 3-fluorophenyl, 4-fluorophenyl, 2-cyanophenyl, 3-cyanophenyl, 4-cyanophenyl, 2-hydroxyphenyl, 3-hydroxyphenyl, 4-hydroxyphenyl, 2-aminophenyl, 3-aminophenyl, 4-aminophenyl, 2-dimethylaminophenyl, 3-dimethylaminophenyl, 4-dimethylaminophenyl, 2-methylaminophenyl, 3-methylaminophenyl, 4-methylaminophenyl, 2-acetylphenyl, 3-acetylphenyl, 4-acetylphenyl, 2-methylsulphinylphenyl, 3-methylsulphinylphenyl, 4-methylsulphinylphenyl, 2-methylsulphonylphenyl, 3-methylsulphonylphenyl, 4-methylsulphonylphenyl, 2-methoxyphenyl, 3-methoxyphenyl, 4-methoxyphenyl, 2-chlorophenyl, 3-chlorophenyl, 4-chlorophenyl, 2-ethoxyphenyl, 3-ethoxyphenyl, 4-ethoxyphenyl, 2-trifluoromethylphenyl and 3-trifluoromethylphenyl, 4-trifluoromethylphenyl, 2-difluoromethylphenyl, 3-difluoromethylphenyl, 4-difluoromethylphenyl, 2-fluoromethylphenyl, 3-fluoromethylphenyl, 4-fluoromethylphenyl, 2-nitrophenyl, 3-nitrophenyl, 4-nitrophenyl, 2-ethylphenyl, 3-ethylphenyl, 4-ethylphenyl, 2-propylphenyl, 3-propylphenyl, 4-propylphenyl, 2-isopropylphenyl, 3-isopropylphenyl, 4-isopropylphenyl, 2-tert-butylphenyl, 3-tert-butylphenyl, 4-tert-butylphenyl, 2-carboxyphenyl, 3-carboxyphenyl, 4-carboxyphenyl, 2-ethenylphenyl, 3-ethenylphenyl, 4-ethenylphenyl, 2-ethinylphenyl, 3-ethinylphenyl, 4-ethinylphenyl, 2-allylphenyl, 3-allylphenyl, 4-allylphenyl, 2-trimethylsilanylethinylphenyl, 3-trimethylsilanylethinylphenyl, 4-trimethylsilanylethinylphenyl, 2-formylphenyl, 3-formylphenyl, 4-formylphenyl, 2-acetaminophenyl, 3-acetaminophenyl, 4-acetaminophenyl, 2-dimethylaminocarbonylphenyl, 3-dimethylaminocarbonylphenyl, 4-dimethylaminocarbonylphenyl, 2-methoxymethylphenyl, 3-methoxymethylphenyl, 4-methoxymethylphenyl, 2-ethoxymethylphenyl and 3-ethoxymethylphenyl, 4-ethoxymethylphenyl, 2-aminocarbonylphenyl, 3-aminocarbonylphenyl, 4-aminocarbonylphenyl, 2-methylaminocarbonylphenyl, 3-methylaminocarbonylphenyl, 4-methylaminocarbonylphenyl, 2-carboxymethylesterphenyl, 3-carboxymethylesterphenyl, 4-carboxymethylesterphenyl, 2-carboxyethylesterphenyl, 3-carboxyethylesterphenyl, 4-carboxyethylesterphenyl, 2-carboxy-tert-butylesterphenyl, 3-carboxy-tert-butylesterphenyl, 4-carboxy-tert-butylesterphenyl, 2-methylmercaptophenyl, 3-methylmercaptophenyl, 4-methylmercaptophenyl, 2-ethylmercaptophenyl, 3-ethylmercaptophenyl, 4-ethylmercaptophenyl, 2-biphenyl, 3-biphenyl, 4-biphenyl, 2-bromophenyl, 3-bromophenyl, 4-bromophenyl, 2-iodophenyl, 3-iodophenyl, 4-iodophenyl, 2-trifluoromethoxyphenyl, 3-trifluoromethoxyphenyl and 4-trifluoromethoxyphenyl, 2-fluoro-3-trifluoromethylphenyl, 2-fluoro-4-methylphenyl, (2,3)-difluorophenyl, (2,3)-dimethylphenyl, (2,3)-dichlorophenyl, 3-fluoro-2-trifluoromethylphenyl, (2,4)-dichlorophenyl, (2,4)-difluorophenyl, 4-fluoro-2-trifluoromethylphenyl, (2,4)-dimethoxyphenyl, 2-chloro-4-fluorophenyl, 2-chloro-4-nitrophenyl, 2-chloro-4-methylphenyl, 2-chloro-5-trifluoromethylphenyl, 2-chloro-5-methoxyphenyl, 2-bromo-5-trifluoromethylphenyl, 2-bromo-5-methoxyphenyl, (2,4)-dibromophenyl, (2,4)-dimethylphenyl, 2-fluoro-4-trifluoromethylphenyl, (2,5)-difluorophenyl, 2-fluoro-5-trifluoromethylphenyl, 5-fluoro-2-trifluoromethylphenyl, 5-chloro-2-trifluoromethylphenyl, 5-bromo-2-trifluoromethylphenyl, (2,5)-dimethoxyphenyl, (2,5)-bis-trifluoromethylphenyl, (2,5)-dichlorophenyl, (2,5)-dibromophenyl, 2-methoxy-5-nitrophenyl, 2-fluoro-6-trifluoromethylphenyl, (2,6)-dimethoxyphenyl, (2,6)-dimethylphenyl, (2,6)-dichlorophenyl, 2-chloro-6-fluorophenyl, 2-bromo-6-chlorophenyl, 2-bromo-6-fluorophenyl, (2,6)-difluorophenyl, (2,6)-difluoro-3-methylphenyl, (2,6)-dibromophenyl, (2,6)-dichlorophenyl, 3-chloro-2-fluorophenyl, 3-chloro-5-methylphenyl, (3,4)-dichlorophenyl, (3,4)-dimethylphenyl, 3-methyl-4-methoxyphenyl, 4-chloro-3-nitrophenyl, (3,4)-dimethoxyphenyl, 4-fluoro-3-trifluoromethylphenyl, 3-fluoro-4-trifluoromethylphenyl, (3,4)-difluorophenyl, 3-cyano-4-fluorophenyl, 3-cyano-4-methylphenyl, 3-cyano-4-methoxyphenyl, 3-bromo-4-fluorophenyl, 3-bromo-4-methylphenyl, 3-bromo-4-methoxyphenyl, 4-chloro-2-fluorophenyl, 4-chloro-3-trifluoromethyl, 4-bromo-3-methylphenyl, 4-bromo-5-methylphenyl, 3-chloro-4-fluorophenyl, 4-fluoro-3-nitrophenyl, 4-bromo-3-nitrophenyl, (3,4)-dibromophenyl, 4-chloro-3-methylphenyl, 4-bromo-3-methylphenyl, 4-fluoro-3-methylphenyl, 3-fluoro-4-methylphenyl, 3-fluoro-5-methylphenyl, 2-fluoro-3-methylphenyl, 4-methyl-3-nitrophenyl, (3,5)-dimethoxyphenyl, (3,5)-dimethylphenyl, (3,5)-bis-trifluoromethylphenyl, (3,5)-difluorophenyl, (3,5)-dinitrophenyl, (3,5)-dichlorophenyl, 3-fluoro-5-trifluoromethylphenyl, 5-fluoro-3-trifluoromethylphenyl, (3,5)-dibromophenyl, 5-chloro-4-fluorophenyl, 5-chloro-4-fluorophenyl, 5-bromo-4-methylphenyl, (2,3,4)-trifluorophenyl, (2,3,4)-trichlorophenyl, (2,3,6)-trifluorophenyl, 5-chloro-2-methoxyphenyl, (2,3)-difluoro-4-methyl, (2,4,5)-trifluorophenyl, (2,4,5)-trichlorophenyl, (2,4)-dichloro-5-fluorophenyl, (2,4,6)-trichlorophenyl, (2,4,6)-trimethylphenyl, (2,4,6)-trifluorophenyl, (2,4,6)-trimethoxyphenyl, (3,4,5)-trimethoxyphenyl, (2,3,4,5)-tetrafluorophenyl, 4-methoxy-(2,3,6)-trimethylphenyl, 4-methoxy-(2,3,6)-trimethylphenyl, 4-chloro-2,5-dimethylphenyl, 2-chloro-6-fluoro-3-methylphenyl, 6-chloro-2-fluoro-3-methyl, (2,4,6)-trimethylphenyl, (2,3,4,5,6)-pentafluorophenyl, 3-methylpyrid-2-yl, 4-methylpyrid-2-yl, 5-methylpyrid-2-yl, 6-methylpyrid-2-yl, 2-methylpyrid-3-yl, 4-methylpyrid-3-yl, 5-methylpyrid-3-yl, 6-methylpyrid-3-yl, 2-methylpyrid-4-yl, 3-methylpyrid-4-yl, 3-fluoropyrid-2-yl, 4-fluoropyrid-2-yl, 5-fluoropyrid-2-yl, 6-fluoropyrid-2-yl, 3-chloropyrid-2-yl, 4-chloropyrid-2-yl, 5-chloropyrid-2-yl, 6-chloropyrid-2-yl, 3-trifluoromethylpyrid-2-yl, 4-trifluoromethylpyrid-2-yl, 5-trifluoromethylpyrid-2-yl, 6-trifluoromethylpyrid-2-yl, 3-methoxypyrid-2-yl, 4-methoxypyrid-2-yl, 5-methoxypyrid-2-yl, 6-methoxypyrid-2-yl, 4-methylthiazol-2-yl, 5-methylthiazol-2-yl, 4-trifluoromethylthiazol-2-yl, 5-trifluoromethylthiazol-2-yl, 4-chlorothiazol-2-yl, 5-chlorothiazol-2-yl, 4-bromothiazol-2-yl, 5-bromothiazol-2-yl, 4-fluorothiazol-2-yl, 5-fluorothiazol-2-yl, 4-cyanothiazol-2-yl, 5-cyanothiazol-2-yl, 4-methoxythiazol-2-yl, 5-methoxythiazol-2-yl and 4-methyloxazol-2-yl, 5-methyloxazol-2-yl, 4-trifluoromethyloxazol-2-yl, 5-trifluoromethyloxazol-2-yl, 4-chloroxazol-2-yl, 5-chloroxazol-2-yl, 4-bromoxazol-2-yl, 5-bromoxazol-2-yl, 4-fluoroxazol-2-yl, 5-fluoroxazol-2-yl, 4-cyano-oxazol-2-yl, 5-cyano-oxazol-2-yl, 4-methoxyoxazol-2-yl, 5-methoxyoxazol-2-yl, 2-methyl-(1,2,4)-thiadiazol-5-yl, 2-trifluoromethyl-(1,2,4)-thiadiazol-5-yl, 2-chloro-(1,2,4)-thiadiazol-5-yl, 2-fluoro-(1,2,4)-thiadiazol-5-yl, 2-methoxy-(1,2,4)-thiadiazol-5-yl, 2-cyano-(1,2,4)-thiadiazol-5-yl, 2-methyl-(1,2,4)-oxadiazol-5-yl, 2-trifluoromethyl-(1,2,4)-oxadiazol-5-yl, 2-chloro-(1,2,4)-oxadiazol-5-yl, 2-fluoro-(1,2,4)-oxadiazol-5-yl, 2-methoxy-(1,2,4)-oxadiazol-5-yl and 2-cyano-(1,2,4)-oxadiazol-5-yl.

7. Compounds as defined in one or more of the claims 1 to 6, **characterised in that** n is equal to 0, 1, 2, 3, or 4, preferably to 0, 1, or 2.

8. Compounds as defined in one or more of claims 1 to 7, **characterised in that**
R⁵ to R²⁵, independently of one another, stand for a linear or branched, saturated or unsaturated, unsubstituted or at least mono-substituted aliphatic C₁₋₈ radical, for an unsubstituted or at least mono-substituted, unsaturated or saturated three-, four-, five-, six-, seven-, eight- or nine-membered cycloaliphatic radical which possibly contains at least one heteroatom as ring member and which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from said group comprising oxygen, sulphur and nitrogen, as chain member(s) and/or possibly condensed with an unsubstituted or at least mono-substituted monocyclic or polycyclic ring system, or for an unsubstituted or at least mono-substituted five- to fourteen-membered aryl or heteroaryl radical, which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s) and/or can be condensed with an unsubstituted or at least mono-substituted monocyclic or polycyclic ring system;
preferably stands for a linear or branched, unsubstituted or at least mono-substituted C₁₋₈ alkyl radical, for a linear or branched, unsubstituted or at least mono-substituted C₂₋₈ alkenyl radical, for a linear or branched, unsubstituted or at least mono-substituted C₂₋₈ alkinyl radical, for an unsubstituted or at least mono-substituted, unsaturated or saturated three-, four-, five-, six-, seven-, eight- or nine-membered cycloaliphatic radical which possibly contains at least one heteroatom as ring member and which can be bonded via a linear or branched, unsubstituted or at least mono-substituted C₁₋₅ alkylene group, C₂₋₅ alkenylene group or C₂₋₅ alkinylene group which possibly contains 1, 2, or 3 heteroatoms selected independently of one another from the group comprising oxygen, sulphur and nitrogen, as chain member(s), or for an unsubstituted or at least mono-substituted five- to fourteen-membered aryl or heteroaryl radical and which can be bonded via a linear or branched, unsubstituted or at least mono-substituted C₁₋₅ alkylene group, C₂₋₅ alkenylene group or C₂₋₅ alkinylene group possibly containing 1, 2, or 3 heteroatoms selected independently of one another from the group comprising oxygen, sulphur and nitrogen, as chain member(s);
particularly preferred stands for an alkyl radical selected from the group comprising methyl, ethyl, n-propyl, isopropyl, n-butyl, sec-butyl, isobutyl and tert-butyl, or for an alkenyl radical selected from the group comprising ethenyl, 1-propenyl, 2-propenyl, 1-butenyl, 2-butenyl and 3-butenyl, or for an alkinyl radical selected from the group comprising ethinyl, 1-propinyl, 2-propinyl, 1-butinyl, 2-butinyl and 3-butinyl, or for a radical selected from the group comprising cyclopropyl, cyclobutyl, cyclopentyl, cyclohexyl, pyrrolidinyl, tetrahydrofuranyl, tetrahydrothiophenyl, piperidinyl, tetrahydropyranyl, piperazinyl, morpholinyl, thiomorpholinyl, dioxolanyl, azepanyl, diazepanyl, imidazolidinyl, phenyl, naphthyl, anthracenyl, pyrrolyl, indolyl, furanyl, benzo[b]furanyl, thiophenyl, benzo[b]thiophenyl, pyrazolyl, imidazolyl, thiazolyl, dithiazolyl, thiadiazolyl, triazolyl, oxazolyl, oxadiazolyl, isoxazolyl, pyridinyl, pyridazinyl, pyrimidinyl, pyrazinyl, pyranyl, indazolyl, purinyl, indolizinyl, quinolinyl, isoquinolinyl and quinazolinyl group, wherein the respective cyclic radical can be bonded via a C₁₋₃ alkylene group, C₂₋₅ alkenylene group or C₂₋₃ alkinylene group possibly containing 1 or 2 heteroatoms selected from the group comprising oxygen, sulphur and nitrogen, as chain member(s), be bonded can and/or unsubstituted or is substituted with 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl, -OH, -O-CH₃, -O-C₂H₅, -O-C₃H₇, -NH₂,-N(CH₃)₂, -N(C₂H₅)₂, -NH-CH₃, -NH-C₂H₅, -NO₂, -CF₃, -O-CF₃, -S-CF₃, -SH, -NH-S(=O)₂-CH₃, -C(=O)-OH, -C(=O)-CH₃, -C(=O)-C₂H₅, -C(=O)-N(CH₃)₂, -C(=O)-NH-CH₃, -NH-C(=O)-CH₃, -NH-C(=O)-C₂H₅, -C(=O)-O-CH₃ and -C(=O)-O-C₂H₅.

9. Compounds as defined in one or more of claims 1 to 8, **characterised in that**
n is equal to 0, 1, 2, 3, or 4,
X stands for N or C-R²,
R¹ stands for a hydrogen radical, a halogen radical selected from the group comprising F, Cl and Br, a CF₃ group, a nitro group, a cyano group, an amino group, a dimethylamino group, a diethylamino group, a hydroxyl group, a thiol group, a carboxyl group, a -C(=O)-O-CH₃ group, a -C(=O)-O-C₂H₅ group, a -C(=O)-NH-R¹⁴ group, a -C(=O)-NR¹⁵R¹⁶ group, an -O-R¹⁷ group, an -S-R¹⁸ group, an -S(=O)-R¹⁹ group, an -S(=O)₂-R²⁰ group, an alkyl radical selected from the group comprising methyl, ethyl, n-propyl, isopropyl, n-butyl, sec-butyl, isobutyl and tert-butyl, for an alkenyl radical selected from the group comprising ethenyl, 1-propenyl, 2-propenyl, 1-butenyl, 2-butenyl and 3-butenyl, for an alkinyl radical selected from the group comprising ethinyl, 1-propinyl, 2-propinyl, 1-butinyl, 2-butinyl and 3-butinyl or for a radical selected from the group comprising (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl, each of which is unsubstituted or substituted with possibly 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl,-OH, -O-CH₃, -O-C₂H₅ and -O-C₃H₇;
R² stands for a hydrogen radical, a halogen radical selected from the group comprising F, Cl and Br, a CF₃ group, a nitro group, a cyano group, an amino group, a dimethylamino group, a diethylamino group, a hydroxyl group, a thiol group, a carboxyl group, a -C(=O)-O-CH₃ group, a -C(=O)-O-C₂H₅ group, a -C(=O)-NH-R¹⁴ group, a -C(=O)-NR¹⁵R¹⁶ group, an -O-R¹⁷ group, for an -S-R¹⁸ group, an -S(=O)-R¹⁹ group, an -S(=O)₂-R²⁰ group, an alkyl radical selected from the group comprising methyl, ethyl, n-propyl, isopropyl, n-butyl, sec-butyl, isobutyl and tert-butyl, for an alkenyl radical selected from the group comprising ethenyl, 1-propenyl, 2-propenyl, 1-butenyl, 2-butenyl and 3-butenyl, for an alkinyl radical selected from the group comprising ethinyl, 1-propinyl, 2-propinyl, 1-butinyl, 2-butinyl and 3-butinyl or for a radical selected from the group comprising (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl, each of which is unsubstituted or substituted with possibly 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl,-OH, -O-CH₃, -O-C₂H₅ and -O-C₃H₇;
R³ stands for an oxo group, an alkyl radical selected from the group comprising methyl, ethyl, n-propyl, isopropyl, n-butyl, sec-butyl, isobutyl and tert-butyl or for a phenyl radical, which is unsubstituted or substituted with 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl, -OH, -O-CH₃, -O-C₂H₅ and -O-C₃H₇;
R⁴ stands for a hydrogen radical, a -C(=O)-NH₂ group, a -C(=O)-NH-R¹⁴ group, a-C(=O)-NR¹⁵R¹⁶ group, an alkyl radical selected from the group comprising methyl, ethyl, n-propyl, isopropyl, n-butyl, sec-butyl, isobutyl, tert-butyl, n-pentyl, 2-pentyl, 3-pentyl, neopentyl, n-hexyl, n-heptyl and n-octyl, a radical selected from the group comprising cyclopropyl, cyclobutyl, cyclopentyl, cyclohexyl, cycloheptyl, cyclopentenyl, cyclohexenyl, pyrrolidinyl, tetrahydrofuranyl, tetrahydrothiophenyl, piperidinyl, tetrahydropyranyl, piperazinyl, morpholinyl, thiomorpholinyl, dioxolanyl, azepanyl, diazepanyl, imidazolidinyl, phenyl, naphthyl, anthracenyl, furanyl, thiophenyl, pyrazolyl, pyrazinyl, pyrimidinyl, pyridinyl, pyrrolyl, oxazolyl, isoxazolyl, thiazolyl, dithiazolyl, oxadiazolyl, triazolyl, imidazolyl, indolyl, benzo[b]thiophenyl, benzo[b]furanyl, quinolinyl, isoquinolinyl and quinazolyl, which in each case can be possibly substituted with 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl, ethenyl, allyl, ethinyl, propinyl,-C=C-Si(CH3)₃, -C=C-Si(C₂H₅)₃, -CH₂-O-CH₃, -CH₂-O-C₂H₅, -OH, -O-CH₃, -O-C₂H₅, -O-C₃H₇, -S-CH₃, -S-C₂H₅, -S(=O)-CH₃, -S(=O)₂-CH₃, -S(=O)-C₂H₅, -S(=O)₂-C₂H₅,-NH₂, -N(CH₃)₂, -N(C₂H₅)₂, -NH-CH₃, -NH-C₂H₅, -NO₂, -CF₃, -CHF₂, -CH₂F, -O-CF₃, -S-CF₃, -SH, -NH-S(=O)₂-CH₃, -C(=O)-OH, -C(=O)-H; -C(=O)-CH₃, -C(=O)-C₂H₅,-C(=O)-NH₂, -C(=O)-N(CH₃)₂, -C(=O)-NH-CH₃, -NH-C(=O)-CH₃, -NH-C(=O)-C₂H₅, -C(=O)-O-CH₃, -C(=O)-O-C₂H₅, -C(=O)-O-C(CH₃)₃ and phenyl;
and
R¹⁴, R¹⁵, R¹⁶, R¹⁷, R¹⁸, R¹⁹ and R²⁰, independently of one another, stand for an alkyl radical selected from the group comprising methyl, ethyl, n-propyl, isopropyl, n-butyl, sec-butyl, isobutyl and tert-butyl,
in each case possibly in the form of one of the pure stereoisomers thereof, particularly enantiomers or diastereomers thereof, the racemates thereof or in the form of a mixture of stereoisomers and particularly of the enantiomers and/or diastereomers in any desired mixture ratio, or in each case in the form of appropriate salts, preferably in the form of corresponding hydrochlorides, or in each case in the form of appropriate solvates.

10. Compounds as defined in one or more of claims 1 to 9, **characterised in that**
n is equal to 0, 1, 2, 3 or 4,
X stands for N or C-R²,
R¹ stands for a hydrogen radical, a halogen radical selected from the group comprising F, Cl and Br, a CF₃ group, a nitro group, a cyano group, an amino group, a dimethylamino group, a diethylamino group, a hydroxyl group, a thiol group, a carboxyl group, a -C(=O)-O-CH₃ group, a -C(=O)-O-C₂H₅ group, a -C(=O)-NH-R¹⁴ group, a -C(=O)-NR¹⁵R¹⁶ group, an -O-R¹⁷ group, an -S-R¹⁸ group, an -S(=O)-R¹⁹ group, an -S(=O)₂-R²⁰ group, an alkyl radical selected from the group comprising methyl, ethyl, n-propyl, isopropyl, n-butyl, sec-butyl, isobutyl and tert-butyl, for an alkenyl radical selected from the group comprising ethenyl, 1-propenyl, 2-propenyl, 1-butenyl, 2-butenyl and 3-butenyl, for an alkinyl radical selected from the group comprising ethinyl, 1-propinyl, 2-propinyl, 1-butinyl, 2-butinyl and 3-butinyl or for a radical selected from the group comprising (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl, each of which is unsubstituted or substituted with possibly 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl,-OH, -O-CH₃, -O-C₂H₅ and -O-C₃H₇;
R² stands for a hydrogen radical, a halogen radical selected from the group comprising F, Cl and Br, a CF₃ group, a nitro group, a cyano group, an amino group, a dimethylamino group, a diethylamino group, a hydroxyl group, a thiol group, a carboxyl group, a -C(=O)-O-CH₃ group, a -C(=O)-O-C₂H₅ group, a -C(=O)-NH-R¹⁴ group, a -C(=O)-NR¹⁵R¹⁶ group, an -O-R¹⁷ group, an -S-R¹⁸ group, an -S(=O)-R¹⁹ group, an -S(=O)₂-R²⁰ group, an alkyl radical selected from the group comprising methyl, ethyl, n-propyl, isopropyl, n-butyl, sec-butyl, isobutyl and tert-butyl, for an alkenyl radical selected from the group comprising ethenyl, 1-propenyl, 2-propenyl, 1-butenyl, 2-butenyl and 3-butenyl, stands for an alkinyl radical selected from the group comprising ethinyl, 1-propinyl, 2-propinyl, 1-butinyl, 2-butinyl and 3-butinyl or for a radical selected from the group comprising (1,3)-dioxolan-2-yl, phenyl, benzyl, phenethyl, oxadiazolyl, 2-pyridyl, 3-pyridyl, 4-pyridyl, 2-thienyl, 3-thienyl, 2-furyl and 3-furyl, each of which is unsubstituted or substituted with possibly 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl,-OH, -O-CH₃, -O-C₂H₅ and -O-C₃H₇;
R³ stands for an oxo group, for an alkyl radical selected from the group comprising methyl, ethyl, n-propyl, isopropyl, n-butyl, sec-butyl, isobutyl and tert-butyl or for a phenyl radical, which is unsubstituted or substituted with 1, 2, 3, 4, or 5 substituents selected independently of one another from the group comprising F, Cl, Br, I, -CN, methyl, ethyl, n-propyl, isopropyl, n-butyl, sec-butyl, isobutyl, tert-butyl, -OH, -O-CH₃, -O-C₂H₅ and -O-C₃H₇;
R⁴ stands for a hydrogen radical, a -C(=O)-NH₂ group, a -C(=O)-NH-R¹⁴ group, a-C(=O)-NR¹⁵R¹⁶ group, an alkyl radical selected from the group comprising methyl, ethyl, n-propyl, isopropyl, n-butyl, sec-butyl, isobutyl, tert-butyl, n-pentyl, sec-pentyl, neopentyl, n-hexyl, n-heptyl and n-octyl, or for a radical selected from the group comprising cyclopropyl, cyclobutyl, cyclopentyl, cyclohexyl, cycloheptyl, cyclopentenyl, cyclohexenyl, phenyl, pyrid-2-yl, pyrid-3-yl, pyrid-4-yl, pyrrol-2-yl, pyrrol-3-yl, thiophen-2-yl, thiophen-3-yl, 4-methylthiophen-2-yl, 3-methylthiophen-2-yl, 5-methylthiophen-2-yl, furan-2-yl, furan-3-yl, imidazol-2-yl, imidazol-4-yl, thiazol-2-yl, thiazol-4-yl, thiazol-5-yl, oxazol-2-yl, oxazol-4-yl, oxazol-5-yl, (1,2,4)-thiadiazol-5-yl, (1,2,4)-oxadiazol-5-yl, naphth-1-yl, naphth-2-yl, anthracen-1-yl, anthracen-2-yl, anthracen-9-yl, indol-2-yl, indol-3-yl, indol-4-yl, indol-5-yl, indol-6-yl, indol-7-yl, 1-methylindol-2-yl, 1-methylindol-3-yl, 1-methylindol-4-yl, 1-methylindol-5-yl, 1-methylindol-6-yl, 1-methylindol-7-yl, quinolin-3-yl and quinolin-4-yl, quinolin-2-yl, quinolin-5-yl, quinolin-6-yl, quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl, isoquinolin-8-yl, 2-methylphenyl, 3-methylphenyl, 4-methylphenyl, 2-fluorophenyl, 3-fluorophenyl, 4-fluorophenyl, 2-cyanophenyl, 3-cyanophenyl, 4-cyanophenyl, 2-hydroxyphenyl, 3-hydroxyphenyl, 4-hydroxyphenyl, 2-aminophenyl, 3-aminophenyl, 4-aminophenyl, 2-dimethylaminophenyl, 3-dimethylaminophenyl, 4-dimethylaminophenyl, 2-methylaminophenyl, 3-methylaminophenyl, 4-methylaminophenyl, 2-acetylphenyl, 3-acetylphenyl, 4-acetylphenyl, 2-methylsulphinylphenyl, 3-methylsulphinylphenyl, 4-methylsulphinylphenyl, 2-methylsulphonylphenyl, 3-methylsulphonylphenyl, 4-methylsulphonylphenyl and 2-methoxyphenyl, 3-methoxyphenyl, 4-methoxyphenyl, 2-chlorophenyl, 3-chlorophenyl, 4-chlorophenyl, 2-ethoxyphenyl, 3-ethoxyphenyl, 4-ethoxyphenyl, 2-trifluoromethylphenyl, 3-trifluoromethylphenyl, 4-trifluoromethylphenyl, 2-difluoromethylphenyl, 3-difluoromethylphenyl, 4-difluoromethylphenyl, 2-fluoromethylphenyl, 3-fluoromethylphenyl, 4-fluoromethylphenyl, 2-nitrophenyl, 3-nitrophenyl, 4-nitrophenyl, 2-ethylphenyl, 3-ethylphenyl, 4-ethylphenyl, 2-propylphenyl, 3-propylphenyl, 4-propylphenyl, 2-isopropylphenyl, 3-isopropylphenyl, 4-isopropylphenyl, 2-tert-butylphenyl, 3-tert-butylphenyl, 4-tert-butylphenyl, 2-carboxyphenyl, 3-carboxyphenyl, 4-carboxyphenyl, 2-ethenylphenyl, 3-ethenylphenyl, 4-ethenylphenyl, 2-ethinylphenyl, 3-ethinylphenyl, 4-ethinylphenyl, 2-allylphenyl, 3-allylphenyl, 4-allylphenyl, 2-trimethylsilanylethinylphenyl, 3-trimethylsilanylethinylphenyl, 4-trimethylsilanylethinylphenyl, 2-formylphenyl, 3-formylphenyl, 4-formylphenyl, 2-acetaminophenyl, 3-acetaminophenyl, 4-acetaminophenyl, 2-dimethylaminocarbonylphenyl, 3-dimethylaminocarbonylphenyl, 4-dimethylaminocarbonylphenyl, 2-methoxymethylphenyl, 3-methoxymethylphenyl, 4-methoxymethylphenyl, 2-ethoxymethylphenyl, 3-ethoxymethylphenyl, 4-ethoxymethylphenyl, 2-aminocarbonylphenyl, 3-aminocarbonylphenyl, 4-aminocarbonylphenyl, 2-methylaminocarbonylphenyl, 3-methylaminocarbonylphenyl, 4-methylaminocarbonylphenyl, 2-carboxymethylesterphenyl, 3-carboxymethylesterphenyl, 4-carboxymethylesterphenyl, 2-carboxyethylesterphenyl, 3-carboxyethylesterphenyl, 4-carboxyethylesterphenyl, 2-carboxy-tert-butylesterphenyl, 3-carboxy-tert-butylesterphenyl, 4-carboxy-tert-butylesterphenyl, 2-methylmercaptophenyl, 3-methylmercaptophenyl, 4-methylmercaptophenyl, 2-ethylmercaptophenyl, 3-ethylmercaptophenyl, 4-ethylmercaptophenyl, 2-biphenyl, 3-biphenyl, 4-biphenyl, 2-bromophenyl, 3-bromophenyl, 4-bromophenyl, 2-iodophenyl, 3-iodophenyl and 4-iodophenyl, 2-trifluoromethoxyphenyl, 3-trifluoromethoxyphenyl, 4-trifluoromethoxyphenyl, 2-fluoro-3-trifluoromethylphenyl, 2-fluoro-4-methylphenyl, (2,3)-difluorophenyl, (2,3)-dimethylphenyl, (2,3)-dichlorophenyl, 3-fluoro-2-trifluoromethylphenyl, (2,4)-dichlorophenyl, (2,4)-difluorophenyl, 4-fluoro-2-trifluoromethylphenyl, (2,4)-dimethoxyphenyl, 2-chloro-4-fluorophenyl, 2-chloro-4-nitrophenyl, 2-chloro-4-methylphenyl, 2-chloro-5-trifluoromethylphenyl, 2-chloro-5-methoxyphenyl, 2-bromo-5-trifluoromethylphenyl, 2-bromo-5-methoxyphenyl, (2,4)-dibromophenyl, (2,4)-dimethylphenyl, 2-fluoro-4-trifluoromethylphenyl, (2,5)-difluorophenyl, 2-fluoro-5-trifluoromethylphenyl, 5-fluoro-2-trifluoromethylphenyl, 5-chloro-2-trifluoromethylphenyl, 5-bromo-2-trifluoromethylphenyl, (2,5)-dimethoxyphenyl, (2,5)-bis-trifluoromethylphenyl, (2,5)-dichlorophenyl, (2,5)-dibromophenyl, 2-methoxy-5-nitrophenyl, 2-fluoro-6-trifluoromethylphenyl, (2,6)-dimethoxyphenyl, (2,6)-dimethylphenyl, (2,6)-dichlorophenyl, 2-chloro-6-fluorophenyl, 2-bromo-6-chlorophenyl, 2-bromo-6-fluorophenyl, (2,6)-difluorophenyl, (2,6)-difluoro-3-methylphenyl, (2,6)-dibromophenyl, (2,6)-dichlorophenyl, 3-chloro-2-fluorophenyl, 3-chloro-5-methylphenyl, (3,4)-dichlorophenyl, (3,4)-dimethylphenyl, 3-methyl-4-methoxyphenyl, 4-chloro-3-nitrophenyl, (3,4)-dimethoxyphenyl, 4-fluoro-3-trifluoromethylphenyl, 3-fluoro-4-trifluoromethylphenyl, (3,4)-difluorophenyl, 3-cyano-4-fluorophenyl, 3-cyano-4-methylphenyl, 3-cyano-4-methoxyphenyl, 3-bromo-4-fluorophenyl, 3-bromo-4-methylphenyl, 3-bromo-4-methoxyphenyl, 4-chloro-2-fluorophenyl, 4-chloro-3-trifluoromethyl, 4-bromo-3-methylphenyl, 4-bromo-5-methylphenyl, 3-chloro-4-fluorophenyl, 4-fluoro-3-nitrophenyl, 4-bromo-3-nitrophenyl, (3,4)-dibromophenyl, 4-chloro-3-methylphenyl, 4-bromo-3-methylphenyl, 4-fluoro-3-methylphenyl, 3-fluoro-4-methylphenyl and 3-fluoro-5-methylphenyl, 2-fluoro-3-methylphenyl, 4-methyl-3-nitrophenyl, (3,5)-dimethoxyphenyl, (3,5)-dimethylphenyl, (3,5)-bis-trifluoromethylphenyl, (3,5)-difluorophenyl, (3,5)-dinitrophenyl, (3,5)-dichlorophenyl, 3-fluoro-5-trifluoromethylphenyl, 5-fluoro-3-trifluoromethylphenyl, (3,5)-dibromophenyl, 5-chloro-4-fluorophenyl, 5-chloro-4-fluorophenyl, 5-bromo-4-methylphenyl, (2,3,4)-trifluorophenyl, (2,3,4)-trichlorophenyl, (2,3,6)-trifluorophenyl, 5-chloro-2-methoxyphenyl, (2,3)-difluoro-4-methyl, (2,4,5)-trifluorophenyl, (2,4,5)-trichlorophenyl, (2,4)-dichloro-5-fluorophenyl, (2,4,6)-trichlorophenyl, (2,4,6)-trimethylphenyl, (2,4,6)-trifluorophenyl, (2,4,6)-trimethoxyphenyl, (3,4,5)-trimethoxyphenyl, (2,3,4,5)-tetrafluorophenyl, 4-methoxy-(2,3,6)-trimethylphenyl, 4-methoxy-(2,3,6)-trimethylphenyl and 4-chloro-2,5-dimethylphenyl, 2-chloro-6-fluoro-3-methylphenyl, 6-chloro-2-fluoro-3-methyl, (2,4,6)-trimethylphenyl, (2,3,4,5,6)-pentafluorophenyl, 3-methylpyrid-2-yl, 4-methylpyrid-2-yl, 5-methylpyrid-2-yl, 6-methylpyrid-2-yl, 2-methylpyrid-3-yl, 4-methylpyrid-3-yl, 5-methylpyrid-3-yl, 6-methylpyrid-3-yl, 2-methylpyrid-4-yl, 3-methylpyrid-4-yl, 3-fluoropyrid-2-yl, 4-fluoropyrid-2-yl, 5-fluoropyrid-2-yl, 6-fluoropyrid-2-yl, 3-chloropyrid-2-yl, 4-chloropyrid-2-yl, 5-chloropyrid-2-yl, 6-chloropyrid-2-yl, 3-trifluoromethylpyrid-2-yl, 4-trifluoromethylpyrid-2-yl, 5-trifluoromethylpyrid-2-yl, 6-trifluoromethylpyrid-2-yl, 3-methoxypyrid-2-yl, 4-methoxypyrid-2-yl, 5-methoxypyrid-2-yl, 6-methoxypyrid-2-yl, 4-methylthiazol-2-yl, 5-methylthiazol-2-yl, 4-trifluoromethylthiazol-2-yl, 5-trifluoromethylthiazol-2-yl, 4-chlorothiazol-2-yl, 5-chlorothiazol-2-yl, 4-bromothiazol-2-yl, 5-bromothiazol-2-yl, 4-fluorothiazol-2-yl, 5-fluorothiazol-2-yl and 4-cyanothiazol-2-yl, 5-cyanothiazol-2-yl, 4-methoxythiazol-2-yl, S-methoxythiazol-2-yl, 4-methyloxazol-2-yl, 5-methyloxazol-2-yl, 4-trifluoromethyloxazol-2-yl, 5-trifluoromethyloxazol-2-yl, 4-chloroxazol-2-yl, 5-chloroxazol-2-yl, 4-bromoxazol-2-yl, 5-bromoxazol-2-yl, 4-fluoroxazol-2-yl, 5-fluoroxazol-2-yl, 4-cyano-oxazol-2-yl, 5-cyano-oxazol-2-yl, 4-methoxyoxazol-2-yl, 5-methoxyoxazol-2-yl, 2-methyl-(1,2,4)-thiadiazol-5-yl, 2-trifluoromethyl-(1,2,4)-thiadiazol-5-yl, 2-chloro-(1,2,4)-thiadiazol-5-yl, 2-fluoro-(1,2,4)-thiadiazol-5-yl, 2-methoxy-(1,2,4)-thiadiazol-S-yl, 2-cyano-(1,2,4)-thiadiazol-5-yl,2-methyl-(1,2,4)-oxadiazol-5-yl,2-trifluoromethyl-(1,2,4)-oxadiazol-5-yl, 2-chloro-(1,2,4)-oxadiazol-5-yl, 2-fluoro-(1,2,4)-oxadiazol-5-yl, 2-methoxy-(1,2,4)-oxadiazol-5-yl and 2-cyano-(1,2,4)-oxadiazol-5-yl;
and
R¹⁴, R¹⁵, R¹⁶, R¹⁷, R¹⁸, R¹⁹ and R²⁰,independently of one another, stand for an alkyl radical selected from the group comprising methyl, ethyl, n-propyl, isopropyl, n-butyl, sec-butyl, isobutyl and tert-butyl;
in each case possibly in the form of one of the pure stereoisomers thereof, particularly enantiomers or diastereomers thereof, the racemates thereof or in the form of a mixture of stereoisomers and particularly of the enantiomers and/or diastereomers in any desired mixture ratio, or in each case in the form of appropriate salts, preferably in the form of corresponding hydrochlorides, or in each case in the form of appropriate solvates.

11. Compounds as defined in one or more of claims 1 to 10, **characterised in that**
n is equal to 0, 1, or 2,
X stands for N or C-R²,
R¹ stands for a hydrogen radical, a halogen radical selected from the group comprising F, Cl and Br, a CF₃ group, a nitro group, a carboxyl group, a -C(=O)-O-CH₃ group, a-C(=O)-O-C₂H₅ group, a -C(=O)-NH-R¹⁴ group, a -C(=O)-NR¹⁵R¹⁶ group, an -O-R¹⁷ group, an -S-R¹⁸ group, an -S(=O)₂-R²⁰ group, an alkyl radical selected from the group comprising methyl, ethyl, n-propyl, isopropyl, n-butyl, sec-butyl, isobutyl and tert-butyl, or stands for a radical selected from the group comprising phenyl and oxadiazolyl, each of which is unsubstituted or substituted with possibly 1, 2, 3, 4, or 5 substituents selected independently of one another from the group comprising F, Cl, Br, I, -O-CH₃, -O-C₂H₅ and -O-C₃H₇;
R² stands for a hydrogen radical, a halogen radical selected from the group comprising F, Cl and Br, a CF₃ group, a nitro group, a carboxyl group, a -C(=O)-O-CH₃ group, a-C(=O)-O-C₂H₅ group, a -C(=O)-NH-R¹⁴ group, a -C(=O)-NR¹⁵R¹⁶ group, an -O-R¹⁷ group, an -S-R¹⁸ group, an -S(=O)₂-R²⁰ group, an alkyl radical selected from the group comprising methyl, ethyl, n-propyl, isopropyl, n-butyl, sec-butyl, isobutyl and tert-butyl, or stands for a radical selected from the group comprising phenyl and oxadiazolyl, each of which is unsubstituted or substituted with possibly 1, 2, 3, 4, or 5 substituents selected independently of one another from the group comprising F, Cl, Br, I, -O-CH₃, -O-C₂H₅ and -O-C₃H₇;
R³ stands for an oxo group, for an alkyl radical selected from the group comprising methyl, ethyl, n-propyl, isopropyl, n-butyl, sec-butyl, isobutyl and tert-butyl or for an unsubstituted phenyl radical;
R⁴ stands for a hydrogen radical, a -C(=O)-NH₂ group, a -C(=O)-NH-R¹⁴ group, a-C(=O)-NR¹⁵R¹⁶ group, an alkyl radical selected from the group comprising methyl, ethyl, n-propyl, isopropyl, n-butyl, sec-butyl, isobutyl, tert-butyl, n-pentyl, sec-pentyl, neopentyl, n-hexyl, n-heptyl and n-octyl or for a radical selected from the group comprising cyclopropyl, cyclobutyl, cyclopentyl, cyclohexyl, cycloheptyl, cyclopentenyl, cyclohexenyl, phenyl, pyrid-2-yl, pyrid-3-yl, pyrid-4-yl, pyrrol-2-yl, pyrrol-3-yl, thiophen-2-yl, thiophen-3-yl, 4-methylthiophen-2-yl, 3-methylthiophen-2-yl, 5-methylthiophen-2-yl, furan-2-yl, furan-3-yl, imidazol-2-yl, imidazol-4-yl, thiazol-2-yl, thiazol-4-yl, thiazol-5-yl, oxazol-2-yl, oxazol-4-yl, oxazol-5-yl, (1,2,4)-thiadiazol-5-yl, (1,2,4)-oxadiazol-5-yl, naphth-1-yl, naphth-2-yl, indol-3-yl, indol-4-yl, indol-5-yl, indol-6-yl, indol-7-yl, 1-methylindol-2-yl, 1-methylindol-3-yl, 1-methylindol-4-yl, 1-methylindol-5-yl, 1-methylindol-6-yl, 1-methylindol-7-yl, quinolin-3-yl, quinolin-4-yl, quinolin-2-yl, quinolin-5-yl, quinolin-6-yl and quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl, isoquinolin-8-yl, 2-methylphenyl, 3-methylphenyl, 4-methylphenyl, 2-fluorophenyl, 3-fluorophenyl, 4-fluorophenyl, 2-cyanophenyl, 3-cyanophenyl, 4-cyanophenyl, 2-hydroxyphenyl, 3-hydroxyphenyl, 4-hydroxyphenyl, 2-aminophenyl, 3-aminophenyl, 4-aminophenyl, 2-dimethylaminophenyl, 3-dimethylaminophenyl, 4-dimethylaminophenyl, 2-methylaminophenyl, 3-methylaminophenyl, 4-methylaminophenyl, 2-acetylphenyl, 3-acetylphenyl, 4-acetylphenyl, 2-methylsulphinylphenyl, 3-methylsulphinylphenyl, 4-methylsulphinylphenyl, 2-methylsulphonylphenyl, 3-methylsulphonylphenyl, 4-methylsulphonylphenyl, 2-methoxyphenyl, 3-methoxyphenyl, 4-methoxyphenyl, 2-chlorophenyl, 3-chlorophenyl, 4-chlorophenyl, 2-ethoxyphenyl, 3-ethoxyphenyl, 4-ethoxyphenyl, 2-trifluoromethylphenyl, 3-trifluoromethylphenyl, 4-trifluoromethylphenyl and 2-difluoromethylphenyl, 3-difluoromethylphenyl, 4-difluoromethylphenyl, 2-fluoromethylphenyl, 3-fluoromethylphenyl, 4-fluoromethylphenyl, 2-nitrophenyl, 3-nitrophenyl, 4-nitrophenyl, 2-ethylphenyl, 3-ethylphenyl, 4-ethylphenyl, 2-propylphenyl, 3-propylphenyl, 4-propylphenyl, 2-isopropylphenyl, 3-isopropylphenyl, 4-isopropylphenyl, 2-tert-butylphenyl, 3-tert-butylphenyl, 4-tert-butylphenyl, 2-carboxyphenyl, 3-carboxyphenyl, 4-carboxyphenyl, 2-ethenylphenyl, 3-ethenylphenyl, 4-ethenylphenyl, 2-ethinylphenyl, 3-ethinylphenyl, 4-ethinylphenyl, 2-allylphenyl, 3-allylphenyl, 4-allylphenyl, 2-trimethylsilanylethinylphenyl, 3-trimethylsilanylethinylphenyl, 4-trimethylsilanylethinylphenyl, 2-formylphenyl, 3-formylphenyl, 4-formylphenyl, 2-acetaminophenyl, 3-acetaminophenyl, 4-acetaminophenyl, 2-dimethylaminocarbonylphenyl, 3-dimethylaminocarbonylphenyl, 4-dimethylaminocarbonylphenyl, 2-methoxymethylphenyl, 3-methoxymethylphenyl, 4-methoxymethylphenyl, 2-ethoxymethylphenyl, 3-ethoxymethylphenyl and 4-ethoxymethylphenyl, 2-aminocarbonylphenyl, 3-aminocarbonylphenyl, 4-aminocarbonylphenyl, 2-methylaminocarbonylphenyl, 3-methylaminocarbonylphenyl, 4-methylaminocarbonylphenyl, 2-carboxymethylesterphenyl, 3-carboxymethylesterphenyl, 4-carboxymethylesterphenyl, 2-carboxyethylesterphenyl, 3-carboxyethylesterphenyl, 4-carboxyethylesterphenyl, 2-carboxy-tert-butylesterphenyl, 3-carboxy-tert-butylesterphenyl, 4-carboxy-tert-butylesterphenyl, 2-methylmercaptophenyl, 3-methylmercaptophenyl, 4-methylmercaptophenyl, 2-ethylmercaptophenyl, 3-ethylmercaptophenyl, 4-ethylmercaptophenyl, 2-biphenyl, 3-biphenyl, 4-biphenyl, 2-bromophenyl, 3-bromophenyl, 4-bromophenyl, 2-iodophenyl, 3-iodophenyl, 4-iodophenyl, 2-trifluoromethoxyphenyl, 3-trifluoromethoxyphenyl, 4-trifluoromethoxyphenyl, 2-fluoro-4-methylphenyl, (2,3)-dimethylphenyl, (2,4)-dichlorophenyl, (2,4)-difluorophenyl, 2-chloro-4-methylphenyl, 2-chloro-5-trifluoromethylphenyl, 2-chloro-5-methoxyphenyl, 2-bromo-5-trifluoromethylphenyl, 2-bromo-5-methoxyphenyl, (2,4)-dimethylphenyl, (2,6)-dimethylphenyl, 3-chloro-5-methylphenyl, (3,4)-dimethylphenyl, 3-methyl-4-methoxyphenyl, (3,4)-difluorophenyl, 3-cyano-4-fluorophenyl, 3-cyano-4-methylphenyl, 3-cyano-4-methoxyphenyl, 3-bromo-4-fluorophenyl, 3-bromo-4-methylphenyl, 3-bromo-4-methoxyphenyl, 4-chloro-2-fluorophenyl, 4-fluoro-3-methylphenyl, 3-fluoro-4-methylphenyl, 3-fluoro-5-methylphenyl, 2-fluoro-3-methylphenyl, (3,5)-dimethylphenyl and (3,5)-dichlorophenyl;
and
R¹⁴, R¹⁵, R¹⁶, R¹⁷, R¹⁸ and R²⁰, independently of one another, stand for an alkyl radical selected from the group comprising methyl, ethyl, n-propyl, isopropyl, n-butyl, sec-butyl, isobutyl and tert-butyl;
in each case possibly in the form of one of the pure stereoisomers thereof, particularly enantiomers or diastereomers thereof, the racemates thereof or in the form of a mixture of stereoisomers and particularly of the enantiomers and/or diastereomers in any desired mixture ratio, or in each case in the form of appropriate salts, preferably in the form of corresponding hydrochlorides, or in each case in the form of appropriate solvates.

12. Compounds as defined in one or more of claims 1 to 11, selected from the group comprising
| | |
|---|---|
| [1] | 1-(3-phenylpropiolyl)-4-(thiazol-2-yl)-piperazine |
| [AAA00100] | 4-(3-methylmercapto-1,2,4-thiadiazol-5-yl)-1-(3-phenylpropiolyl)-piperazine |
| [AAA00101] | 4-(3-methansulphonyl-1,2,4-thiadiazol-5-yl)-1-(3-phenylpropiolyl)-piperazine |
| [AAA00102] | 4-(3-methoxy-1,2,4-thiadiazol-5-yl)-1-(3-phenylpropiolyl)-piperazine |
| [AAA00103] | 4-(1,2,4-thiadiazol-5-yl)-1-(3-phenylpropiolyl)piperazine |
| [AAA00104] | 4-(3-methyl-1,2,4-thiadiazol-5-yl)-1-(3-phenylpropiolyl)piperazine |
| [AAA00105] | 4-(3-trifluoromethyl-1,2,4-thiadiazol-5-yl)-1-(3-phenylpropiolyl)-piperazine |
| [AAA00106] | 4-(5-(3-methyl-1,2,4-oxadiazol-5-yl)thiazol-2-yl)-1-(3-phenyl-propiolyl)piperazine |
| [AAA00107] | 4-(4-tert-butylthiazol-2-yl)-1-(3-(4-aminophenyl)propiolyl)-piperazine |
| [AAA1] | 4-(thiazol-2-yl)-1-(3-(2-carboxyphenyl)propiolyl)piperazine methyl ester |
| [AAA10] | 4-(thiazol-2-yl)-1-(3-(2-thienyl)propiolyl)piperazine |
| [AAA11] | 4-(thiazol-2-yl)-1-(3-(3-thienyl)propiolyl)piperazine |
| [AAA12] | 4-(thiazol-2-yl)-1-(3-(2-methoxyphenyl)propiolyl)piperazine |
| [AAA13] | 4-(thiazol-2-yl)-1-(3-(3-methoxyphenyl)propiolyl)piperazine |
| [AAA14] | 4-(thiazol-2-yl)-1-(3-(4-methoxyphenyl)propiolyl)piperazine |
| [AAA15] | 4-(thiazol-2-yl)-1-(3-(2-cyanophenyl)propiolyl)piperazine |
| [AAA16] | 4-(thiazol-2-yl)-1-(3-(3-cyanophenyl)propiolyl)piperazine |
| [AAA17] | 4-(thiazol-2-yl)-1-(3-(4-cyanophenyl)propiolyl)piperazine |
| [AAA18] | 4-(thiazol-2-yl)-1-(3-(2,4-dimethylphenyl)propiolyl)piperazine |
| [AAA19] | 4-(thiazol-2-yl)-1-(3-(3,5-dimethylphenyl)propiolyl)piperazine |
| [AAA2] | 4-(thiazol-2-yl)-1-(3-(4-carboxyphenyl)propiolyl)piperazine methyl ester |
| [AAA20] | 4-(thiazol-2-yl)-1-(3-(2,6-dimethylphenyl)propiolyl)piperazine |
| [AAA21] | 4-(thiazol-2-yl)-1-(3-(2-fluorophenyl)propiolyl)piperazine |
| [AAA22] | 4-(thiazol-2-yl)-1-(3-(3-fluorophenyl)propiolyl)piperazine |
| [AAA23] | 4-(thiazol-2-yl)-1-(3-(2-chlorophenyl)propiolyl)piperazine |
| [AAA24] | 4-(thiazol-2-yl)-1-(3-(3-chlorophenyl)propiolyl)piperazine |
| [AAA25] | 4-(thiazol-2-yl)-1-(3-naphthylpropiolyl)piperazine |
| [AAA26] | 4-(thiazol-2-yl)-1-(3-(2,3-dimethylphenyl)propiolyl)piperazine |
| [AAA27] | 4-(thiazol-2-yl)-1-(3-(3,4-dimethylphenyl)propiolyl)piperazine |
| [AAA28] | 4-(thiazol-2-yl)-1-(3-(3-nitrophenyl)propiolyl)piperazine |
| [AAA29] | 4-(thiazol-2-yl)-1-(3-(2-nitrophenyl)propiolyl)piperazine |
| [AAA3] | 4-(thiazol-2-yl)-1-(3-(3-carboxyphenyl)propiolyl)piperazine ethyl ester |
| [AAA30] | 4-(thiazol-2-yl)-1-(3-(3-formylphenyl)prophenyl)piperazine |
| [AAA31] | 4-(thiazol-2-yl)-1-(3-(3-ethenylphenyl)propiolyl)piperazine |
| [AAA32] | 4-(thiazol-2-yl)-1-(3-pyrid-2-ylpropiolyl)piperazine |
| [AAA33] | 4-(thiazol-2-yl)-1-(3-pyrid-3-ylpropiolyl)piperazine |
| [AAA34] | 4-(thiazol-2-yl)-1-(3-pyrid-4-ylpropiolyl)piperazine |
| [AAA35] | 4-(thiazol-2-yl)-1-(3-(quinolin-6-yl)propiolyl)piperazine |
| [AAA36] | 4-(thiazol-2-yl)-1-(3-(3-isopropylphenyl)propiolyl)piperazine |
| [AAA37] | 4-(thiazol-2-yl)-1-(3-biphenyl-3-ylpropiolyl)piperazine |
| [AAA38] | 4-(thiazol-2-yl)-1-(3-naphth-2-ylpropiolyl)piperazine |
| [AAA39] | 4-(thiazol-2-yl)-1-(3-(1-methylindol-5-yl)propiolyl)piperazine |
| [AAA4] | 4-(thiazol-2-yl)-1-(3-(2-hydroxyphenyl)propiolyl)piperazine |
| [AAA40] | 4-(thiazol-2-yl)-1-(3-(3-methylmercaptophenyl)propiolyl)-piperazine |
| [AAA41] | 4-(thiazol-2-yl)-1-(3-(3-cyano-4-fluorophenyl)propiolyl)piperazine |
| [AAA42] | 4-(thiazol-2-yl)-1-(3-(3-methoxymethylphenyl)propiolyl)piperazine |
| [AAA43] | 4-(thiazol-2-yl)-1-(3-(3-hydroxyphenyl)propiolyl)piperazine |
| [AAA44] | 4-(thiazol-2-yl)-1-(3-(3-acetaminophenyl)propiolyl)piperazine |
| [AAA45] | 4-(thiazol-2-yl)-1-(3-(4-acetaminophenyl)propiolyl)piperazine |
| [AAA46] | 4-(thiazol-2-yl)-1-(3-(2-carboxyphenyl)propiolyl)piperazine |
| [AAA47] | 4-(thiazol-2-yl)-1-(3-(3-carboxyphenyl)propiolyl)piperazine |
| [AAA48] | 4-(thiazol-2-yl)-1-(3-(4-carboxyphenyl)propiolyl)piperazine |
| [AAA49] | 4-(thiazol-2-yl)-1-(3-(3-carboxyphenyl)propiolyl)piperazine methyl ester |
| [AAA5] | 4-(thiazol-2-yl)-1-(3-(4-hydroxyphenyl)propiolyl)piperazine |
| [AAA50] | 4-(thiazol-2-yl)-1-(3-(3-aminocarbonylphenyl)propiolyl)piperazine |
| [AAA51] | 4-(thiazol-2-yl)-1-(3-(3-methylaminocarbonylphenyl)propiolyl)-piperazine |
| [AAA52] | 4-(thiazol-2-yl)-1-(3-(3-dimethylaminocarbonylphenyl)propiolyl)-piperazine |
| [AAA53] | 4-(thiazol-2-yl)-1-(3-(2-tolyl)propiolyl)piperazine |
| [AAA54] | 4-(thiazol-2-yl)-1-(3-(3-tolyl)propiolyl)piperazine hydrochloride |
| [AAA55] | 4-(thiazol-2-yl)-1-(3-(4-tolyl)propiolyl)piperazine hydrochloride |
| [AAA56] | 4-(thiazol-2-yl)-1-(3-phenylpropiolyl)piperazine hydrochloride |
| [AAA57] | 4-(thiazol-2-yl)-1-(3-(2-trifluoromethylphenyl)propiolyl)piperazin hydro chloride |
| [AAA58] | 4-(thiazol-2-yl)-1-(3-(3-trifluoromethylphenyl)propiolyl)piperazine hydrochloride |
| [AAA59] | 4-(thiazol-2-yl)-1-(3-(4-trifluoromethylphenyl)propiolyl)piperazin hydrochloride |
| [AAA6] | 4-(thiazol-2-yl)-1-(3-(2-aminophenyl)propiolyl)piperazine |
| [AAA60] | 4-(thiazol-2-yl)-1-(3-pentylpropiolyl)piperazine |
| [AAA61] | 4-(thiazol-2-yl)-1-(3-(4-fluorophenyl)propiolyl)piperazine |
| [AAA62] | 4-(thiazol-2-yl)-1-(3-(4-chlorophenyl)propiolyl)piperazine |
| [AAA63] | 2-methyl-4-(thiazol-2-yl)-1-(3-phenylpropiolyl)piperazine |
| [AAA64] | (S)2-methyl-4-(thiazol-2-yl)-1-(3-phenylpropiolyl)piperazine |
| [AAA65] | (R)2-methyl-4-(thiazol-2-yl)-1-(3-phenylpropiolyl)piperazine |
| [AAA66] | 2-methyl-4-(thiazol-2-yl)-1-(3-phenylpropiolyl)piperazine hydrochloride |
| [AAA67] | 2-methyl-4-(thiazol-2-yl)-1-(3-(3-tolyl)propiolyl)piperazine |
| [AAA68] | 2-methyl-4-(thiazol-2-yl)-1-(3-(3-tolyl)propiolyl)piperazine hydrochloride |
| [AAA69] | 4-(thiazol-2-yl)-1-(3-cyclohexylpropiolyl)piperazine |
| [AAA7] | 4-(thiazol-2-yl)-1-(3-(3-aminophenyl)propiolyl)piperazine |
| [AAA170] | 4-(thiazol-2-yl)-1-(3-methylpropiolyl)piperazine |
| [AAA71] | 2-ethyl-4-(thiazol-2-yl)-1-(3-phenylpropiolyl)piperazine |
| [AAA72] | 2-phenyl-4-(thiazol-2-yl)-1-(3-phenylpropiolyl)piperazine |
| [AAA73] | 4-(thiazol-2-yl)-1-(3-(2-furyl)propiolyl)piperazine |
| [AAA74] | 4-(thiazol-2-yl)-1-(3-(3-furyl)propiolyl)piperazine |
| [AAA75] | cis-2,6-dimethyl-4-(thiazol-2-yl)-1-(3-phenylpropiolyl)piperazine |
| [AAA76] | 4-(5-carboxy-thiazol-2-yl)-1-(3-phenylpropiolyl)piperazine ethyl ester |
| [AAA77] | 4-(4-carboxy-thiazol-2-yl)-1-(3-phenylpropiolyl)piperazine ethyl ester |
| [AAA78] | 4-(5-carboxy-thiazol-2-yl)-1-(3-phenylpropiolyl)piperazine |
| [AAA79] | 4-(4-carboxy-thiazol-2-yl)-1-(3-phenylpropiolyl)piperazine |
| [AAA1] | 4-(thiazol-2-yl)-1-(3-(4-aminophenyl)propiolyl)piperazine |
| [AAA80] | 4-(5-methylaminocarbonylthiazol-2-yl)-1-(3-phenylpropiolyl)-piperazine |
| [AAA81] | 4-(5-dimethylaminocarbonylthiazol-2-yl)-1-(3-phenylpropiolyl)-piperazine |
| [AAA82] | 4-(3-phenyl-1,2,4-thiadiazol-5-yl)-1-(3-phenylpropiolyl)piperazine |
| [AAA83] | 4-(thiazol-2-yl)-1-(3-(quinol-7-yl)propiolyl)piperazine |
| [AAA84] | 4-(thiazol-2-yl)-1-(3-phenylpropiolyl)piperazine-3-on |
| [AAA85] | 4-(3-(4-fluorobenzyl)-1,2,4-thiadiazol-5-yl)-1-(3-phenylpropiolyl)-piperazine |
| [AAA86] | 4-(5-nitro-thiazol-2-yl)-1-(3-phenylpropiolyl)piperazine |
| [AAA87] | 4-(4-tert-butylthiazol-2-yl)-1-(3-phenylpropiolyl)piperazine |
| [AAA188] | 4-(5-methylthiazol-2-yl)-1-(3-phenylpropiolyl)piperazine |
| [AAA89] | 4-(4,5-dimethylthiazol-2-yl)-1-(3-phenylpropiolyl)piperazine |
| [AAA9] | 4-(thiazol-2-yl)-1-(3-(indol-5-yl)propiolyl)piperazine |
| [AAA90] | 4-(5-bromo-4-phenylthiazol-2-yl)-1-(3-phenylpropiolyl)piperazine |
| [AAA91] | 4-(5-bromo-4-methylthiazol-2-yl)-1-(3-phenylpropiolyl)piperazine |
| [AAA92] | 4-(4-methylthiazol-2-yl)-1-(3-phenylpropiolyl)piperazine |
| [AAA93] | 4-(4-trifluorornethylthiazol-2-yl)-1-(3-phenylpropiolyl)piperazine |
| [AAA94] | 4-(4-chloro-thiazol-2-yl)-1-(3-phenylpropiolyl)piperazine |
| [AAA95] | 4-(5-chloro-thiazol-2-yl)-1-(3-phenylpropiolyl)piperazine |
| [AAA96] | 4-(4-bromo-thiazol-2-yl)- 1-(3-phenylpropiolyl)piperazine |
| [AAA97] | 4-(5-bromo-thiazol-2-yl)-1-(3-phenylpropiolyl)piperazine |
| [AAA98] | 4-(5-phenylthiazol-2-yl)-1-(3-phenylpropiolyl)piperazine |
| [AAA99] | 4-(4phenylthiazol-2-yl)-1-(3-phenylpropiolyl)piperazine |
| [BBB2] | 4-(thiazol-2-yl)-1-propiolylpiperazine; |
| [CCC1] | 4-(3-phenyl-1,2,4-thiadiazol-5-yl)-1-(3-(2-methoxyphenyl)-propiolyl)piperazine |
| [CCC2] | 4-(3-phenyl-1,2,4-thiadiazol-5-yl)-1-(3-(4-methoxyphenyl)-propiolyl)piperazine |
| [CCC3] | 4-(3-phenyl-1,2,4-thiadiazol-5-yl)-1-(3-(2-fluorophenyl)propiolyl)-piperazine |
| [CCC4] | 4-(3-phenyl-1,2,4-thiadiazol-5-yl)-1-(3-(3-fluorophenyl)propiolyl)-piperazine |
| [CCC5] | 4-(3-phenyl-1,2,4-thiadiazol-5-yl)-1-(3-(4-fluorophenyl)propiolyl)-piperazine |
| [CCC6] | 4-(3-phenyl-1,2,4-thiadiazol-5-yl)-1-(3-(2,4-dichlorophenyl)-propiolyl)piperazine |
| [CCC7] | 4-(3-phenyl-1,2,4-thiadiazol-5-yl)-1-(3-(3,5-dichlorophenyl)-propiolyl)piperazine |
| [CCC8] | 4-(3-(4-fluorophenyl)-1,2,4-thiadiazol-5-yl)-1-(3-(tol-2-yl)-propiolyl)piperazine |
| [CCC9] | 4-(3-(4-fluorophenyl)-1,2,4-thiadiazol-5-yl)-1-(3-(3-methoxyphenyl)propiolyl)piperazine |
| [CCC10] | 4-(3-(4-fluorophenyl)-1,2,4-thiadiazol-5-yl)-1-(3-(3-fluoro-4-methylphenyl)propiolyl)piperazine |
| [CCC11] | 4-(3-(4-fluorophenyl)-1,2,4-thiadiazol-5-yl)-1-(3-(2,4-difluorophenyl)propiolyl)piperazine |
| [CCC12] | 4-(3-(4-fluorophenyl)-1,2,4-thiadiazol-5-yl)-1-(3-(tol-3-yl)-propiolyl)piperazine |
| [CCC13] | 4-(3-(4-fluorophenyl)-1,2,4-thiadiazol-5-yl)-1-(3-(4-trifluoromethylphenyl)propiolyl)piperazine |
| [CCC14] | 4-(3-(4-fluorophenyl)-1,2,4-thiadiazol-5-yl)-1-(3-(3-trifluoromethylphenyl)propiolyl)piperazine |
| [CCC15] | 4-(3-(4-fluorophenyl)-1,2,4-thiadiazol-5-yl)-1-(3-(2-chloro-5-trifluoromethylphenyl)propiolyl)piperazine |
| [CCC16] | 4-(3-tert-butyl-1,2,4-thiadiazol-5-yl)-1-(3-(thiophen-2-yl)-propiolyl)piperazine |
| [CCC17] | 4-(3-tert-butyl-1,2,4-thiadiazol-5-yl)-1-(3-(3-trifluoromethylphenyl)propiolyl)piperazine |
| [CCC18] | 4-(3-tert-butyl-1,2,4-thiadiazol-5-yl)-1-(3-(2-methoxyphenyl)-propiolyl)piperazine |
| [CCC19] | 4-(3-tert-butyl-1,2,4-thiadiazol-5-yl)-1-(3-(tol-2-yl)propiolyl)-piperazine |
| [CCC20] | 4-(3-tert-butyl-1,2,4-thiadiazol-5-yl)-1-(3-(3-fluoro-4-methylphenyl)propiolyl)piperazine |
| [CCC21] | 4-(3-tert-butyl-1,2,4-thiadiazol-5-yl)-1-(3-(2-chloro-5-trifluoromethylphenyl)propiolyl)piperazine |
| [CCC22] | 4-(thiazol-2-yl)-1-(3-(2,4-difluorophenyl)propiolyl)piperazine |
| [CCC23] | 4-(thiazol-2-yl)-1-(3-(2-bromo-5-methoxyphenyl)propiolyl)-piperazine |
| [CCC24] | 4-(thiazol-2-yl)-1-(3-(3-bromo-4-methoxyphenyl)propiolyl)-piperazine |
| [CCC25] | 4-(thiazol-2-yl)-1-(3-(3,5-dichlorophenyl)propiolyl)piperazine |
| [CCC26] | 4-(thiazol-2-yl)-1-(3-(4-fluoro-3-methylphenyl)propiolyl)piperazine |
| [CCC27] | 4-(4-tert-butylthiazol-2-yl)-1-(3-(3-cyanophenyl)propiolyl)-piperazine |
| [CCC28] | 4-(4-tert-butylthiazol-2-yl)-1-(3-(2-trifluoromethylphenyl)-propiolyl)piperazine |
| [CCC29] | 4-(4-phenylthiazol-2-yl)-1-(3-(2,3-dimethylphenyl)propiolyl)-piperazine |
| [CCC30] | 4-(4-phenylthiazol-2-yl)-1-(3-(4-fluorophenyl)propiolyl)piperazine |
| [CCC31] | 4-(4-methylthiazol-2-yl)-1-(3-(tol-3-yl)propiolyl)piperazine |
| [CCC32] | 4-(4-methylthiazol-2-yl)-1-(3-(thiophen-2-yl)propiolyl)piperazine |
| [CCC33] | 4-(5-methylthiazol-2-yl)-1-(3-(tol-4-yl)propiolyl)piperazine |
| [CCC34] | 4-(5-methylthiazol-2-yl)-1-(3-(2-cyanophenyl)propiolyl)piperazine |
| [CCC35] | 4-(4,5-dimethylthiazol-2-yl)-1-(3-(3-cyanophenyl)propiolyl)-piperazine |
| [CCC36] | 4-(4,5-dimethylthiazol-2-yl)-1-(3-(3,4-dimethylphenyl)propiolyl)-piperazine |
| [CCC37] | 4-(4-(4-methoxyphenyl)thiazol-2-yl)-1-(3-(2,4-dimethylphenyl)-propiolyl)piperazine |
| [CCC38] | 4-(4-(4-methoxyphenyl)thiazol-2-yl)-1-(3-(4-trifluoromethylphenyl)propiolyl)piperazine |
| [CCC39] | 4-(4-(4-fluorophenyl)thiazol-2-yl)-1-(3-(2-cyanophenyl)propiolyl)-piperazine |
| [CCC40] | 4-(4-(4-chlorophenyl)thiazol-2-yl)-1-(3-(4-cyanophenyl)propiolyl)-piperazine |
| [AAA00108] | 4-(thiazol-2-yl)-1-(3-(1-Methylindol-6-yl)propiolyl)piperazine |
| [AAA00109] | 4-(thiazol-2-yl)-1-(3-(3-acetylphenyl)propiolyl)piperazine |
| [AAA00110] | 4-(thiazol-2-yl)-1-(3-(3-fluoro-5-methylphenyl)propiolyl)piperazine |
| [AAA00111] | 4-(thiazol-2-yl)-1-(3-(2-fluoro-3-methylphenyl)propiolyl)piperazine |
| [AAA00112] | 4-(thiazol-2-yl)-1-(3-(3-methylaminophenyl)propiolyl)piperazine |
| [AAA00113] | 4-(thiazol-2-yl)-1-(3-(3-dimethylaminophenyl)propiolyl)piperazine |
| [AAA00114] | 4-(thiazol-2-yl)-1-(dimethylcarbamoylpropiolyl)piperazine |
| [AAA00115] | 4-(thiazol-2-yl)-1-(3-(3-methylsulphinylphenyl)propiolyl)-piperazin |
| [AAA00116] | 4-(thiazol-2-yl)-1-(3-(3-methylsulphonylphenyl)propiolyl)-piperazine |
| [AAA00117] | 4-(thiazol-2-yl)-1-(3-(3-ethinylphenyl)propiolyl)piperazine |
| [AAA00118] | 4-(thiazol-2-yl)-1-(3-(4-methylthiophen-2-yl)propiolyl)piperazine |
| [AAA00119] | 2-methyl-4-(thiazol-2-yl)-1-(3-(3-chlorophenyl)propiolyl)-piperazine |
| [AAA00120] | 4-(thiazol-2-yl)-1-(3-(3-ethylphenyl)propiolyl)piperazine |
| [AAA00121] | 2-tert-butyl-4-(thiazol-2-yl)-1-(3-phenylpropiolyl)piperazine |
| [AAA00122] | 4-(thiazol-2-yl)-1-(3-(3-difluoromethylphenyl)propiolyl)piperazine |
| [AAA00123] | 2-methyl-4-(thiazol-2-yl)-1-(3-(3-cyanophenyl)propiolyl)piperazine |
| [AAA00124] | 2-isopropyl-4-(thiazol-2-yl)-1-(3-phenylpropiolyl)piperazine |
| [AAA00125] | 4-(thiazol-2-yl)-1-(3-(3-trimethylsilanylethinylphenyl)propiolyl)-piperazine; |
in each case possibly in the form of pure stereoisomers thereof, particularly enantiomers or diastereomers, the racemates thereof or in the form of a mixture of stereoisomers, particularly the enantiomers and/or diastereomers, in any desired mixture ratio, or in each case in the form of appropriate salts thereof, or in each case in the form of appropriate solvates thereof.

13. A process for the preparation of compounds of the general formula 1 as defined in one or more of claims 1 to 12, **characterised in that**
at least one compound of the general formula II, 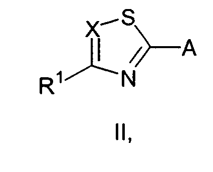 in which the radicals X and R¹ have the meanings defined in one or more of claims 1 to 12 and A stands for a leaving group, preferably for a halogen radical or a sulphonic acid ester, more preferred for a chlorine or bromine radical, is caused to react with at least one compound of the general formula III, 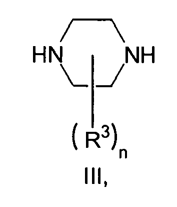 in which R³ and n have the meanings defined in one or more of claims 1 to 12, possibly in a reaction medium, possibly in the presence of at least one base and/or at least one organometallic compound and/or at least one metallic hydride reagent, preferably at a temperature of from -70 °C to 300 °C, preferably from -70 °C to 150°C, to produce at least one corresponding compound of the general formula IV, possibly in the form of an appropriate salt thereof, 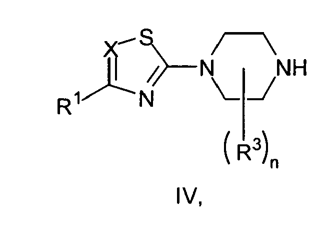 in which X, R¹, R³ and n have the meanings stated above and said product is possibly purified and/or isolated;
or at least one compound of the general formula II is caused to react with at least one compound of the general formula V, 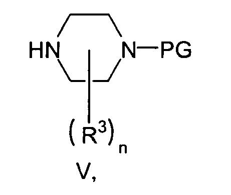 in which R³ and n have the meanings defined in one or more of claims 1 to 12 and PG stands for a protective group, preferably a protective group selected from the group comprising tert-butyloxycarbonyl, benzyl, carbobenzoxy and 9-fluorenylmethyloxycarbonyl, possibly in a reaction medium, possibly in the presence of at least one base and/or at least one organometallic compound and/or at least one metallic hydride reagent, preferably at a temperature of from -70 °C to 300 °C to produce at least one corresponding compound of the general formula VI, 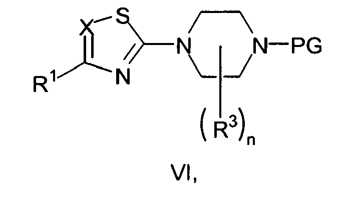 in which R¹, R³, X, n and PG have the meanings stated above and the resulting product is possibly purified and/or isolated;
or at least one compound of the general formula VII, 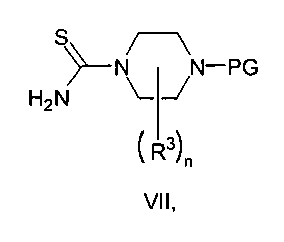 in which R³ and n have the meanings defined in one or more of claims 1 to 12 and PG stands for a protective group, preferably a protective group selected from the group comprising tert-butyloxycarbonyl, benzyl, carbobenzoxy and 9-fluorenylmethyloxycarbonyl, is caused to react with at least one compound of the general formula R'-C(=O)-CH₂-A or (C₁₋₅ alkyl-O)₂-CH-CH₂-A, in which R¹ has the meaning defined in one or more of claims 1 to 12 and A stands for a leaving group, preferably for a halogen radical, more preferred for a bromine atom, in a reaction medium, possibly in the presence of at least one organic base or in the presence of at least one acid, preferably in the presence of at least one base selected from the group comprising triethylamine, diisopropylethylamine, N-methylmorpholine, dimethylaminopyridine and pyridine or in the presence of at least one acid selected from the group comprising acetic acid, trifluoroacetic acid and hydrochloric acid, preferably at a temperature between -70 °C and 300 °C, to form at least one corresponding compound of the general formula VI, possibly in the form of an appropriate salt in which R¹, R³ and n have the meanings defined in one or more of claims 1 to 12, PG has the meaning stated above and X stands for CH and the said product is possibly purified and/or isolated;
and at least one compound of the general formula VI, when PG stands for a tert-butoxycarbonyl or 9-fluorenylmethyloxycarbonyl group, in a reaction medium, in the presence of at least one acid, preferably in the presence of at least one acid selected from the group comprising hydrochloric acid and trifluoroacetic acid, preferably at a temperature between -70 °C and 100 °C or, when PG stands for a benzyl group or benzyloxycarbonyl group, in a reaction medium, in the presence of hydrogen and in the presence of at least one catalyst, preferably in the presence of palladium on carbon, preferably at a temperature between -70 °C and 100 °C is converted to at least one corresponding compound of the general formula IV, possibly in the form of an appropriate salt thereof, in which X, R¹, R³ and n have the meanings defined in one or more of claims 1 to 12 and the resulting product is possibly purified and/or isolated;
and at least one compound of the general formula IV is converted with at least one compound of the general formula R⁴-C≡C-C(=O)-OH, in which R⁴ has the meaning defined in one or more of claims 1 to 12, in a reaction medium, possibly in the presence of at least one suitable coupling agent, possibly in the presence of at least one base, preferably at a temperature of from -70 °C to 100 °C, or is converted with at least one compound of the general formula R⁴-C≡C-C(=O)-A, in which R⁴ has the meaning stated above and A stands for a leaving group, preferably for a halogen radical, more preferred a chlorine or bromine atom, in a reaction medium, possibly in the presence of at least one base, preferably at a temperature of from -70 °C to 100 °C, into at least one corresponding compound of the general formula I, possibly in the form of an appropriate salt thereof, 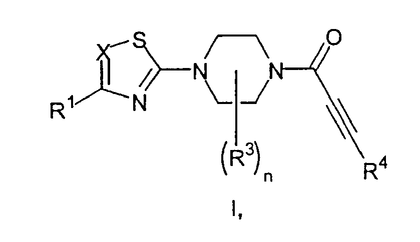 in which X and R¹, R³, R⁴ and n have the meanings stated above and the resulting product is possibly purified and/or isolated;
or at least one compound of the general formula IV is converted with propynoic acid [HC≡C-C(=O)-OH] in a reaction medium, possibly in the presence of at least one suitable coupling agent, possibly in the presence of at least one base, preferably at a temperature of from -70 °C to 100 °C, or is converted with at least one compound of the general formula HC≡C-C(=O)-A in which A stands for a leaving group, preferably for a halogen radical, more preferred a chlorine or bromine radical, in a reaction medium, possibly in the presence of at least one base, preferably at a temperature of from -70 °C to 100 °C, into at least one corresponding compound of the general formula VIII, possibly in the form of an appropriate salt thereof, 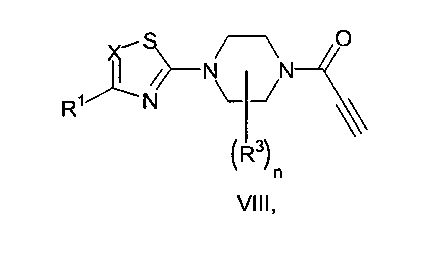 in which R¹, R³, X and n have the meanings stated above and the resulting product is possibly purified and/or isolated;
and at least one compound of the general formula VIII is converted with at least one compound of the general formula R⁴-A in which R⁴ has the meaning defined in one or more of claims 10 to 13 with the exception of hydrogen and A stands for a leaving group, preferably for a halogen radical or a sulphonic acid ester, particularly preferred for iodine, bromine or triflate, in a reaction medium, possibly in the presence of at least one catalyst, preferably in the presence of a palladium catalyst selected from the group comprising palladium chloride [PdCl₂], palladium acetate [Pd(OAc)₂], tetrakis(triphenylphosphine)palladium [Pd(PPh₃)₄], bis(triphenylphosphine)palladium dichloride [Pd(PPh₃)₂Cl₂] and bis(triphenylphosphine)palladium acetate [Pd(PPh₃)₂(OAc)₂], possibly in the presence of at least one ligand, preferably in the presence of at least one ligand selected from the group comprising triphenylphosphine, triphenylarsine and tri-2-furylphosphine, possibly in the presence of at least one inorganic salt, preferably in the presence of at least one inorganic salt selected from the group comprising lithium chloride and zinc chloride, possibly in the presence of at least one copper salt, preferably in the presence of copper iodide, possibly in the presence of at least one organic or inorganic base, preferably in the presence of at least one base selected from the group comprising triethylamine, [1,4]-diazabicyclo-[2,2,2]-octane, diisopropylamine, diisopropylethylamine, potassium carbonate and sodium hydrogencarbonate, preferably at a temperature between -70 °C and 300 °C, into at least one corresponding compound of the general formula I, possibly in the form of an appropriate salt thereof and the resulting product is possibly purified and/or isolated.

14. A process for the preparation of compounds of the general formula I as defined in one or more of claims 1 to 12, **characterised in that**
at least one compound of the general formula III, 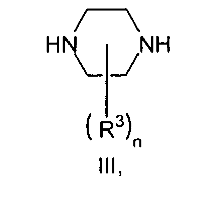 in which R³ and n have the meanings defined in one or more of claims 1 to 12, is converted with at least one compound of the general formula R⁴-C≡C-C(=O)-OH, in which R⁴ has the meaning stated above, in a reaction medium, possibly in the presence of at least one suitable coupling agent, possibly in the presence of at least one base, preferably at a temperature ranging from -70 °C to 100 °c, or is converted with at least one compound of the general formula R⁴-C≡C-C(=O)-A, in which R⁴ has the meaning stated above and A stands for a leaving group, preferably for a halogen radical, more preferred a chlorine or bromine radical, in a reaction medium, possibly in the presence of at least one base, preferably at a temperature ranging from -70 °C to 100 °C, into at least one corresponding compound of the general formula IX, possibly in the form of 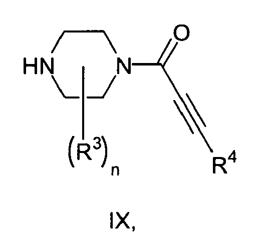 an appropriate salt thereof,
in which R³, R⁴ and n have the meanings stated above and the resulting product is possibly purified and/or isolated;
or at least one compound of the general formula V, 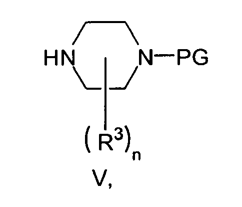 in which R³ and n have the meanings defined in one or more of claims 1 to 12 and PG stands for a protective group, preferably a protective group selected from the group comprising tert-butyloxycarbonyl, benzyl, carbobenzoxy and 9-fluorenylmethyloxycarbonyl, is converted with at least one compound of the general formula R⁴-C≡C-C(=O)-OH, in which R⁴ has the meaning defined in one or more of claims 1 to 12, in a reaction medium, possibly in the presence of at least one suitable coupling agent, possibly in the presence of at least one base, preferably at a temperature ranging from -70 °C to 100 °C, or is converted with at least one compound of the general formula R⁴-C≡C-C(=O)-A, in which R⁴ has the meaning stated above and A stands for a leaving group, preferably for a halogen radical, more preferred a chlorine or bromine radical, in a reaction medium, possibly in the presence of at least one base, preferably at a temperature ranging from -70 °C to 100 °C, into at least one corresponding compound of the general formula XI, possibly in the form of an appropriate salt thereof, 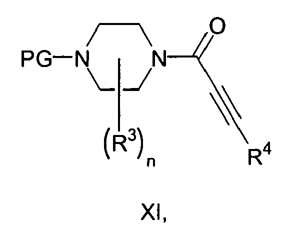 in which R³, R⁴ and n and PG have the meanings stated above and the resulting product is possibly purified and/or isolated;
and at least one compound of the general formula XI, when PG stands for a tert-butoxycarbonyl group or 9-fluorenylmethyloxycarbonyl group, in a reaction medium, in the presence of at least one acid, preferably in the presence of at least one acid selected from the group comprising hydrochloric acid and trifluoroacetic acid, preferably at a temperature ranging from -70 °C to 100 °C or, when PG stands for a benzyl group or a benzyloxycarbonyl group, in a reaction medium, in the presence of hydrogen and in the presence of at least one catalyst, preferably in the presence of palladium on carbon, preferably at a temperature ranging from -70 °C to 100 °C , into at least one corresponding compound of the general formula IX, possibly in the form of an appropriate salt thereof, in which R¹, R³ and n have the meanings stated above and the resulting product is possibly purified and/or isolated;
and at least one compound of the general formula IX is converted with at least one compound of the general formula II, 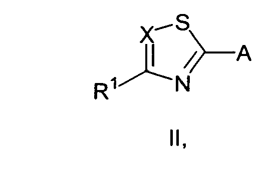 in which the radicals X and R¹ have the meanings defined in one or more of claims 1 to 12 and A stands for a leaving group, preferably for a halogen radical or a sulphonic acid ester, more preferred for a chlorine radical or bromine radical, in a reaction medium, possibly in the presence of at least one base and/or at least one organometallic compound and/or at least one metallic hydride reagent, preferably at a temperature ranging from -70 °C to 300 °C, into at least one corresponding compound of the general formula I, possibly in the form of an appropriate salt thereof and the resulting product is possibly purified and/or isolated.

15. A medication comprising at least one compound, as defined in one or more of claims 1 to 12, and possibly one or more physiologically acceptable adjuvants.

16. Medication as defined in claim 15 for the treatment and/or prophylaxis of pain, preferably pain selected from the group comprising acute pain, chronic pain, neuropathic pain and visceral pain; migraine; depressions; neurodegenerative diseases, preferably selected from the group comprising multiple sclerosis, Alzheimer's disease, Parkinson's disease and Huntington's disease; cognitive diseases, preferably cognitive deficiencies, particularly preferred attention deficit syndrome (ADS); anxiety conditions; panic attacks; epilepsy; coughing; urinary incontinence; diarrhoea; pruritus; schizophrenia; cerebral ischemias; muscle spasms; cramps; eating disorders, preferably selected from the group comprising bulimia, cachexia, anorexia and obesity; alcohol dependency; medication dependency; drug dependency, preferably nicotine and/or cocaine dependency; alcohol abuse; medication abuse; drug abuse; preferably nicotine and/or cocaine abuse; withdrawal symptoms following alcohol dependency, medication dependency and/or drug (in particular nicotine and/or cocaine) dependency; development of tolerance to medications, preferably to natural and synthetic opioids; gastro-oesophageal reflux syndrome; for diuresis; for antinatriuresis; influencing the cardiovascular system; increasing vigilance; increasing libido; modulating motive activity, or for local anesthesia.

17. Use of at least one compound as defined in one or more of claims 1 to 12 for the preparation of a medication for the treatment and/or prophylaxis of pain, preferably pain selected from the group comprising acute pain, chronic pain, neuropathic pain and visceral pain; migraine; depressions; neurodegenerative diseases, preferably selected from the group comprising multiple sclerosis, Alzheimer's disease, Parkinson's disease and Huntington's disease; cognitive diseases, preferably cognitive deficiencies, particularly preferred attention deficit syndrome (ADS); anxiety conditions; panic attacks; epilepsy; coughing; urinary incontinence; diarrhoea; pruritus; schizophrenia; cerebral ischemias; muscle spasms; cramps; eating disorders, preferably selected from the group comprising bulimia, cachexia, anorexia and obesity; alcohol dependency; medication dependency; drug dependency, preferably nicotine and/or cocaine dependency; alcohol abuse; medication abuse; drug abuse; preferably nicotine and/or cocaine abuse; withdrawal symptoms following alcohol dependency, medication dependency and/or drug (in particular nicotine and/or cocaine) dependency; development of tolerance to medications, particularly to natural and synthetic opioids; gastro-oesophageal reflux syndrome; for diuresis; for antinatriuresis; influencing the cardiovascular system; increasing vigilance; increasing libido; modulating motive activity, or for local anesthesia.

18. Use as defined in claim 17 for the preparation of a medication for the treatment and/or prophylaxis of pain, preferably of pain selected from the group comprising acute pain, chronic pain, neuropathic pain and visceral pain; depressions; Parkinson's disease; anxiety conditions; panic attacks; epilepsy; alcohol dependency; medication dependency; drug dependency, preferably nicotine and/or cocaine dependency; alcohol abuse; medication abuse; drug abuse; preferably nicotine and/or cocaine abuse; withdrawal symptoms following alcohol, medication dependency and/or drug (in particular nicotine and/or cocaine) dependency; development of tolerance to medications and/or to drugs, in particular to natural and synthetic opioids, or for local anesthesia.

19. Use as defined in claim 17 or 18 for the preparation of a medication for the treatment of pain, preferably of pain selected from the group comprising acute pain, chronic pain, neuropathic pain or visceral pain, anxiety conditions and panic attacks.

## Revendications

1. 1-propiolyl-pipérazines substituées de formule générale I, 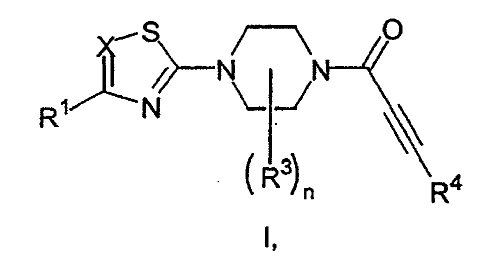 dans laquelle
X est N ou C-R²,
R¹ et R² représentent chacun indépendamment de l'autre un atome d'hydrogène, un radical halogéno, un groupe nitro, un groupe cyano, un groupe amino, un groupe hydroxy, un groupe thiol, un groupe carboxy, un groupe formyle, un groupe -NH-C(=O)-H, un groupe -NH-R⁵, un groupe -NR⁶R⁷, un groupe -C(=O)-R⁸, un groupe -C(=O)-O-R⁹, un groupe -O-C(=O)-R¹⁰, un groupe -NH-C(=O)-R¹¹, un groupe -NR¹²-C(=O)-R¹³, un groupe -C(=O)-NH₂, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰, un groupe -NH-C(=O)-NH-R²¹, un groupe -NH-C(=S)-NH-R²², un groupe -NH-S(=O)₂-R²³, un groupe -NR²⁴-S(=O)₂-R²⁵, un radical aliphatique à chaîne droite ou ramifiée, saturé ou insaturé, non substitué ou au moins monosubstitué, comportant éventuellement au moins un hétéroatome en tant que chaînon de la chaîne aliphatique, un radical cycloaliphatique non substitué ou au moins monosubstitué, insaturé ou saturé, comportant éventuellement au moins un hétéroatome en tant que chaînon du cycle, radical qui peut être lié par l'intermédiaire d'un groupe alkylène, alcénylène ou alcynylène, à chaîne droite ou ramifiée, non substitué ou au moins monosubstitué, comportant éventuellement au moins un hétéroatome en tant que chaînon de la chaîne aliphatique, et/ou pouvant être condensé à au moins un système cyclique monocyclique ou polycyclique, non substitué ou au moins monosubstitué, ou un radical aryle ou hétéroaryle non substitué ou au moins monosubstitué, qui peut être lié par l'intermédiaire d'un groupe alkylène, alcénylène ou alcynylène à chaîne droite ou ramifiée, non substitué ou au moins monosubstitué, comportant éventuellement au moins un hétéroatome en tant que chaînon de la chaîne aliphatique et/ou pouvant être condensé à un système cyclique monocyclique ou polycyclique non substitué ou au moins monosubstitué,
R³ est un radical halogéno, un groupe nitro, un groupe cyano, un groupe amino, un groupe hydroxy, un groupe thiol, un groupe carboxy, un groupe formyle, un groupe -NH-C(=O)-H, un groupe oxo (=O), un groupe -NH-R⁵, un groupe -NR⁶R⁷, un groupe -C(=O)-R⁸, un groupe -C(=O)-O-R⁹, un groupe -O-C(=O)-R¹⁰, un groupe -NH-C(=O)-R¹¹, un groupe -NR¹²-C(=O)-R¹³, un groupe -C(=O)-NH₂, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰, un groupe -NH-C(=O)-NH-R²¹, un groupe -NH-C(=S)-NH-R²², un groupe -NH-S(=O)2-R²³, un groupe -NR²⁴-S(=O)₂-R²⁵, un radical aliphatique à chaîne droite ou ramifiée, saturé ou insaturé, non substitué ou au moins monosubstitué, comportant éventuellement au moins un hétéroatome en tant que chaînon de la chaîne aliphatique, un radical cycloaliphatique non substitué ou au moins monosubstitué, insaturé ou saturé, comportant éventuellement au moins un hétéroatome en tant que chaînon du cycle, radical qui peut être lié par l'intermédiaire d'un groupe alkylène, alcénylène ou alcynylène à chaîne droite ou ramifiée, non substitué ou au moins monosubstitué, comportant éventuellement au moins un hétéroatome en tant que chaînon de la chaîne aliphatique, et/ou pouvant être condensé à un système cyclique monocyclique ou polycyclique non substitué ou au moins monosubstitué, ou un radical aryle ou hétéroaryle non substitué ou au moins monosubstitué, qui peut être lié par l'intermédiaire d'un groupe alkylène, alcénylène ou alcynylène à chaîne droite ou ramifiée, non substitué ou au moins monosubstitué, comportant éventuellement au moins un hétéroatome en tant que chaînon de la chaîne aliphatique et/ou pouvant être condensé à un système cyclique monocyclique ou polycyclique non substitué ou au moins monosubstitué, R⁴ est un atome d'hydrogène, un atome de fluor, un atome de chlore, un atome de brome, un groupe nitro, un groupe cyano, un groupe amino, un groupe hydroxy, un groupe thiol, un groupe carboxy, un groupe formyle, un groupe -NH-C(=O)-H, un groupe -NH-R⁵, un groupe -NR⁶R⁷, un groupe -C(=O)-R⁸, un groupe -C(=O)-O-R⁹, un groupe -O-C(=O)-R¹⁰, un groupe -NH-C(=O)-R¹¹, un groupe -NR¹²-C(=O)-R¹³, un groupe -C(=O)-NH₂, un groupe -C(=O)-NH-R¹⁴ un groupe -C(=O)-NR¹⁵R¹⁶, un groupe -O-R¹⁷, un groupe -S-R¹⁸, un groupe -S(=O)-R¹⁹, un groupe -S(=O)₂-R²⁰, un groupe -NH-C (=O)-NH-R²¹, un groupe -NH-C (=S)-NH-R²², un groupe -NH-S(=O)₂-R²³, un groupe -NR²⁴-S(=O)₂-R²⁵ un radical aliphatique à chaîne droite ou ramifiée, saturé ou insaturé, non substitué ou au moins monosubstitué, comportant éventuellement au moins un hétéroatome en tant que chaînon de la chaîne aliphatique, un radical cycloaliphatique non substitué ou au moins monosubstitué, insaturé ou saturé, comportant éventuellement au moins un hétéroatome en tant que chaînon du cycle, radical qui peut être lié par l'intermédiaire d'un groupe alkylène, alcénylène ou alcynylène à chaîne droite ou ramifiée, non substitué ou au moins monosubstitué, comportant éventuellement au moins un hétéroatome en tant que chaînon de la chaîne aliphatique, et/ou pouvant être condensé à un système cyclique monocyclique ou polycyclique non substitué ou au moins monosubstitué, ou un radical hétéroaryle, qui peut être lié par l'intermédiaire d'un groupe alkylène, alcénylène ou alcynylène à chaîne droite ou ramifiée, non substitué ou au moins monosubstitué, comportant éventuellement au moins un hétéroatome en tant que chaînon de la chaîne aliphatique, et/ou pouvant être condensé à un système cyclique monocyclique ou polycyclique non substitué ou au moins monosubstitué,
R⁵ à R²⁵ représentent chacun indépendamment de l'autre un radical aliphatique à chaîne droite ou ramifiée, saturé ou insaturé, non substitué ou au moins monosubstitué, comportant éventuellement au moins un hétéroatome en tant que chaînon de la chaîne aliphatique, un radical cycloaliphatique non substitué ou au moins monosubstitué, insaturé ou saturé, comportant éventuellement au moins un hétéroatome en tant que chaînon du cycle, radical qui peut être lié par l'intermédiaire d'un groupe alkylène, alcénylène ou alcynylène, à chaîne droite ou ramifiée, non substitué ou au moins monosubstitué, comportant éventuellement au moins un hétéroatome en tant que chaînon de la chaîne aliphatique et/ou pouvant être condensé à un système cyclique monocyclique ou polycyclique non substitué ou au moins monosubstitué, ou un radical aryle ou hétéroaryle non substitué ou au moins monosubstitué, qui peut être lié par l'intermédiaire d'un groupe alkylène, alcénylène ou alcynylène, à chaîne droite ou ramifiée, non substitué ou au moins monosubstitué, comportant éventuellement au moins un hétéroatome en tant que chaînon de la chaîne aliphatique,
et n vaut 0, 1, 2, 3, 4, 5, 6, 7 ou 8,
dans chaque cas éventuellement sous forme d'un de leurs stéréoisomères purs, en particulier de leurs énantiomères ou diastéréoisomères, de leurs racémates,
ou sous forme d'un mélange de stéréoisomères, en particulier des énantiomères et/ou des diastéréoisomères, selon un rapport de mélange quelconque, ou dans chaque cas sous forme des sels correspondants, de préférence sous forme des chlorhydrates correspondants, ou dans chaque cas sous forme des produits de solvatation correspondants.

2. Composés selon la revendication 1, **caractérisés en ce que**
X est N ou C-R²
n vaut 0, 1, 2, 3, 4, 5, 6, 7 ou 8, R¹ et R² représentent chacun indépendamment de l'autre un atome d'hydrogène, un radical halogéno, un groupe nitro, un groupe cyano, un groupe amino, un groupe hydroxy, un groupe thiol, un groupe carboxy, un groupe formyle, un groupe -NH-C(=O)-H, un groupe -NH-R⁵ un groupe -NR⁶R⁷, un groupe -C(=O)-R⁸, un groupe -C(=O)-O-R⁹, un groupe -O-C(=O)-R¹⁰, un groupe -NH-C(=O)-R¹¹, un groupe -NR¹²-C(=O)-R¹³, un groupe -C(=O)-NH₂, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰, un groupe -NH-C(=O)-NH-R²¹, un groupe -NH-C(=S)-NH-R²², un groupe -NH-S(=O)₂-R²³, un groupe -NR²⁴-S(=O)₂-R²⁵, un radical aliphatique en C₁-C₈ à chaîne droite ou ramifiée, saturé ou insaturé, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote, un radical cycloaliphatique à 3, 4, 5, 6, 7, 8 ou 9 chaînons, non substitué ou au moins monosubstitué, insaturé ou saturé, comportant éventuellement un hétéroatome en tant que chaînon du cycle, radical qui peut être lié par l'intermédiaire d'un groupe alkylène en C₁₋₅, alcénylène en C₂₋₅ ou alcynylène en C₂₋₅, à chaîne droite ou ramifiée, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, l'azote et le soufre et/ou pouvant être condensés à un système cyclique monocyclique ou polycyclique non substitué ou au moins monosubstitué, ou un radical aryle ou hétéroaryle à 5 à 14 chaînons, non substitué ou au moins monosubstitué, qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon (s) de la chaîne aliphatique éventuellement 1, 2 ou 3 hétéroatomes choisis indépendamment les uns des autres dans le groupe consistant en l'oxygène, le soufre et l'azote, et/ou pouvant être condensé à un système cyclique monocyclique ou polycyclique non substitué ou au moins monosubstitué,
R³ est un radical halogéno, un groupe nitro, un groupe cyano, un groupe amino, un groupe hydroxy, un groupe thiol, un groupe carboxy, un groupe formyle, un groupe -NH-C(=O)-H, un groupe oxo (=O), un groupe -NH-R⁵, un groupe -NR⁶R⁷, un groupe -C(=O)-R⁸, un groupe -C(=O)-O-R⁹, un groupe -O-C(=O)-R¹⁰, un groupe -NH-C(=O)-R¹¹, un groupe -NR¹²-C(=O)-R¹³, un groupe -C(=O)-NH₂, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰, un groupe -NH-C(=O)-NH-R²¹, un groupe -NH-C(=S)-NH-R²², un groupe -NH-S(=O)₂-R²³, un groupe -NR²⁴-S(=O)₂-R²⁵, un radical aliphatique en C₁₋₈ à chaîne droite ou ramifiée, saturé ou insaturé, non substitué ou au moins monosubstitué, comportant en tant que chaînon(s) éventuellement 1, 2 ou 3 hétéroatomes choisis indépendamment les uns des autres dans le groupe consistant en l'oxygène, le soufre et l'azote, un radical cycloaliphatique à 3, 4, 5, 6, 7, 8 ou 9 chaînons, non substitué ou au moins monosubstitué, insaturé ou saturé, comportant éventuellement au moins un hétéroatome en tant que chaînon du cycle, radical qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote, et/ou pouvant être condensé à un système cyclique monocyclique ou polycyclique non substitué ou au moins monosubstitué, ou un radical aryle ou hétéroaryle à 5 à 14 chaînons, non substitué ou au moins monosubstitué, qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) éventuellement 1, 2 ou 3 hétéroatomes choisis indépendamment les uns des autres dans le groupe consistant en l'oxygène, le soufre et l'azote, et/ou pouvant être condensé à un système cyclique monocyclique ou polycyclique non substitué ou au moins monosubstitué,
R⁴ est un atome d'hydrogène, un groupe -C(=O)-NH₂, un groupe -C(=O)-NH-R¹⁴, un groupe -C(=O)-NR¹⁵R¹⁶, un radical aliphatique en C₁₋₈ à chaîne droite ou ramifiée, saturé ou insaturé, non substitué ou au moins monosubstitué, un radical cycloaliphatique à 3, 4, 5, 6, 7, 8 ou 9 chaînons, non substitué ou au moins monosubstitué, insaturé ou saturé, comportant éventuellement au moins un hétéroatome en tant que chaînon du cycle, radical qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon (s) de la chaîne aliphatique éventuellement 1, 2 ou 3 hétéroatomes choisis indépendamment les uns des autres dans le groupe consistant en l'oxygène, le soufre et l'azote, et/ou pouvant être condensé à un système cyclique monocyclique ou polycyclique non substitué ou au moins monosubstitué, ou un radicale aryle ou hétéroaryle à 5 à 14 chaînons, non substitué ou au moins monosubstitué, qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) éventuellement 1, 2 ou 3 hétéroatomes choisis indépendamment les uns des autres dans le groupe consistant en l'oxygène, le soufre et l'azote, et/ou pouvant être condensé à un système cyclique monocyclique ou polycyclique non substitué ou au moins monosubstitué,
et
R⁵ à R²⁵ représentent chacun indépendamment des autres un radical aliphatique en C₁₋₈ à chaîne droite ou ramifiée, saturé ou insaturé, non substitué ou au moins monosubstitué, un radical cycloaliphatique à 3, 4, 5, 6, 7, 8 ou 9 chaînons, non substitué ou au moins monosubstitué, insaturé ou saturé, comportant au moins un hétéroatome en tant que chaînon du cycle, radical qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) éventuellement 1, 2 ou 3 hétéroatomes choisis indépendamment les uns des autres dans le groupe consistant en l'oxygène, le soufre et l'azote, et/ou pouvant être condensé à un système cyclique monocyclique ou polycyclique non substitué ou au moins monosubstitué, ou un groupe aryle ou hétéroaryle à 5 à 14 chaînons, non substitué ou au moins monosubstitué, qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) éventuellement 1, 2 ou 3 hétéroatomes choisis indépendamment les uns des autres dans le groupe consistant en l'oxygène, le soufre et l'azote, et/ou pouvant être condensé à un système cyclique monocyclique ou polycyclique non substitué ou au moins monosubstitué,
les radicaux cycloaliphatique mentionnés ci-dessus pouvant éventuellement comporter 1, 2, 3, 4 ou 5 hétéroatome(s) en tant que chaînon(s), dont chacun peut indépendamment des autres être choisi dans le groupe consistant en l'azote, l'oxygène et le soufre,
les noyaux des systèmes cycliques monocycliques ou bicycliques mentionnés ci-dessus ayant chacun 4, 5 ou 6 chaînons, et comportant chacun éventuellement 0, 1, 2, 3, 4 ou 5 hétéroatome(s) en tant que chaînon(s), qui sont choisis indépendamment les uns des autres dans le groupe consistant en l'oxygène, l'azote et le soufre ;
et
les radicaux hétéroaryle mentionnés ci-dessus pouvant éventuellement comporter 1, 2, 3, 4 ou 5 hétéroatome(s) en tant que chaînon(s) ;
dans chaque cas éventuellement sous forme d'un de leurs stéréoisomères purs, en particulier de leurs énantiomères ou diastéréoisomères, de leurs racémates,
ou sous forme d'un mélange de stéréoisomères, en particulier des énantiomères et/ou des diastéréoisomères, selon un rapport de mélange quelconque, ou dans chaque cas sous forme des sels correspondants, de préférence sous forme des chlorhydrates correspondants, ou dans chaque cas sous forme des produits de solvatation correspondants.

3. Composés selon la revendication 1 ou 2, **caractérisés en ce que** R¹ est un atome d'hydrogène, un radical halogéno, un groupe nitro, un groupe cyano, un groupe amino, un groupe hydroxy, un groupe thiol, un groupe carboxy, un groupe formyle, un groupe -NH-C(=O)-H, un groupe -NH-R⁵, un groupe -NR⁶R⁷, un groupe -C(=O)-R⁸ un groupe -C(=O)-O-R⁹, un groupe -O-C(=O)-R¹⁰, un groupe -NH-C(=O)-R¹¹, un groupe -NR¹²-C(=O)-R¹³, un groupe -C(=O)-NH₂, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰, un groupe -NH-C(=O)-NH-R²¹, un groupe -NH-C(=S)-NH-R²², un groupe -NH-S (=O)₂-R²¹, un groupe -NR²⁴-S(O)₂-R²⁵, un radical aliphatique en C₁₋₈, à chaîne droite ou ramifiée, saturé ou insaturé, non substitué ou au moins monosubstitué, comportant en tant que chaînon (s) de la chaîne aliphatique éventuellement 1, 2 ou 3 hétéroatomes choisis indépendamment les uns des autres dans le groupe consistant en l'oxygène, le soufre et l'azote, un radical cycloaliphatique à 3, 4, 5, 6, 7, 8 ou 9 chaînons, non substitué ou au moins monosubstitué, non saturé ou saturé, comportant éventuellement au moins un hétéroatome en tant que chaînon du cycle, radical qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote, et/ou pouvant être condensé à un système cyclique monocyclique ou polycyclique non substitué ou éventuellement substitué, ou un radical aryle ou hétéroaryle à 5 à 14 chaînons, non substitué ou au moins monosubstitué, qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote, et/ou peut être condensé à un système cyclique monocyclique ou polycyclique non substitué ou au moins monosubstitué,
de préférence un atome d'hydrogène, un radical halogéno choisi dans le groupe consistant en F, Cl, Br et I, un groupe nitro, un groupe CF₃, un groupe cyano, un groupe amino, un groupe hydroxy, un groupe thiol, un groupe carboxy, un groupe -NH-R⁵, un groupe -NR⁶R⁷, un groupe -C(=O)-R⁸, un groupe -C(=O)-O-R⁹, un groupe -O-C(=O)-R¹⁰, un groupe -C(=O)-NH₂, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂R²⁰, un radical alkyle en C₁₋₈ à chaîne droite ou ramifiée, un radical alcényle en C₂₋₈ à chaîne droite ou ramifiée, un radical alcynyle en C₂₋₈ à chaîne droite ou ramifiée, un radical cycloaliphatique à 3, 4, 5, 6 ou 7 chaînons, non substitué ou au moins monosubstitué, insaturé ou saturé, comportant en tant que chaînon(s) du cycle éventuellement 1, 2 ou 3 hétéroatomes choisis indépendamment les uns des autres dans le groupe consistant en l'oxygène, le soufre et l'azote, radical qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, ou un radical aryle ou hétéroaryle à 5 à 14 chaînons, non substitué ou au moins monosubstitué, qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué,
d'une manière particulièrement préférée un atome d'hydrogène, un radical halogéno choisi dans le groupe consistant en F, Cl et Br, un groupe CF₃, un groupe nitro, un groupe cyano, un groupe amino, un groupe hydroxy, un groupe thiol, un groupe carboxy, un groupe -NH-R⁵ un groupe -NR⁶R⁷, un groupe -C(=O)-R⁸, un groupe -C-(=O)-O-R⁹, un groupe -O-C(=O)-R¹⁰, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰ un radical alkyle en C₁₋₄ à chaîne droite ou ramifiée, un radical alcényle en C₂₋₄ à chaîne droite ou ramifiée, un radical alcynyle en C₂₋₄ à chaîne droite ou ramifiée, ou un radical choisi dans le groupe consistant en les groupes cyclopropyle, cyclobutyle, cyclopentyle, cyclohexyle, pyrrolidinyle, tétrahydrofurannyle, tétrahydrothiophényle, pipéridinyle, tétrahydropyrannyle, pipérazinyle, morpholinyle, thiomorpholinyle, dioxolannyle, azépannyle, diazépannyle, imidazolidinyle, phényle, naphtyle, anthracényle, pyrrolyle, indolyle, furannyle, benzo[b]furannyle, thiophényle, benzo[b]thiophényle, pyrazolyle, imidazolyle, thiazolyle, dithiazolyle, thiadiazolyle, triazolyle, oxazolyle, oxadiazolyle, isoxazolyle, pyridinyle, pyridazinyle, pyrimidinyle, pyrazinyle, pyrannyle, indazolyle, purinyle, indolizinyle, quinoléinyle, isoquinoléinyle et quinazolinyle, dont chacun peut être lié par l'intermédiaire d'un groupe alkylène en C₁₋₃, alcénylène en C₂₋₃ ou alcynylène en C₂₋₃ et/ou est non substitué, ou est substitué par éventuellement 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, -OH, -O-CH₃, -O-C₂H₅, -O-C₃H₇, -NH₂, -N(CH₃)₂, -N(C₂H₅)₂, -NH-CH₃, -NH-C₂H₅, -NO₂, -CF₃, -O-CF₃, -S-CF₃, -SH, -NH-S(=O)₂-CH₃, -C(=O)-OH, -C(=O)-CH₃, -C(=O)-C₂H₅, -C(=O)-N(CH₃)₂, -C(=O)-NH-CH₃, -NH-C(=O)-CH₃, -NH-C(=O)-C₂H₅, -C(=O)-O-CH₃ et -C(=O)-O-C₂H₅,
d'une manière tout particulièrement préférée un atome d'hydrogène, un radical halogéno choisi dans le groupe consistant en F, Cl et Br, un groupe CF₃, un groupe nitro, un groupe cyano, un groupe amino, un groupe diméthylamino, un groupe diéthylamino, un groupe monométhylamino, un groupe monoéthylamino, un groupe hydroxy, un groupe thiol, un groupe carboxy, un groupe -C(=O)-O-CH₃, un groupe -C(=O)-O-C₂H₅, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰, un radical alkyle choisi dans le groupe consistant en les groupes méthyle, éthyle, n-propyle, isopropyle, n-butyle, sec-butyle, isobutyle et tert-butyle, un radical alcényle choisi dans le groupe consistant en les groupes éthényle, 1-propényle, 2-propényle, 1-butényle, 2-butényle et 3-butényle, un radical alcynyle choisi dans le groupe consistant en les groupes éthynyle, 1-propynyle, 2-propynyle, 1-butynyle, 2-butynyle et 3-butynyle, ou un radical choisi dans le groupe consistant en les groupes (1,3)-dioxolann-2-yle, phényle, benzyle, phénéthyle, oxadiazolyle, 2-pyridyle, 3-pyridyle, 4-pyridyle, 2-thiényle, 3-thiényle, 2-furyle et 3-furyle, dont chacun est non substitué ou est substitué par éventuellement 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, -OH, -O-CH₃, -O-C₂H₅ et -O-C₃H₇.

4. Composés selon l'une ou plusieurs des revendications 1 à 3, **caractérisés en ce que** R² est un atome d'hydrogène, un radical halogéno, un groupe nitro, un groupe cyano, un groupe amino, un groupe hydroxy, un groupe thiol, un groupe carboxy, un groupe formyle, un groupe -NH-C(=O)-H, un groupe -NH-R⁵ un groupe -NR⁶R⁷ un groupe -C(=O)-R⁸, un groupe -C(=O)-O-R⁹, un groupe -O-C(=O)-R¹⁰, un groupe -NH-C(=O)-R¹¹, un groupe -NR¹²-C(=O)-R¹³, un groupe -C(=O)-NH₂, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰, un groupe -NH-C(=O)-NH-R²¹, un groupe -NH-C(=S)-NH-R²², un groupe -NH-S(=O)₂-R²³, un groupe -NR²⁴-S(=O)₂-R²⁵, un radical aliphatique en C₁₋₈ à chaîne droite ou ramifiée, saturé ou insaturé, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote, un radical cycloaliphatique à 3, 4, 5, 6, 7, 8 ou 9 chaînons, non substitué ou au moins monosubstitué, insaturé ou saturé, comportant éventuellement au moins un hétéroatome en tant que chaînon du cycle, radical qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) éventuellement 1, 2 ou 3 hétéroatomes choisis indépendamment les uns des autres dans le groupe consistant en l'oxygène, le soufre et l'azote, et/ou pouvant être condensé à un système cyclique monocyclique ou polycyclique non substitué ou au moins monosubstitué, ou un radical aryle ou hétéroaryle à 5 à 14 chaînons, non substitué ou au moins monosubstitué, qui peut être lié par l'intermédiaire d'un groupe alkylène en C₁₋₅, alcénylène en C₂₋₅ ou alcynylène en C₂₋₅, à chaîne droite ou ramifiée, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote, et/ou peut être condensé à un système cyclique monocyclique ou polycyclique non substitué ou au moins monosubstitué,
de préférence un atome d'hydrogène, un radical halogéno choisi dans le groupe consistant en F, Cl, Br et I, un groupe nitro, un groupe CF₃, un groupe cyano, un groupe amino, un groupe hydroxy, un groupe thiol, un groupe carboxy, un groupe -NH-R⁵, un groupe NR⁶R⁷, un groupe -C(=O)-R⁸, un groupe -C(=O)-O-R⁹, un groupe -O-C(=O)R¹⁰, un groupe -C(=O)-NH₂, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰, un radical alkyle en C₁₋₈ à chaîne droite ou ramifiée, un radical alcényle en C₂₋₈ à chaîne droite ou ramifiée ou un radical alcynyle en C₂₋₈ à chaîne droite ou ramifiée, un radical cycloaliphatique à 3, 4, 5, 6 ou 7 chaînons, non substitué ou au moins monosubstitué, insaturé ou saturé, comportant en tant que chaînon(s) du cycle éventuellement 1, 2 ou 3 hétéroatomes choisis indépendamment les uns des autres dans le groupe consistant en l'oxygène, le soufre et l'azote, radical qui peut être lié par l'intermédiaire d'un groupe alkylène en C₁₋₅, alcénylène en C₂₋₅ ou alcynylène en C₂₋₅, à chaîne droite ou ramifiée, non substitué ou au moins monosubstitué, ou un radical aryle ou hétéroaryle à 5 à 14 chaînons non substitué ou au moins monosubstitué, qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué,
d'une manière particulièrement préférée un atome d'hydrogène, un radical halogéno choisi dans le groupe consistant en F, Cl et Br, un groupe CF₃, un groupe nitro, un groupe cyano, un groupe amino, un groupe hydroxy, un groupe thiol, un groupe carboxy, un groupe -NH-R⁵, un groupe -NR⁶R⁷, un groupe C(=O)-R⁸, un groupe -C(=O)-O-R⁹, un groupe -O-C(=O)-R¹⁰, un groupe -C(=O)-NH-R¹⁴, un groupe -C(=O)-NR¹⁵R¹⁶, un groupe -S(=O)-R¹⁹, un groupe -S(=O)₂R²⁰, un groupe -O-R¹⁷, un groupe -S-R¹⁸, un radical alkyle en C₁₋₄ à chaîne droite ou ramifiée, un radical alcényle en C₁₋₄ à chaîne droite ou ramifiée, un radical alcynyle en C₂₋₄ à chaîne droite ou ramifiée ou un radical choisi dans le groupe consistant en les groupes cyclopropyle, cyclobutyle, cyclopentyle, cyclohexyle, pyrrolidinyle, tétrahydrofurannyle, tétrahydrothiophényle, pipéridinyle, tétrahydropyrannyle, pipérazinyle, morpholinyle, thiomorpholinyle, dioxolannyle, azépannyle, diazépannyle, imidazolidinyle, phényle, naphtyle, anthracényle, pyrrolyle, indolyle, furannyle, benzo[b]furannyle, thiophényle, benzo[b]thiophényle, pyrazolyle, imidazolyle, thiazolyle, dithiazolyle, thiadiazolyle, triazolyle, oxazolyle, oxadiazolyle, isoxazolyle, pyridinyle, pyridazinyle, pyrimidinyle, pyrazinyle, pyrannyle, indazolyle, purinyle, indolizinyle, quinoléinyle, isoquinoléinyle et quinazolinyle, dont chacun peut être lié par l'intermédiaire d'un groupe alkylène en C₁₋₃, alcénylène en C₂₋₃ ou alcynylène en C₂₋₃ et/ou est non substitué ou est substitué par éventuellement 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, -OH, -O-CH₃, -O-C₂H₅, -O-C₃H₇, -NH₂, -N(CH₃)₂, -N(C₂H₅)₂, -NH-CH₃, -NH-C₂H₅, -NO₂, -CF₃, -O-CF₃, -S-CF₃, -SH, -NH-S(=O)₂-CH₃, -C(=O)-OH, -C(=O)-CH₃, -C(=O)-C₂H₅, -C(=O)-N(CH₃)₂, -C(=O)-NH-CH₃, -NH-C(=O)-CH₃, -NH-C(=O)-C₂H₅, -C(=O)-O-CH₃ et -C(=O)-O-C₂H₅,
d'une manière tout particulièrement préférée un atome d'hydrogène, un radical halogéno choisi dans le groupe consistant en F, Cl et Br, un groupe CF₃, un groupe nitro, un groupe cyano, un groupe amino, un groupe diméthylamino, un groupe diéthylamino, un groupe monométhylamino, un groupe monoéthylamino, un groupe hydroxy, un groupe thiol, un groupe carboxy, un groupe -C(=O)-O-CH₃, un groupe -C(=O)-O-C₂H₅, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰, un radical alkyle choisi dans le groupe consistant en les groupes méthyle, éthyle, n-propyle, isopropyle, n-butyle, sec-butyle, isobutyle et tert-butyle, un radical alcényle choisi dans le groupe consistant en les groupes éthényle, 1-propényle, 2-propényle, 1-butényle, 2-butényle et 3-butényle, un radical alcynyle choisi dans le groupe consistant en les groupes éthynyle, 1-propynyle, 2-propynyle, 1-butynyle, 2-butynyle et 3-butynyle, ou un radical choisi dans le groupe consistant en les groupes (1,3)-dioxolann-2-yle, phényle, benzyle, phénéthyle, oxadiazolyle, 2-pyridyle, 3-pyridyle, 4-pyridyle, 2-thiényle, 3-thiényle, 2-furyle et 3-furyle, dont chacun est non substitué ou est substitué par éventuellement 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, -OH, -O-CH₃, -O-C₂H₅ et -O-C₃H₇.

5. Composés selon l'une ou plusieurs des revendications 1 à 4, **caractérisés en ce que** R³ est un radical halogéno, un groupe nitro, un groupe cyano, un groupe amino, un groupe hydroxy, un groupe thiol, un groupe carboxy, un groupe formyle, un groupe -NH-C(=O)-H, un groupe oxo (=O), un groupe -NH-R⁵, un groupe -NR⁶R⁷, un groupe -C(=O)-R⁸, un groupe -C(=O)-O-R⁹, un groupe -O-C(=O)-R¹⁰, un groupe -NH-C(=O)-R¹¹, un groupe -NR¹²C(=O)-R¹³, un groupe -C(=O)-NH₂, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰, un groupe -NH-C(=O)-NH-R²¹, un groupe -NH-C(=S)-NH-R²², un groupe -NH-S(=O)₂-R²³, un groupe -NR²⁴-S(=O)₂-R²⁵, un radical aliphatique en C₁₋₈ à chaîne droite ou ramifiée, saturé ou insaturé, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote, un radical cycloaliphatique à 3, 4, 5, 6, 7, 8 ou 9 chaînons, non substitué ou au moins monosubstitué, insaturé ou saturé, comportant éventuellement au moins un hétéroatome en tant que chaînon du cycle, radical qui peut être lié par l'intermédiaire d'un groupe alkylène en C₁₋₅, alcénylène en C₂₋₅ ou alcynylène en C₂₋₅, à chaîne droite ou ramifiée, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote, et/ou peut être condensé à un système cyclique monocyclique ou polycyclique non substitué ou au moins monosubstitué, ou un radical aryle ou hétéroaryle à 5 à 14 chaînons, non substitué ou au moins monosubstitué, qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote, et/ou qui peut être condensé à un système cyclique monocyclique ou polycyclique non substitué ou au moins monosubstitué,
de préférence un radical halogéno choisi dans le groupe consistant en F, Cl et Br, un groupe nitro, un groupe cyano, un groupe amino, un groupe hydroxy, un groupe thiol, un groupe carboxy, un groupe oxo (=O), ou un radical alkyle en C₁₋₄ à chaîne droite ou ramifiée, ou un radical choisi dans le groupe consistant en les groupes cyclopropyle, cyclobutyle, cyclopentyle, cyclohexyle, pyrrolidinyle, tétrahydrofurannyle, tétrahydrothiophényle, pipéridinyle, tétrahydropyrannyle, pipérazinyle, morpholinyle, thiomorpholinyle, dioxolannyle, azépannyle, diazépannyle, imidazolidinyle, phényle, naphtyle, anthracényle, pyrrolyle, indolyle, furannyle, benzo[b]furannyle, thiophényle, benzo[b]thiophényle, pyrazolyle, imidazolyle, thiazolyle, dithiazolyle, thiadiazolyle, triazolyle, oxazolyle, oxadiazolyle, isoxazolyle, pyridinyle, pyridazinyle, pyrimidinyle, pyrazinyle, pyrannyle, indazolyle, purinyle, indolizinyle, quinoléinyle, isoquinoléinyle et quinazolinyle, qui est non substitué ou est substitué par éventuellement 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, -OH, -O-CH₃, -O-C₂H₅ et -O-C₃H₇, d'une manière particulièrement préférée un groupe oxo (=O), un radical alkyle choisi dans le groupe consistant en les groupes méthyle, éthyle, n-propyle, isopropyle, n-butyle, sec-butyle, isobutyle et tert-butyle, ou un radical phényle, qui est non substitué ou est substitué par 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, -OH, -O-CH₃, -O-C₂H₅ et -O-C₃H₇.

6. Composés selon l'une ou plusieurs des revendications 1 à 5, **caractérisés en ce que** R⁴ est un atome d'hydrogène, un groupe -C(=O)-NH₂, un groupe -C(=O)-NH-R¹⁴,un groupe -C(=O)-NR¹⁵R¹⁶, un radical aliphatique en C₁₋₈ à chaîne droite ou ramifiée, saturé ou insaturé, non substitué ou au moins monosubstitué, un radical cycloaliphatique à 3, 4, 5, 6, 7, 8 ou 9 chaînons, non substitué ou au moins monosubstitué, insaturé ou saturé, comportant éventuellement au moins un hétéroatome en tant que chaînon du cycle, radical qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote, et/ou qui peut être condensé à un système cyclique monocyclique ou polycyclique non substitué ou au moins monosubstitué, ou un radical aryle ou hétéroaryle à 5 à 14 chaînons, non substitué ou au moins monosubstitué, qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote, et/ou qui peut être condensé à un système cyclique monocyclique ou polycyclique non substitué ou au moins monosubstitué,
de préférence un atome d'hydrogène, un groupe -C(=O)-NH₂, un groupe -C(=O)-NH-R¹⁴, un groupe -C(=O)-NR¹⁵R¹⁶, un radical alkyle en C₁₋₈ à chaîne droite ou ramifiée, non substitué ou au moins monosubstitué, un radical alcényle en C₂₋₈ à chaîne droite ou ramifiée, non substitué ou au moins monosubstitué, un radical alcynyle en C₂₋₈ à chaîne droite ou ramifiée, non substitué ou au moins monosubstitué, un radical cycloaliphatique à 4, 5, 6 ou 7 chaînons, non substitué ou au moins monosubstitué, insaturé ou saturé, qui peut être condensé à un système cyclique monocyclique ou bicyclique insaturé, saturé ou aromatique, non substitué ou au moins monosubstitué, chacun des cycles pouvant comporter 1, 2 ou 3 hétéroatomes, qui peuvent être choisis indépendamment les uns des autres dans le groupe consistant en l'oxygène, l'azote et le soufre, les noyaux du système cyclique monocyclique ou bicyclique ayant chacun 4, 5 ou 6 chaînons, ou un radical aryle ou hétéroaryle à 5 ou 6 chaînons, non substitué ou au moins monosubstitué, qui peut être condensé à un système cyclique monocyclique ou bicyclique saturé, insaturé ou aromatique, non substitué ou au moins monosubstitué, le radical hétéroaryle et éventuellement l'un des noyaux, ou les deux, du système cyclique monocyclique ou bicyclique, comportant chacun 1, 2 ou 3 hétéroatomes, qui peuvent être choisis indépendamment les uns des autres dans le groupe consistant en l'oxygène, l'azote et le soufre, les noyaux du système cyclique monocyclique ou bicyclique ayant chacun 4, 5 ou 6 chaînons,
d'une manière particulièrement préférée un atome d'hydrogène, un groupe -C(=O)-NH₂, un groupe -C(=O)-NH-R¹⁴, un groupe -C(=O)-NR¹⁵R¹⁶, un radical alkyle choisi dans le groupe consistant en les groupes méthyle, éthyle, n-propyle, isopropyle, n-butyle, sec-butyle, isobutyle, tert-butyle, n-pentyle, 2-pentyle, 3-pentyle, néopentyle, n-hexyle, n-heptyle et n-octyle, ou un radical choisi dans le groupe consistant en les groupes cyclopropyle, cyclobutyle, cyclopentyle, cyclohexyle, cycloheptyle, cyclopentényle, cyclohexényle, pyrrolidinyle, tétrahydrofurannyle, tétrahydrothiophényle, pipéridinyle, tétrahydropyrannyle, pipérazinyle, morpholinyle, thiomorpholinyle, dioxolannyle, azépannyle, diazépannyle, imidazolidinyle, phényle, naphtyle, anthracényle, furannyle, thiophényle, pyrazolyle, pyrazinyle, pyrimidinyle, pyridinyle, pyrrolyle, oxazolyle, isoxazolyle, thiazolyle, dithiazolyle, oxadiazolyle, triazolyle, imidazolyle, indolyle, benzo[b]thiophényle, benzo[b]furannyle, quinoléinyle, isoquinoléinyle et quinazolyle, dont chacun peut être substitué éventuellement par 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, éthényle, allyle, éthynyle, propynyle, -C≡C-Si(CH3)₃, -C=C-Si(C₂H₅)₃, -CH₂-O-CH₃, -CH₂-O-C₂H₅, -OH, -O-CH₃, -O-C₂H₅, -O-C₃H₇, -S-CH₃, -S-C₂H₅, -S(=O)-CH₃, -S(=O)₂-CH₃, -S(=O)-C₂H₅, -S(=O)₂-C₂H₅, -NH₂, -N(CH₃)₂, -NH-CH₃, -NH-C₂H₅, -NO₂, -CF₃, -CH₂F, -CHF₂, -O-CF₃, -S-CF₃, -SH, -NH-S(=O)₂-CH3, -C(=O)-OH, -C(=O)-H ; -C(=O)-CH₃, -C(=O)-C₂H₅, -C(=O)-NH₂, -C(=O)-N(CH₃)₂, -C(=O)-NH-CH₃, -NH-C(=O)-CH₃, -NH-C(=O)-C₂H₅, -C(=O)-O-CH₃, -C(=O)-O-C₂H₅, -C(=O)-O-C(CH₃) et phényle,
d'une manière tout particulièrement préférée un atome d'hydrogène, un groupe -C(=O)-NH₂, un groupe -C(=O)-NH-R¹⁴, un groupe -C(=O)-NR¹⁵R¹⁶, un radical alkyle choisi dans le groupe consistant en les groupes méthyle, éthyle, n-propyle, isopropyle, n-butyle, sec-butyle, isobutyle, tert-butyle, n-pentyle, sec-pentyle, néopentyle, n-hexyle, n-heptyle et n-octyle, ou un radical choisi dans le groupe consistant en les groupes cyclopropyle, cyclobutyle, cyclopentyle, cyclohexyle, cycloheptyle, cyclopentényle, cyclohexényle, phényle, pyrid-2-yle, pyrid-3-yle, pyrid-4-yle, pyrrol-2-yle, pyrrol-3-yle, thiophèn-2-yle, thiophèn-3-yle, 4-méthylthiophèn-2-yle, 3-méthyl-thiophèn-2-yle, 5-méthylthiophèn-2-yle, furann-2-yle, furann-3-yle, imidazol-2-yle, imidazol-4-yle, thiazol-2-yle, thiazol-4-yle, thiazol-5-yle, oxazol-2-yle, oxazol-4-yle, oxazol-5-yle, (1,2,4)-thiadiazol-5-yle, (1,2,4)-oxadiazol-5-yle, napht-1-yle, napht-2-yle, anthracèn-1-yle, anthracèn-2-yle, anthracèn-9-yle, indol-2-yle, indol-3-yle, indol-4-yle, indol-5-yle, indol-6-yle, indol-7-yle, 1-méthylindol-2-yle, 1-méthyl-indol-3-yle, 1-méthyl-indol-4-yle, 1-méthyl-indol-5-yle, 1-méthyl-indol-6-yle, 1-méthyl-indol-7-yle, quinoléin-3-yle, quinoléin-4-yle, quinoléin-2-yle, quinoléin-5-yle, quinoléin-6-yle, quinoléin-7-yle, quinoléin-8-yle, isoquinoléin-1-yle, isoquinoléin-3-yle, isoquinoléin-4-yle, isoquinoléin-5-yle, isoquinoléin-6-yle, isoquinoléin-7-yle, isoquinoléin-8-yle, 2-méthyl-phényle, 3-méthyl-phényle, 4-méthyl-phényle, 2-fluoro-phényle, 3-fluoro-phényle, 4-fluoro-phényle, 2-cyano-phényle, 3-cyano-phényle, 4-cyano-phényle, 2-hydroxy-phényle, 3-hydroxy-phényle, 4-hydroxy-phényle, 2-amino-phényle, 3-amino-phényle, 4-amino-phényle, 2-diméthylamino-phényle, 3-diméthylamino-phényle, 4-diméthylamino-phényle, 2-méthylamino-phényle, 3-méthylamino-phényle, 4-méthylamino-phényle, 2-acétyl-phényle, 3-acétyl-phényle, 4-acétyl-phényle, 2-méthylsulfinyl-phényle, 3-méthylsulfinyl-phényle, 4-méthylsulfinyl-phényle, 2-méthylsulfonyl-phényle, 3-méthylsulfonyl-phényle, 4-méthylsulfonyl-phényle, 2-méthoxy-phényle, 3-méthoxy-phényle, 4-méthoxy-phényle, 2-chloro-phényle, 3-chloro-phényle, 4-chloro-phényle, 2-éthoxy-phényle, 3-éthoxy-phényle, 4-éthoxy-phényle, 2-trifluorométhyl-phényle, 3-trifluorométhyl-phényle, 4-trifuorométhyl-phényle, 2-difluorométhyl-phényle, 3-difluorométhyl-phényle, 4-difluorométhyl-phényle, 2-fluorométhyl-phényle, 3-fluorométhyl-phényle, 4-fluorométhyl-phényle, 2-nitro-phényle, 3-nitro-phényle, 4-nitro-phényle, 2-éthyl-phényle, 3-éthyl-phényle, 4-éthyl-phényle, 2-propyl-phényle, 3-propyl-phényle, 4-propyl-phényle, 2-isopropyl-phényle, 3-isopropyl-phényle, 4-isopropyl-phényle, 2-tert-butyl-phényle, 3-tert-butyl-phényle, 4-tert-butyl-phényle, 2-carboxy-phényle, 3-carboxy-phényle, 4-carboxy-phényle, 2-éthényl-phényle, 3-éthényl-phényle, 4-éthényl-phényle, 2-éthynyl-phényle, 3-éthynyl-phényle, 4-éthynyl-phényle, 2-allyl-phényle, 3-allyl-phényle, 4-allyl-phényle, 2-triméthylsilanyléthynyl-phényle, 3-triméthylsilanyléthynyl-phényle, 4-triméthylsilanyléthynyl-phényle, 2-formyl-phényle, 3-formyl-phényle, 4-formyl-phényle, 2-acétamino-phényle, 3-acétamino-phényle, 4-acétamino-phényle, 2-diméthylaminocarbonyl-phényle, 3-diméthylaminocarbonyl-phényle, 4-diméthylaminocarbonyl-phényle, 2-méthoxyméthyl-phényle, 3-méthoxyméthyl-phényle, 4-méthoxyméthyl-phényle, 2-éthoxyméthyl-phényle, 3-éthoxyméthyl-phényle, 4-éthoxyméthyl-phényle, 2-aminocarbonyl-phényle, 3-aminocarbonyl-phényle, 4-aminocarbonyl-phényle, 2-méthylaminocarbonyl-phényle, 3-méthylaminocarbonyl-phényle, 4-méthylaminocarbonyl-phényle, 2-carboxyméthylester-phényle, 3-carboxyméthylester-phényle, 4-carboxyméthylester-phényle, 2-carboxyéthylester-phényle, 3-carboxyéthylester-phényle, 4-carboxyéthylester-phényle, 2-carboxy-tert-butylester-phényle, 3-carboxy-tert-butylester-phényle, 4-carboxy-tert-butylester-phényle, 2-méthylmercapto-phényle, 3-méthylmercapto-phényle, 4-méthylmercapto-phényle, 2-éthylmercapto-phényle, 3-éthylmercapto-phényle, 4-éthylmercapto-phényle, 2-biphényle, 3-biphényle, 4-biphényle, 2-bromophényle, 3-bromophényle, 4-bromophényle, 2-iodo-phényle, 3-iodo-phényle, 4-iodo-phényle, 2-trifluorométhoxy-phényle, 3-trifluorométhoxy-phényle, 4-trifluorométhoxy-phényle, 2-fluoro-3-trifluorométhyl-phényle, 2-fluoro-4-méthyl-phényle, (2,3)-difluorophényle, (2,3)-diméthyl-phényle, (2,3)-dichlorophényle, 3-fluoro-2-trifluorométhyl-phényle, (2,4)-dichlorophényle, (2,4)-difluoro-phényle, 4-fluoro-2-trifluorométhyl-phényle, (2,4)-diméthoxy-phényle, 2-chloro-4-fluoro-phényle, 2-chloro-4-nitro-phényle, 2-chloro-4-méthyl-phényle, 2-chloro-5-trifluorométhyl-phényle, 2-chloro-5-méthoxy-phényle, 2-bromo-5-trifluorométhyl-phényle, 2-bromo-5-méthoxy-phényle, (2,4)-dibromo-phényle, (2,4)-diméthyl-phényle, 2-fluoro-4-trifluorométhyl-phényle, (2,5)-difluoro-phényle, 2-fluoro-5-trifluorométhyl-phényle, 5-fluoro-2-trifluorométhyl-phényle, 5-chloro-2-trifluorométhyl-phényle, 5-bromo-2-trifluorométhyl-phényle, (2,5)-diméthoxy-phényle, (2,5)-bis-trifluorométhyl-phényle, (2,5)-dichlorophényle, (2,5)-dibromo-phényle, 2-méthoxy-5-nitro-phényle, 2-fluoro-6-trifluorométhyl-phényle, (2,6)-diméthoxy-phényle, (2,6)-diméthyl-phényle, (2,6)-dichloro-phényle, 2-chloro-6-fluoro-phényle, 2-bromo-6-chloro-phényle, 2-bromo-6-fluoro-phényle, (2,6)-difluoro-phényle, (2,6)-difluoro-3-méthyl-phényle, (2,6)-dibromo-phényle, (2,6)-dichlorophényle, 3-chloro-2-fluoro-phényle, 3-chloro-5-méthyl-phényle, (3,4)-dichlorophényle, (3,4)-diméthyl-phényle, 3-méthyl-4-méthoxy-phényle, 4-chloro-3-nitro-phényle, (3,4)-diméthoxy-phényle, 4-fluoro-trifluorométhyl-phényle, 3-fluoro-4-trifluorométhyl-phényle, (3,4)-difluorophényle, 3-cyano-4-fluoro-phényle, 3-cyano-4-méthyl-phényle, 3-cyano-4-méthoxy-phényle, 3-bromo-4-fluoro-phényle, 3-bromo-4-méthyl-phényle, 3-bromo-4-méthoxy-phényle, 4-chloro-2-fluoro-phényle, 4-chloro-3-trifluorométhyle, 4-bromo-3-méthyl-phényle, 4-bromo-5-méthyl-phényle, 3-chloro-4-fluoro-phényle, 4-fluoro-3-nitro-phényle, 4-bromo-3-nitro-phényle, (3,4)-dibromo-phényle, 4-chloro-3-méthyl-phényle, 4-bromo-3-méthyl-phényle, 4-fluoro-3-méthyl-phényle, 3-fluoro-4-méthyl-phényle, 3-fluoro-5-méthyl-phényle, 2-fluoro-3-méthyl-phényle, 4-méthyle-3-nitro-phényle, (3,5)-diméthoxy-phényle, (3,5)-diméthyl-phényle, (3,5)-bis-trifluorométhyl-phényle, (3,5)-difluoro-phényle, (3,5)-dinitrophényle, (3,5)-dichloro-phényle, 3-fluoro-5-trifluorométhyl-phényle, 5-fluoro-3-trifluorométhyl-phényle, (3,5)-dibromo-phényle, 5-chloro-4-fluoro-phényle, 5-chloro-4-fluoro-phényle, 5-bromo-4-méthyl-phényle, (2,3,4)-trifluoro-phényle, (2,3,4)-trichloro-phényle, (2,3,6)-trifluoro-phényle, 5-chloro-2-méthoxy-phényle, (2,3)-difluoro-4-méthyle, (2,4,5)-trifluoro-phényle, (2,4,5)-trichloro-phényle, (2,4)-dichloro-5-fluoro-phényle, (2,4,6)-trichloro-phényle, (2,4,6)-triméthylphényle, (2,4,6)-trifluoro-phényle, (2,4,6)-triméthoxy-phényle, (3,4,5)-triméthoxy-phényle, (2,3,4,5)-tétrafluoro-phényle, 4-méthoxy-(2,3,6)-triméthylphényle, 4-méthoxy-(2,3,6)-triméthyl-phényle, 4-chloro-2,5-diméthyl-phényle, 2-chloro-6-fluoro-3-méthyl-phényle, 6-chloro-2-fluoro-3-méthyle, (2,4,6)-triméthyl-phényle, (2,3,4,5,6)-pentafluoro-phényle, 3-méthyl-pyrid-2-yle, 4-méthyl-pyrid-2-yle, 5-méthylpyrid-2-yle, 6-méthyl-pyrid-2-yle, 2-méthyl-pyrid-3-yle, 4-méthyl-pyrid-3-yle, 5-méthyl-pyrid-3-yle, 6-méthyl-pyrid-3-yle, 2-méthyl-pyrid-4-yle, 3-méthylpyrid-4-yle, 3-fluoro-pyrid-2-yle, 4-fluoro-pyrid-2-yle, 5-fluoro-pyrid-2-yle, 6-fluoro-pyrid-2-yle, 3-chloro-pyrid-2-yle, 4-chloro-pyrid-2-yle, 5-chloropyrid-2-yle, 6-chloro-pyrid-2-yle, 3-trifluorométhylpyrid-2-yle, 4-trifluorométhyl-pyrid-2-yle, 5-trifluorométhyl-pyrid-2-yle, 6-trifluorométhyl-pyrid-2-yle,3-méthoxy-pyrid-2-yle, 4-méthoxy-pyrid-2-yle, 5-méthoxy-pyrid-2-yle, 6-méthoxy-pyrid-2-yle, 4-méthylthiazol-2-yle, 5-méthyl-thiazol-2-yle, 4-trifluorométhyl-thiazol-2-yle, 5-trifluorométhyl-thiazol-2-yle, 4-chloro-thiazol-2-yle, 5-chloro-thiazol-2-yle, 4-bromo-thiazol-2-yle, 5-bromo-thiazol-2-yle, 4-fluorothiazol-2-yle, 5-fluoro-thiazol-2-yle, 4-cyano-thiazol-2-yle, 5-cyano-thiazol-2-yle, 4-méthoxy-thiazol-2-yle, 5-méthoxy-thiazol-2-yle, 4-méthyl-oxazol-2-yle, 5-méthyl-oxazol-2-yle, 4-trifluorométhyl-oxazol-2-yle, 5-trifluorométhyl-oxazol-2-yle, 4-chloro-oxazol-2-yle, 5-chloro-oxazol-2-yle, 4-bromo-oxazol-2-yle, 5-bromooxazol-2-yle, 4-fluoro-oxazol-2-yle, 5-fluoro-oxazol-2-yle, 4-cyano-oxazol-2-yle, 5-cyano-oxazol-2-yle, 4-méthoxy-oxazol-2-yle, 5-méthoxy-oxazol-2-yle, 2-méthyl-(1,2,4)-thiadiazol-5-yle, 2-trifluorométhyl-(1,2,4)-thiadiazol-5-yle, 2-chloro-(1,2,4)-thiadiazol-5-yle, 2-fluoro-(1,2,4)-thiadiazol-5-yle, 2-méthoxy-(1,2,4)-thiadiazol-5-yle, 2-cyano-(1,2,4)-thiadiazol-5-yle, 2-méthyl-(1,2,4)-oxadiazol-5-yle, 2-trifluorométhyl-(1,2,4)-oxadiazol-5-yle, 2-chloro-(1,2,4)-oxadiazol-5-yle, 2-fluoro-(1,2,4)-oxadiazol-5-yle, 2-méthoxy-(1,2,4)-oxadiazol-5-yle et 2-cyano-(1,2,4)-oxadiazol-5-yle.

7. Composés selon l'une ou plusieurs des revendications 1 à 6, **caractérisés en ce que** n vaut 0, 1, 2, 3 ou 4 et vaut de préférence 0, 1 ou 2.

8. Composés selon l'une ou plusieurs des revendications 1 à 7, **caractérisés en ce que** R⁵ à R²⁵, indépendamment les uns des autres, représentent chacun un radical aliphatique en C₁₋₈ à chaîne droite ou ramifiée, saturé ou insaturé, non substitué ou au moins monosubstitué, un radical cycloaliphatique à 3, 4, 5, 6, 7, 8 ou 9 chaînons, non substitué ou au moins monosubstitué, insaturé ou saturé, comportant éventuellement au moins un hétéroatome en tant que chaînon du cycle, radical qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon (s) de la chaîne aliphatique éventuellement 1, 2 ou 3 hétéroatomes choisis indépendamment les uns des autres dans le groupe consistant en l'oxygène, le soufre et l'azote, et/ou qui peut être condensé à un système cyclique monocyclique ou polycyclique non substitué ou au moins monosubstitué, ou un radical aryle ou hétéroaryle à 5 à 14 chaînons, non substitué ou au moins monosubstitué, qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote, et/ou qui peut être condensé à un système cyclique monocyclique ou polycyclique non substitué ou au moins monosubstitué,
de préférence un radical alkyle en C₁₋₈ à chaîne droite ou ramifiée, non substitué ou au moins monosubstitué, un radical alcényle en C₂₋₈ à chaîne droite ou ramifiée, non substitué ou au moins monosubstitué, un radical alcynyle en C₂₋₈ à chaîne droite ou ramifiée, non substitué ou au moins monosubstitué, un radical cycloaliphatique à 3, 4, 5, 6, 7, 8 ou 9 chaînons, non substitué ou au moins monosubstitué, insaturé ou saturé, comportant éventuellement au moins un hétéroatome en tant que chaînon du cycle, radical qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote, ou un radical aryle ou hétéroaryle à 5 à 14 chaînons, non substitué ou au moins monosubstitué, qui peut être lié par l'intermédiaire d'un groupe alkylène en C₁₋₅, alcénylène en C₂₋₅ ou alcynylène en C₂₋₅ à chaîne droite ou ramifiée, non substitué ou au moins monosubstitué, comportant en tant que chaînon(s) de la chaîne aliphatique éventuellement 1, 2 ou 3 hétéroatomes choisis indépendamment les uns des autres dans le groupe consistant en l'oxygène, le soufre et l'azote,
d'une manière particulièrement préférée un radical alkyle choisi dans le groupe consistant en les groupes méthyle, éthyle, n-propyle, isopropyle, n-butyle, sec-butyle, isobutyle et tert-butyle, un radical alcényle choisi dans le groupe consistant en les groupes éthényle, 1-propényle, 2-propényle, 1-butényle, 2-butényle et 3-butényle, un radical alcynyle choisi dans le groupe consistant en les groupes éthynyle, 1-propynyle, 2-propynyle, 1-butynyle, 2-butynyle et 3-butynyle, ou un radical choisi dans le groupe consistant en les groupes cyclopropyle, cyclobutyle, cyclopentyle, cyclohexyle, pyrrolidinyle, tétrahydrofurannyle, tétrahydrothiophényle, pipéridinyle, tétrahydropyrannyle, pipérazinyle, morpholinyle, thiomorpholinyle, dioxolannyle, azépannyle, diazépannyle, imidazolidinyle, phényle, naphtyle, anthracényle, pyrrolyle, indolyle, furannyle, benzo[b]furannyle, thiophényle, benzo[b]thiophényle, pyrazolyle, imidazolyle, thiazolyle, dithiazolyle, thiadiazolyle, triazolyle, oxazolyle, oxadiazolyle, isoxazolyle, pyridinyle, pyridazinyle, pyrimidinyle, pyrazinyle, pyrannyle, indazolyle, purinyle, indolizinyle, quinoléinyle, isoquinoléinyle et quinazolinyle, chaque radical cyclique pouvant être lié par l'intermédiaire d'un groupe alkylène en C₁₋₃, alcénylène en C₂₋₃ ou alcynylène en C₂₋₃ comportant en tant que chaînon(s) de la chaîne aliphatique éventuellement 1 ou 2 hétéroatomes choisis dans le groupe consistant en l'oxygène, le soufre et l'azote, et/ou est non substitué ou est substitué par 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, -OH, -O-CH₃, -O-C₂H₅, -O-C₃H₇, -NH₂, -N(CH₃)₂, -N(C₂H₅)₂, -NH-CH₃, -NH-C₂H₅, -NO₂, -CF₃, -O-CF₃, -S-CF₃, -SH, -NH-S(=O)₂-CH₃, -C(=O)-OH, -C(=O)-CH₃, -C(=O)-C₂H₅, -C(=O)-N(CH₃)₂, -C(=O)-NH-CH₃, -NH-C(=O)-CH₃, -NH-C(=O)-C₂H₅, -C(=O)-O-CH₃ et -C(=O)-O-C₂H₅.

9. Composés selon l'une ou plusieurs des revendications 1 à 8, **caractérisés en ce que**
n vaut 0, 1, 2, 3 ou 4,
X est N ou C-R²,
R¹ est un atome d'hydrogène, un radical halogéno choisi dans le groupe consistant en F, Cl et Br, un groupe CF₃, un groupe nitro, un groupe cyano, un groupe amino, un groupe diméthylamino, un groupe diéthylamino, un groupe hydroxy, un groupe thiol, un groupe carboxy, un groupe -C(=O)-O-CH₃, un groupe -C(=O)-O-C₂H₅, un groupe -C(=O)-NH-R¹⁴, un groupe -C(=O)-NR¹⁵R¹⁶ un groupe -O-R¹⁷, un groupe -S-R¹⁸, un groupe -S(=O)-R¹⁹, un groupe -S(=O)₂-R²⁰ un radical alkyle choisi dans le groupe consistant en les groupes méthyle, éthyle, n-propyle, isopropyle, n-butyle, sec-butyle, isobutyle et tert-butyle, un radical alcényle choisi dans le groupe consistant en les groupes éthényle, 1-propényle, 2-propényle, 1-butényle, 2-butényle et 3-butényle, un radical alcynyle choisi dans le groupe consistant en les groupes éthynyle, 1-propynyle, 2-propynyle, 1-butynyle, 2-butynyle et 3-butynyle, ou un radical choisi dans le groupe consistant en les groupes (1,3)-dioxolann-2-yle, phényle, benzyle, phénéthyle, oxadiazolyle, 2-pyridyle, 3-pyridyle, 4-pyridyle, 2-thiényle, 3-thiényle, 2-furyle et 3-furyle, dont chacun est non substitué ou est substitué par éventuellement 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, -OH, -O-CH₃, -O-C₂H₅ et -O-C₃H₇,
R² est un atome d'hydrogène, un radical halogéno choisi dans le groupe consistant en F, Cl et Br, un groupe CF₃, un groupe nitro, un groupe cyano, un groupe amino, un groupe diméthylamino, un groupe diéthylamino, un groupe hydroxy, un groupe thiol, un groupe carboxy, un groupe -C(=O)-O-CH₃, un groupe -C(=O)-O-C₂H₅, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰, un radical alkyle choisi dans le groupe consistant en les groupes méthyle, éthyle, n-propyle, isopropyle, n-butyle, sec-butyle, isobutyle et tert-butyle, un radical alcényle choisi dans le groupe consistant en les groupes éthényle, 1-propényle, 2-propényle, 1-butényle, 2-butényle et 3-butényle, un radical alcynyle choisi dans le groupe consistant en les groupes éthynyle, 1-propynyle, 2-propynyle, 1-butynyle, 2-butynyle et 3-butynyle, ou un radical choisi dans le groupe consistant en les groupes (1,3)-dioxolann-2-yle, phényle, benzyle, phénéthyle, oxadiazolyle, 2-pyridyle, 3-pyridyle, 4-pyridyle, 2-thiényle, 3-thiényle, 2-furyle et 3-furyle, dont chacun est non substitué ou est substitué par éventuellement 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, -OH, -O-CH₃, -O-C₂H₅ et -O-C₃H₇,
R³ est un groupe oxo, un radical alkyle choisi dans le groupe consistant en les groupes méthyle, éthyle, n-propyle, isopropyle, n-butyle, sec-butyle, isobutyle et tert-butyle, ou un radical phényle qui est non substitué ou est substitué par 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, -OH, -O-CH₃, -O-C₂H₅ et -O-C₃H₇,
R⁴ est un atome d'hydrogène, un groupe -C(=O)-NH₂, un groupe -C(=O)-NH-R¹⁴, un groupe -C(=O)-NR¹⁵R¹⁶, un radical alkyle choisi dans le groupe consistant en les groupes méthyle, éthyle, n-propyle, isopropyle, n-butyle, sec-butyle, isobutyle, tert-butyle, n-pentyle, 2-pentyle, 3-pentyle, néopentyle, n-hexyle, n-heptyle et n-octyle, ou un radical choisi dans le groupe consistant en les groupes cyclopropyle, cyclobutyle, cyclopentyle, cyclohexyle, cycloheptyle, cyclopentényle, cyclohexényle, pyrrolidinyle, tétrahydrofurannyle, tétrahydrothiophényle, pipéridinyle, tétrahydropyrannyle, pipérazinyle, morpholinyle, thiomorpholinyle, dioxolannyle, azépannyle, diazépannyle, imidazolidinyle, phényle, naphtyle, anthracényle, furannyle, thiophényle, pyrazolyle, pyrazinyle, pyrimidinyle, pyridinyle, pyrrolyle, oxazolyle, isoxazolyle, thiazolyle, dithiazolyle, oxadiazolyle, triazolyle, imidazolyle, indolyle, benzo[b]thiophényle, benzo[b]furannyle, quinoléinyle, isoquinoléinyle et quinazolyle, dont chacun peut être éventuellement substitué par 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, éthényle, allyle, éthynyle, propynyle, -C=C-Si(CH₃)₃, -C≡C-Si(C₂H₅)₃, -CH₂-O-CH₃, -CH₂-O-C₂H₅, -OH, -O-CH₃, -O-C₂H₅, -O-C₃H₇, -S-CH₃, -S-C₂H₅, -S(=O)-CH₃, -S(=O)₂-CH₃, -S(=O)-C₂H₅, -S(=O)₂-C₂H₅, -NH₂, -N(CH₃)₂, -N(C₂H₅)₂, -NH-CH₃, -NH-C₂H₅, -NO₂, -CF₃, -CHF₂, -CH₂F, -O-CF₃, -S-CF₃, -SH, -NH-S(=O)₂-CH₃, -C(=O)-OH, -C(=O)-H; -C(=O)-CH₃, -C(=O)-C₂H₅, -C(=O)-NH₂, -C(=O)-N(CH₃)₂, -C(=O)-NH-CH₃, -NH-C(=O)-CH₃, -NH-C(=O)-C₂H₅, -C(=O)-O-CH₃, -C(=O)-O-C₂H₅, -C(=O)-O-C(CH₃)₃, et phényle,
et
R¹⁹, R¹⁵, R¹⁶, R¹⁷ , R¹⁸, R¹⁹ et R²⁰ représentent chacun indépendamment des autres un radical alkyle choisi dans le groupe consistant en le groupe méthyle, éthyle, n-propyle, isopropyle, n-butyle, sec-butyle, isobutyle et tert-butyle ;
dans chaque cas éventuellement sous forme d'un de leurs stéréoisomères purs, en particulier de leurs énantiomères ou diastéréoisomères, de leurs racémates, ou sous forme d'un mélange de stéréoisomères, en particulier des énantiomères et/ou des diastéréoisomères, selon un rapport de mélange quelconque, ou dans chaque cas sous forme des sels correspondants, de préférence sous forme des chlorhydrates correspondants, ou dans chaque cas sous forme des produits de solvatation correspondants.

10. Composés selon l'une ou plusieurs des revendications 1 à 9, **caractérisés en ce que**
n vaut 0, 1, 2, 3 ou 4,
X est N ou C-R²
R¹ est un atome d'hydrogène, un radical halogéno choisi dans le groupe consistant en F, Cl et Br, un groupe CF₃, un groupe nitro, un groupe cyano, un groupe amino, un groupe diméthylamino, un groupe diéthylamino, un groupe hydroxy, un groupe thiol, un groupe carboxy, un groupe -C(=O)-O-CH₃, un groupe -C(=O)-O-C₂H₅, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰, un radical alkyle choisi dans le groupe consistant en les groupes méthyle, éthyle, n-propyle, isopropyle, n-butyle, sec-butyle, isobutyle et tert-butyle, un radical alcényle choisi dans le groupe consistant en les groupes éthényle, 1-propényle, 2-propényle, 1-butényle, 2-butényle et 3-butényle, un radical alcynyle choisi dans le groupe consistant en les groupes éthynyle, 1-propynyle, 2-propynyle, 1-butynyle, 2-butynyle et 3-butynyle, ou un radical choisi dans le groupe consistant en les groupes (1,3)-dioxolann-2-yle, phényle, benzyle, phénéthyle, oxadiazolyle, 2-pyridyle, 3-pyridyle, 4-pyridyle, 2-thiényle, 3-thiényle, 2-furyle et 3-furyle, dont chacun est non substitué ou est substitué par éventuellement 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, -OH, -O-CH₃, -O-C₂H₅ et -O-C₃H₇,
R² est un atome d'hydrogène, un radical halogéno choisi dans le groupe consistant en F, Cl et Br, un groupe CF₃, un groupe nitro, un groupe cyano, un groupe amino, un groupe diméthylamino, un groupe diéthylamino, un groupe hydroxy, un groupe thiol, un groupe carboxy, un groupe -C(=O)-O-CH₃, un groupe -C(=O)-O-C₂H₅, un groupe -C(=O)-NH-R¹⁴, un groupe -C(=O)-NR¹⁵R¹⁶, un groupe -O-R¹⁷, un groupe -S-R¹⁸, un groupe -S(=O)-R¹⁹, un groupe -S(=O)₂-R²⁰ ,un radical alkyle choisi dans le groupe consistant en les groupes méthyle, éthyle, n-propyle, isopropyle, n-butyle, sec-butyle, isobutyle et tert-butyle, un radical alcényle choisi dans le groupe consistant en les groupes éthényle, 1-propényle, 2-propényle, 1-butényle, 2-butényle et 3-butényle, un radical alcynyle choisi dans le groupe consistant en les groupes éthynyle, 1-propynyle, 2-propynyle, 1-butynyle, 2-butynyle et 3-butynyle, ou un radical choisi dans le groupe consistant en les groupes (1,3)-dioxolann-2-yle, phényle, benzyle, phénéthyle, oxadiazolyle, 2-pyridyle, 3-pyridyle, 4-pyridyle, 2-thiényle, 3-thiényle, 2-furyle et 3-furyle, dont chacun est non substitué ou est substitué par éventuellement 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, -OH, -O-CH₃, -O-C₂H₅ et -O-C₃H₇,
R³ est un groupe oxo, un radical alkyle choisi dans le groupe consistant en les groupes méthyle, éthyle, n-propyle, isopropyle, n-butyle, sec-butyle, isobutyle et tert-butyle, ou un radical phényle qui est non substitué ou est substitué par 1, 2, 3, 4 ou 5 substituants choisis indépendamment les uns des autres dans le groupe consistant en F, Cl, Br, I, -CN, les groupes méthyle, éthyle, n-propyle, isopropyle, n-butyle, sec-butyle, isobutyle, tert-butyle, -OH, -O-CH₃, -O-C₂H₅ et -O-C₃H₇,
R⁴ est un atome d'hydrogène, un groupe -C(=O)-NH₂, un groupe -C(=O)-NH-R¹⁴ un groupe -C(=O)-NR¹⁵R¹⁶ ,un radical alkyle choisi dans le groupe consistant en les groupes méthyle, éthyle, n-propyle, isopropyle, n-butyle, sec-butyle, isobutyle, tert-butyle, n-pentyle, sec-pentyle, néopentyle, n-hexyle, n-heptyle et n-octyle, ou un radical choisi dans le groupe consistant en les groupes cyclopropyle, cyclobutyle, cyclopentyle, cyclohexyle, cycloheptyle, cyclopentényle, cyclohexényle, phényle, pyrid-2-yle, pyrid-3-yle, pyrid-4-yle, pyrrol-2-yle, pyrrol-3-yle, thiophèn-2-yle, thiophèn-3-yle, 4-méthyl-thiophèn-2-yle, 3-méthylthiophèn-2-yle, 5-méthyl-thiophèn-2-yle, furann-2-yle, furann-3-yle, imidazol-2-yle, imidazol-4-yle, thiazol-2-yle, thiazol-4-yle, thiazol-5-yle, oxazol-2-yle, oxazol-4-yle, oxazol-5-yle, (1,2,4)-thiadiazol-5-yle, (1,2,4)-oxadiazol-5-yle, napht-1-yle, napht-2-yle, anthracèn-1-yle, anthracèn-2-yle, anthracèn-9-yle, indol-2-yle, indol-3-yle, indol-4-yle, indol-5-yle, indol-6-yle, indol-7-yle, 1-méthyl-indol-2-yle, 1-méthyl-indol-3-yle, 1-méthyl-indol-4-yle, 1-méthylindol-5-yle, 1-méthyl-indol-6-yle, 1-méthyl-indol-7-yle, quinoléin-3-yle, quinoléin-4-yle, quinoléin-2-yle, quinoléin-5-yle, quinoléin-6-yle, quinoléin-7-yle, quinoléin-8-yle, isoquinoléin-1-yle, isoquinoléin-3-yle, isoquinoléin-4-yle, isoquinoléin-5-yle, isoquinoléin-6-yle, isoquinoléin-7-yle, isoquinoléin-8-yle, 2-méthyl-phényle, 3-méthyl-phényle, 4-méthyl-phényle, 2-fluoro-phényle, 3-fluoro-phényle, 4-fluoro-phényle, 2-cyano-phényle, 3-cyano-phényle, 4-cyano-phényle, 2-hydroxy-phényle, 3-hydroxy-phényle, 4-hydroxy-phényle, 2-amino-phényle, 3-amino-phényle, 4-amino-phényle, 2-diméthylamino-phényle, 3-diméthylamino-phényle, 4-diméthylamino-phényle, 2-méthylamino-phényle, 3-méthylamino-phényle, 4-méthylamino-phényle, 2-acétyl-phényle, 3-acétyl-phényle, 4-acétyl-phényle, 2-méthylsulfinyl-phényle, 3-méthylsulfinyl-phényle, 4-méthylsulfinyl-phényle, 2-méthylsulfonyl-phényle, 3-méthylsulfonyl-phényle, 4-méthylsulfonyl-phényle, 2-méthoxy-phényle, 3-méthoxy-phényle, 4-méthoxy-phényle, 2-chloro-phényle, 3-chloro-phényle, 4-chloro-phényle, 2-éthoxy-phényle, 3-éthoxy-phényle, 4-éthoxy-phényle, 2-trifluorométhyl-phényle, 3-trifluorométhyl-phényle, 4-trifuorométhyl-phényle, 2-difluorométhyl-phényle, 3-difluorométhyl-phényle, 4-difluorométhyl-phényle, 2-fluorométhyl-phényle, 3-fluorométhyl-phényle, 4-fluorométhyl-phényle, 2-nitro-phényle, 3-nitro-phényle, 4-nitro-phényle, 2-éthyl-phényle, 3-éthyl-phényle, 4-éthyl-phényle, 2-propyl-phényle, 3-propyl-phényle, 4-propyl-phényle, 2-isopropyl-phényle, 3-isopropyl-phényle, 4-isopropyl-phényle, 2-tert-butyl-phényle, 3-tert-butyl-phényle, 4-tert-butyl-phényle, 2-carboxy-phényle, 3-carboxy-phényle, 4-carboxy-phényle, 2-éthényl-phényle, 3-éthényl-phényle, 4-éthényl-phényle, 2-éthynyl-phényle, 3-éthynyl-phényle, 4-éthynyl-phényle, 2-allyl-phényle, 3-allyl-phényle, 4-allyl-phényle, 2-triméthylsilanyléthynyl-phényle, 3-triméthylsilanyléthynyl-phényle, 4-triméthylsilanyléthynyl-phényle, 2-formyl-phényle, 3-formyl-phényle, 4-formyl-phényle, 2-acétamino-phényle, 3-acétamino-phényle, 4-acétamino-phényle, 2-diméthylaminocarbonyl-phényle, 3-diméthylaminocarbonyl-phényle, 4-diméthylaminocarbonyl-phényle, 2-méthoxyméthyl-phényle, 3-méthoxyméthyl-phényle, 4-méthoxyméthyl-phényle, 2-éthoxyméthyl-phényle, 3-éthoxyméthyl-phényle, 4-éthoxyméthyl-phényle, 2-aminocarbonyl-phényle, 3-aminocarbonyl-phényle, 4-aminocarbonyl-phényle, 2-méthylaminocarbonyl-phényle, 3-méthylaminocarbonyl-phényle, 4-méthylaminocarbonyl-phényle, 2-carboxyméthylester-phényle, 3-carboxyméthylester-phényle, 4-carboxyméthylester-phényle, 2-carboxyéthylester-phényle, 3-carboxyéthylester-phényle, 4-carboxyéthylester-phényle, 2-carboxy-tert-butylester-phényle, 3-carboxy-tert-butylester-phényle, 4-carboxy-tert-butylester-phényle, 2-méthylmercapto-phényle, 3-méthylmercapto-phényle, 4-méthylpercapto-phényle, 2-éthylmercapto-phényle, 3-éthylmercapto-phényle, 4-éthylmercapto-phényle, 2-biphényle, 3-biphényle, 4-biphényle, 2-bromophényle, 3-bromophényle, 4-bromophényle, 2-iodo-phényle, 3-iodo-phényle, 4-iodo-phényle, 2-trifluorométhoxy-phényle, 3-trifluorométhoxy-phényle, 4-trifluorométhoxy-phényle, 2-fluoro-3-trifluorométhylphényle, 2-fluoro-4-méthyl-phényle, (2,3)-difluorophényle, (2,3)-diméthyl-phényle, (2,3)-dichlorophényle, 3-fluoro-2-trifluorométhyl-phényle, (2,4)-dichloro-phényle, (2,4)-difluoro-phényle, 4-fluoro-2-trifluorométhyl-phényle, (2,4)-diméthoxy-phényle, 2-chloro-4-fluoro-phényle, 2-chloro-4-nitro-phényle, 2-chloro-4-méthyl-phényle, 2-chloro-5-trifluorométhyl-phényle, 2-chloro-5-méthoxy-phényle, 2-bromo-5-trifluorométhyl-phényle, 2-bromo-5-méthoxy-phényle, (2,4)-dibromo-phényle, (2,4)-diméthyl-phényle, 2-fluoro-4-trifluorométhyl-phényle, (2,5)-difluorophényle, 2-fluoro-5-trifluorométhyl-phényle, 5-fluoro-2-trifluorométhyl-phényle, 5-chloro-2-trifluorométhyl-phényle, 5-bromo-2-trifluorométhyl-phényle, (2,5)-diméthoxy-phényle, (2,5)-bis-trifluorométhyl-phényle, (2,5)-dichloro-phényle, (2,5)-dibromo-phényle, 2-méthoxy-5-nitro-phényle, 2-fluoro-6-trifluorométhyl-phényle, (2,6)-diméthoxy-phényle, (2,6)-diméthyl-phényle, (2,6)-dichloro-phényle, 2-chloro-6-fluoro-phényle, 2-bromo-6-chloro-phényle, 2-bromo-6-fluoro-phényle, (2,6)-difluoro-phényle, (2,6)-difluoro-3-méthyl-phényle, (2,6)-dibromo-phényle, (2,6)-dichlorophényle, 3-chloro-2-fluoro-phényle, 3-chloro-5-méthyl-phényle, (3,4)-dichlorophényle, (3,4)-diméthyl-phényle, 3-méthyl-4-méthoxy-phényle, 4-chloro-3-nitro-phényle, (3,4)-diméthoxy-phényle, 4-fluoro-3-trifluorométhyl-phényle, 3-fluoro-4-trifluorométhyl-phényle, (3,4)-difluoro-phényle, 3-cyano-4-fluoro-phényle, 3-cyano-4-méthyl-phényle, 3-cyano-4-méthoxy-phényle, 3-bromo-4-fluoro-phényle, 3-bromo-4-méthyl-phényle, 3-bromo-4-méthoxy-phényle, 4-chloro-2-fluoro-phényle, 4-chloro-3-trifluorométhyle, 4-bromo-3-méthyl-phényle, 4-bromo-5-méthyl-phényle, 3-chloro-4-fluoro-phényle, 4-fluoro-3-nitro-phényle, 4-bromo-3-nitro-phényle, (3,4)-dibromo-phényle, 4-chloro-3-méthyl-phényle, 4-bromo-3-méthyl-phényle, 4-fluoro-3-méthyl-phényle, 3-fluoro-4-méthyl-phényle, 3-fluoro-5-méthyl-phényle, 2-fluoro-3-méthyl-phényle, 4-méthyle-3-nitro-phényle, (3,5)-diméthoxy-phényle, (3,5)-diméthyl-phényle, (3,5)-bis-trifluorométhyl-phényle, (3,5)-difluoro-phényle, (3,5)-dinitrophényle, (3,5)-dichloro-phényle, 3-fluoro-5-trifluorométhyl-phényle, 5-fluoro-3-trifluorométhyl-phényle, (3,5)-dibromo-phényle, 5-chloro-4-fluoro-phényle, 5-chloro-4-fluoro-phényle, 5-bromo-4-méthyl-phényle, (2,3,4)-trifluoro-phényle, (2,3,4)-trichloro-phényle, (2,3,6)-trifluoro-phényle, 5-chloro-2-méthoxy-phényle, (2,3)-difluoro-4-méthyle, (2,4,5)-trifluoro-phényle, (2,4,5)-trichloro-phényle, (2,4)-dichloro-5-fluoro-phényle, (2,4,6)-trichloro-phényle, (2,4,6)-triméthylphényle, (2,4,6)-trifluoro-phényle, (2,4,6)-triméthoxy-phényle, (3,4,5)-triméthoxy-phényle, (2,3,4,5)-tétrafluoro-phényle, 4-méthoxy-(2,3,6)-triméthylphényle, 4-méthoxy-(2,3,6)-triméthyl-phényle, 4-chloro-2,5-diméthyl-phényle, 2-chloro-6-fluoro-3-méthyl-phényle, 6-chloro-2-fluoro-3-méthyle, (2,4,6)-triméthyl-phényle, (2,3,4,5,6)-pentafluoro-phényle, 3-méthyl-pyrid-2-yle, 4-méthyl-pyrid-2-yle, 5-méthylpyrid-2-yle, 6-méthyl-pyrid-2-yle, 2-méthyl-pyrid-3-yle, 4-méthyl-pyrid-3-yle, 5-méthyl-pyrid-3-yle, 6-méthyl-pyrid-3-yle, 2-méthyl-pyrid-4-yle, 3-méthylpyrid-4-yle, 3-fluoro-pyrid-2-yle, 4-fluoro-pyrid-2-yle, 5-fluoro-pyrid-2-yle, 6-fluoro-pyrid-2-yle, 3-chloro-pyrid-2-yle, 4-chloro-pyrid-2-yle, 5-chloropyrid-2-yle, 6-chloro-pyrid-2-yle, 3-trifluorométhylpyrid-2-yle, 4-trifluorométhyl-pyrid-2-yle, 5-trifluorométhyl-pyrid-2-yle, 6-trifluorométhyl-pyrid-2-yle,3-méthoxy-pyrid-2-yle, 4-méthoxy-pyrid-2-yle, 5-méthoxy-pyrid-2-yle, 6-méthoxy-pyrid-2-yle, 4-méthylthiazol-2-yle, 5-méthyl-thiazol-2-yle, 4-trifluorométhyl-thiazol-2-yle, 5-trifluorométhyl-thiazol-2-yle, 4-chloro-thiazol-2-yle, 5-chloro-thiazol-2-yle, 4-bromo-thiazol-2-yle, 5-bromo-thiazol-2-yle, 4-fluorothiazol-2-yle, 5-fluoro-thiazol-2-yle, 4-cyano-thiazol-2-yle, 5-cyano-thiazol-2-yle, 4-méthoxy-thiazol-2-yle, 5-méthoxy-thiazol-2-yle, 4-méthyl-oxazol-2-yle, 5-méthyl-oxazol-2-yle, 4-trifluorométhyl-oxazol-2-yle, 5-trifluorométhyl-oxazol-2-yle, 4-chloro-oxazol-2-yle, 5-chloro-oxazol-2-yle, 4-bromo-oxazol-2-yle, 5-bromooxazol-2-yle, 4-fluoro-oxazol-2-yle, 5-fluoro-oxazol-2-yle, 4-cyano-oxazol-2-yle, 5-cyano-oxazol-2-yle, 4-méthoxy-oxazol-2-yle, 5-méthoxy-oxazol-2-yle, 2-méthyl-(l,2,4)-thiadiazol-5-yle, 2-trifluorométhyl-(1,2,4)-thiadiazol-5-yle, 2-chloro-(1,2,4)-thiadiazol-5-yle, 2-fluoro-(1,2,4)-thiadiazol-5-yle, 2-méthoxy-(1,2,4)-thiadiazol-5-yle, 2-cyano-(1,2,4)-thiadiazol-5-yle, 2-méthyl-(1,2,4)-oxadiazol-5-yle, 2-trifluorométyl-(l,2,4)-oxadiazol-5-yle, 2-chloro-(1,2,4)-oxadiazol-5-yle, 2-fluoro-(1,2,4)-oxadiazol-5-yle, 2-méthoxy-(l,2,4)-oxadiazol-5-yle et 2-cyano-(1,2,4)-oxadiazol-5-yle,
et
R¹⁴, R¹⁵, R¹⁶, R¹⁷, R¹⁸, R¹⁹ et R²⁰ représentent chacun indépendamment des autres un radical alkyle choisi dans le groupe consistant en les groupes méthyle, éthyle, n-propyle, isopropyle, n-butyle, sec-butyle, isobutyle et tert-butyle,
dans chaque cas éventuellement sous forme d'un de leurs stéréoisomères purs, en particulier de leurs énantiomères ou diastéréoisomères, de leurs racémates, ou sous forme d'un mélange de stéréoisomères, en particulier des énantiomères et/ou des diastéréoisomères, selon un rapport de mélange quelconque, ou dans chaque cas sous forme des sels correspondants, de préférence sous forme des chlorhydrates correspondants, ou dans chaque cas sous forme des produits de solvatation correspondants.

11. Composés selon l'une ou plusieurs des revendications 1 à 10, **caractérisés en ce que**
n vaut 0, 1 ou 2,
X est N ou C-R²,
R¹ est un atome d'hydrogène, un radical halogéno choisi dans le groupe consistant en F, Cl et Br, un groupe CF₃, un groupe nitro, un groupe carboxy, un groupe -C(=O)-O-CH₃, un groupe C(=O)-O-C2H₅, un groupe -C(=O)-NH-R¹⁴, un groupe -C(=O)-NR¹⁵R¹⁶, un groupe -O-R¹⁷, un groupe -S-R¹⁸, un groupe -S(=O)₂-R²⁰, un radical alkyle choisi dans le groupe consistant en les groupes méthyle, éthyle, n-propyle, isopropyle, n-butyle, sec-butyle, isobutyle et tert-butyle, ou un radical choisi dans le groupe consistant en les groupes phényle et oxadiazolyle, dont chacun est non substitué ou est substitué par éventuellement 1, 2, 3, 4 ou 5 substituants choisis indépendamment les uns des autres dans le groupe consistant en F, Cl, Br, I, -O-CH₃, -O-C₂H₅ et -O-C₃H₇,
R² est un atome d'hydrogène, un radical halogéno choisi dans le groupe consistant en F, Cl et Br, un groupe CF₃, un groupe nitro, un groupe carboxy, un groupe -C(=O)-O-CH₃, un groupe C(=O)-O-C₂H₅, un groupe -C(=O)-NH-R¹⁴, un groupe -C(=O)-NR¹⁵R¹⁶, un groupe -O-R¹⁷, un groupe -S-R¹⁸, un groupe -S(=O)₂-R²⁰, un radical alkyle choisi dans le groupe consistant en les groupes méthyle, éthyle, n-propyle, isopropyle, n-butyle, sec-butyle, isobutyle et tert-butyle, ou un radical choisi dans le groupe consistant en les groupes phényle et oxadiazolyle, dont chacun est non substitué ou est substitué par éventuellement 1, 2, 3, 4 ou 5 substituants choisis indépendamment les uns des autres dans le groupe consistant en F, Cl, Br, I, -O-CH₃, -O-C₂H₅ et -O-C₃H₇,
R³ est un groupe oxo, un radical alkyle choisi dans le groupe consistant en les groupes méthyle, éthyle, n-propyle, isopropyle, n-butyle, sec-butyle, isobutyle et tert-butyle, ou un radical phényle non substitué,
R⁴ est un atome d'hydrogène, un groupe -C(=O)-NH₂, un groupe -C (=O) -NH- R¹⁴ un groupe -C(=O)-NR¹⁵R¹⁶, un radical alkyle choisi dans le groupe consistant en les groupes méthyle, éthyle, n-propyle, isopropyle, n-butyle, sec-butyle, isobutyle, tert-butyle, n-pentyle, sec-pentyle, néopentyle, n-hexyle, n-heptyle et n-octyle, ou un radical choisi dans le groupe consistant en les groupes cyclopropyle, cyclobutyle, cyclopentyle, cyclohexyle, cycloheptyle, cyclopentényle, cyclohexényle, phényle, pyrid-2-yle, pyrid-3-yle, pyrid-4-yle, pyrrol-2-yle, pyrrol-3-yle, thiophèn-2-yle, thiophèn-3-yle, 4-méthyl-thiophèn-2-yle, 3-méthylthiophèn-2-yle, 5-méthyl-thiophèn-2-yle, furann-2-yle, furann-3-yle, imidazol-2-yle, imidazol-4-yle, thiazol-2-yle, thiazol-4-yle, thiazol-5-yle, oxazol-2-yle, oxazol-4-yle, oxazol-5-yle, (1,2,4)-thiadiazol-5-yle, (1,2,4)-oxadiazol-5-yle, napht-1-yle, napht-2-yle, indol-3-yle, indol-4-yle, indol-5-yle, indol-6-yle, indol-7-yle, 1-méthyl-indol-2-yle, 1-méthyl-indol-3-yle, 1-méthyl-indol-4-yle, 1-méthyl-indol-5-yle, 1-méthyl-indol-6-yle, 1-méthyl-indol-7-yle, quinoléin-3-yle, quinoléin-4-yle, quinoléin-2-yle, quinoléin-5-yle, quinoléin-6-yle, quinoléin-7-yle, quinoléin-8-yle, isoquinoléin-1-yle, isoquinoléin-3-yle, isoquinoléin-4-yle, isoquinoléin-5-yle, isoquinoléin-6-yle, isoquinoléin-7-yle, isoquinoléin-8-yle, 2-méthyl-phényle, 3-méthyl-phényle, 4-méthyl-phényle, 2-fluoro-phényle, 3-fluoro-phényle, 4-fluoro-phényle, 2-cyano-phényle, 3-cyano-phényle, 4-cyano-phényle, 2-hydroxy-phényle, 3-hydroxy-phényle, 4-hydroxy-phényle, 2-amino-phényle, 3-amino-phényle, 4-amino-phényle, 2-diméthylamino-phényle, 3-diméthylamino-phényle, 4-diméthylamino-phényle, 2-méthylamino-phényle, 3-méthylamino-phényle, 4-méthylamino-phényle, 2-acétyl-phényle, 3-acétyl-phényle, 4-acétyl-phényle, 2-méthylsulfinyl-phényle, 3-méthylsulfinyl-phényle, 4-méthylsulfinyl-phényle, 2-méthylsulfonyl-phényle, 3-méthylsulfonyl-phényle, 4-méthylsulfonyl-phényle, 2-méthoxy-phényle, 3-méthoxy-phényle, 4-méthoxy-phényle, 2-chloro-phényle, 3-chloro-phényle, 4-chloro-phényle, 2-éthoxy-phényle, 3-éthoxy-phényle, 4-éthoxy-phényle, 2-trifluorométhyl-phényle, 3-trifluorométhyl-phényle, 4-trifluorométhyl-phényle, 2-difluorométhyl-phényle, 3-difluorométhyl-phényle, 4-difluorométhyl-phényle, 2-fluorométhyl-phényle, 3-fluorométhyl-phényle, 4-fluorométhyl-phényle, 2-nitro-phényle, 3-nitro-phényle, 4-nitro-phényle, 2-éthyl-phényle, 3-éthyl-phényle, 4-éthyl-phényle, 2-propyl-phényle, 3-propyl-phényle, 4-propyl-phényle, 2-isopropyl-phényle, 3-isopropyl-phényle, 4-isopropyl-phényle, 2-tert-butyl-phényle, 3-tert-butyl-phényle, 4-tert-butyl-phényle, 2-carboxy-phényle, 3-carboxy-phényle, 4-carboxy-phényle, 2-éthényl-phényle, 3-éthényl-phényle, 4-éthényl-phényle, 2-éthynyl-phényle, 3-éthynyl-phényle, 4-éthynyl-phényle, 2-allyl-phényle, 3-allyl-phényle, 4-allyl-phényle, 2-triméthylsilanyléthynyl-phényle, 3-triméthylsilanyléthynyl-phényle, 4-triméthylsilanyléthynyl-phényle, 2-formyl-phényle, 3-formyl-phényle, 4-formyl-phényle, 2-acétamino-phényle, 3-acétamino-phényle, 4-acétamino-phényle, 2-acétamino-phényle, 3-acétamino-phényle, 4-acétamino-phényle, 2-diméthylaminocarbonyl-phényle, 3-diméthylaminocarbonyl-phényle, 4-diméthylaminocarbonyl-phényle, 2-méthoxyméthyl-phényle, 3-méthoxyméthyl-phényle, 4-méthoxyméthyl-phényle, 2-éthoxyméthyl-phényle, 3-éthoxyméthyl-phényle, 4-éthoxyméthyl-phényle, 2-aminocarbonyl-phényle, 3-aminocarbonyl-phényle, 4-aminocarbonyl-phényle, 2-méthylaminocarbonyl-phényle, 3-méthylaminocarbonyl-phényle, 4-méthylaminocarbonyl-phényle, 2-carboxyméthylester-phényle, 3-carboxyméthylester-phényle, 4-carboxyméthylester-phényle, 2-carboxyéthylester-phényle, 3-carboxyéthylester-phényle, 4-carboxyéthylester-phényle, 2-carboxy-tert-butylester-phényle, 3-carboxy-tert-butylester-phényle, 4-carboxy-tert-butylester-phényle, 2-méthylmercapto-phényle, 3-méthylmercapto-phényle, 4-méthylmercapto-phényle, 2-éthylmercapto-phényle, 3-éthylmercapto-phényle, 4-éthylmercapto-phényle, 2-biphényle, 3-biphényle, 4-biphényle, 2-bromophényle, 3-bromophényle, 4-bromophényle, 2-iodo-phényle, 3-iodo-phényle, 4-iodo-phényle, 2-trifluorométhoxy-phényle, 3-trifluorométhoxy-phényle, 4-trifluorométhoxy-phényle, 2-fluoro-4-méthyl-phényle, (2,3)-diméthyl-phényle, (2,4)-dichloro-phényle, (2,4)-difluorophényle, 2-chloro-4-méthyl-phényle, 2-chloro-5-trifluorométhyl-phényle, 2-chloro-5-méthoxy-phényle, 2-bromo-5-trifluorométhyl-phényle, 2-bromo-5-méthoxy-phényle, (2,4)-diméthyl-phényle, (2,6)-diméthyl-phényle, 3-chloro-5-méthyl-phényle, (3,4)-diméthyl-phényle, 3-méthyl-4-méthoxy-phényle, (3,4)-difluoro-phényle, 3-cyano-4-fluoro-phényle, 3-cyano-4-méthyl-phényle, 3-cyano-4-méthoxy-phényle, 3-bromo-4-fluoro-phényle, 3-bromo-4-méthyl-phényle, 3-bromo-4-méthoxy-phényle, 4-chloro-2-fluoro-phényle, 4-fluoro-3-méthyl-phényle, 3-fluoro-4-méthyl-phényle, 3-fluoro-5-méthyl-phényle, 2-fluoro-3-méthyl-phényle, (3,5)-diméthyl-phényle et (3,5)-dichloro-phényle,
et
R¹⁴, R¹⁵, R¹⁶, R¹⁷, R¹⁸ et R²⁰ représentent chacun indépendamment des autres un radical alkyle choisi dans le groupe consistant en les groupes méthyle, éthyle, n-propyle, isopropyle, n-butyle, sec-butyle, isobutyle et tert-butyle ;
dans chaque cas éventuellement sous forme d'un de leurs stéréoisomères purs, en particulier de leurs énantiomères ou diastéréoisomères, de leurs racémates, ou sous forme d'un mélange de stéréoisomères, en particulier des énantiomères et/ou des diastéréoisomères, selon un rapport de mélange quelconque, ou dans chaque cas sous forme des sels correspondants, de préférence sous forme des chlorhydrates correspondants, ou dans chaque cas sous forme des produits de solvatation correspondants.

12. Composés selon l'une ou plusieurs des revendications 1 à 11, choisis dans le groupe consistant en les groupes suivants :
| | |
|---|---|
| [1] | 1-(3-phényl-propiolyl)-4-(thiazol-2-yl)-pipérazine |
| [AAA00100] | 4-(3-méthylmercapto-1,2,4-thiadiazol-5-yl)-1-(3-phényl-propiolyl)-pipérazine |
| [AAA00101] | 4-(3-méthanesulfonyl-1,2,4-thiadiazol-5-yl)-1-(3-phényl-propiolyl)-pipérazine |
| [AAA00102] | 4-(3-méthoxy-1,2,4-thiadiazol-5-yl)-1-(3-phényl-propiolyl)-pipérazine, |
| [AAA00103] | 4-(1,2,4-thiadiazol-5-yl)-1-(3-phényl-propiolyl)-pipérazin |
| [AAA00104] | 4-(3-méthyl-1,2,4-thiadiazol-5-yl)-1-(3-phényl-propiolyl)-pipérazine |
| [AAA00105] | 4-(3-trifluorométhyl-1,2,4-thiadiazol-5-yl)-1-(3-phényl-propiolyl)-pipérazine |
| [AAA00106] | 4-(5-(3-méthyl-1,2,4-oxadiazol-5-yl)-thiazol-2-yl)-1-(3-phényl-propiolyl)-pipérazine |
| [AAA00107] | 4-(4-tert-butyl-thiazol-2-yl)-1-(3-(4-amino-phényl)-propiolyl)-pipérazine |
| [AAA1] | ester méthylique de la 4-(thiazol-2-yl)-1-(3-(2-carboxy-phényl)-propiolyl)-pipérazine |
| [AAA10] | 4-(thiazol-2-yl)-1-(3-(2-thiényl)-propiolyl)-pipérazin |
| [AAA11] | 4-(thiazol-2-yl)-1-(3-(3-thiényl)-propiolyl)-pipérazine |
| [AAA12] | 4-(thiazol-2-yl)-1-(3-(2-méthoxy-phényl)-propiolyl)-pipérazine |
| [AAA13] | 4-(thiazol-2-yl)-1-(3-(3-méthoxy-phényl)-propiolyl)-pipérazine |
| [AAA14] | 4-(thiazol-2-yl)-1-(3-(4-méthoxy-phényl)-propiolyl)-pipérazine |
| [AAA15] | 4-(thiazol-2-yl)-1-(3-(2-cyano-phényl)-propiolyl)-pipérazine |
| [AAA16] | 4-(thiazol-2-yl)-1-(3-(3-cyano-phényl)-propiolyl)-pipérazine |
| [AAA17] | 4-(thiazol-2-yl)-1-(3-(4-cyano-phényl)-propiolyl)-pipérazine |
| [AAA18] | 4-(thiazol-2-yl)-1-(3-(2,4-diméthyl-phényl)-propiolyl)-pipérazine |
| [AAA19] | 4-(thiazol-2-yl)-1-(3-(3,5-diméthyl-phényl)-propiolyl)-pipérazine |
| [AAA2] | ester méthylique de la 4-(thiazol-2-yl)-1-(3-(4-carboxy-phényl)-propiolyl)-pipérazine |
| [AAA20] | 4-(thiazol-2-yl)-1-(3-(2,6-diméthyl-phényl)-propiolyl)-pipérazine |
| [AAA21] | 4-(thiazol-2-yl)-1-(3-(2-fluoro-phényl)-propiolyl)-pipérazine |
| [AAA22] | 4-(thiazol-2-yl)-1-(3-(3-fluoro-phényl)-propiolyl)-pipérazine |
| [AAA23] | 4-(thiazol-2-yl)-1-(3-(2-chloro-phényl)-propiolyl)-pipérazine |
| [AAA24] | 4-(thiazol-2-yl)-1-(3-(3-chloro-phényl)-propiolyl)-pipérazine |
| [AAA25] | 4-(thiazol-2-yl)-1-(3-naphtyl-propiolyl)-pipérazine |
| [AAA26] | 4-(thiazol-2-yl)-1-(3-(2,3-diméthyl-phényl)-propiolyl)-pipérazine |
| [AAA27] | 4-(thiazol-2-yl)-1-(3-(3,4-diméthyl-phényl)-propiolyl)-pipérazine |
| [AAA28] | 4-(thiazol-2-yl)-1-(3-(3-nitro-phényl)-propiolyl)-pipérazine |
| [AAA29] | 4-(thiazol-2-yl)-1-(3-(2-nitro-phényl)-propiolyl)-pipérazine |
| [AAA3] | ester éthylique de la 4-(thiazol-2-yl)-1-(3-(3-carboxy-phényl)-propiolyl)-pipérazine |
| [AAA30] | 4-(thiazol-2-yl)-1-(3-(3-formyl-phényl)-propiolyl)-pipérazine |
| [AAA31] | 4-(thiazol-2-yl)-1-(3-(3-éthényl-phényl)-propiolyl)-pipérazine |
| [AAA32] | 4-(thiazol-2-yl)-1-(3-pyrid-2-yl-propiolyl)-pipérazine |
| [AAA33] | 4-(thiazol-2-yl)-1-(3-pyrid-3-yl-propiolyl)-pipérazine |
| [AAA34] | 4-(thiazol-2-yl)-1-(3-pyrid-4-yl-propiolyl)-pipérazine |
| [AAA35] | 4-(thiazol-2-yl)-1-(3-(quinoléin-6-yl)-propiolyl)-pipérazine |
| [AAA36] | 4-(thiazol-2-yl)-1-(3-(3-isopropyl-phényl)-propiolyl)-pipérazine |
| [AAA37] | 4-(thiazol-2-yl)-1-(3-biphényl-3-yl-propiolyl)-pipérazine |
| [AAA38] | 4-(thiazol-2-yl)-1-(3-napht-2-yl-propiolyl)-pipérazine |
| [AAA39] | 4-(thiazol-2-yl)-1-(3-(1-méthyl-indol-5-yl)-propiolyl)-pipérazine |
| [AAA4] | 4-(thiazol-2-yl)-1-(3-(2-hydroxy-phényl)-propiolyl)-pipérazine |
| [AAA40] | 4-(thiazol-2-yl)-1-(3-(3-méthylmercapto-phényl)-propiolyl)-pipérazine |
| [AAA41] | 4-(thiazol-2-yl)-1-(3-(3-cyano-4-fluoro-phényl)-propiolyl)-pipérazine |
| [AAA42] | 4-(thiazol-2-yl)-1-(3-(3-méthoxyméthyl-phényl)-propiolyl)-pipérazine |
| [AAA43] | 4-(thiazol-2-yl)-1-(3-(3-hydroxy-phényl)-propiolyl)-pipérazine |
| [AAA44] | 4-(thiazol-2-yl)-1-(3-(3-acétamino-phényl)-propiolyl)-pipérazine |
| [AAA45] | 4-(thiazol-2-yl)-1-(3-(4-acétamino-phényl)-propiolyl)-pipérazine |
| [AAA46] | 4-(thiazol-2-yl)-1-(3-(2-carboxy-phényl)-propiolyl)-pipérazine |
| [AAA47] | 4-(thiazol-2-yl)-1-(3-(3-carboxy-phényl)-propiolyl)-pipérazine |
| [AAA48] | 4-(thiazol-2-yl)-1-(3-(4-carboxy-phényl)-propiolyl)-pipérazin |
| [AAA49] | ester méthylique de la 4-(thiazol-2-yl)-1-(3-(3-carboxy-phényl)-propiolyl)-pipérazine |
| [AAA5] | 4-(thiazol-2-yl)-1-(3-(4-hydroxy-phényl)-propiolyl)-pipérazine |
| [AAA50] | 4-(thiazol-2-yl)-1-(3-(3-aminocarbonyl-phényl)-propiolyl)-pipérazine |
| [AAA51] | 4-(thiazol-2-yl)-1-(3-(3-méthylaminocarbonyl-phényl)-propiolyl)-pipérazine |
| [AAA52] | 4-(thiazol-2-yl)-1-(3-(3-diméthylamino-carbonyl-phényl)-propiolyl)-pipérazine |
| [AAA53] | chlorhydrate de 4-(thiazol-2-yl)-1-(3-(2-tolyl)-propiolyl)-pipérazine |
| [AAA54] | chlorhydrate de 4-(thiazol-2-yl)-1-(3-(3-tolyl)-propiolyl)-pipérazine |
| [AAA55] | chlorhydrate de 4-(thiazol-2-yl)-1-(3-(4-tolyl)-propiolyl)-pipérazine |
| [AAA56] | chlorhydrate de 4-(thiazol-2-yl)-1-(3-phényl-propiolyl)-pipérazine |
| [AAA57] | chlorhydrate de 4-(thiazol-2-yl)-1-(3-(2-trifluorométhyl-phényl)-propiolyl)-pipérazine |
| [AAA58] | chlorhydrate de 4-(thiazol-2-yl)-1-(3-(3-trifluorométhyl-phényl)-propiolyl)-pipérazine |
| [AAA59] | chlorhydrate de 4-(thiazol-2-yl)-1-(3-(4-trifluorométhyl-phényl)-propiolyl)-pipérazine |
| [AAA6] | 4-(thiazol-2-yl)-1-(3-(2-amino-phényl)-propiolyl)-pipérazine |
| [AAA60] | 4-(thiazol-2-yl)-1-(3-pentyl-propiolyl)-pipérazine |
| [AAA61] | 4-(thiazol-2-yl)-1-(3-(4-fluoro-phényl)-propiolyl)-pipérazine |
| [AAA62] | 4-(thiazol-2-yl)-1-(3-(4-chloro-phényl)-propiolyl)-pipérazine |
| [AAA63] | 2-méthyl-4-(thiazol-2-yl)-1-(3-phényl-propiolyl)-pipérazine |
| [AAA64] | (S)-2-méthyl-4-(thiazol-2-yl)-1-(3-phényl-propiolyl)-pipérazine |
| [AAA65] | (R)-2-méthyl-4-(thiazol-2-yl)-1-(3-phényl-propiolyl)-pipérazine |
| [AAA66] | chlorhydrate de 2-méthyl-4-(thiazol-2-yl)-1-(3-phényl-propiolyl)-pipérazine |
| [AAA67] | 2-méthyl-4-(thiazol-2-yl)-1-(3-(3-tolyl)-propiolyl)-pipérazine |
| [AAA68] | chlorhydrate de 2-méthyl-4-(thiazol-2-yl)-1-(3-(3-tolyl)-propiolyl)-pipérazine |
| [AAA69] | 4-(thiazol-2-yl)-1-(3-cyclohexyl-propiolyl)-pipérazine |
| [AAA7] | 4-(thiazol-2-yl)-1-(3-(3-amino-phényl)-propiolyl)-pipérazine |
| [AAA70] | 4-(thiazol-2-yl)-1-(3-méthyl-propiolyl)-pipérazine |
| [AAA71] | 2-éthyl-4-(thiazol-2-yl)-1-(3-phényl-propiolyl)-pipérazine |
| [AAA72] | 2-phényl-4-(thiazol-2-yl)-1-(3-phényl-propiolyl)-pipérazine |
| [AAA73] | 4-(thiazol-2-yl)-1-(3-(2-furyl)-propiolyl)-pipérazine |
| [AAA74] | 4-(thiazol-2-yl)-1-(3-(3-furyl)-propiolyl)-pipérazine |
| [AAA75] | cis-2,6-diméthyl-4-(thiazol-2-yl)-1-(3-phényl-propiolyl)-pipérazine |
| [AAA76] | ester éthylique de la 4-(5-carboxy-thiazol-2-yl)-1-(3-phényl-propiolyl)-pipérazine |
| [AAA77] | ester éthylique de la 4-(4-carboxy-thiazol-2-yl)-1-(3-phényl-propiolyl)-pipérazine |
| [AAA78] | 4-(5-carboxy-thiazol-2-yl)-1-(3-phényl-propiolyl)-pipérazine |
| [AAA79] | 4-(4-carboxy-thiazol-2-yl)-1-(3-phénylpropiolyl)-pipérazine |
| [AAA8] | 4-(thiazol-2-yl)-1-(3-(4-amino-phényl)-propiolyl)-pipérazine |
| [AAA80] | 4-(5-méthylaminocarbonyl-thiazol-2-yl)-1-(3-phényl-propiolyl)-pipérazine |
| [AAA81] | 4-(5-diméthylaminocarbonyl-thiazol-2-yl)-1-(3-phényl-propiolyl)-pipérazine |
| [AAA82] | 4-(3-phényl-1,2,4-thiadiazol-5-yl)-1-(3-phényl-propiolyl)-pipérazine |
| [AAA83] | 4-(thiazol-2-yl)-1-(3-(quinol-7-yl)-propiolyl)-pipérazine |
| [AAA84] | 4-(thiazol-2-yl)-1-(3-phényl-propiolyl)-pipérazine-3-one |
| [AAA85] | 4-(3-(4-fluorobenzyl)-1,2,4-thiadiazol-5-yl)-1-(3-phényl-propiolyl)-pipérazine |
| [AAA86] | 4-(5-nitro-thiazol-2-yl)-1-(3-phénylpropiolyl)-pipérazine |
| [AAA87] | 4-(4-tert-butyl-thiazol-2-yl)-1-(3-phénylpropiolyl)-pipérazine |
| [AAA88] | 4-(5-méthyl-thiazol-2-yl)-1-(3-phénylpropiolyl)-pipérazine |
| [AAA89] | 4-(4,5-diméthyl-thiazol-2-yl)-1-(3-phénylpropiolyl)-pipérazine |
| [AAA9] | 4-(thiazol-2-yl)-1-(3-(indol-5-yl)-propiolyl)-pipérazine |
| [AAA90] | 4-(5-bromo-4-phényl-thiazol-2-yl)-1-(3-phényl-propiolyl)-pipérazine |
| [AAA91] | 4-(5-bromo-4-méthyl-thiazol-2-yl)-1-(3-phényl-propiolyl)-pipérazine |
| [AAA92] | 4-(4-méthyl-thiazol-2-yl)-1-(3-phénylpropiolyl)-pipérazine |
| [AAA93] | 4-(4-trifluorométhyl-thiazol-2-yl)-1-(3-phényl-propiolyl)-pipérazine |
| [AAA94] | 4-(4-chloro-thiazol-2-yl)-1-(3-phénylpropiolyl)-pipérazine |
| [AAA195] | 4-(5-chloro-thiazol-2-yl)-1-(3-phénylpropiolyl)-pipérazine |
| [AAA96] | 4-(4-bromo-thiazol-2-yl)-1-(3-phénylpropiolyl)-pipérazine |
| [AAA97] | 4-(5-bromo-thiazol-2-yl)-1-(3-phénylpropiolyl)-pipérazine |
| [AAA98] | 4-(5-phényl-thiazol-2-yl)-1-(3-phénylpropiolyl)-pipérazine |
| [AAA99] | 4-(4-phényl-thiazol-2-yl)-1-(3-phénylpropiolyl)-pipérazine |
| [BBB2] | 4-(thiazol-2-yl)-1-propiolyl-pipérazine ; |
| [CCC1] | 4-(3-phényl-1,2,4-thiadiazol-5-yl)-1-(3-(2-méthoxy-phényl)-propiolyl)-pipérazine |
| [CCC2] | 4-(3-phényl, 1,2,4-thiadiazol-5-yl)-1-(3-(4-méthoxy-phényl)-propiolyl)-pipérazine |
| [CCC3] | 4-(3-phényl-1,2,4-thiadiazol-5-yl)-1-(3-(2-fluoro-phényl)-propiolyl)-pipérazine |
| [CCC4] | 4-(3-phényl-1,2,4-thiadiazol-5-yl)-1-(3-(3-fluoro-phényl)-propiolyl)-pipérazine |
| [CCC5] | 4-(3-phényl-1,2,4-thiadiazol-5-yl)-1-(3-(4-fluoro-phényl)-propiolyl)-pipérazine |
| [CCC6] | 4-(3-phényl-1,2,4-thiadiazol-5-yl)-1-(3-(2,4-dichloro-phényl)-propiolyl)-pipérazine |
| [CCC7] | 4-(3-phényl-1,2,4-thiadiazol-5-yl)-1-(3-(3,5-dichloro-phényl)-propiolyl)-pipérazine |
| [CCC8] | 4-(3-(4-fluoro-phényl)-1,2,4-thiadiazol-5-yl)-1-(3-(tol-2-yl)-propiolyl)-pipérazine |
| [CCC9] | 4-(3-(4-fluoro-phényl)-1,2,4-thiadiazol-5-yl)-1-(3-(3-méthoxy-phényl)-propiolyl)-pipérazine |
| [CCC10] | 4-(3-(4-fluoro-phényl)-1,2,4-thiadiazol-5-yl)-1-(3-(3-fluoro-4-méthyl-phényl)-propiolyl)-pipérazine |
| [CCC11] | 4-(3-(4-fluoro-phényl)-1,2,4-thiadiazol-5-yl)-1-(3-(2,4-difluoro-phényl)-propiolyl)-pipérazine |
| [CCC12] | 4-(3-(4-fluoro-phényl)-1,2,4-thiadiazol-5-yl)-1-(3-(tol-3-yl)-propiolyl)-pipérazine |
| [CCC13] | 4-(3-(4-fluoro-phényl)-1,2,4-thiadiazol-5-yl)-1-(3-(4-trifluorométhyl-phényl)-propiolyl)-pipérazine |
| [CCC14] | 4-(3-(4-fluoro-phényl)-1,2,4-thiadiazol-5-yl)-1-(3-(3-trifluorométhyl-phényl)-propiolyl)-pipérazine |
| [CCC15] | 4-(3-(4-fluoro-phényl)-1,2,4-thiadiazol-5-yl)-1-(3-(2-chloro-5-trifluorométhyl-phényl)-propiolyl)-pipérazine |
| [CCC16] | 4-(3-tert-butyl-1,2,4-thiadiazol-5-yl)-1-(3-thiophèn-2-yl)-propiolyl)-pipérazine |
| [CCC17] | 4-(3-tert-butyl-1,2,4-thiadiazol-5-yl)-1-(3-(3-trifluorométhyl-phényl)-propiolyl)-pipérazine |
| [CCC18] | 4-(3-tert-butyl-1,2,4-thiadiazol-5-yl)-1-(3-(2-méthoxy-phényl)-propiolyl)-pipérazine |
| [CCC19] | 4-(3-tert-butyl-1,2,4-thiadiazol-5-yl)-1-(3-(tol-2-yl)-propiolyl)-pipérazine |
| [CCC20] | 4-(3-tert-butyl-1,2,4-thiadiazol-5-yl)-1-(3-(3-fluoro-4-méthyl-phényl)-propiolyl)-pipérazine |
| [CCC21] | 4-(3-tert-butyl-1,2,4-thiadiazol-5-yl)-1-(3-(2-chloro-5-trifluorométhyl-phényl)-propiolyl)-pipérazine |
| [CCC22] | 4-(thiazol-2-yl)-1-(3-(2,4-difluorophényl)-propiolyl)-pipérazine |
| [CCC23] | 4-(thiazol-2-yl)-1-(3-(2-bromo-5-méthoxyphényl)-propiolyl)-pipérazine |
| [CCC24] | 4-(thiazol-2-yl)-1-(3-(3-bromo-4-méthoxyphényl)-propiolyl)-pipérazine |
| [CCC25] | 4-(thiazol-2-yl)-1-(3-(3,5-dichlorophényl)-propiolyl)-pipérazine |
| [CCC26] | 4-(thiazol-2-yl)-1-(3-(4-fluoro-3-méthylphényl)-propiolyl)-pipérazine |
| [CCC27] | 4-(4-tert-butyl-thiazol-2-yl)-1-(3-(3-cyano-phényl)-propiolyl)-pipérazine |
| [CCC28] | 4-(4-tert-butyl-thiazol-2-yl)-1-(3-(2-trifluorométhyl-phényl)-propiolyl)-pipérazine |
| [CCC29] | 4-(4-phényl-thiazol-2-yl)-1-(3-(2,3-diméthyl-phényle)-propiolyl)-pipérazine |
| [CCC30] | 4-(4-phényl-thiazol-2-yl)-1-(3-(4-fluorophényl)-propiolyl)-pipérazine |
| [CCC31] | 4-(4-méthyl-thiazol-2-yl)-1-(3-(tol-3-yl)-propiolyl)-pipérazine |
| [CCC32] | 4-(4-méthyl-thiazol-2-yl)-1-(3-(thiophèn-2-yl)-propiolyl)-pipérazine |
| [CCC33] | 4-(5-méthyl-thiazol-2-yl)-1-(3-(tol-4-yl)-propiolyl)-pipérazine |
| [CCC34] | 4-(5-méthyl-thiazol-2-yl)-1-(3-(2-cyanophényl)-propiolyl)-pipérazine |
| [CCC35] | 4-(4,5-diméthyl-thiazol-2-yl)-1-(3-(3-cyano-phényl)-propiolyl)-pipérazine |
| [CCC36] | 4-(4,5-diméthyl-thiazol-2-yl)-1-(3-(3,4-diméthyl-phényl)-propiolyl)-pipérazine |
| [CCC37] | 4-(4-(4-méthoxy-phényl)-thiazol-2-yl)-1-(3-(2,4-diméthyl-phényl)-propiolyl)-pipérazine |
| [CCC38] | 4-(4-(4-méthoxy-phényl)-thiazol-2-yl)-1-(3-(4-trifluorométhyl-phényl)-propiolyl)-pipérazine |
| [CCC39] | 4-(4-(4-fluoro-phényle)-thiazol-2-yl)-1-(3-(2-cyano-phényl)-propiolyl)-pipérazine |
| [CCC40] | 4-(4-(4-chloro-phényl)-thiazol-2-yl)-1-(3-(4-cyano-phényl)-propiolyl)-pipérazine |
| [AAA00108] | 4-(thiazol-2-yl)-1-(3-(1-méthyl-indol-6-yl)-propiolyl)-pipérazine |
| [AAA00109] | 4-(thiazol-2-yl)-1-(3-(3-acétyl-phényl)-propiolyl)-pipérazine |
| [AAA00110] | 4-(thiazol-2-yl)-1-(3-(3-fluoro-5-méthylphényl)-propiolyl)-pipérazine |
| [AAA00111] | 4-(thiazol-2-yl)-1-(3-(2-fluoro-3-méthyl-phényl)-propiolyl)-pipérazin |
| [AAA00112] | 4-(thiazol-2-yl)-1-(3-(3-méthylaminophényl)-propiolyl)-pipérazine |
| [AAA0113] | 4-(thiazol-2-yl)-1-(3-(3-diméthylaminophényl)-propiolyl)-pipérazine |
| [AAA00114] | 4-(thiazol-2-yl)-1-(diméthylcarbamoylpropiolyl)-pipérazine |
| [AAA00115] | 4-(thiazol-2-yl)-1-(3-(3-méthylsulfinylphényl)-propiolyl)-pipérazine |
| [AAA00116] | 4-(thiazol-2-yl)-1-(3-(3-méthylsulfonylphényl)-propiolyl)-pipérazine |
| [AAA00117] | 4-(thiazol-2-yl)-1-(3-(3-éthynyl-phényl)-propiolyl)-pipérazine |
| [AAA00118] | 4-(thiazol-2-yl)-1-(3-(4-méthyl-thiophèn-2-yl)-propiolyl)-pipérazine |
| [AAA00119] | 2-méthyl-4-(thiazol-2-yl)-1-(3-(3-chlorophényl)-propiolyl)-pipérazine |
| [AAA00120] | 4-(thiazol-2-yl)-1-(3-(3-éthyl-phényl)-propiolyl)-pipérazine |
| [AAA00121] | 2-tert-butyl-4-(thiazol-2-yl)-1-(3-phénylpropiolyl)-pipérazine |
| [AAA00122] | 4-(thiazol-2-yl)-1-(3-(3-difluorométhylphényl)-propiolyl)-pipérazine |
| [AAA00123] | 2-méthyl-4-(thiazol-2-yl)-1-(3-(3-cyanophényl)-propiolyl)-pipérazine |
| [AAA00124] | 2-isopropyl-4-(thiazol-2-yl)-1-(3-phénylpropiolyl)-pipérazine |
| [AAA00125] | 4-(thiazol-2-yl)-1-(3-(3-triméthylsilanyléthynyl-phényl)-propiolyl)-pipérazine ; |
dans chaque cas éventuellement sous forme d'un de leurs stéréoisomères purs, en particulier de leurs énantiomères ou diastéréoisomères, de leurs racémates, ou sous forme d'un mélange de stéréoisomères, en particulier des énantiomères et/ou des diastéréoisomères, selon un rapport de mélange quelconque, ou dans chaque cas sous forme des sels correspondants, de préférence sous forme des chlorhydrates correspondants, ou dans chaque cas sous forme des produits de solvatation correspondants.

13. Procédé de préparation de composés de formule générale I selon l'une ou plusieurs des revendications 1 à 12, **caractérisé en ce qu'**on fait réagir au moins un composé de formule générale II, 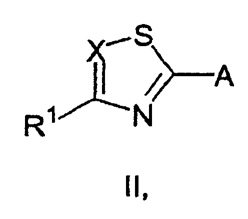 dans laquelle les radicaux X et R¹ ont les significations selon l'une ou plusieurs des revendications 1 à 12 et A est un groupe partant, de préférence un radical halogéno ou un ester d'acide sulfonique, d'une manière particulièrement préférée un radical chloro ou bromo, avec au moins un composé de formule générale III, 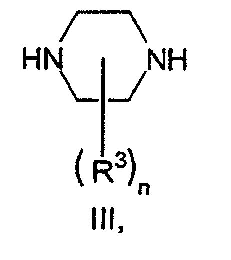 dans laquelle R³ et n ont les significations selon l'une ou plusieurs des revendications 1 à 12, éventuellement dans un milieu réactionnel, éventuellement en présence d'au moins une base et/ou d'au moins un composé organométallique et/ou d'au moins un réactif de type hydrure métallique, de préférence à une température de -70 à 300°C, de préférence de -70 à 150°C, pour obtenir au moins un composé correspondant de formule générale IV, éventuellement sous forme d'un sel correspondant, 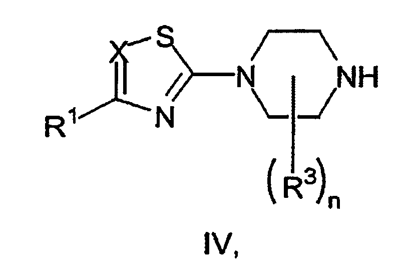 où X, R¹, R³ et n ont les significations données ci-dessus, et éventuellement on purifie et/ou isole ce dernier ;
ou on fait réagir au moins un composé de formule générale II avec au moins un composé de formule générale V, 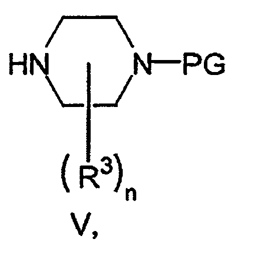 où R³ et n ont les significations selon l'une ou plusieurs des revendications 1 à 12 et PG est un groupe protecteur, de préférence un groupe protecteur choisi dans le groupe consistant en les groupes tert-butyloxycarbonyle, benzyle, benzyloxycarbonyle et 9-fluorénylméthyloxycarbonyle, éventuellement dans un milieu réactionnel, éventuellement en présence d'au moins une base et/ou d'au moins un composé organométallique et/ou d'au moins un réactif de type hydrure métallique, de préférence à une température de -70 à 300°C, pour obtenir au moins un composé correspondant de formule générale VI, 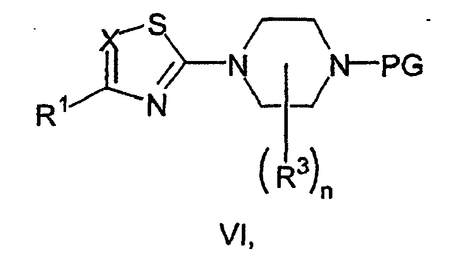 où R¹, R³, X, n et PG ont les significations données ci-dessus, et éventuellement on purifie et/ou isole ce dernier ;
ou on convertit un composé de formule générale VII, 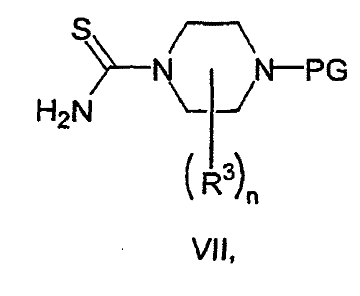 où R³ et n ont les significations selon l'une ou plusieurs des revendications 1 à 12 et PG est un groupe protecteur, de préférence un groupe protecteur choisi dans le groupe consistant en les groupes tert-butyloxycarbonyle, benzyle, benzyloxycarbonyle et 9-fluorénylméthyloxycarbonyle, par réaction avec au moins un composé de formule générale R¹-C(=O)-CH₂-A ou (alkyle en C₁₋₅-O)₂-CH-CH₂-A, où R¹ a les significations selon l'une ou plusieurs des revendications 1 à 12 et A est un groupe partant, de préférence un radical halogéno, d'une manière particulièrement préférée un atome de brome, dans un milieu réactionnel, de préférence en présence d'au moins une base organique, ou en présence d'au moins un acide, de préférence en présence d'au moins une base choisie dans le groupe consistant en la triéthylamine, la diisopropyléthylamine, la N-méthylmorpholine, la diméthylaminopyridine et la pyridine ou en présence d'au moins un acide choisi dans le groupe consistant en l'acide acétique, l'acide trifluoracétique et l'acide chlorhydrique, de préférence à une température comprise entre -70 et 300°C, en au moins un composé correspondant de formule générale VI, éventuellement sous forme d'un sel correspondant, où R¹, R³ et n ont les significations selon l'une ou plusieurs des revendications 1 à 12, PG a les significations données ci-dessus et X représente CH, et éventuellement on purifie et/ou isole ce dernier ;
et on fait réagir au moins un composé de formule générale VI, dans le cas dans lequel PG est un groupe tert-butoxycarbonyle ou 9-fluorénylméthyloxycarbonyle, dans un milieu réactionnel, en présence d'au moins un acide, de préférence en présence d'au moins un acide choisi dans le groupe consistant en l'acide chlorhydrique et l'acide trifluoracétique, de préférence à une température comprise entre -70 et 100°C, ou dans le cas dans lequel PG est un groupe benzyle ou un groupe benzyloxycarbonyle, dans un milieu réactionnel, en présence d'hydrogène et en présence d'au moins un catalyseur, de préférence en présence de catalyseur sur charbon, de préférence à une température comprise entre -70 et 100°C, en au moins un composé correspondant de formule générale IV, éventuellement sous forme d'un sel correspondant, où X, R¹, R³ et n ont les significations selon l'une ou plusieurs des revendications 1 à 12, et éventuellement on purifie et/ou isole ce dernier ;
et on convertit au moins un composé de formule générale IV, par réaction avec au moins un composé de formule générale R⁴-C≡C-C(=O)-OH, où R⁴ a les significations selon l'une ou plusieurs des revendications 1 à 12, dans un milieu réactionnel, éventuellement en présence d'au moins un agent de couplage approprié, éventuellement en présence d'au moins une base, de préférence à une température de -70 à 100°C, ou par réaction avec au moins un composé de formule générale R⁴-C≡C-C(=O)-A, où R⁴ a les significations données ci-dessus et A est un groupe partant, de préférence un radical halogéno, d'une manière particulièrement préférée un radical chloro ou bromo, dans un milieu réactionnel, éventuellement en présence d'une base, de préférence à une température de -70 à 100°C, en au moins un composé correspondant de formule générale I, éventuellement sous forme d'un sel correspondant, 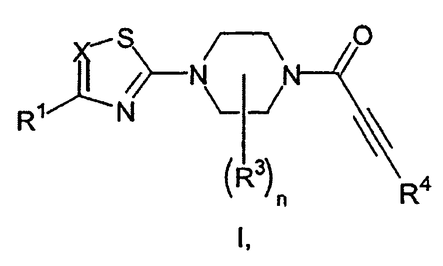 où X et R¹, R³, R⁴ et n ont les significations données ci-dessus, et éventuellement on purifie et/ou isole ce dernier ;
ou on convertit au moins un composé de formule générale IV par réaction avec de l'acide propynique [HC≡C-C(=O)-OH] dans un milieu réactionnel, éventuellement en présence d'au moins un agent de couplage approprié, éventuellement en présence d'au moins une base, de préférence à une température de -70 à 100°C, ou par réaction avec au moins un composé de formule générale HC=C-C(=O)-A, où A est un groupe partant, de préférence un radical halogéno, d'une manière particulièrement préférée un radical chloro ou bromo, dans un milieu réactionnel, éventuellement en présence d'au moins une base, de préférence à une température de -70 à 100°C, en au moins un composé correspondant de formule générale VIII, éventuellement sous forme d'un sel correspondant, 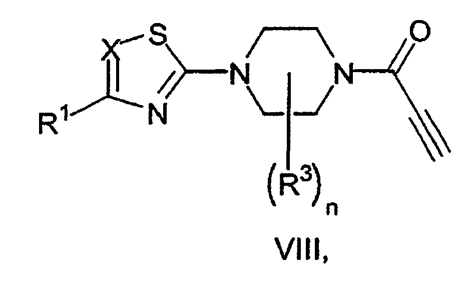 où R¹, R³, X et n ont les significations données ci-dessus, et éventuellement on purifie et/ou isole ce dernier,
et on convertit au moins un composé de formule générale VIII par réaction avec au moins un composé de formule générale R⁴-A, où R⁴ a les significations selon l'une ou plusieurs des revendications 10 à 13 à l'exception de l'hydrogène, et A est un groupe partant, de préférence un radical halogéno ou un ester d'acide sulfonique, d'une manière particulièrement préférée l'iode, le brome ou le triflate, dans un milieu réactionnel, éventuellement en présence d'au moins un catalyseur, de préférence en présence d'un catalyseur au palladium choisi dans le groupe consistant en le chlorure de palladium [PdCl₂], l'acétate de palladium [Pd(OAc)₂], le tétrakistriphénylphosphinepalladium [Pd(PPh₃)₄], le dichlorure de bistriphénylphosphinepalladium [Pd(PPh₃)₂Cl₂] et l'acétate de bistriphénylphosphinepalladium [Pd(PPh₃)₂(OAc)₂], éventuellement en présence d'au moins un ligand, de préférence en présence d'au moins un ligand choisi dans le groupe consistant en la triphénylphosphine, la triphénylarsine et la tri-2-furyl-phosphine, éventuellement en présence d'au moins un sel inorganique, de préférence en présence d'au moins un sel inorganique choisi dans le groupe consistant en le chlorure de lithium et le chlorure de zinc, éventuellement en présence d'au moins un sel de cuivre, de préférence en présence d'iodure de cuivre, éventuellement en présence d'au moins une base organique ou inorganique, de préférence en présence d'au moins une base choisie dans le groupe consistant en la triéthylamine, le [1,4]-diazabicyclo-[2.2.2]-octane, la diisopropylamine, la diisopropyléthylamine, le carbonate de potassium et l'hydrogénocarbonate de sodium, de préférence à une température comprise entre -70 et 300°C, en au moins un composé correspondant de formule générale I, éventuellement sous forme d'un sel correspondant, et éventuellement on purifie et/ou isole ce dernier.

14. Procédé de préparation de composés de formule générale I selon l'une ou plusieurs des revendications 1 à 12, **caractérisé en ce qu'**on convertit au moins un composé de formule générale III, 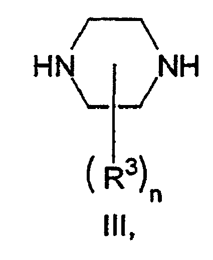 dans laquelle R³ et n ont les significations selon l'une ou plusieurs des revendication 1 à 12, par réaction avec au moins un composé de formule générale R⁴-C≡C-C(=O)-OH, où R⁴ a les significations selon l'une ou plusieurs des revendications 1 à 12, dans un milieu réactionnel, éventuellement en présence d'au moins un agent de couplage approprié, éventuellement en présence d'au moins une base, de préférence à une température comprise entre -70 et 100°C, ou par réaction avec au moins un composé de formule générale R⁴-C≡C-C(=O)-A, où R⁴ a les significations données ci-dessus et A est un groupe partant, de préférence un radical halogéno, d'une manière particulièrement préférée un radical chloro ou bromo, dans un milieu réactionnel, éventuellement en présence d'au moins une base, de préférence à une température comprise entre -70 et 100°C, en au moins un composé correspondant de formule générale IX, éventuellement sous forme d'un sel correspondant, 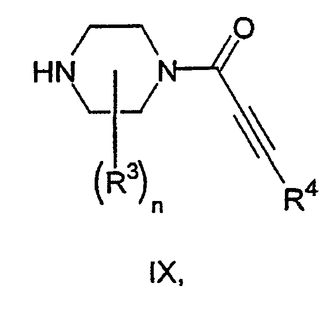 où R³, R⁴ et n ont les significations données ci-dessus, et éventuellement on purifie et/ou on isole ce dernier ;
ou on convertit au moins un composé de formule générale V, 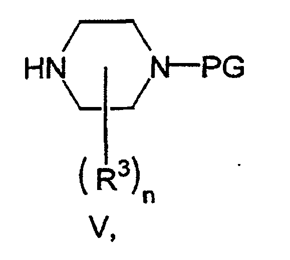 où R³ et n ont les significations selon l'une ou plusieurs des revendications 1 à 12 et PG est un groupe protecteur, de préférence un groupe protecteur choisi dans le groupe consistant en les groupes tert-butyloxycarbonyle, benzyle, benzyloxycarbonyle et 9-fluorénylméthyloxycarbonyle, par réaction avec au moins un composé de formule générale R⁴-C≡C-C(=O)-OH, où R⁴ a les significations selon l'une ou plusieurs des revendications 1 à 12, dans un milieu réactionnel, éventuellement en présence d'au moins un agent de couplage approprié, éventuellement en présence d'au moins une base, de préférence à une température comprise entre -70 et 100°C, ou par réaction avec au moins un composé de formule générale R⁴-C≡C-C(=O)-A, où R⁴ a les significations données ci-dessus et A est un groupe partant, de préférence un radical halogéno, d'une manière particulièrement préférée un radical chloro ou bromo, dans un milieu réactionnel, éventuellement en présence d'au moins une base, de préférence à une température comprise entre -70 et 100°C, en au moins un composé correspondant de formule générale XI, éventuellement sous forme d'un sel correspondant, 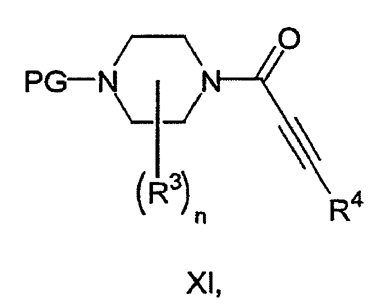 où R³, R⁴, n et PG ont les significations données ci-dessus, et éventuellement on purifie et/ou isole ce dernier ;
et on convertit au moins un composé de formule générale XI, pour le cas dans lequel PG est un groupe tert-butoxycarbonyle ou 9-fluorénylméthyloxycarbonyle, dans un milieu réactionnel, en présence d'au moins un acide, de préférence en présence d'au moins un acide choisi dans le groupe consistant en l'acide chlorhydrique et l'acide trifluoracétique, de préférence à une température comprise entre -70 et 100°C, ou dans le cas dans lequel PG est un groupe benzyle ou benzyloxycarbonyle, dans un milieu réactionnel, en présence d'hydrogène et en présence d'au moins un catalyseur, de préférence en présence de palladium sur charbon, de préférence à une température comprise entre -70 et 100°C, en au moins un composé correspondant de formule générale IX, éventuellement sous forme d'un sel correspondant, où R¹, R³ et n ont les significations données ci-dessus, et éventuellement on purifie et/ou isole ce dernier ; et on convertit au moins un composé de formule générale IX par réaction avec au moins un composé de formule générale II, 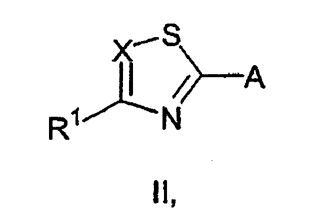 où les radicaux X et R¹ ont les significations selon l'une ou plusieurs des revendications 1 à 12 et A est un groupe partant, de préférence un radical halogéno ou un ester d'acide sulfonique, d'une manière particulièrement préférée un radical chloro ou bromo, dans un milieu réactionnel, éventuellement en présence d'au moins une base et/ou d'au moins un composé organométallique et/ou d'au moins un réactif de type hydrure métallique, de préférence à une température comprise entre -70 et 300°C, en au moins un composé correspondant de formule générale I, éventuellement sous forme d'un sel correspondant, et éventuellement on purifie et/ou isole ce dernier.

15. Médicament contenant au moins un composé selon l'une ou plusieurs des revendications 1 à 12 et éventuellement un ou plusieurs adjuvants acceptables d'un point de vue physiologique.

16. Médicament selon la revendication 15, pour le traitement et/ou la prophylaxie de la douleur, de préférence d'une douleur choisie dans le groupe consistant en une douleur aiguë, une douleur chronique, une douleur neuropathique et une douleur viscérale ; la migraine ; les dépressions ; les maladies neurodégénératives, choisies de préférence dans le groupe consistant en la sclérose en plaques, la maladie d'Alzheimer, la maladie de Parkinson et la maladie de Huntington ; les maladies cognitives, de préférence les états de déficience cognitive, d'une manière particulièrement préférée les troubles du déficit de l'attention (ADS) ; les états d'angoisse ; les crises de panique ; l'épilepsie ; la toux ; l'incontinence urinaire ; la diarrhée ; le prurit ; la schizophrénie ; les ischémies cérébrales ; les spasmes musculaires ; les crampes ; les troubles de l'ingestion, choisis de préférence dans le groupe consistant en la boulimie, la cachexie, l'anorexie et l'obésité ; la dépendance à l'alcool ; la dépendance aux médicaments ; la dépendance aux drogues, de préférence la dépendance à la nicotine et/ou à la cocaïne ; l'alcoolisme ; la pharmacodépendance ; la toxicomanie ; de préférence le tabagisme et/ou la cocaïnomanie ; les phénomènes de sevrage en présence d'une dépendance à l'alcool, aux médicaments et/ou aux drogues (en particulier à la nicotine et/ou à la cocaïne) ; le développement d'une tolérance à des médicaments, de préférence à des opioïdes naturels ou synthétiques ; le syndrome de reflux gastro-oesophagien ; pour la diurèse ; pour l'anti-natriurèse, pour influer sur le système cardiovasculaire ; pour augmenter la vigilance ; pour augmenter la libido ; pour moduler l'activité motrice
ou pour l'anesthésie locale.

17. Utilisation d'au moins un composé selon l'une ou plusieurs des revendications 1 à 12 pour préparer un médicament pour le traitement et/ou la prophylaxie de la douleur, de préférence d'une douleur choisie dans le groupe consistant en une douleur aiguë, une douleur chronique, une douleur neuropathique et une douleur viscérale ; la migraine ; les dépressions ; les maladies neurodégénératives, choisies de préférence dans le groupe consistant en la sclérose en plaques, la maladie d'Alzheimer, la maladie de Parkinson et la maladie de Huntington ; les maladies cognitives, de préférence les états de déficience cognitive, d'une manière particulièrement préférée les troubles du déficit de l'attention (ADS) ; les états d'angoisse ; les crises de panique ; l'épilepsie ; la toux ; l'incontinence urinaire ; la diarrhée ; le prurit ; la schizophrénie ; les ischémies cérébrales ; les spasmes musculaires ; les crampes ; les troubles de l'ingestion, choisis de préférence dans le groupe consistant en la boulimie, la cachexie, l'anorexie et l'obésité ; la dépendance à l'alcool ; la dépendance aux médicaments ; la dépendance aux drogues, de préférence la dépendance à la nicotine et/ou à la cocaïne ; l'alcoolisme ; la pharmacodépendance ; la toxicomanie ; de préférence le tabagisme et/ou la cocaïnomanie ; les phénomènes de sevrage en présence d'une dépendance à l'alcool, aux médicaments et/ou aux drogues (en particulier à la nicotine et/ou à la cocaïne) ; le développement d'une tolérance à des médicaments, de préférence à des opioïdes naturels ou synthétiques ; le syndrome de reflux gastro-oesophagien ; pour la diurèse ; pour l'anti-natriurèse, pour influer sur le système cardiovasculaire ; pour augmenter la vigilance ; pour augmenter la libido ; pour moduler l'activité motrice ou pour l'anesthésie locale.

18. Utilisation selon la revendication 17, pour préparer un médicament pour le traitement et/ou la prophylaxie de la douleur, de préférence d'une douleur choisie dans le groupe consistant en une douleur aiguë, une douleur chronique, une douleur neuropathique et une douleur viscérale ; les dépressions ; la maladie de Parkinson ; les états d'angoisse ; les crises de panique ; l'épilepsie ; la dépendance à l'alcool ; la dépendance aux médicaments ; les troubles de l'ingestion, choisis de préférence dans le groupe consistant en la boulimie, la cachexie, l'anorexie et l'obésité ; la dépendance aux drogues, de préférence la dépendance à la nicotine et/ou à la cocaïne ; l'alcoolisme ; la pharmacodépendance ; la toxicomanie ; de préférence le tabagisme et/ou la cocaïnomanie ; les phénomènes de sevrage en présence d'une dépendance à l'alcool, aux médicaments et/ou aux drogues (en particulier à la nicotine et/ou à la cocaïne) ; le développement d'une tolérance à des médicaments et/ou des drogues, en particulier à des opioïdes naturels ou synthétiques, ou pour une anesthésie locale.

19. Utilisation selon la revendication 17 ou 18 pour préparer un médicament pour le traitement de la douleur, de préférence d'une douleur choisie dans le groupe consistant en une douleur aiguë, une douleur chronique, une douleur neuropathique et une douleur viscérale, les états d'angoisse et les crises de panique.
